(19) Europäisches Patentamt
European Patent Office
Office européen des brevets

(11) EP 4 055 016 B1

(12) **EUROPEAN PATENT SPECIFICATION**

(45) Date of publication and mention of the grant of the patent:
**29.04.2026 Bulletin 2026/18**

(21) Application number: **20804658.1**

(22) Date of filing: **09.11.2020**

(51) International Patent Classification (IPC):
***C07D 471/04*** $^{(2006.01)}$ ***C07D 487/04*** $^{(2006.01)}$
***C07D 498/04*** $^{(2006.01)}$ ***A61P 25/18*** $^{(2006.01)}$
***A61K 31/437*** $^{(2006.01)}$

(52) Cooperative Patent Classification (CPC):
**C07D 471/04; A61P 25/18; C07D 487/04; C07D 498/04**

(86) International application number:
**PCT/GB2020/052832**

(87) International publication number:
**WO 2021/090030 (14.05.2021 Gazette 2021/19)**

(54) **GPR52 MODULATOR COMPOUNDS**

GPR52-MODULATORVERBINDUNGEN

COMPOSÉS MODULATEURS DE GPR52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2019 GB 201916298**
**20.12.2019 GB 201918974**
**20.12.2019 GB 201918992**

(43) Date of publication of application:
**14.09.2022 Bulletin 2022/37**

(73) Proprietor: **Nxera Pharma UK Limited**
**Cambridge, Cambridgeshire CB21 6DG (GB)**

(72) Inventors:
- **BUCKNELL, Sarah Joanne**
**Cambridge Cambridgeshire CB21 6DG (GB)**
- **WATSON, Stephen Paul**
**Cambridge Cambridgeshire CB21 6DG (GB)**
- **O'BRIEN, Michael Alistair**
**Cambridge Cambridgeshire CB21 6DG (GB)**
- **DE GRAAF, Chris**
**Cambridge Cambridgeshire CB21 6DG (GB)**
- **SWAIN, Nigel Alan**
**Cambridge Cambridgeshire CB21 6DG (GB)**

(74) Representative: **Gill Jennings & Every LLP**
**The Broadgate Tower**
**20 Primrose Street**
**London EC2A 2ES (GB)**

(56) References cited:
**WO-A1-2016/176571**
**WO-A1-2018/098561**
**WO-A1-2019/079485**
**KR-A- 20100 097 077**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

This application relates to novel compounds and their use as G-protein coupled receptor 52 (GPR52) modulators. Compounds described herein may be useful in the treatment or prevention of diseases in which GPR52 receptors are involved or in which modulation of GPR52 receptors may be beneficial. The application is also directed to pharmaceutical compositions comprising these compounds.

## BACKGROUND OF THE INVENTION

G-protein coupled receptor 52 (GPR52) is a constitutively active Gs coupled orphan receptor which is highly expressed in the striatum and cortex. In the striatum GPR52 is expressed exclusively on dopamine D2 medium spiny neurons and in the cortex it is found on cortical pyramidal neurons expressing dopamine D1 receptors (Komatsu et al, 2014, PLoS One 9:e90134). Based on its localization and functional coupling, GPR52 is proposed to play a role in the modulation of fronto-striatal and limbic dopamine and may therefore have utility in the treatment of neuropsychiatric disorders. GPR52 agonists are thought to be particularly relevant to the treatment of schizophrenia, where they are hypothesized to improve cognition and negative symptoms indirectly by potentiating D1 signalling but alleviate positive symptoms through inhibition of D2-mediated signalling in the striatum.

GPR52 agonists could be used to treat psychiatric disorders related to dysfunction of the mesolimbic and mesocortical pathways. Examples include treatment of the positive, negative and cognitive symptoms of schizophrenia, depression, attention-deficit hyperactivity disorder, anxiety disorders (generalised anxiety disorder, obsessive compulsive disorder, panic disorder), bipolar disorder, addiction/impulse-control disorders and autism spectrum disorders. Neuropsychiatric symptoms (e.g. psychosis, anhedonia, agitation, etc) of neurodegenerative diseases (e.g. Alzheimer's disease, Parkinson's disease, Huntington's disease, etc) could also be treated by GPR52 agonists. GPR52 expression in the pituitary gland and hypothalamus suggests utility for GPR52 modulators in pituitary and hypothalamic disorders, and there is preclinical evidence (Xiong et al, 2016, WO2016/176571) to suggest that GPR52 agonists could be useful in the treatment of hyperprolactinemia.

WO 2016/176571 describes various 1-heteroaryl-indoline-4-carboxamide compounds, which may act as modulators of GPR52, as well as their use in the treatment of related conditions.

## THE INVENTION

The present invention provides compounds as defined in independent claim 1 having activity as G protein-coupled receptor 52 (GPR52) modulators.

The present invention provides a compound of Formula (1):

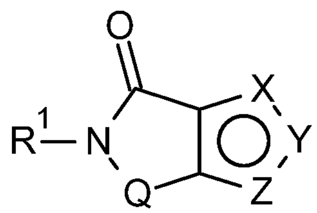

(1)

or a salt thereof, wherein;

X is N or $CR^2$;

Y is N, $NR^3$ or $CR^2$;

Z is N or $NR^3$;

wherein one but not both of Y and Z is $NR^3$;

Q is selected from $-CR^4R^5-$, $-CR^4R^5CR^6R^7-$, $-CR^4R^5CR^6R^7CR^8R^9-$, $-CR^4R^5CR^6R^7O-$, $- OCR^4R^5-$, $-OCR^4R^5CR^6R^7-$ and $-CR^4R^5O-$;

$R^1$ is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group $- C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;

$R^2$ is H, halo, CN, $C_{1-6}$ alkyl optionally substituted with OH or 1 to 6 fluorine atoms, $C_{1-6}$ alkoxy optionally substituted

with OH or 1 to 6 fluorine atoms, or $C_{3-6}$ cycloalkyl optionally substituted with OH or 1 to 6 fluorine atoms wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;

$R^4$, $R^5$, $R^6$, $R^7$, $R^8$ and $R^9$ are independently selected from H and $C_{1-3}$ alkyl;

wherein $R^3$ is a group of the formula:

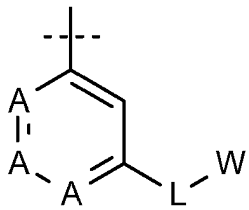

wherein, each A is independently N or $CR^{10}$;

L is selected from $CH_2$, CHD, $CD_2$, CHF, $CF_2$, C=O, CHOH, O and NH;

$R^{10}$ is selected from H, halo and $C_{1-3}$ alkyl optionally substituted with 1 to 6 fluorine atoms;

and W is either:

(i) a 6-membered optionally substituted aryl or heteroaryl ring;

or

(ii) a 9-10-membered optionally substituted heterobicyclic ring system, where one or more of the rings is aromatic.

Compounds of the present invention are for use as GPR52 modulators. Compounds of the present invention may be used as GPR52 agonists. Compounds of the present invention may be used in the manufacture of medicaments. The compounds or medicaments may be for use in treating, preventing, ameliorating, controlling or reducing the risk of diseases or disorders in which GPR52 receptors are involved. The compounds or medicaments may be for use in treating, preventing, ameliorating, controlling or reducing the risk of diseases or disorders in which modulation of GPR52 receptors may be beneficial. Compounds of the present invention may be useful in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; hypothalamic disorders; pituitary disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

Compounds of the present invention may be useful in the treatment of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, anhedonia, agitation, Alzheimer's disease, Parkinson's disease, Huntington's disease, vascular dementia, Lewy body disease, frontotemporal dementia, Tourette's syndrome, hyperprolactinemia, pituitary adenoma, prolactinoma, craniopharyngioma, Cushing's disease, diabetes insipidus, non-functioning tumours, obesity, posttraumatic stress disorder (PTSD), akathisia and associated movements, athetosis, ataxia, ballismus, hemiballismus, chorea, choreoathetosis, dyskinesia, tardive dyskinesia, neuroleptic-induced dyskinesia, myoclonus, mirror movement disorder, paroxysmal kinesigenic dyskinesia, restless legs syndrome, spasms, stereotypic movement disorder, sterotypy, Tic disorder, tremor, Wilson's disease, schizotypal personality disorder, delusional disorder, brief psychotic disorder, schizophreniform disorder, schizoaffective disorder, substance- or medication-induced psychotic disorder, delusions, hallucinations, disorganized thinking, grossly disorganized or abnormal motor behavior, catatonia, major depressive disorder, bipolar I disorder, bipolar II disorder, cyclothymic disorder, substance- or medication-induced bipolar and related disorders, bipolar and related disorders due to another medical condition, separation anxiety disorder, selective mutism, specific phobia, social anxiety disorder, panic disorder, agoraphobia, generalized anxiety disorder, substance- or medication-induced anxiety disorder, anxiety disorders due to another medical condition, delirium, major neurocognitive disorder, minor neurocognitive disorder, amnesia, dementia, developmental coordination disorder, stereotypic movement disorder, a post-stroke effect, dentatorubral-pallidoluysian atrophy, diminished emotional expression, avolition, alogia and asociality.

## DETAILED DESCRIPTION OF THE INVENTION

The invention relates to novel compounds as defined in independent claim 1. The novel compounds are for use as modulators of the GPR52 receptor. The compounds of the present invention may be used in the manufacture of medicaments for use as GPR52 modulators. Compounds of the present invention may be used as GPR52 agonists. The compounds or medicaments may be for use in treating, preventing, ameliorating, controlling or reducing the risk of diseases or disorders in which GPR52 receptors are involved. The compounds or medicaments may be for use in treating, preventing, ameliorating, controlling or reducing the risk of diseases or disorders in which modulation of GPR52 receptors may be beneficial.

The invention further relates to compounds as defined in independent claim 1, compositions and medicaments thereof that may be useful in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

The present invention provides a compound of Formula (1):

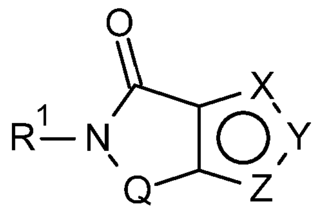

(1)

or a salt thereof, wherein;

X is N or $CR^2$;

Y is N, $NR^3$ or $CR^2$;

Z is N or $NR^3$;

wherein one but not both of Y and Z is $NR^3$;

Q is selected from $-CR^4R^5-$, $-CR^4R^5CR^6R^7-$, $-CR^4R^5CR^6R^7CR^8R^9-$, $-CR^4R^5CR^6R^7O-$, $-OCR^4R^5-$ and $-OCR^4R^5CR^6R^7-$;

$R^1$ is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;

$R^2$ is H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;

$R^4$, $R^5$, $R^6$, $R^7$, $R^8$ and $R^9$ are independently selected from H and $C_{1-3}$ alkyl;

wherein $R^3$ is a group of the formula:

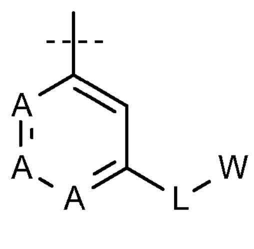

wherein, each A is independently N or $CR^{10}$;

L is selected from $CH_2$, CHD, $CD_2$, CHF, $CF_2$, C=O, CHOH, O and NH;
$R^{10}$ is selected from H, halo and $C_{1-3}$ alkyl optionally substituted with 1 to 6 fluorine atoms;
and W is either:

(i) a 6-membered optionally substituted aryl or heteroaryl ring;
or

(ii) a 9-10-membered optionally substituted heterobicyclic ring system, where one or more of the rings is aromatic.

Also described herein is a compound of Formula (1'):

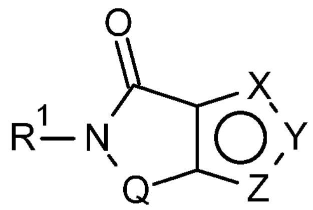

(1')

or a salt thereof, wherein;
X is N or $CR^2$;
Y is N, $NR^3$ or $CR^2$;
Z is N or $NR^3$;
wherein one but not both of Y and Z is $NR^3$;
Q is selected from $-CR^4R^5-$, $-CR^4R^5CR^6R^7-$, $-CR^4R^5CR^6R^7CR^8R^9-$, $-CR^4R^5CR^6R^7O-$, - $OCR^4R^5-$, $-OCR^4R^5CR^6R^7-$ and $-CR^4R^5O-$;
$R^1$ is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group - $C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;
$R^2$ is H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;
$R^3$ is a group -V-L-W;
$R^4$, $R^5$, $R^6$, $R^7$, $R^8$ and $R^9$ are independently selected from H and $C_{1-3}$ alkyl;
wherein V is a 6-membered optionally substituted aryl or heteroaryl ring substituted with L at the meta-position relative to the position of attachment of $R^3$ to the remainder of the molecule;
L is selected from $CH_2$, CHD, $CD_2$, CHF, $CF_2$, C=O, CHOH, O and NH;
and W is an optionally substituted monocyclic or polycyclic ring system.

Also described herein is a compound of Formula (1"):

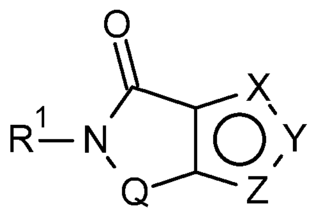

(1'')

or a salt thereof, wherein;
X is N or $CR^2$;

Y is N, $NR^3$ or $CR^2$;
Z is N or $NR^3$;
wherein one but not both of Y and Z is $NR^3$;
Q is selected from $-CR^4R^5-$, $-CR^4R^5CR^6R^7-$, $-CR^4R^5CR^6R^7CR^8R^9-$, $-CR^4R^5CR^6R^7O-$, $-OCR^4R^5-$ and $-OCR^4R^5CR^6R^7-$;
$R^1$ is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;
$R^2$ is H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;
$R^3$ is a group -V-L-W;
$R^4$, $R^5$, $R^6$, $R^7$, $R^8$ and $R^9$ are independently selected from H and $C_{1-3}$ alkyl;
wherein V is a 6-membered optionally substituted aryl or heteroaryl ring substituted with L at the meta-position relative to the position of attachment of $R^3$ to the remainder of the molecule;
L is selected from $CH_2$, CHD, $CD_2$, CHF, $CF_2$, C=O, CHOH, O and NH;
and W is an optionally substituted monocyclic or polycyclic ring system.

Also described herein is a compound of Formula (1a):

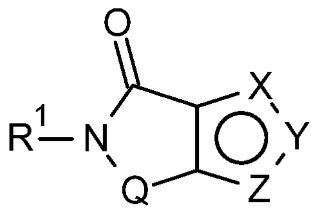

(1a)

or a salt thereof, wherein;
X is N or $CR^2$;
Y is N, $NR^3$ or $CR^2$;
Z is N or $NR^3$;
wherein one but not both of Y and Z is $NR^3$;
Q is selected from $-CR^4R^5-$, $-CR^4R^5CR^6R^7-$, $-CR^4R^5CR^6R^7CR^8R^9-$, $-CR^4R^5CR^6R^7O-$, $- OCR^4R^5-$, $-OCR^4R^5CR^6R^7-$ and $-CR^4R^5O-$;
$R^1$ is H, $C_{1-3}$ alkyl or $C_{3-6}$ cycloalkyl;
$R^2$ is H, halo, $C_{1-3}$ alkyl or $C_{3-6}$ cycloalkyl;
$R^3$ is a group -V-L-W;
$R^4$, $R^5$, $R^6$, $R^7$, $R^8$ and $R^9$ are independently selected from H and $C_{1-3}$ alkyl;
wherein V is a 6-membered optionally substituted aryl or heteroaryl ring substituted with L at the meta-position;
L is selected from $CH_2$, CHD, $CD_2$, CHF, $CF_2$, C=O, CHOH, O and NH;
and W is an optionally substituted monocyclic or polycyclic ring system.

Also provided is a compound of Formula (1b):

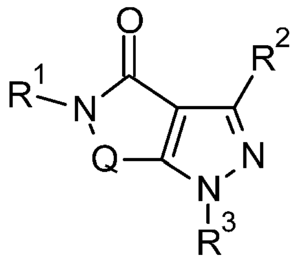

(1b)

or a salt thereof, wherein Q, $R^1$, $R^2$ and $R^3$ are as defined herein.

Also provided is a compound of Formula (1b):

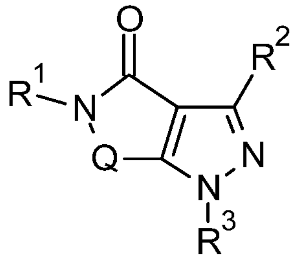

(1b)

or a salt thereof, wherein;

Q is selected from $-CR^4R^5-$, $-CR^4R^5CR^6R^7-$, $-CR^4R^5CR^6R^7CR^8R^9-$, $-CR^4R^5CR^6R^7O-$,$-OCR^4R^5-$ and $-OCR^4R^5CR^6R^7-$

$R^1$ is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;

$R^2$ is H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;

$R^4$, $R^5$, $R^6$, $R^7$, $R^8$ and $R^9$ are independently selected from H and $C_{1-3}$ alkyl;

$R^3$ is a group of the formula:

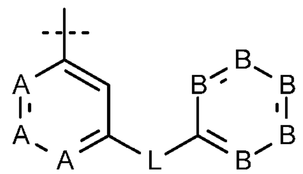

wherein, each A is independently N or $CR^{10}$;

L is selected from $CH_2$, CHD, $CD_2$, CHF, $CF_2$, C=O, CHOH, O and NH;

each B is independently N, $CR^{11}$, $CR^{12}$, $CR^{13}$, $CR^{15}$ or $CR^{16}$;

$R^{10}$ is selected from H, halo and $C_{1-3}$ alkyl optionally substituted with 1 to 6 fluorine atoms;

$R^{11}$, $R^{12}$, $R^{13}$, $R^{15}$ and $R^{16}$ are independently selected from H, CN, $SF_5$, halo, a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms or optionally substituted with $CF_3$.

Also provided is a compound of Formula (1b):

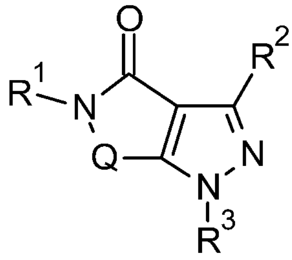

(1b)

or a salt thereof, wherein;

Q is a $C_{1-3}$ alkyl linker;

$R^1$ is H or $C_{1-3}$ alkyl;

$R^2$ is selected from CN, halo, $C_{1-6}$ alkyl optionally substituted with OH or 1 to 6 fluorine atoms and $C_{1-6}$ alkoxy optionally substituted with OH or 1 to 6 fluorine atoms, wherein one atom of the $C_{1-6}$ alkyl or $C_{1-6}$ alkoxy group may be optionally replaced by a heteroatom selected from O, N, S and oxidised forms thereof;

$R^3$ is a group of the formula:

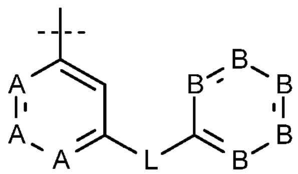

wherein, A is N or $CR^{10}$;

L is selected from $CH_2$, CHD, $CD_2$, CHF, $CF_2$, C=O, CHOH, O and NH;

B is N, $CR^{11}$, $CR^{12}$ or $CR^{13}$;

$R^{10}$ is selected from H, halo and $C_{1-3}$ alkyl optionally substituted with 1 to 6 fluorine atoms;

$R^{11}$, $R^{12}$ and $R^{13}$ are independently selected from H, CN, halo, $C_{1-6}$ alkyl optionally substituted with 1 to 6 fluorine atoms and $C_{1-6}$ alkoxy optionally substituted with 1 to 6 fluorine atoms, wherein one atom of the $C_{1-6}$ alkyl or $C_{1-6}$ alkoxy group may be optionally replaced by a heteroatom selected from O, N, S and oxidised forms thereof.

In the compounds herein, X can be N or $CR^2$. X can be N. X can be $CR^2$.

In the compounds herein, Y can be N, $NR^3$ or $CR^2$. Y can be N. Y can be $NR^3$. Y can be $CR^2$. When Y is $NR^3$, Z is N.

In the compounds herein, Z can be N or $NR^3$. Z can be N. Z can be $NR^3$. When Z is $NR^3$, Y is N or $CR^2$.

In the compounds herein, one but not both of Y and Z is $NR^3$. Y can be $NR^3$ and Z can be N. Z can be $NR^3$ and Y can be N. Z can be $NR^3$ and Y can be $CR^2$.

In the compounds herein, $R^1$ can be H, $C_{1-6}$ alkyl optionally substituted with 1 to 6 fluorine atoms, $C_{3-6}$ cycloalkyl optionally substituted with 1 to 6 fluorine atoms, a group - $C(R^{14})_2C(R^{18})_2OR^{17}$ or a group $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring. $R^1$ can be H, $C_{1-3}$ alkyl or $C_{3-6}$ cycloalkyl. $R^1$ can be H or a $C_{1-3}$ alkyl group. $R^1$ can be H. $R^1$ can be a $C_{1-6}$ alkyl group optionally substituted with 1 to 6 fluorine atoms. $R^1$ can be a $C_{1-3}$ alkyl group optionally substituted with 1 to 6 fluorine atoms. $R^1$ can be a $C_{3-6}$ cycloalkyl group optionally substituted with 1 to 6 fluorine atoms. $R^1$ can be a $C_{1-6}$ alkyl group. $R^1$ can be a $C_{1-3}$ alkyl group. $R^1$ can be a $C_{3-6}$ cycloalkyl group. $R^1$ can be $-C(R^{14})_2C(R^{18})_2OR^{17}$ or $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring. $R^1$ can be H, methyl, $-CH_2CH_2OCH_3$, $-CH_2CH_2OH$, $CH(CH_3)CH_2OCH_3$, $-CH(CH_3)CH_2OH$, $-CH_2CH(CH_3)OCH_3$ or $-CH_2CH(CH_3)OH$. $R^1$ can be H, methyl, $-CH_2CH_2OCH_3$ or $-CH_2CH_2OH$. $R^1$ can be H or methyl. $R^1$ can be methyl. $R^1$ can be an oxolane ring. $R^1$ can be a tetrahydrofuran ring. $R^1$ can be an oxetane ring. $R^1$ can be

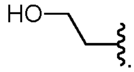

$R^1$ can be

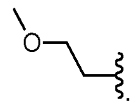

$R^1$ can be

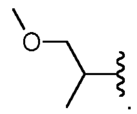

$R^1$ can be

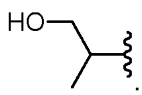

$R^1$ can be

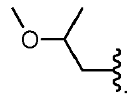

$R^1$ can be

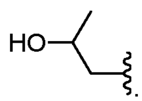

$R^1$ can be

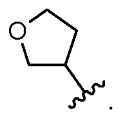

$R^1$ can be

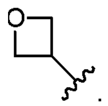

In the compounds herein, $R^2$ can be H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof.

$R^2$ can be H, halo, CN, $C_{1-6}$ alkyl optionally substituted with OH or 1 to 6 fluorine atoms, $C_{1-6}$ alkoxy optionally substituted with OH or 1 to 6 fluorine atoms, or $C_{3-6}$ cycloalkyl optionally substituted with OH or 1 to 6 fluorine atoms.

$R^2$ can be H, halo, $C_{1-3}$ alkyl or $C_{3-6}$ cycloalkyl. $R^2$ can be H or a $C_{1-3}$ alkyl group. $R^2$ can be H. $R^2$ can be halo. $R^2$ can be a $C_{1-3}$ alkyl group. $R^2$ can be a $C_{3-6}$ cycloalkyl group. $R^2$ can be H or methyl. $R^2$ can be methyl. $R^2$ can be F. $R^2$ can be Cl. $R^2$ can be Br. $R^2$ can be selected from CN, halo, $C_{1-6}$ alkyl optionally substituted with OH or 1 to 6 fluorine atoms and $C_{1-6}$ alkoxy optionally substituted with OH or 1 to 6 fluorine atoms, wherein one atom of the $C_{1-6}$ alkyl or $C_{1-6}$ alkoxy group may be optionally replaced by a heteroatom selected from O, N, S and oxidised forms thereof. $R^2$ can be selected from methyl, F, Cl, CN, $CF_3$ and $CH_2OH$. $R^2$ can be $C_{1-6}$ alkyl optionally substituted with OH. $R^2$ can be $C_{1-6}$ alkyl optionally substituted with 1 to 6 fluorine atoms. $R^2$ can be $C_{1-3}$ alkyl optionally substituted with OH. $R^2$ can be $C_{1-3}$ alkyl optionally substituted with 1 to 6 fluorine atoms.

In the compounds herein $R^3$ can be a group -V-L-W, such that $R^3$ is a group of the formula:

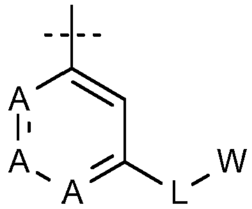

.

V can be a phenyl ring substituted with L at the meta-position. V can be a pyridine ring substituted with L at the meta-position. V can be a pyrimidine ring substituted with L at the meta-position. V can be a pyridazine ring substituted with L at the meta-position. The term "meta-position" as used herein in relation to L should be interpreted to mean that substituent -L-W is positioned at the meta-position (or 3-position) of ring V relative to the point of attachment of ring V to Z or Y, i.e. relative to the position of attachment of $R^3$ to the remainder of the molecule.

In the compounds herein, L can be selected from $CH_2$, CHD, $CD_2$, CHF, $CF_2$, C=O, CHOH, O or NH. L can be $CH_2$. L can be CHD. L can be $CD_2$. L can be CHF. L can be $CF_2$. L can be C=O. L can be CHOH. L can be O. L can be NH.

In the compounds herein, W can be a 6-membered optionally substituted aryl or heteroaryl ring. W can be a 6-membered optionally substituted aryl ring. W can be a 6-membered optionally substituted heteroaryl ring. W can be an optionally substituted phenyl ring. W can be an optionally substituted pyridine ring. W can be a 9-10-membered optionally substituted heterobicyclic ring system, where one or more of the rings is aromatic. W can be a 9-membered optionally substituted heterobicyclic ring system, where one or more of the rings is aromatic. W can be a 10-membered optionally substituted heterobicyclic ring system, where one or more of the rings is aromatic.

W can be a 9-10-membered optionally substituted heterobicyclic ring system selected from:

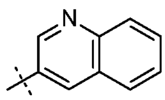 ; 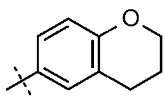 ; 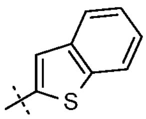 ; 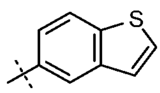 .

W can be an optionally substituted polycyclic ring system selected from: quinoline, 3,4-dihydro-2H-1-benzopyran, 1-benzothiophene.

In the compounds herein, $R^3$ is a group of the formula:

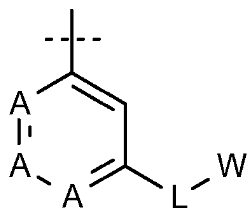

wherein, A is N or $CR^{10}$;
L is selected from $CH_2$, CHD, $CD_2$, CHF, $CF_2$, C=O, CHOH, O and NH;
$R^{10}$ is selected from H, halo and $C_{1-3}$ alkyl optionally substituted with 1 to 6 fluorine atoms; and W is either:

(i) a 6-membered optionally substituted aryl or heteroaryl ring;
(ii) a 9-10-membered optionally substituted heterobicyclic ring system, where one or more of the rings is aromatic.

In the compounds herein $R^3$ can be a group of the formula:

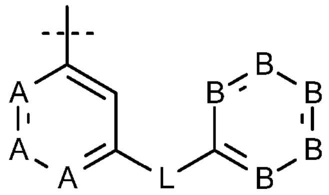

wherein, A is N or $CR^{10}$;
L is selected from $CH_2$, CHD, $CD_2$, CHF, $CF_2$, C=O, CHOH, O and NH;
B is N, $CR^{11}$, $CR^{12}$ or $CR^{13}$;
$R^{10}$ is selected from H, halo and $C_{1-3}$ alkyl optionally substituted with 1 to 6 fluorine atoms;
$R^{11}$, $R^{12}$ and $R^{13}$ are independently selected from H, CN, $SF_5$, halo, a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms or optionally substituted with $CF_3$.

In the compounds herein $R^3$ can be a group of the formula:

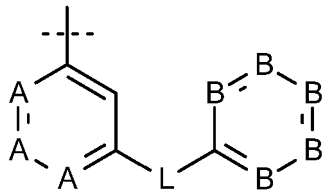

wherein, each A is independently N or $CR^{10}$;
L is selected from $CH_2$, CHD, $CD_2$, CHF, $CF_2$, C=O, CHOH, O and NH;
each B is independently N, $CR^{11}$, $CR^{12}$, $CR^{13}$, $CR^{15}$ or $CR^{16}$;
$R^{10}$ is selected from H, halo and $C_{1-3}$ alkyl optionally substituted with 1 to 6 fluorine atoms;
$R^{11}$, $R^{12}$, $R^{13}$, $R^{15}$ and $R^{16}$ are independently selected from H, CN, $SF_5$, halo, a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms or optionally substituted with $CF_3$.

In the compounds herein, A can be N or $CR^{10}$. A can be N. A can be $CR^{10}$. Each A can independently be N or $CR^{10}$.

In the compounds herein, $R^{10}$ can be H, halo or $C_{1-3}$ alkyl optionally substituted with 1 to 6 fluorine atoms. $R^{10}$ can be H. $R^{10}$ can be halo. $R^{10}$ can be F. $R^{10}$ can be Cl. $R^{10}$ can be Br. $R^{10}$ can be a $C_{1-3}$ alkyl group optionally substituted with 1 to 6 fluorine atoms. $R^{10}$ can be a $C_{1-3}$ alkyl group. $R^{10}$ can be methyl. $R^{10}$ can be $CF_3$.

In the compounds herein, B can be selected from N, $CR^{11}$, $CR^{12}$ or $CR^{13}$. B can be N. B can be $CR^{11}$. B can be $CR^{12}$. B can be $CR^{13}$. Each B can independently be N, $CR^{11}$, $CR^{12}$ or $CR^{13}$. B can be selected from N, $CR^{11}$, $CR^{12}$, $CR^{13}$, $CR^{15}$ or $CR^{16}$. B can be N. B can be $CR^{11}$. B can be $CR^{12}$. B can be $CR^{13}$. B can be $CR^{15}$. B can be $CR^{16}$. Each B can independently be N, $CR^{11}$, $CR^{12}$, $CR^{13}$, $CR^{15}$ or $CR^{16}$.

In the compounds herein, $R^3$ can be selected from group consisting of:

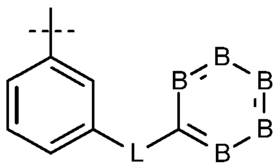
;
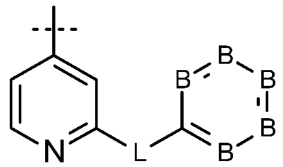
;
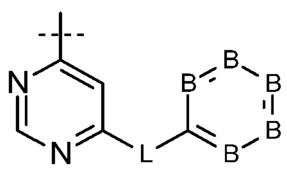
;

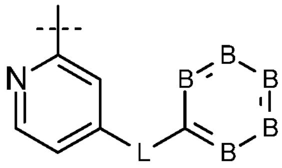 ; 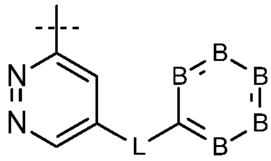 ; 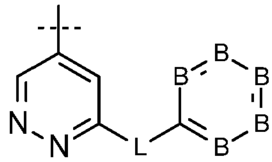 ;

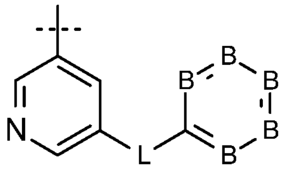 .

In the compounds herein, the group:

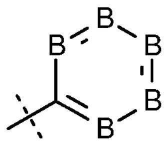

can be selected from the group consisting of:

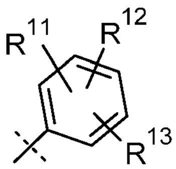 ; 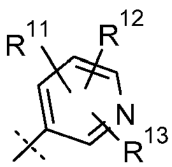 ; 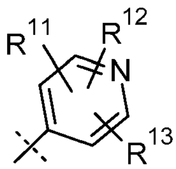 .

In the compounds herein, the group:

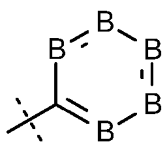

can be selected from the group consisting of:

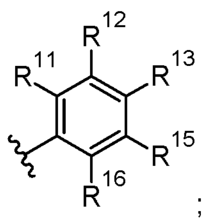 ; 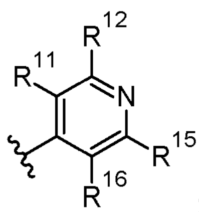 ; 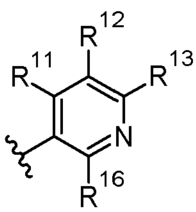 .

In the compounds herein, W can be selected from the group consisting of:

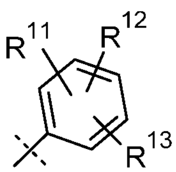 ; 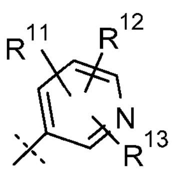 ; 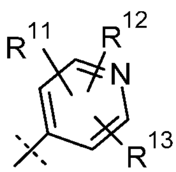 .

In the compounds herein, W can be selected from the group consisting of:

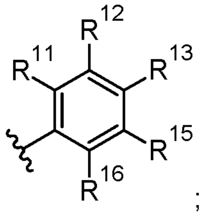 ; 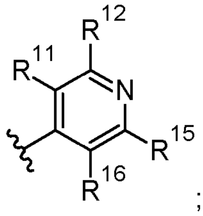 ; 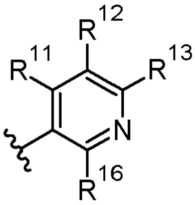 .

In the compounds herein, $R^{11}$, $R^{12}$ and $R^{13}$ can independently be selected from H, CN, $SF_5$, halo, a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms or optionally substituted with $CF_3$. $R^{11}$, $R^{12}$ and $R^{13}$ can independently be H, CN, halo, $C_{1-6}$ alkyl optionally substituted with 1 to 6 fluorine atoms wherein one atom of the $C_{1-6}$ alkyl group may be optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms. $R^{11}$, $R^{12}$ and $R^{13}$ can be independently selected from H, CN, halo, $C_{1-6}$ alkyl optionally substituted with 1 to 6 fluorine atoms and $C_{1-6}$ alkoxy optionally substituted with 1 to 6 fluorine atoms, wherein one atom of the $C_{1-6}$ alkyl or $C_{1-6}$ alkoxy group may be optionally replaced by a heteroatom selected from O, N, S and oxidised forms thereof. $R^{11}$, $R^{12}$ and $R^{13}$ can independently be selected from H, CN, $SF_5$, halo, a $C_{1-6}$ saturated hydrocarbon group optionally substituted with 1 to 6 fluorine atoms or $OC_{1-6}$ alkyl optionally substituted with 1 to 6 fluorine atoms.

In the compounds herein, $R^{11}$, $R^{12}$, $R^{13}$, $R^{15}$ and $R^{16}$ can independently be selected from H, CN, $SF_5$, halo, a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms or optionally substituted with $CF_3$. $R^{11}$, $R^{12}$, $R^{13}$, $R^{15}$ and $R^{16}$ can independently be H, CN, halo, $C_{1-6}$ alkyl optionally substituted with 1 to 6 fluorine atoms wherein one atom of the $C_{1-6}$ alkyl group may be optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms. $R^{11}$, $R^{12}$, $R^{13}$, $R^{15}$ and $R^{16}$ can be independently selected from H, CN, halo, $C_{1-6}$ alkyl optionally substituted with 1 to 6 fluorine atoms and $C_{1-6}$ alkoxy optionally substituted with 1 to 6 fluorine atoms, wherein one atom of the $C_{1-6}$ alkyl or $C_{1-6}$ alkoxy group may be optionally replaced by a heteroatom selected from O, N, S and oxidised forms thereof. $R^{11}$, $R^{12}$, $R^{13}$, $R^{15}$ and $R^{16}$ can independently be selected from H, CN, $SF_5$, halo, a $C_{1-6}$ saturated hydrocarbon group optionally substituted with 1 to 6 fluorine atoms or $OC_{1-6}$ alkyl optionally substituted with 1 to 6 fluorine atoms.

In the compounds herein, $R^{11}$, $R^{12}$ and $R^{13}$ can independently be H, CN, $SF_5$, F, Cl, methyl, ethyl, isopropyl, cyclopropyl, $CF_3$, $CF_2H$, $OCF_2H$, OMe and $SO_2Me$. $R^{11}$, $R^{12}$ and $R^{13}$ can be independently selected from H, F and $CF_3$.

In the compounds herein, $R^{11}$, $R^{12}$, $R^{13}$, $R^{15}$ and $R^{16}$ can independently be H, CN, $SF_5$, F, Cl, methyl, ethyl, isopropyl, cyclopropyl, $CF_3$, $CF_2H$, $OCF_2H$, OMe and $SO_2Me$. $R^{11}$, $R^{12}$, $R^{13}$, $R^{15}$ and $R^{16}$ can be independently selected from H, F and $CF_3$.

In the compounds herein, $R^{11}$ can be H. $R^{11}$ can be CN. $R^{11}$ can be halo. $R^{11}$ can be F or Cl. $R^{11}$ can be F. $R^{11}$ can be $SF_5$. $R^{11}$ can be a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof. $R^{11}$ can be a $C_{1-6}$ alkyl group which is optionally substituted with 1 to 6 fluorine atoms wherein one atom of the $C_{1-6}$ alkyl group may be optionally replaced by a heteroatom selected from O, N, S and oxidised forms thereof. $R^{11}$ can be a $C_{1-6}$ alkyl group which is optionally substituted with 1 to 6 fluorine atoms. $R^{11}$ can be a $C_{1-6}$ alkyl group. $R^{11}$ can be a $OC_{1-6}$ alkyl group which is optionally substituted with 1 to 6 fluorine atoms. $R^{11}$ can be a $OC_{1-6}$ alkyl group. $R^{11}$ can be a $C_{1-6}$ alkoxy group which is optionally substituted with 1 to 6 fluorine atoms, $R^{11}$ can be a $C_{1-6}$ alkoxy group. $R^{11}$ can be a $SO_2C_{1-6}$ alkyl group which is optionally substituted with 1 to 6 fluorine atoms. $R^{11}$ can be a $SO_2C_{1-6}$ alkyl group. $R^{11}$ can be a $C_{3-6}$ cycloalkyl group which is optionally substituted with 1 to 6 fluorine atoms or optionally substituted with $CF_3$. $R^{11}$ can be a $C_{3-6}$ cycloalkyl group. $R^{11}$ can be H. $R^{11}$ can be CN. $R^{11}$ can be F. $R^{11}$ can be Cl. $R^{11}$ can be methyl. $R^{11}$ can be ethyl. $R^{11}$ can be isopropyl. $R^{11}$ can be cyclopropyl. $R^{11}$ can be $CF_3$. $R^{11}$ can be $OCF_2H$. $R^{11}$ can be $SO_2Me$. $R^{11}$ can be $CF_2H$. $R^{11}$ can be OMe. $R^{11}$ can be

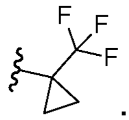

In the compounds herein, $R^{12}$ can be H. $R^{12}$ can be CN. $R^{12}$ can be halo. $R^{12}$ can be F or Cl. $R^{12}$ can be F. $R^{12}$ can be $SF_5$. $R^{12}$ can be a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof. $R^{12}$ can be a $C_{1-6}$ alkyl group which is optionally substituted with 1 to 6 fluorine atoms wherein one atom of the $C_{1-6}$ alkyl group may be optionally replaced by a heteroatom selected from O, N, S and oxidised forms thereof. $R^{12}$ can be a $C_{1-6}$ alkyl group which is optionally substituted with 1 to 6 fluorine atoms. $R^{12}$ can be a $C_{1-6}$ alkyl group. $R^{12}$ can be a $OC_{1-6}$ alkyl group which is optionally substituted with 1 to 6 fluorine atoms. $R^{12}$ can be a $OC_{1-6}$ alkyl group. $R^{12}$ can be a $C_{1-6}$ alkoxy group which is optionally substituted with 1 to 6 fluorine atoms, $R^{12}$ can be a $C_{1-6}$ alkoxy group. $R^{12}$ can be a $SO_2C_{1-6}$ alkyl group which is optionally substituted with 1 to 6 fluorine atoms. $R^{12}$ can be a $SO_2C_{1-6}$ alkyl group. $R^{12}$ can be a $C_{3-6}$ cycloalkyl group which is optionally substituted with 1 to 6 fluorine atoms or optionally substituted with $CF_3$. $R^{12}$ can be a $C_{3-6}$ cycloalkyl group. $R^{12}$ can be H. $R^{12}$ can be CN. $R^{12}$ can be F. $R^{12}$ can be Cl. $R^{12}$ can be methyl. $R^{12}$ can be ethyl. $R^{12}$ can be isopropyl. $R^{12}$ can be cyclopropyl. $R^{12}$ can be $CF_3$. $R^{12}$ can be $OCF_2H$. $R^{12}$ can be $SO_2Me$. $R^{12}$ can be $CF_2H$. $R^{12}$ can be OMe. $R^{12}$ can be

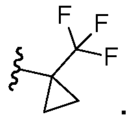

In the compounds herein, $R^{13}$ can be H. $R^{13}$ can be CN. $R^{13}$ can be halo. $R^{13}$ can be F or Cl. $R^{13}$ can be F. $R^{13}$ can be $SF_5$. $R^{13}$ can be a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof. $R^{13}$ can be a $C_{1-6}$ alkyl group which is optionally substituted with 1 to 6 fluorine atoms wherein one atom of the $C_{1-6}$ alkyl group may be optionally replaced by a heteroatom selected from O, N, S and oxidised forms thereof. $R^{13}$ can be a $C_{1-6}$ alkyl group which is optionally substituted with 1 to 6 fluorine atoms. $R^{13}$ can be a $C_{1-6}$ alkyl group. $R^{13}$ can be a $OC_{1-6}$ alkyl group which is optionally substituted with 1 to 6 fluorine atoms. $R^{13}$ can be a $OC_{1-6}$ alkyl group. $R^{13}$ can be a $C_{1-6}$ alkoxy group which is optionally substituted with 1 to 6 fluorine atoms, $R^{13}$ can be a $C_{1-6}$ alkoxy group. $R^{13}$ can be a $SO_2C_{1-6}$ alkyl group which is optionally substituted with 1 to 6 fluorine atoms. $R^{13}$ can be a $SO_2C_{1-6}$ alkyl group. $R^{13}$ can be a $C_{3-6}$ cycloalkyl group which is optionally substituted with 1 to 6 fluorine atoms or optionally substituted with $CF_3$. $R^{13}$ can be a $C_{3-6}$ cycloalkyl group. $R^{13}$ can be H. $R^{13}$ can be CN. $R^{13}$ can be F. $R^{13}$ can be Cl. $R^{13}$ can be methyl. $R^{13}$ can be ethyl. $R^{13}$ can be isopropyl. $R^{13}$ can be cyclopropyl. $R^{13}$ can be $CF_3$. $R^{13}$ can be $OCF_2H$. $R^{13}$ can be $SO_2Me$. $R^{13}$ can be $CF_2H$. $R^{13}$ can be OMe. $R^{13}$ can be

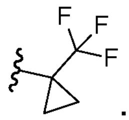

In the compounds herein, $R^{15}$ can be H. $R^{15}$ can be CN. $R^{15}$ can be halo. $R^{15}$ can be F or Cl. $R^{15}$ can be F. $R^{15}$ can be $SF_5$. $R^{15}$ can be a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof. $R^{15}$ can be a $C_{1-6}$ alkyl group which is optionally substituted with 1 to 6 fluorine atoms wherein one atom of the $C_{1-6}$ alkyl group may be optionally replaced by a heteroatom selected from O, N, S and oxidised forms thereof. $R^{15}$ can be a $C_{1-6}$ alkyl group which is optionally substituted with 1 to 6 fluorine atoms. $R^{15}$ can be a $C_{1-6}$ alkyl group. $R^{15}$ can be a $OC_{1-6}$ alkyl group which is optionally substituted with 1 to 6 fluorine atoms. $R^{15}$ can be a $OC_{1-6}$ alkyl group. $R^{15}$ can be a $C_{1-6}$ alkoxy group which is optionally substituted with 1 to 6 fluorine atoms, $R^{15}$ can be a $C_{1-6}$ alkoxy group. $R^{15}$ can be a $SO_2C_{1-6}$ alkyl group which is optionally substituted with 1 to 6 fluorine atoms. $R^{15}$ can be a $SO_2C_{1-6}$ alkyl group. $R^{15}$ can be a $C_{3-6}$ cycloalkyl group which is optionally substituted with 1 to 6 fluorine atoms or optionally substituted with $CF_3$. $R^{15}$ can be a $C_{3-6}$ cycloalkyl group. $R^{15}$ can be H. $R^{15}$ can be CN. $R^{15}$ can be F. $R^{15}$ can be Cl. $R^{15}$ can be methyl. $R^{15}$ can be ethyl. $R^{15}$ can be isopropyl. $R^{15}$ can be cyclopropyl. $R^{15}$ can be $CF_3$. $R^{15}$ can be $OCF_2H$. $R^{15}$ can be $SO_2Me$. $R^{15}$ can be $CF_2H$. $R^{15}$ can be OMe. $R^{15}$ can be

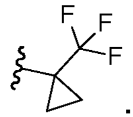.

In the compounds herein, $R^{16}$ can be H. $R^{16}$ can be CN. $R^{16}$ can be halo. $R^{16}$ can be F or Cl. $R^{16}$ can be F. $R^{16}$ can be $SF_5$. $R^{16}$ can be a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof. $R^{16}$ can be a $C_{1-6}$ alkyl group which is optionally substituted with 1 to 6 fluorine atoms wherein one atom of the $C_{1-6}$ alkyl group may be optionally replaced by a heteroatom selected from O, N, S and oxidised forms thereof. $R^{16}$ can be a $C_{1-6}$ alkyl group which is optionally substituted with 1 to 6 fluorine atoms. $R^{16}$ can be a $C_{1-6}$ alkyl group. $R^{16}$ can be a $OC_{1-6}$ alkyl group which is optionally substituted with 1 to 6 fluorine atoms. $R^{16}$ can be a $OC_{1-6}$ alkyl group. $R^{16}$ can be a $C_{1-6}$ alkoxy group which is optionally substituted with 1 to 6 fluorine atoms, $R^{16}$ can be a $C_{1-6}$ alkoxy group. $R^{16}$ can be a $SO_2C_{1-6}$ alkyl group which is optionally substituted with 1 to 6 fluorine atoms. $R^{16}$ can be a $SO_2C_{1-6}$ alkyl group. $R^{16}$ can be a $C_{3-6}$ cycloalkyl group which is optionally substituted with 1 to 6 fluorine atoms or optionally substituted with $CF_3$. $R^{16}$ can be a $C_{3-6}$ cycloalkyl group. $R^{16}$ can be H. $R^{16}$ can be CN. $R^{16}$ can be F. $R^{16}$ can be Cl. $R^{16}$ can be methyl. $R^{16}$ can be ethyl. $R^{16}$ can be isopropyl. $R^{16}$ can be cyclopropyl. $R^{16}$ can be $CF_3$. $R^{16}$ can be $OCF_2H$. $R^{16}$ can be $SO_2Me$. $R^{16}$ can be $CF_2H$. $R^{16}$ can be OMe. $R^{16}$ can be

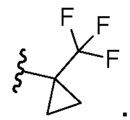.

In the compounds herein, each $R^{14}$ can independently be H, F or $C_{1-3}$ alkyl optionally substituted with 1 to 6 fluorine atoms. $R^{14}$ can be H, F or $C_{1-3}$ alkyl optionally substituted with 1 to 6 fluorine atoms. $R^{14}$ can be H. $R^{14}$ can be F. $R^{14}$ can be methyl. One $R^{14}$ can be joined to $R^{17}$ to form an oxolane or oxetane ring. $R^{14}$ can be joined to $R^{17}$ to form an oxolane or oxetane ring.

In the compounds herein, $R^{17}$ can be $C_{1-3}$ alkyl optionally substituted with 1 to 6 fluorine atoms. $R^{17}$ can be methyl. $R^{17}$ can be joined to one $R^{14}$ to form an oxolane or oxetane ring. $R^{17}$ can be joined to $R^{14}$ to form an oxolane or oxetane ring.

In the compounds herein, each $R^{18}$ can independently be H, F or $C_{1-3}$ alkyl optionally substituted with 1 to 6 fluorine atoms. $R^{18}$ can be H, F or $C_{1-3}$ alkyl optionally substituted with 1 to 6 fluorine atoms. $R^{18}$ can be H. $R^{18}$ can be F. $R^{18}$ can be methyl.

In the compounds herein, each $R^{19}$ can independently be H or $C_{1-3}$ alkyl optionally substituted with 1 to 6 fluorine atoms. $R^{19}$ can be H or $C_{1-3}$ alkyl optionally substituted with 1 to 6 fluorine atoms. $R^{19}$ can be H. $R^{19}$ can be methyl.

In the compounds herein, $R^3$ can be selected from the group consisting of:

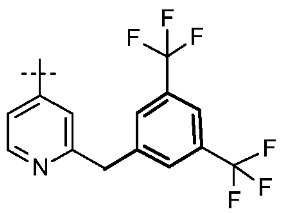 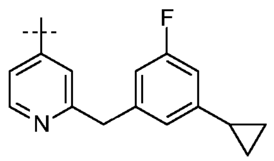 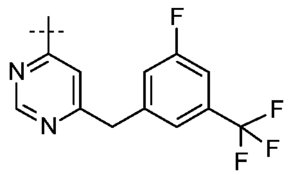

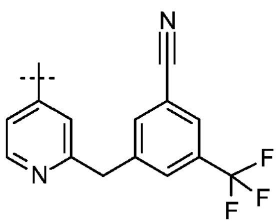 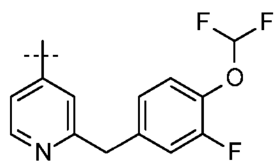 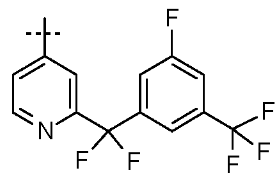

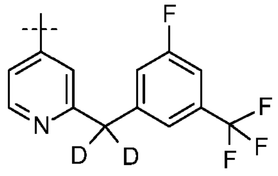
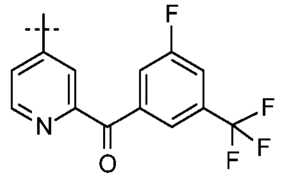
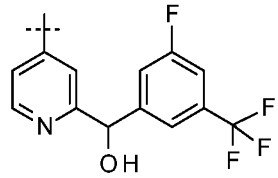

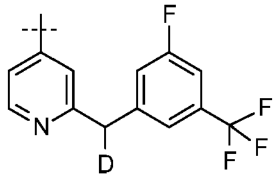
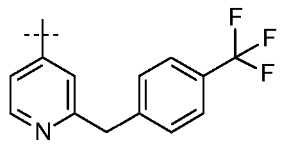
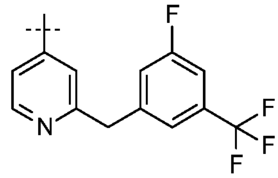

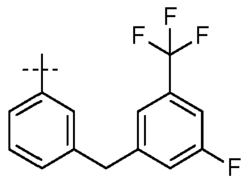
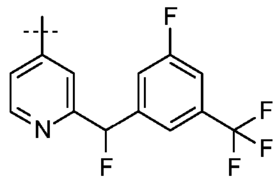
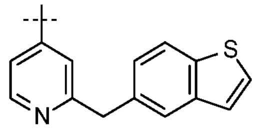

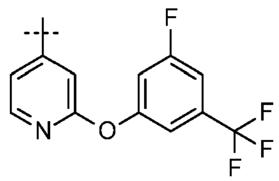
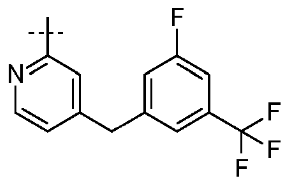
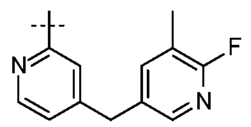

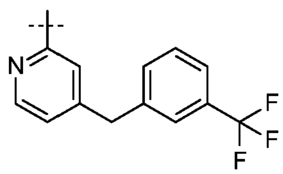
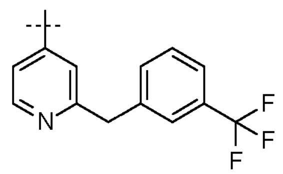
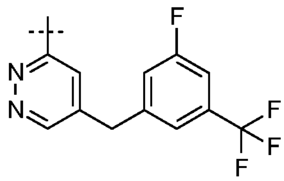

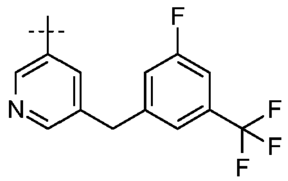
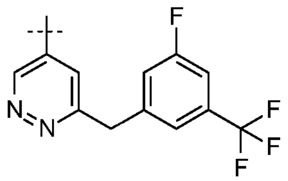
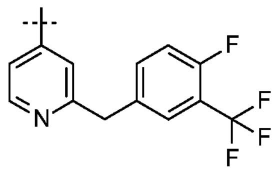

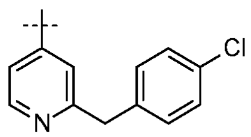
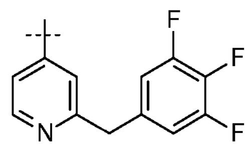
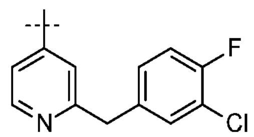

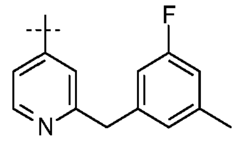
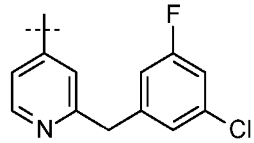
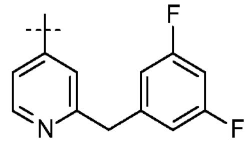

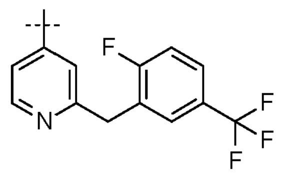 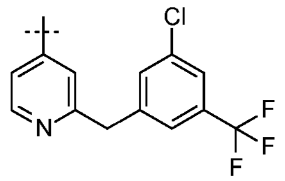 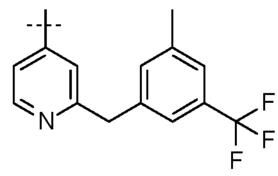

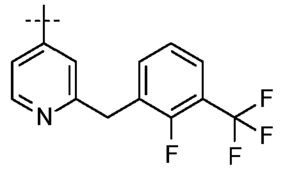 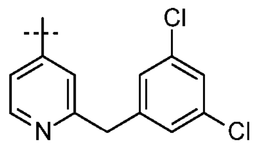 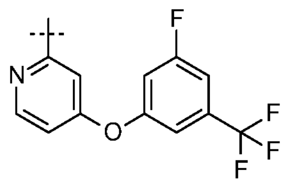

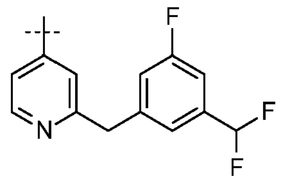 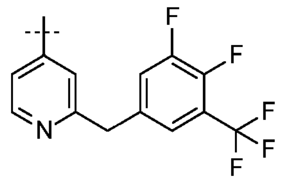 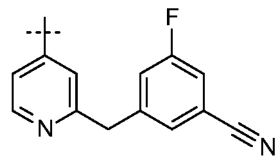

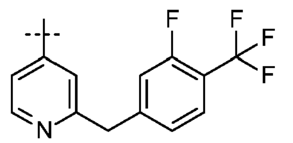 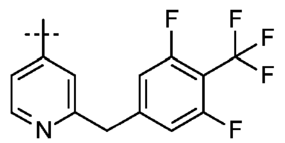 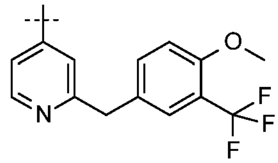

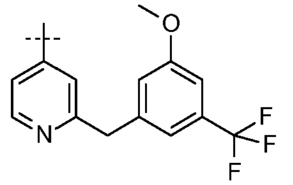 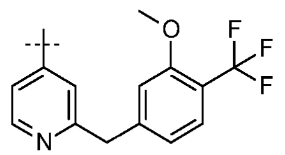 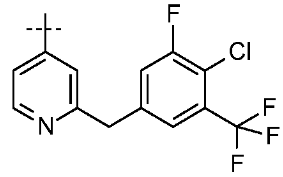

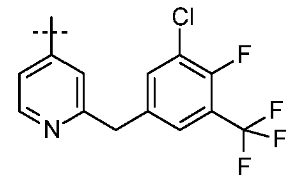 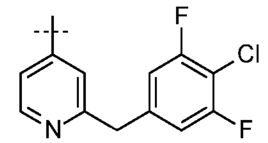 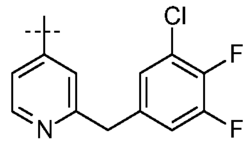

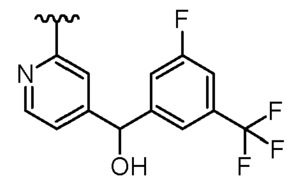 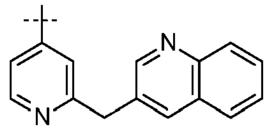 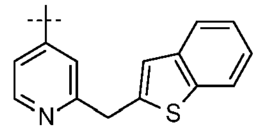

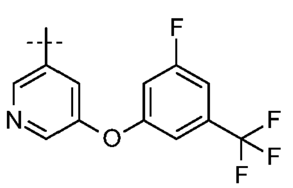 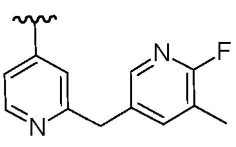 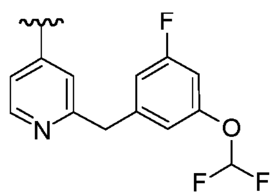

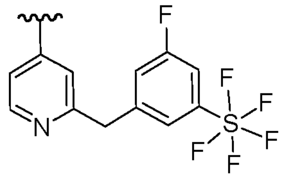
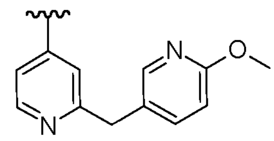
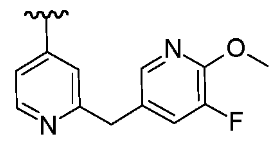
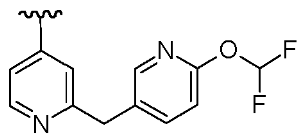
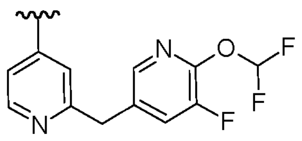
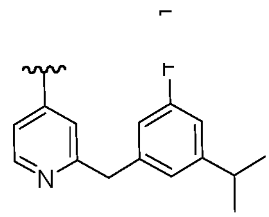
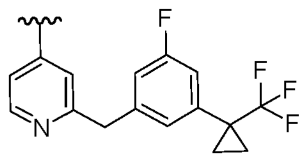
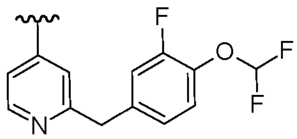
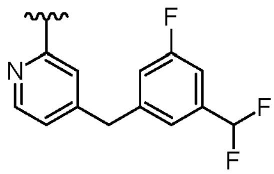
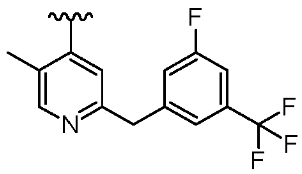

In the compounds herein, Q can be selected from $-CR^4R^5-$, $-CR^4R^5CR^6R^7-$, $-CR^4R^5CR^6R^7CR^8R^9-$, $-CR^4R^5CR^6R^7O-$, $-OCR^4R^5-$, $-OCR^4R^5CR^6R^7-$ and $-CR^4R^5O-$. Q can be selected from $-CR^4R^5-$, $-CR^4R^5CR^6R^7-$, $-CR^4R^5CR^6R^7CR^8R^9-$, $-CR^4R^5CR^6R^7O-$, $-OCR^4R^5-$ and $-OCR^4R^5CR^6R^7-$. Q can be $-CR^4R^5-$. Q can be $-CR^4R^5CR^6R^7-$. Q can be $- CR^4R^5CR^6R^7CR^8R^9-$. Q can be $-CR^4R^5CR^6R^7O-$. Q can be $-OCR^4R^5-$. Q can be $- OCR^4R^5CR^6R^7-$. Q can be $-CR^4R^5O-$.

In the compounds herein, Q can be selected from $-CH_2CH_2-$, $-CH_2CH_2CH_2-$, $-CH_2-$, $-OCH_2-$, $-CH_2O-$, $-CH_2CH_2O-$, $-OCH_2CH_2-$, $-CH(CH_3)CH_2-$ and $-CH_2CH(CH_3)-$. Q can be selected from the group consisting of $-CH_2CH_2-$, $-CH_2CH_2CH_2-$, $-CH_2-$, $-OCH_2-$, $-CH_2CH_2O-$, $-CH(CH_3)CH_2-$ and $-CH_2CH(CH_3)-$. Q can be $-CH_2CH_2-$. Q can be $-CH_2CH_2CH_2-$. Q can be $-CH_2-$. Q can be $-OCH_2-$. Q can be $-CH_2O-$. Q can be $-CH_2CH_2O-$. Q can be $CH_2CH(CH_3)-$. Q can be $-OCH_2CH_2-$. Q can be $-CH(CH_3)CH_2-$. Q can be a $C_{1-3}$ alkyl linker. Q can be selected from $-CH_2-$, $-CH_2CH_2-$ and $-CH_2CH_2CH_2-$. Q can be $-CH_2-$. Q can be $-CH_2CH_2-$. Q can be $-CH_2CH_2CH_2-$.

In the compounds herein, $R^4$, $R^5$, $R^6$, $R^7$, $R^8$ and $R^9$ can independently be H or a $C_{1-3}$ alkyl group. $R^4$, $R^5$, $R^6$, $R^7$, $R^8$ and $R^9$ can independently be H or methyl.

In the compounds herein, $R^4$ can be H. $R^4$ can be a $C_{1-3}$ alkyl group. $R^4$ can be methyl.

In the compounds herein, $R^5$ can be H. $R^5$ can be a $C_{1-3}$ alkyl group. $R^5$ can be methyl.

In the compounds herein, $R^6$ can be H. $R^6$ can be a $C_{1-3}$ alkyl group. $R^6$ can be methyl.

In the compounds herein, $R^7$ can be H. $R^7$ can be a $C_{1-3}$ alkyl group. $R^7$ can be methyl.

In the compounds herein, $R^8$ can be H. $R^8$ can be a $C_{1-3}$ alkyl group. $R^8$ can be methyl.

In the compounds herein, $R^9$ can be H. $R^9$ can be a $C_{1-3}$ alkyl group. $R^9$ can be methyl.

Particular compounds include compounds of Formula (2a), (2b), (2c), (2d), (2e), (2f) and (2g):

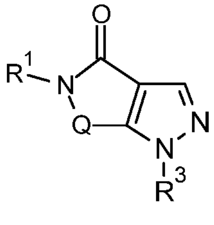 (2a); 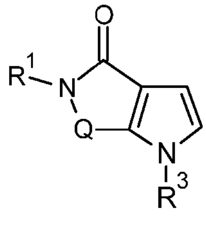 (2b); 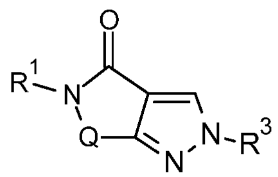 (2c);

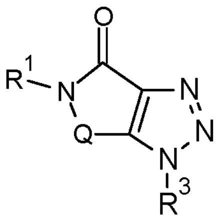 (2d); 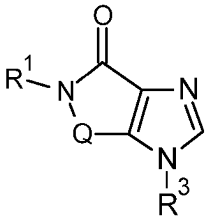 (2e); 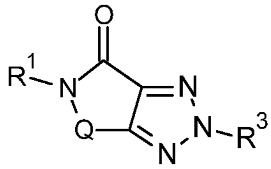 (2f);

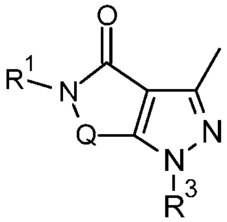 (2g);

or a salt thereof, wherein $R^1$, $R^3$ and Q are as defined above.

Particular compounds include compounds of Formula (3a), (3b), (3c), (3d), (3e), (3f) and (3g):

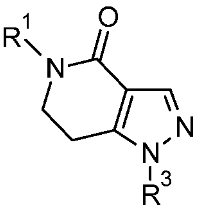 (3a); 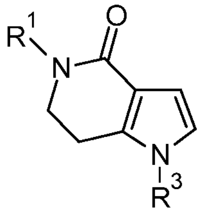 (3b); 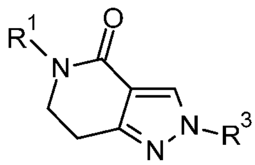 (3c);

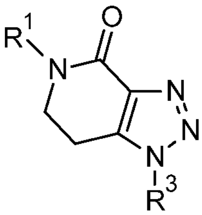 (3d); 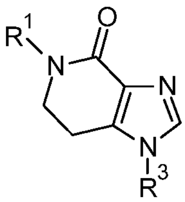 (3e); 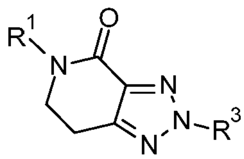 (3f);

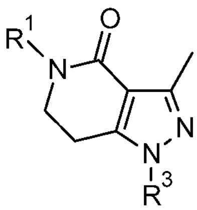 (3g);

or a salt thereof, wherein R' and $R^3$ are as defined above.

Particular compounds include compounds of formula (4a), (4b), (4c), (4d), (4e), (4f), (4g), (4h), (4i) or (4j):

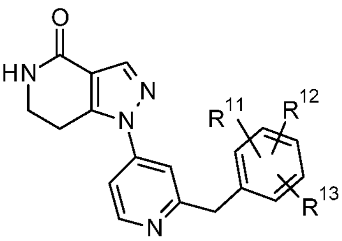 (4a); 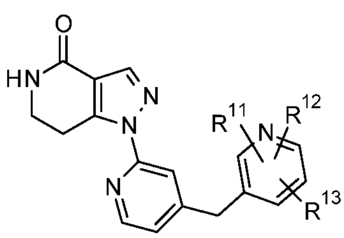 (4b);

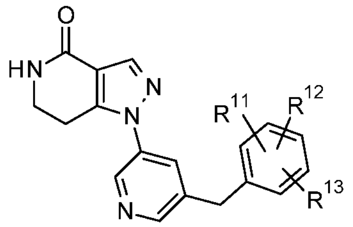 (4c); 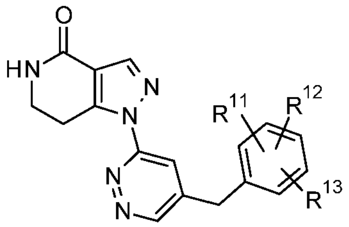 (4d);

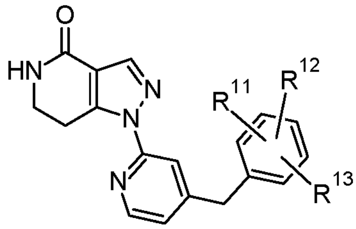 (4e); 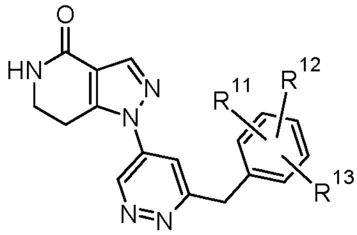 (4f);

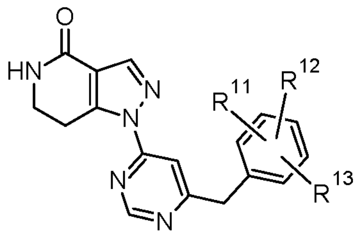 (4g); 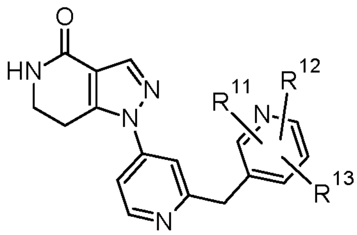 (4h);

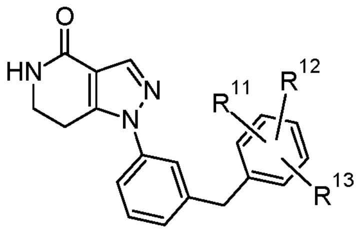 (4i); 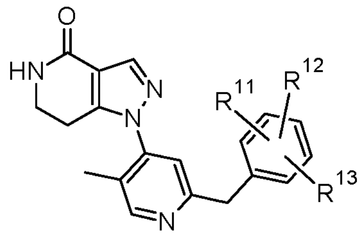 (4j);

or a salt thereof, wherein $R^{11}$, $R^{12}$ and $R^{13}$ are as defined above.

Particular compounds include compounds of formula (5a):

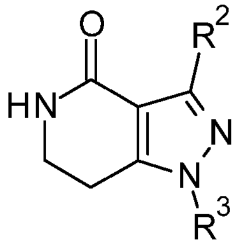 (5a);

or a salt thereof, wherein $R^2$ and $R^3$ are as defined above.

The compound can be a compound of formula (6a), (6b), (6c), (6d), (6e), (6f), (6g), (6h), (6i) or (6j):

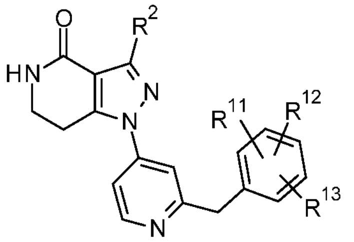 (6a); 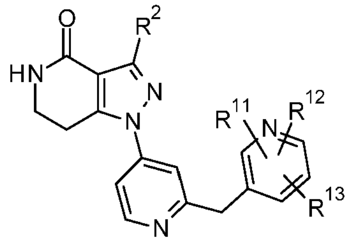 (6b);

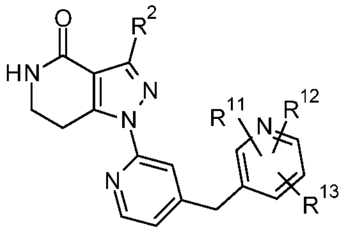 (6c); 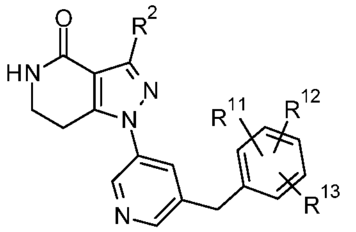 (6d);

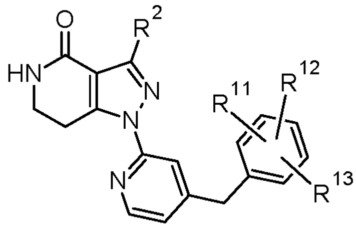 (6e); 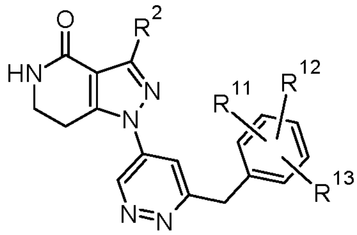 (6f);

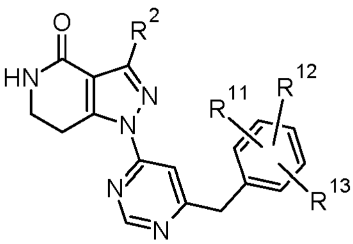 (6g); 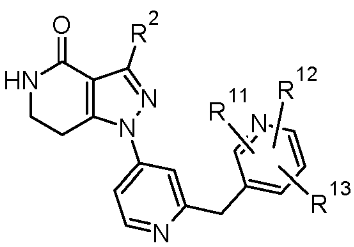 (6h);

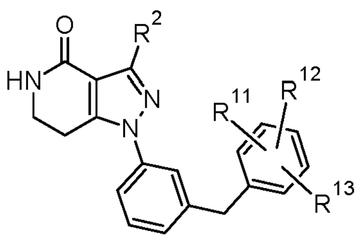 (6i); 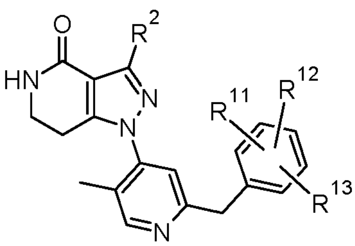 (6j);

or a salt thereof, wherein $R^2$, $R^{11}$, $R^{12}$ and $R^{13}$ are as defined above.

The compound can be a compound of formula (6a):

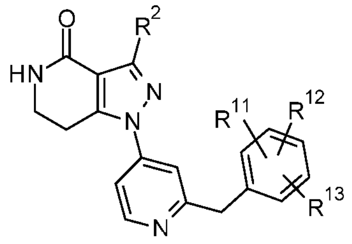
(6a);

or a salt thereof, wherein $R^2$, $R^{11}$, $R^{12}$ and $R^{13}$ are as defined above.

The compound can be a compound of formula (8a), (8b) or (8c):

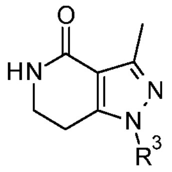
(8a);
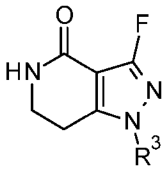
(8b);
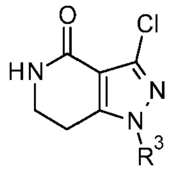
(8c);

or a salt thereof, wherein $R^3$ is as defined above.

Particular compounds include compounds of formula (9a), (9b), (9c), (9d), (9e), (9f), (9g), (9h), (9i) or (9j):

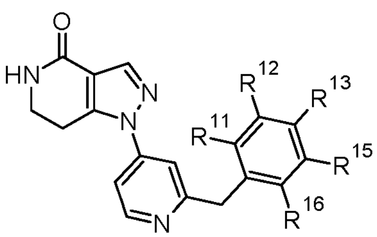
(9a);
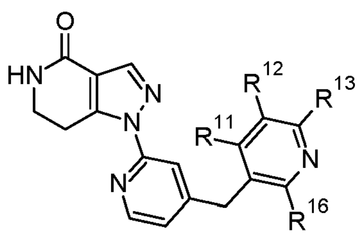
(9b);

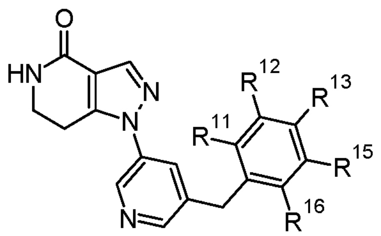
(9c);
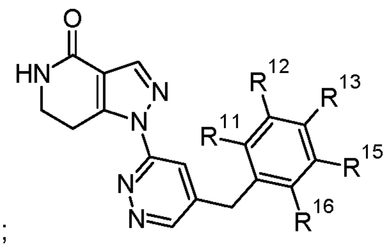
(9d);

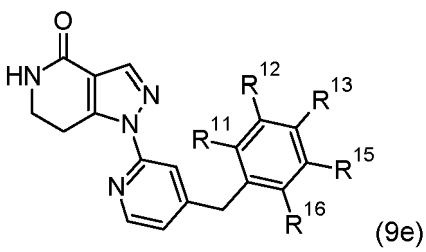
(9e);
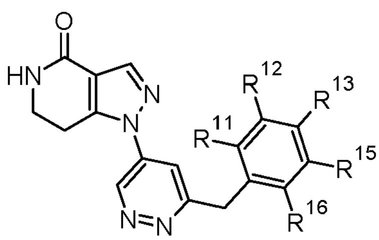
(9f);

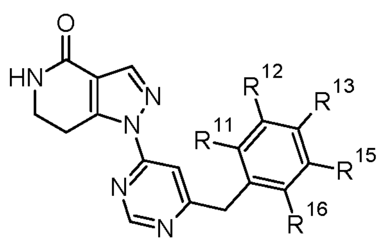
(9g);
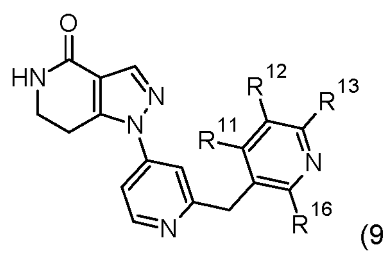
(9h);

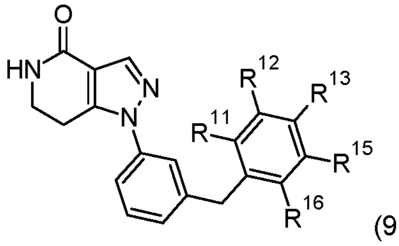 (9i); 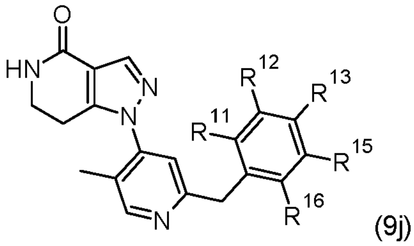 (9j);

or a salt thereof, wherein $R^{11}$, $R^{12}$, $R^{13}$, $R^{15}$ and $R^{16}$ are as defined above.

The compound can be a compound of formula (10a), (10b), (10c), (10d), (10e), (10f), (10g), (10h), (10i) or (10j):

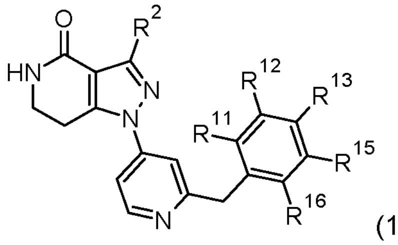 (10a); 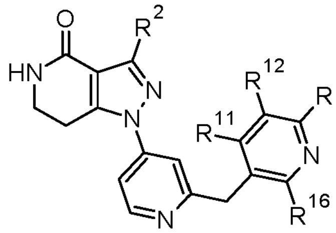 (10b);

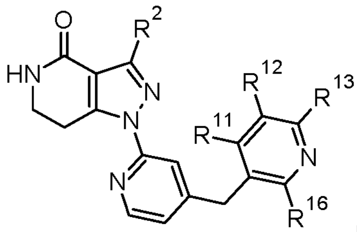 (10c); 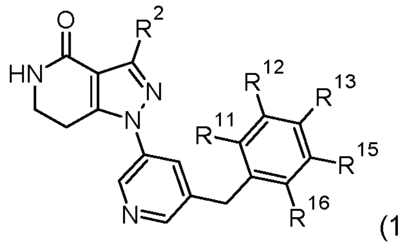 (10d);

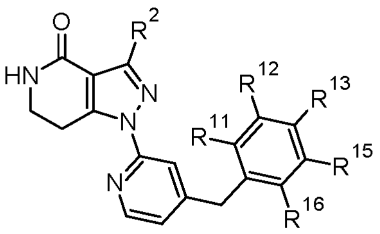 (10e); 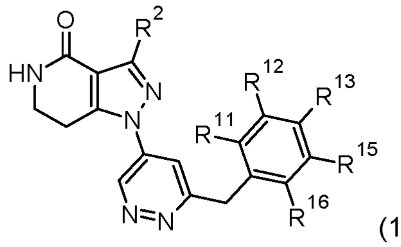 (10f);

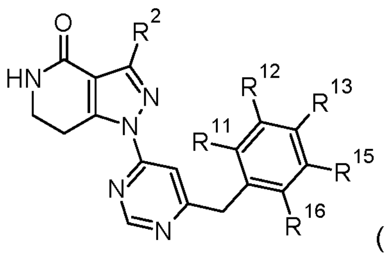 (10g); 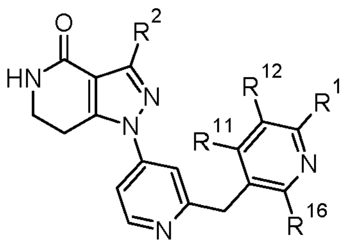 (10h);

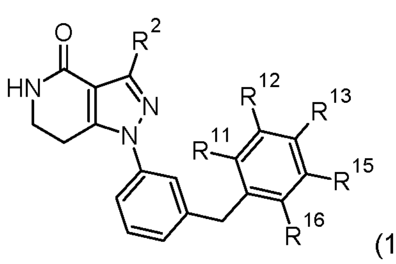 (10i); 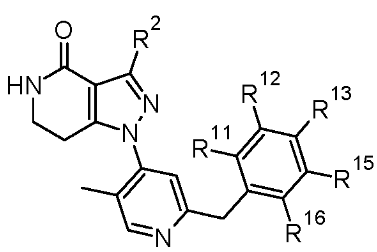 (10j);

or a salt thereof, wherein $R^2$, $R^{11}$, $R^{12}$, $R^{13}$, $R^{15}$ and $R^{16}$ are as defined above.

The compound can be a compound of formula (10a):

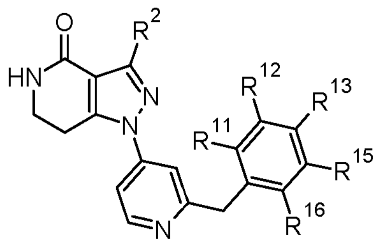
(10a);

or a salt thereof, wherein $R^2$, $R^{11}$, $R^{12}$, $R^{13}$, $R^{15}$ and $R^{16}$ are as defined above.

Particular compounds include compounds of Formula (11a), (11b) and (11c):

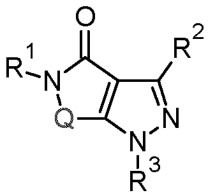
(11a);
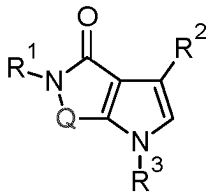
(11b);
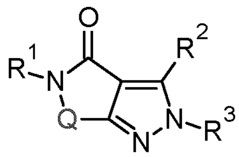
(11c);

or a salt thereof, wherein $R^1$, $R^2$, $R^3$ and Q are as defined above.

Particular compounds include compounds of Formula (12a), (12b) and (12c):

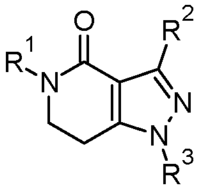
(12a);
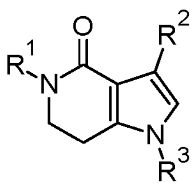
(12b);
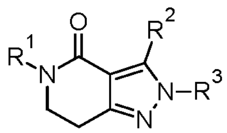
(12c);

or a salt thereof, wherein $R^1$, $R^2$ and $R^3$ are as defined above.

The compound can be a compound of formula (13a), (13b), (13c), (13d), (13e), (13f), (13g), (13h), (13i) or (13j):

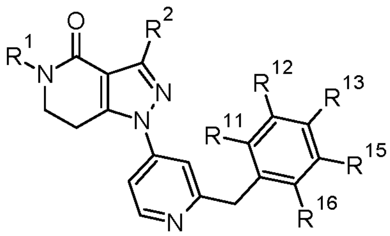
(13a);
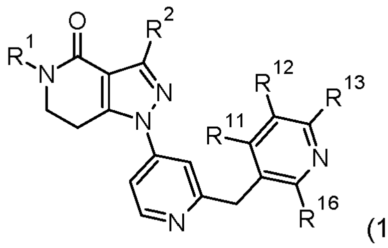
(13b);
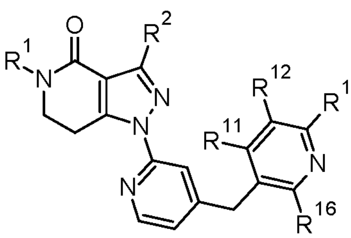
(13c);
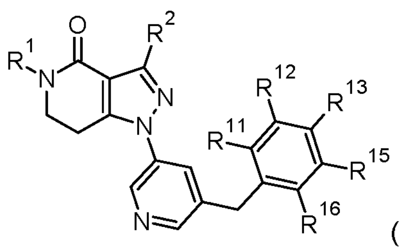
(13d);

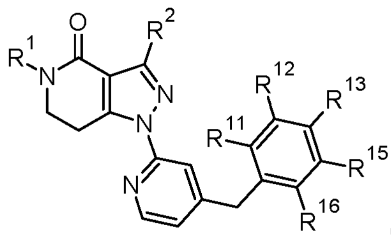 (13e); 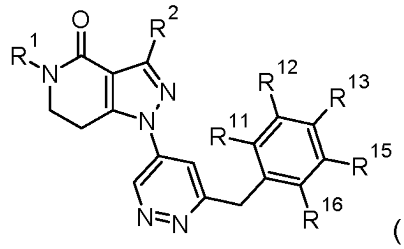 (13f);

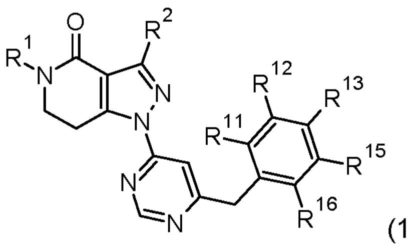 (13g); 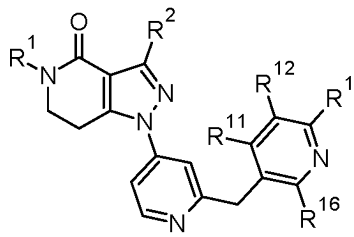 (13h);

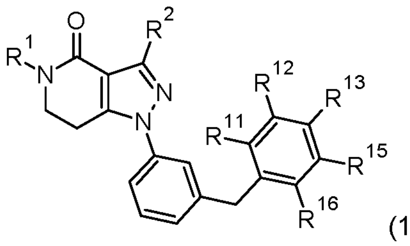 (13i); 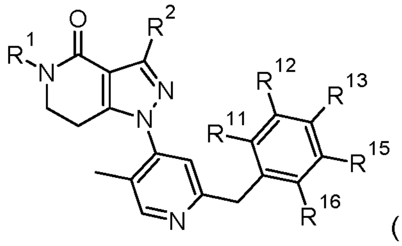 (13j);

or a salt thereof, wherein $R^1$, $R^2$, $R^{11}$, $R^{12}$, $R^{13}$, $R^{15}$ and $R^{16}$ are as defined above.

The compound can be a compound of formula (14a), (14b), (14c), (14d), (14e), (14f), (14g), (14h), (14i) or (14j):

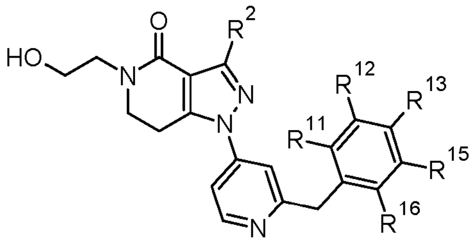 (14a); 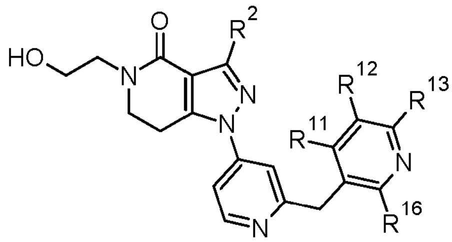 (14b);

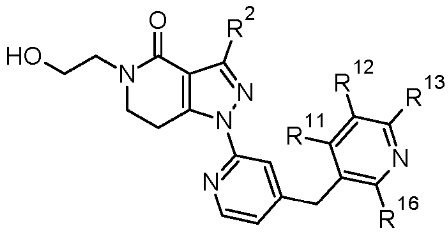 (14c); 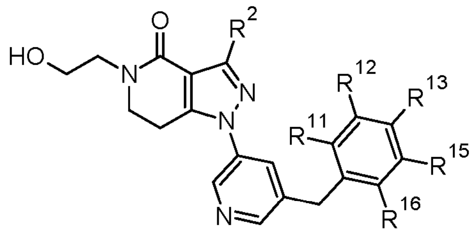 (14d);

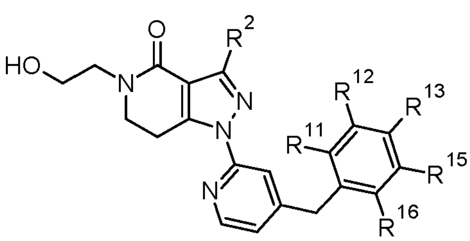 (14e); 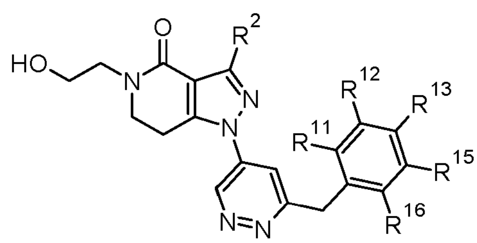 (14f);

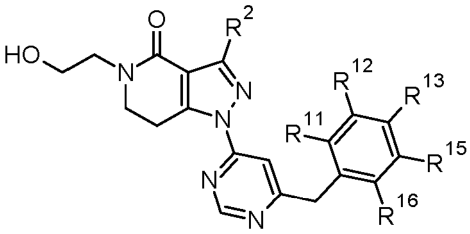 (14g);

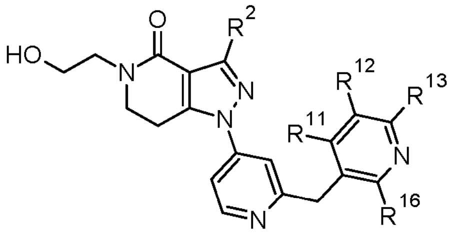 (14h);

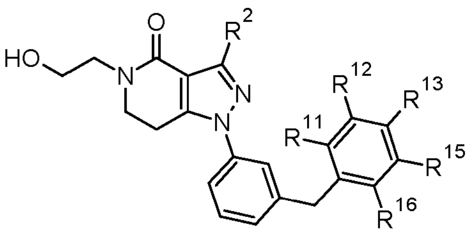 (14i);

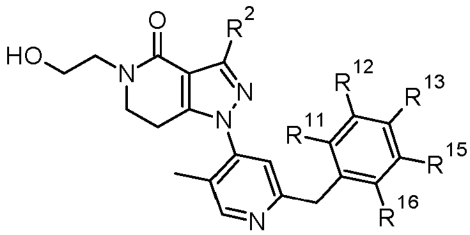 (14j);

or a salt thereof, wherein $R^2$, $R^{11}$, $R^{12}$, $R^{13}$, $R^{15}$ and $R^{16}$ are as defined above.

The compound can be selected from any one of Examples 1 to 118 as shown in Table 1 or a salt thereof.

The compound can be selected from the group consisting of:

1-(2-(3-fluoro-5-(trifluoromethyl)benzyl)pyridin-4-yl)-1,5,6,7-tetrahydro-4*H*-pyrazolo[4,3-*c*]pyridin-4-one;

1-(4-(3-(trifluoromethyl)benzyl)pyridin-2-yl)-1,5,6,7-tetrahydro-4*H*-pyrazolo[4,3-*c*]pyridin-4-one;

5-methyl-1-(4-(3-(trifluoromethyl)benzyl)pyridin-2-yl)-1,5,6,7-tetrahydro-4*H*-pyrazolo[4,3-*c*]pyridin-4-one;

1-(4-(3-fluoro-5-(trifluoromethyl)benzyl)pyridin-2-yl)-1,5,6,7-tetrahydro-4*H*-pyrazolo[4,3-*c*]pyridin-4-one;

1-(4-(3-fluoro-5-(trifluoromethyl)benzyl)pyridin-2-yl)-5-methyl-1,5,6,7-tetrahydro-4*H*-pyrazolo[4,3-*c*]pyridin-4-one;

1-(4-((6-fluoro-5-methylpyridin-3-yl)methyl)pyridin-2-yl)-1,5,6,7-tetrahydro-4*H*-pyrazolo[4,3-*c*]pyridin-4-one;

1-(4-((6-fluoro-5-methylpyridin-3-yl)methyl)pyridin-2-yl)-5-methyl-1,5,6,7-tetrahydro-4*H*-pyrazolo[4,3-*c*]pyridin-4-one;

2-(4-(3-(trifluoromethyl)benzyl)pyridin-2-yl)-2,5,6,7-tetrahydro-4*H*-pyrazolo[4,3-*c*]pyridin-4-one;

2-(2-(3-fluoro-5-(trifluoromethyl)benzyl)pyridin-4-yl)-2,5,6,7-tetrahydro-4*H*-pyrazolo[4,3-*c*]pyridin-4-one;

1-(4-(3-fluoro-5-(trifluoromethyl)benzyl)pyridin-2-yl)-5,6,7,8-tetrahydropyrazolo[4,3-*c*]azepin-4(1*H*)-one;

1-(4-(3-(trifluoromethyl)benzyl)pyridin-2-yl)-5,6,7,8-tetrahydropyrazolo[4,3-*c*]azepin-4(1*H*)-one;

1-(2-(3-fluoro-5-(trifluoromethyl)benzyl)pyridin-4-yl)-5,6,7,8-tetrahydropyrazolo[4,3-*c*]azepin-4(1*H*)-one;

1-(4-(3-(trifluoromethyl)benzyl)pyridin-2-yl)-6,7-dihydro-1*H*-pyrazolo[4,3-*f*][1,4]oxazepin-4(5*H*)-one;

1-(4-(3-fluoro-5-(trifluoromethyl) benzyl) pyridin-2-yl)-6, 7-dihydro-1*H*-pyrazolo[4,3-*f*][1,4]oxazepin-4(5*H*)-one;

1-(5-(3-fluoro-5-(trifluoromethyl)benzyl)pyridazin-3-yl)-1,5,6,7-tetrahydro-4*H*-pyrazolo[4,3-*c*]pyridin-4-one;

1-(5-(3-fluoro-5-(trifluoromethyl)benzyl)pyridin-3-yl)-1,5,6,7-tetrahydro-4*H*-pyrazolo[4,3-*c*]pyridin-4-one;

1-(6-(3-fluoro-5-(trifluoromethyl)benzyl)pyridazin-4-yl)-1,5,6,7-tetrahydro-4*H*-pyrazolo[4,3-*c*]pyridin-4-one;

1-{4-[3-fluoro-5-(trifluoromethyl)phenoxy]pyridin-2-yl}-1,5,6,7-tetrahydro-4*H*-pyrazolo[4,3-*c*]pyridin-4-one;

1-(2-(4-chlorobenzyl)pyridin-4-yl)-1,5,6,7-tetrahydro-4*H*-pyrazolo[4,3-*c*]pyridin-4-one;

1-(2-(3-chloro-5-fluorobenzyl)pyridin-4-yl)-1,5,6,7-tetrahydro-4*H*-pyrazolo[4,3-*c*]pyridin-4-one;

1-(2-(3,5-difluorobenzyl)pyridin-4-yl)-1,5,6,7-tetrahydro-4*H*-pyrazolo[4,3-*c*]pyridin-4-one;

1-(2-(3-(trifluoromethyl)benzyl)pyridin-4-yl)-1,5,6,7-tetrahydro-4*H*-pyrazolo[4,3-*c*]pyridin-4-one;

1-(2-(4-fluoro-3-(trifluoromethyl)benzyl)pyridin-4-yl)-1,5,6,7-tetrahydro-4*H*-pyrazolo[4,3-*c*]pyridin-4-one;

1-(2-(3,4,5-trifluorobenzyl)pyridin-4-yl)-1,5,6,7-tetrahydro-4*H*-pyrazolo[4,3-*c*]pyridin-4-one;

1-(2-(3-chloro-4-fluorobenzyl)pyridin-4-yl)-1,5,6,7-tetrahydro-4*H*-pyrazolo[4,3-*c*]pyridin-4-one;

1-(2-(3-fluoro-5-methylbenzyl)pyridin-4-yl)-1,5,6,7-tetrahydro-4*H*-pyrazolo[4,3-*c*]pyridin-4-one;

1-(2-(3-chloro-5-(trifluoromethyl)benzyl)pyridin-4-yl)-1,5,6,7-tetrahydro-4*H*-pyrazolo[4,3-*c*]pyridin-4-one;

1-(2-(3-methyl-5-(trifluoromethyl)benzyl)pyridin-4-yl)-1,5,6,7-tetrahydro-4*H*-pyrazolo[4,3-*c*]pyridin-4-one;

1-(2-(2-fluoro-3-(trifluoromethyl)benzyl)pyridin-4-yl)-1,5,6,7-tetrahydro-4*H*-pyrazolo[4,3-*c*]pyridin-4-one;

1-(2-(2-fluoro-5-(trifluoromethyl)benzyl)pyridin-4-yl)-1,5,6,7-tetrahydro-4*H*-pyrazolo[4,3-*c*]pyridin-4-one;

1-(2-(3,5-dichlorobenzyl)pyridin-4-yl)-1,5,6,7-tetrahydro-4*H*-pyrazolo[4,3-*c*]pyridin-4-one;

1-(2-(4-(trifluoromethyl)benzyl)pyridin-4-yl)-1,5,6,7-tetrahydro-4*H*-pyrazolo[4,3-*c*]pyridin-4-one;

3-((4-(4-oxo-4,5,6,7-tetrahydro-1*H*-pyrazolo[4,3-*c*]pyridin-1-yl)pyridin-2-yl)methyl)-5-(trifluoromethyl)benzonitrile;

1-(2-(3,5-bis(trifluoromethyl)benzyl)pyridin-4-yl)-1,5,6,7-tetrahydro-4*H*-pyrazolo[4,3-*c*]pyridin-4-one;

1-(2-(3-fluoro-5-(trifluoromethyl)benzoyl)pyridin-4-yl)-1,5,6,7-tetrahydro-4*H*-pyrazolo[4,3-*c*]pyridin-4-one;

1-(2-((3-fluoro-5-(trifluoromethyl)phenyl)(hydroxy)methyl)pyridin-4-yl)-1,5,6,7-tetrahydro-4*H*-pyrazolo[4,3-*c*]pyridin-4-one;

1-(2-(fluoro(3-fluoro-5-(trifluoromethyl)phenyl)methyl)pyridin-4-yl)-1,5,6,7-tetrahydro-4*H*-pyrazolo[4,3-*c*]pyridin-4-one;

1-(2-(difluoro(3-fluoro-5-(trifluoromethyl)phenyl)methyl)pyridin-4-yl)-1,5,6,7-tetrahydro-4*H*-pyrazolo[4,3-*c*]pyridin-4-one;

1-(2-((3-fluoro-5-(trifluoromethyl)phenyl)methyl-$d_2$)pyridin-4-yl)-1,5,6,7-tetrahydro-4*H*-pyrazolo[4,3-]pyridin-4-one;

1-(2-(3-fluoro-5-(trifluoromethyl)benzyl)pyridin-4-yl)-6-methyl-1,5,6,7-tetrahydro-4*H*-pyrazolo[4,3-*c*]pyridin-4-one;

1-(4-(3-(trifluoromethyl)benzyl)pyridin-2-yl)-5,6,7,8-tetrahydro-[1,2,3]triazolo[4,5-*c*]azepin-4(1*H*)-one;

1-(4-(3-(trifluoromethyl)benzyl)pyridin-2-yl)-1,5,6,7-tetrahydro-4*H*-[1,2,3]triazolo[4,5-*c*]pyridin-4-one;

2-(4-(3-(trifluoromethyl)benzyl)pyridin-2-yl)-2,5,6,7-tetrahydro-4*H*-[1,2,3]triazolo[4,5-*c*]pyridin-4-one;

5-methyl-2-(4-(3-(trifluoromethyl)benzyl)pyridin-2-yl)-2,5,6,7-tetrahydro-4*H*-[1,2,3]triazolo[4,5-c]pyridin-4-one;

2-(4-(3-(trifluoromethyl)benzyl)pyridin-2-yl)-5,6,7,8-tetrahydro-[1,2,3]triazolo[4,5-c]azepin-4(2*H*)-one;

1-(4-(3-fluoro-5-(trifluoromethyl)benzyl)pyridin-2-yl)-5,6,7,8-tetrahydro-[1,2,3]triazolo[4,5-*c*]azepin-4(1*H*)-one;

1-(4-(3-fluoro-5-(trifluoromethyl)benzyl)pyridin-2-yl)-1,7-dihydropyrazolo[4,3-*d*][1,2]oxazin-4(5*H*)-one;

1-(2-(3-fluoro-5-(trifluoromethyl)benzyl)pyridin-4-yl)-5,6-dihydropyrrolo[3,4-*c*]pyrazol-4(1*H*)-one;

1-(2-((3-fluoro-5-(trifluoromethyl)phenyl)methyl-d)pyridin-4-yl)-1,5,6,7-tetrahydro-4*H*-pyrazolo[4,3-*c*]pyridin-4-one;

1-(2-(3-fluoro-5-(trifluoromethyl)benzyl)pyridin-4-yl)-7-methyl-1,5,6,7-tetrahydro-4*H*-pyrazolo[4,3-*c*]pyridin-4-one;

1-(2-(3-fluoro-5-(trifluoromethyl)benzyl)pyridin-4-yl)-1,5,6,7-tetrahydro-4*H*-pyrrolo[3,2-*c*]pyridin-4-one;

1-(2-(3-fluoro-5-(trifluoromethyl)phenoxy)pyridin-4-yl)-1,5,6,7-tetrahydro-4*H*-pyrazolo[4,3-*c*]pyridin-4-one;

1-(2-(3-cyclopropyl-5-fluorobenzyl)pyridin-4-yl)-1,5,6,7-tetrahydro-4*H*-pyrazolo[4,3-*c*]pyridin-4-one;

1-(2-(3-fluoro-4-(trifluoromethyl)benzyl)pyridin-4-yl)-1,5,6,7-tetrahydro-4*H*-pyrazolo[4,3-*c*]pyridin-4-one;

1-(2-(4-methoxy-3-(trifluoromethyl)benzyl)pyridin-4-yl)-1,5,6,7-tetrahydro-4*H*-pyrazolo[4,3-*c*]pyridin-4-one;

1-(6-(3-fluoro-5-(trifluoromethyl)benzyl)pyrimidin-4-yl)-1,5,6,7-tetrahydro-4*H*-pyrazolo[4,3-*c*]pyridin-4-one;

1-(2-(3-methoxy-5-(trifluoromethyl)benzyl)pyridin-4-yl)-1,5,6,7-tetrahydro-4*H*-pyrazolo[4,3-*c*]pyridin-4-one;

1-(2-(4-chloro-3,5-difluorobenzyl)pyridin-4-yl)-1,5,6,7-tetrahydro-4*H*-pyrazolo[4,3-*c*]pyridin-4-one;

1-(2-(benzo[*b*]thiophen-5-ylmethyl)pyridin-4-yl)-1,5,6,7-tetrahydro-4*H*-pyrazolo[4,3-c]pyridin-4-one;

1-(2-(3-methoxy-4-(trifluoromethyl)benzyl)pyridin-4-yl)-1,5,6,7-tetrahydro-4*H*-pyrazolo[4,3-*c*]pyridin-4-one;

1-(2-(3-fluoro-5-(pentafluoro-$\lambda^6$-sulfaneyl)benzyl)pyridin-4-yl)-1,5,6,7-tetrahydro-4*H*-pyrazolo[4,3-*c*]pyridin-4-one;

1-(2-(3-(difluoromethoxy)-5-fluorobenzyl)pyridin-4-yl)-1,5,6,7-tetrahydro-4*H*-pyrazolo[4,3-*c*]pyridin-4-one;

1-(2-(4-(difluoromethoxy)-3-fluorobenzyl)pyridin-4-yl)-1,5,6,7-tetrahydro-4*H*-pyrazolo[4,3-*c*]pyridin-4-one;

1-(2-(3,4-difluoro-5-(trifluoromethyl)benzyl)pyridin-4-yl)-1,5,6,7-tetrahydro-4*H*-pyrazolo[4,3-*c*]pyridin-4-one;

1-(2-(3-(difluoromethyl)-5-fluorobenzyl)pyridin-4-yl)-1,5,6,7-tetrahydro-4*H*-pyrazolo[4,3-*c*]pyridin-4-one;

1-(2-(3-chloro-4,5-difluorobenzyl)pyridin-4-yl)-1,5,6,7-tetrahydro-4*H*-pyrazolo[4,3-*c*]pyridin-4-one;

1-(2-(quinolin-3-ylmethyl)pyridin-4-yl)-1,5,6,7-tetrahydro-4*H*-pyrazolo[4,3-*c*]pyridin-4-one;

1-(2-(4-chloro-3-fluoro-5-(trifluoromethyl)benzyl)pyridin-4-yl)-1,5,6,7-tetrahydro-4*H*-pyrazolo[4,3-*c*]pyridin-4-one;

1-(2-(benzo[b]thiophen-2-ylmethyl)pyridin-4-yl)-1,5,6,7-tetrahydro-4*H*-pyrazolo[4,3-*c*]pyridin-4-one;

1-(2-(3,5-difluoro-4-(trifluoromethyl)benzyl)pyridin-4-yl)-1,5,6,7-tetrahydro-4*H*-pyrazolo[4,3-*c*]pyridin-4-one;

1-(2-(3-fluoro-5-isopropylbenzyl)pyridin-4-yl)-1,5,6,7-tetrahydro-4*H*-pyrazolo[4,3-*c*]pyridin-4-one;

1-(2-((5-fluoro-6-methoxypyridin-3-yl)methyl)pyridin-4-yl)-1,5,6,7-tetrahydro-4*H*-pyrazolo[4,3-*c*]pyridin-4-one;

1-(2-(3-chloro-4-fluoro-5-(trifluoromethyl)benzyl)pyridin-4-yl)-1,5,6,7-tetrahydro-4*H*-pyrazolo[4,3-c]pyridin-4-one;

1-(2-(3-fluoro-5-(1-(trifluoromethyl)cyclopropyl)benzyl)pyridin-4-yl)-1,5,6,7-tetrahydro-4*H*-pyrazolo[4,3-*c*]pyridin-4-one;

1-(2-(3-fluoro-5-(trifluoromethyl)benzyl)pyridin-4-yl)-3-methyl-1,5,6,7-tetrahydro-4*H*-pyrazolo[4,3-*c*]pyridin-4-one;

1-(2-((6-fluoro-5-methylpyridin-3-yl)methyl)pyridin-4-yl)-3-methyl-1,5,6,7-tetrahydro-4*H*-pyrazolo[4,3-*c*]pyridin-4-one;

3-methyl-1-(2-(3,4,5-trifluorobenzyl)pyridin-4-yl)-1,5,6,7-tetrahydro-4*H*-pyrazolo[4,3-*c*]pyridin-4-one;

3-methyl-1-(2-(3-(trifluoromethyl)benzyl)pyridin-4-yl)-1,5,6,7-tetrahydro-4*H*-pyrazolo[4,3-*c*]pyridin-4-one;

1-(2-(3-chloro-5-fluorobenzyl)pyridin-4-yl)-3-methyl-1,5,6,7-tetrahydro-4*H*-pyrazolo[4,3-*c*]pyridin-4-one;

1-(2-(3-chloro-4-fluorobenzyl)pyridin-4-yl)-3-methyl-1,5,6,7-tetrahydro-4*H*-pyrazolo[4,3-*c*]pyridin-4-one;

1-(2-(4-(difluoromethoxy)-3-fluorobenzyl)pyridin-4-yl)-3-methyl-1,5,6,7-tetrahydro-4*H*-pyrazolo[4,3-*c*]pyridin-4-one;

1-(5-(3-fluoro-5-(trifluoromethyl)benzyl)pyridin-3-yl)-3-methyl-1,5,6,7-tetrahydro-4*H*-pyrazolo[4,3-*c*]pyridin-4-one;

1-(4-((6-fluoro-5-methylpyridin-3-yl)methyl)pyridin-2-yl)-3-methyl-1,5,6,7-tetrahydro-4*H*-pyrazolo[4,3-*c*]pyridin-4-one;

1-(2-(3-chloro-4,5-difluorobenzyl)pyridin-4-yl)-3-methyl-1,5,6,7-tetrahydro-4*H*-pyrazolo[4,3-*c*]pyridin-4-one;

3-fluoro-5-((4-(3-methyl-4-oxo-4,5,6,7-tetrahydro-1*H*-pyrazolo[4,3-*c*]pyridin-1-yl)pyridin-2-yl)methyl)benzonitrile;

1-(2-(3,4-difluoro-5-(trifluoromethyl)benzyl)pyridin-4-yl)-3-methyl-1,5,6,7-tetrahydro-4*H*-pyrazolo[4,3-*c*]pyridin-4-one;

1-(2-(4-chloro-3-fluoro-5-(trifluoromethyl)benzyl)pyridin-4-yl)-3-methyl-1,5,6,7-tetrahydro-4*H*-pyrazolo[4,3-*c*]pyridin-4-one;

1-(2-(3-chloro-4-fluoro-5-(trifluoromethyl)benzyl)pyridin-4-yl)-3-methyl-1,5,6,7-tetrahydro-4*H*-pyrazolo[4,3-*c*]pyridin-4-one;

1-(2-(3-(difluoromethoxy)-5-fluorobenzyl)pyridin-4-yl)-3-methyl-1,5,6,7-tetrahydro-4*H*-pyrazolo[4,3-*c*]pyridin-4-one;

1-(2-(4-chloro-3,5-difluorobenzyl)pyridin-4-yl)-3-methyl-1,5,6,7-tetrahydro-4*H*-pyrazolo[4,3-*c*]pyridin-4-one;

1-(2-(3-(difluoromethyl)-5-fluorobenzyl)pyridin-4-yl)-3-methyl-1,5,6,7-tetrahydro-4*H*-pyrazolo[4,3-*c*]pyridin-4-one;

1-(2-((6-methoxypyridin-3-yl)methyl)pyridin-4-yl)-3-methyl-1,5,6,7-tetrahydro-4*H*-pyrazolo[4,3-*c*]pyridin-4-one;

1-(2-((5-fluoro-6-methoxypyridin-3-yl)methyl)pyridin-4-yl)-3-methyl-1,5,6,7-tetrahydro-4*H*-pyrazolo[4,3-*c*]pyridin-4-one;

1-(2-((6-(difluoromethoxy)pyridin-3-yl)methyl)pyridin-4-yl)-3-methyl-1,5,6,7-tetrahydro-4*H*-pyrazolo[4,3-*c*]pyridin-4-one;

1-(4-(3-fluoro-5-(trifluoromethyl)benzyl)pyridin-2-yl)-3-methyl-1,5,6,7-tetrahydro-4*H*-pyrazolo[4,3-*c*]pyridin-4-one;

3-fluoro-5-((4-(4-oxo-4,5,6,7-tetrahydro-1*H*-pyrazolo[4,3-*c*]pyridin-1-yl)pyridin-2-yl)methyl)benzonitrile;

1-(2-(3-fluoro-5-(trifluoromethyl)benzyl)pyridin-4-yl)-5,6,7,8-tetrahydro-[1,2,3]triazolo[4,5-*c*]azepin-4(1*H*)-one;

2-(4-(3-fluoro-5-(trifluoromethyl)benzyl)pyridin-2-yl)-2,5,6,7-tetrahydro-4*H*-[1,2,3]triazolo[4,5-*c*]pyridin-4-one;

1-(4-(3-fluoro-5-(trifluoromethyl)benzyl)pyridin-2-yl)-1,5,6,7-tetrahydro-4*H*-[1,2,3]triazolo[4,5-*c*]pyridin-4-one;

1-(2-(3-fluoro-5-(trifluoromethyl)benzyl)pyridin-4-yl)-1,5,6,7-tetrahydro-4*H*-[1,2,3]triazolo[4,5-*c*]pyridin-4-one;

2-(2-(3-fluoro-5-(trifluoromethyl)benzyl)pyridin-4-yl)-2,5,6,7-tetrahydro-4*H*-[1,2,3]triazolo[4,5-*c*]pyridin-4-one;

1-(4-(3-(difluoromethyl)-5-fluorobenzyl)pyridin-2-yl)-1,5,6,7-tetrahydro-4*H*-pyrazolo[4,3-*c*]pyridin-4-one;

1-(5-(3-fluoro-5-(trifluoromethyl)phenoxy)pyridin-3-yl)-1,5,6,7-tetrahydro-4*H*-pyrazolo[4,3-*c*]pyridin-4-one;

1-(2-(3-fluoro-5-(trifluoromethyl)benzyl)pyridin-4-yl)-5-(2-methoxyethyl)-1,5,6,7-tetrahydro-4*H*-pyrazolo[4,3-*c*]pyridin-4-one;

1-(2-(3-fluoro-5-(trifluoromethyl)benzyl)pyridin-4-yl)-5-(2-hydroxyethyl)-1,5,6,7-tetrahydro-4*H*-pyrazolo[4,3-*c*]pyridin-4-one;

1-(4-(3-fluoro-5-(trifluoromethyl)benzyl)pyridin-2-yl)-5-(2-methoxyethyl)-1,5,6,7-tetrahydro-4*H*-pyrazolo[4,3-*c*]pyridin-4-one;

1-(4-(3-fluoro-5-(trifluoromethyl)benzyl)pyridin-2-yl)-5-(2-hydroxyethyl)-1,5,6,7-tetrahydro-4*H*-pyrazolo[4,3-*c*]pyridin-4-one;

1-(2-(3-fluoro-5-(trifluoromethyl)benzyl)-5-methylpyridin-4-yl)-1,5,6,7-tetrahydro-4*H*-pyrazolo[4,3-*c*]pyridin-4-one;

1-(3-(3-fluoro-5-(trifluoromethyl)benzyl)phenyl)-1,5,6,7-tetrahydro-4*H*-pyrazolo[4,3-*c*]pyridin-4-one;

1-(2-((6-(difluoromethoxy)-5-fluoropyridin-3-yl)methyl)pyridin-4-yl)-1,5,6,7-tetrahydro-4*H*-pyrazolo[4,3-*c*]pyridin-4-one;

1-(2-((6-(difluoromethoxy)-5-fluoropyridin-3-yl)methyl)pyridin-4-yl)-3-methyl-1,5,6,7-tetrahydro-4*H*-pyrazolo[4,3-*c*]pyridin-4-one;

2-(2-(3-fluoro-5-(trifluoromethyl)benzyl)pyridin-4-yl)-6,7-dihydro-2H-pyrazolo[4,3-*f*][1,4]oxazepin-4(5*H*)-one;

1-(4-(3-fluoro-5-(trifluoromethyl)benzyl)pyridin-2-yl)-3-(hydroxymethyl)-1,5,6,7-tetrahydro-4*H*-pyrazolo[4,3-*c*]pyridin-4-one;

1-(2-(3-fluoro-5-(trifluoromethyl)benzyl)pyridin-4-yl)-3-(hydroxymethyl)-1,5,6,7-tetrahydro-4*H*-pyrazolo[4,3-*c*]pyridin-4-one;

1-(2-(3-fluoro-5-(trifluoromethyl)benzyl)pyridin-4-yl)-4-oxo-4,5,6, 7 -tetrahydro-1*H*-pyrazolo[4,3-*c*]pyridine-3-carbonitrile;

1-(2-(3-fluoro-5-(trifluoromethyl)benzyl)pyridin-4-yl)-3-(trifluoromethyl)-1,5,6,7-tetrahydro-4*H*-pyrazolo[4,3-*c*]pyridin-4-one;

1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one;

1-(4-((3-fluoro-5-(trifluoromethyl)phenyl)(hydroxy)methyl)pyridin-2-yl)-3-methyl-1,5,6,7-tetrahydro-4*H*-pyrazolo[4,3-c]pyridin-4-one;

or a salt thereof.

Further embodiments of the invention include the use of a compound of Formula (1) or a salt thereof or a pharmaceutical composition comprising a compound of Formula (1) as a GPR52 receptor modulator or a GPR52 receptor agonist. Compounds of the present invention may be used as GPR52 modulators. Compounds of the present invention may be used as GPR52 agonists. General references to Formula 1 throughout the specification include all compounds of Formula (1x) including (1'), (1a), (1b).

Compounds of the present invention may be used in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders;

depressive disorders; anxiety disorders; obsessive-compulsive disorder (OCD); autism spectrum disorders; depressive disorders; hypothalamic disorders; pituitary disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

Compounds of the present invention may be used in the treatment of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, anhedonia, agitation, Alzheimer's disease, Parkinson's disease, Huntington's disease, vascular dementia, Lewy body disease, frontotemporal dementia, Tourette's syndrome, hyperprolactinemia, pituitary adenoma, prolactinoma, craniopharyngioma, Cushing's disease, diabetes insipidus, non-functioning tumours, obesity, posttraumatic stress disorder (PTSD), akathisia and associated movements, athetosis, ataxia, ballismus, hemiballismus, chorea, choreoathetosis, dyskinesia, tardive dyskinesia, neuroleptic-induced dyskinesia, myoclonus, mirror movement disorder, paroxysmal kinesigenic dyskinesia, restless legs syndrome, spasms, stereotypic movement disorder, sterotypy, Tic disorder, tremor, Wilson's disease, schizotypal personality disorder, delusional disorder, brief psychotic disorder, schizophreniform disorder, schizoaffective disorder, substance- or medication-induced psychotic disorder, delusions, hallucinations, disorganized thinking, grossly disorganized or abnormal motor behavior, catatonia, major depressive disorder, bipolar I disorder, bipolar II disorder, cyclothymic disorder, substance- or medication-induced bipolar and related disorders, bipolar and related disorders due to another medical condition, separation anxiety disorder, selective mutism, specific phobia, social anxiety disorder, panic disorder, agoraphobia, generalized anxiety disorder, substance- or medication-induced anxiety disorder, anxiety disorders due to another medical condition, delirium, major neurocognitive disorder, minor neurocognitive disorder, amnesia, dementia, developmental coordination disorder, stereotypic movement disorder, a post-stroke effect, dentatorubral-pallidoluysian atrophy, diminished emotional expression, avolition, alogia and asociality.

Compounds of the present invention may be used in the treatment of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, neurocognitive disorder, delirium, anhedonia, agitation, Alzheimer's disease, Parkinson's disease, Huntington's disease, vascular dementia, Lewy body disease, frontotemporal dementia, Tourette's syndrome, hyperprolactinemia, obesity, and posttraumatic stress disorder (PTSD). Compounds of the present invention may be used in the treatment of schizophrenia.

## DEFINITIONS

In this application, the following definitions apply, unless indicated otherwise.

The term "GPR52 modulator" as used herein refers to any compound which binds to and modulates the function of the GPR52 receptor. The term "modulator" should be interpreted to include modulation by modalities including, but not limited to, agonists, partial agonists and inverse agonists.

The term "treatment", in relation to the uses of any of the compounds described herein, including those of Formula (1), (1'), (1a), (1b), is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

Terms such as "alkyl", "hydrocarbon", "alkoxy", "halo", "aryl", "heteroaryl", "monocyclic", "polycyclic" and "cycloalkyl" are all used in their conventional sense (e.g. as defined in the IUPAC Gold Book), unless indicated otherwise. "optionally substituted" as applied to any group means that the said group may if desired be substituted with one or more substituents, which may be the same or different.

Examples of heteroatom replacements for carbon atoms include replacement of a carbon atom in a $-CH_2-CH_2-CH_2-$ chain with oxygen or sulfur to give an ether $-CH_2-O-CH_2-$ or thioether $-CH_2-S-CH_2-$, replacement of a carbon atom in a group $CH_2-C{\equiv}C-H$ with nitrogen to give a nitrile (cyano) group $CH_2-C{\equiv}N$, replacement of a carbon atom in a group $-CH_2-CH_2-CH_2-$ with C=O to give a ketone $-CH_2-C(O)-CH_2-$, replacement of a carbon atom in a group $-CH_2-CH=CH_2$ with C=O to give an aldehyde $-CH_2-C(O)H$, replacement of a carbon atom in a group $-CH_2-CH_2-CH_3$ with O to give an alcohol $-CH_2-CH_2-CH_2OH$, replacement of a carbon atom in a group $-CH_2-CH_2-CH_3$ with O to give an ether $-CH_2-O-CH_3$, replacement of a carbon atom in a group $-CH_2-CH_2-CH_3$ with S to give an thiol $-CH_2-CH_2-CH_2SH$, replacement of a carbon atom in a group $-CH_2-CH_2-CH_2-$ with S=O or $SO_2$ to give a sulfoxide $-CH_2-S(O)-CH_2-$ or sulfone

$-CH_2-S(O)_2-CH_2-$, replacement of a carbon atom in a $-CH_2-CH_2-CH_2-$ chain with C(O)NH to give an amide $-CH_2-CH_2-C(O)-NH-$, replacement of a carbon atom in a $-CH_2-CH_2-CH_2-$ chain with nitrogen to give an amine $-CH_2-NH-CH_2-$, and replacement of a carbon atom in a $-CH_2-CH_2-CH_2-$ chain with C(O)O to give an ester (or carboxylic acid) $-CH_2-CH_2-C(O)-O-$. In each such replacement, at least one carbon atom of the alkyl group must remain.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared by exchanging a counter-ion of a compound in the form of a salt with another counter-ion, for example using a suitable ion exchange resin.

Examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), $\alpha$-oxoglutaric, glycolic, hippuric, hydrobromic, hydrochloric, hydriodic, isethionic, lactic (e.g. (+)-L-lactic and ($\pm$)-DL-lactic), lactobionic, maleic, malic (e.g. (-)-L-malic), malonic, ($\pm$)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

**The solvates** can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may take the form, for example, of tablets, dragees, powders, elixirs, syrups, liquid preparations including suspensions, sprays, inhalants, tablets, lozenges, emulsions, solutions, cachets, granules, capsules and suppositories, as well as liquid preparations for injections, including liposome preparations.

The compounds of the invention may contain one or more isotopic substitutions, and a reference to a particular element includes within its scope all isotopes of the element. For example, a reference to hydrogen includes within its scope $^{1}H$, $^{2}H$ (D), and $^{3}H$ (T). Similarly, references to carbon and oxygen include within their scope respectively $^{12}C$, $^{13}C$ and $^{14}C$ and $^{16}O$ and $^{18}O$. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

Therapeutic dosages may be varied depending upon the requirements of the patient, the severity of the condition

being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

The magnitude of an effective dose of a compound will, of course, vary with the nature of the severity of the condition to be treated and with the particular compound and its route of administration. The selection of appropriate dosages is within the ability of one of ordinary skill in this art, without undue burden. In general, the daily dose range may be from about 10 μg to about 30 mg per kg body weight of a human and non-human animal, preferably from about 50 μg to about 30 mg per kg of body weight of a human and non-human animal, for example from about 50 μg to about 10 mg per kg of body weight of a human and non-human animal, for example from about 100 μg to about 30 mg per kg of body weight of a human and non-human animal, for example from about 100 μg to about 10 mg per kg of body weight of a human and non-human animal and most preferably from about 100 μg to about 1 mg per kg of body weight of a human and non-human animal.

## PHARMACEUTICAL FORMULATIONS

While it is possible for the active compound to be administered alone, it is preferable to present it as a pharmaceutical composition (e.g. formulation).

Accordingly, in one embodiment of the invention, there is provided a pharmaceutical composition comprising at least one compound of Formula (1) as defined above together with at least one pharmaceutically acceptable excipient.

The composition may be a tablet composition. The composition may be a capsule composition.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the Formula (1), (1'), (1a), (1b) can be formulated in accordance with known techniques, see for example, Remington's Pharmaceutical Sciences, Mack Publishing Company, Easton, PA, USA. The pharmaceutical compositions can be in any form suitable for oral, parenteral, topical, intranasal, intrabronchial, sublingual, ophthalmic, otic, rectal, intra-vaginal, or transdermal administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95%, preferably% (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically excipient or combination of excipients. The pharmaceutical compositions comprise from approximately 1% to approximately 95%, preferably from approximately 20% to approximately 90%, active ingredient. Pharmaceutical compositions according to

the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds of the Formula (1) (1'), (1a), (1b) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 milligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

## EXAMPLES

The invention will now be illustrated, but not limited, by reference to the following examples shown in **Table 1.** NMR and LCMS properties are set out in **Table 3.** Intermediates used are listed in **Table 2.**

**Table 1 - Examples**

| | | |
|---|---|---|
| 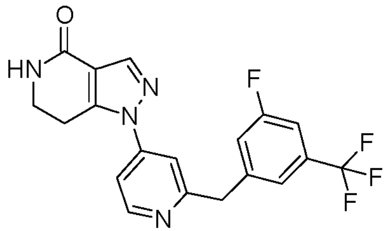 Example 1 | 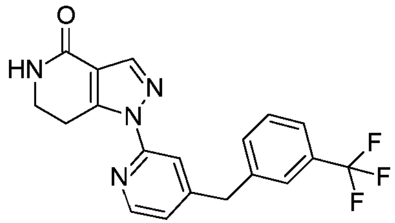 Example 2 | 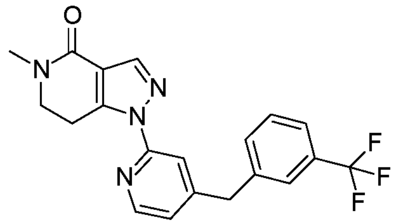 Example 3 |
| 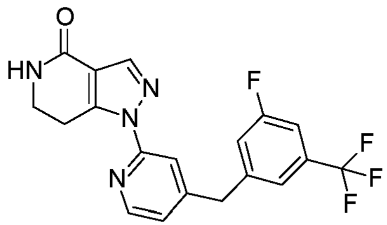 Example 4 | 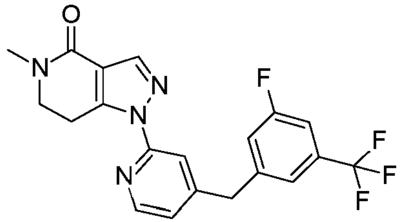 Example 5 | 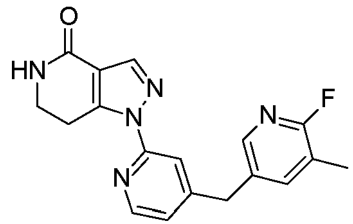 Example 6 |
| 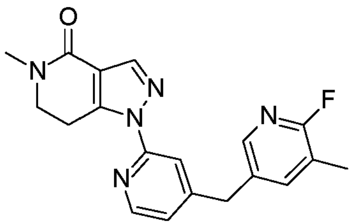 Example 7 | 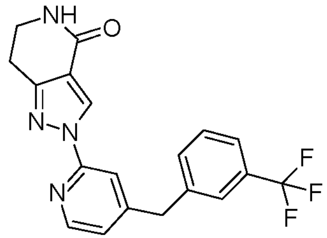 Example 8 | 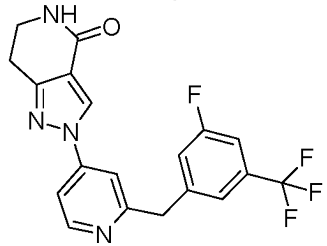 Example 9 |
| 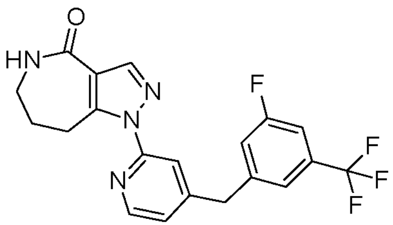 Example 10 | 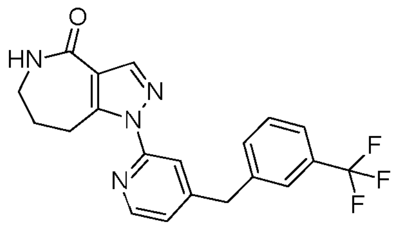 Example 11 | 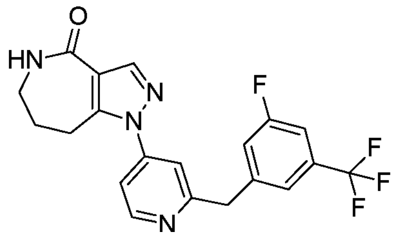 Example 12 |

(continued)

| | | |
|---|---|---|
| 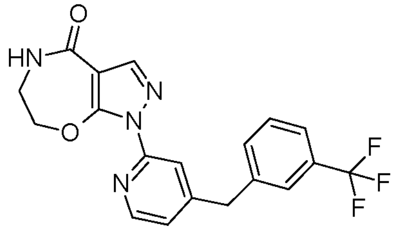 Example 13 | 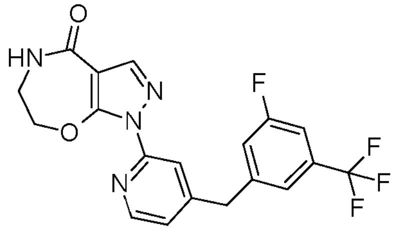 Example 14 | 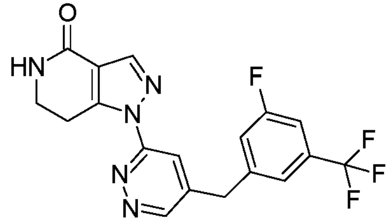 Example 15 |
| 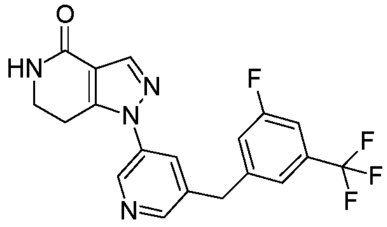 Example 16 | 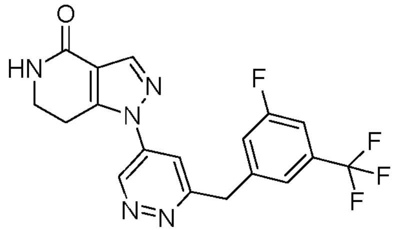 Example 17 | 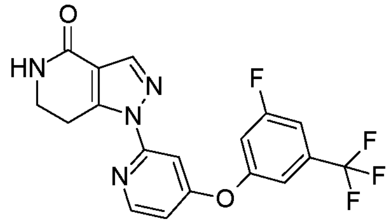 Example 18 |
| 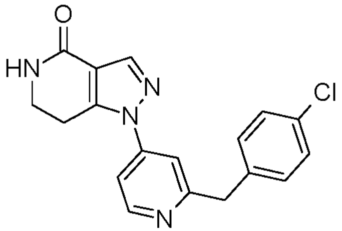 Example 19 | 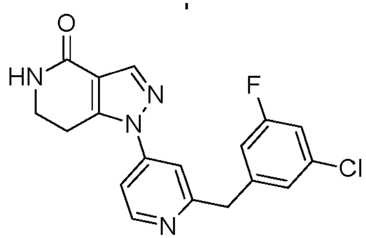 Example 20 | 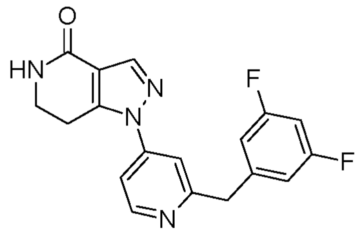 Example 21 |
| 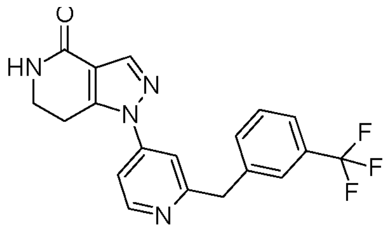 Example 22 | 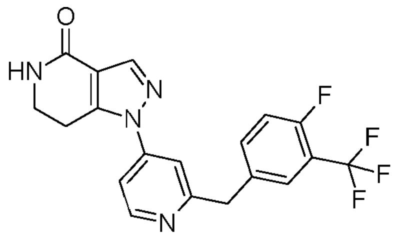 Example 23 | 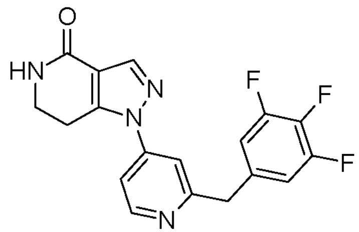 Example 24 |

(continued)

| | | |
|---|---|---|
| 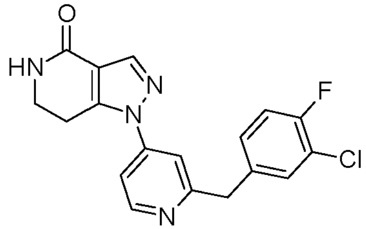 Example 25 | 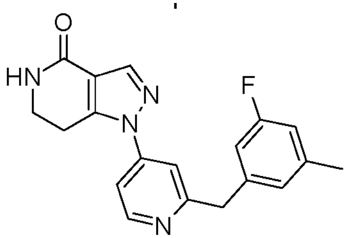 Example 26 | 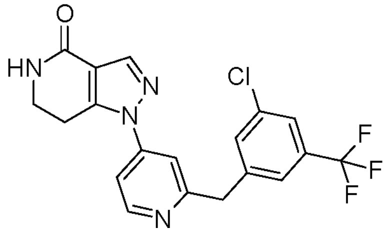 Example 27 |
| 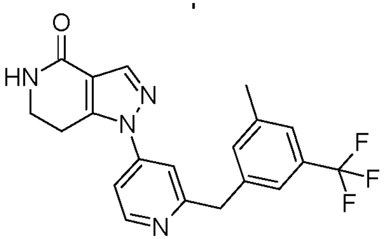 Example 28 | 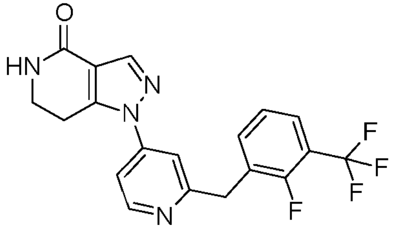 Example 29 | 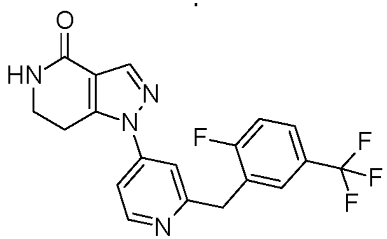 Example 30 |
| 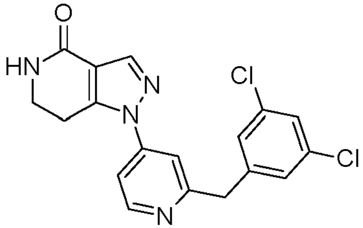 Example 31 | 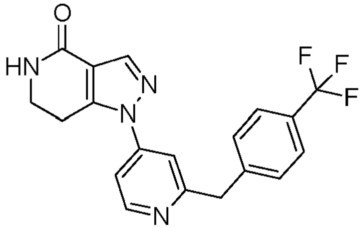 Example 32 | 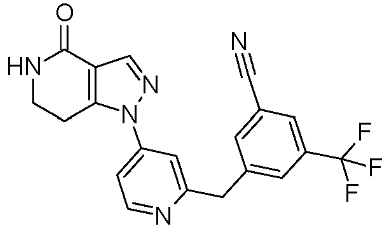 Example 33 |
| 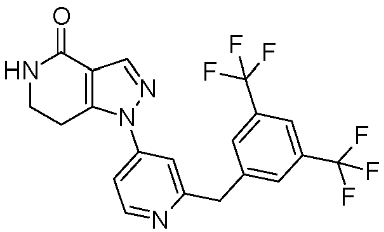 Example 34 | 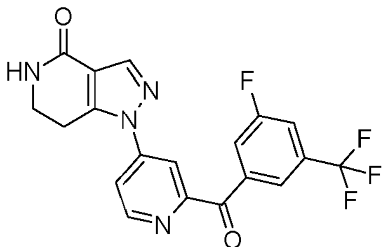 Example 35 | 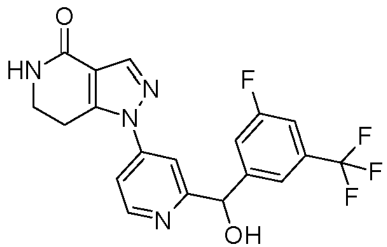 Example 36 |

(continued)

| | | |
|---|---|---|
| 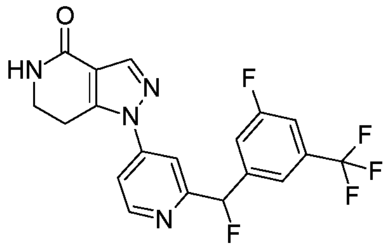 Example 37 | 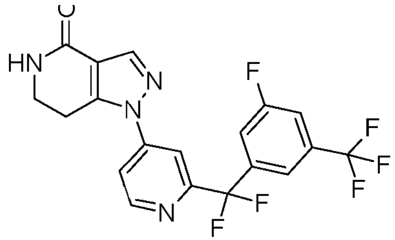 Example 38 | 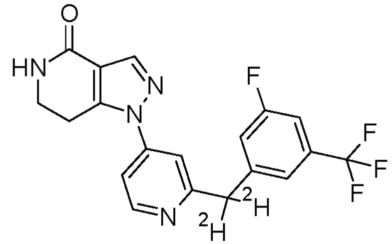 Example 39 |
| 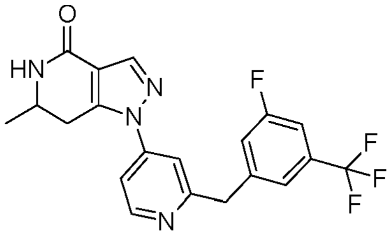 Example 40 | 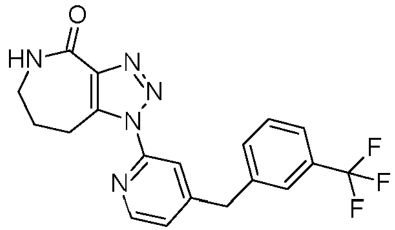 Example 41 | 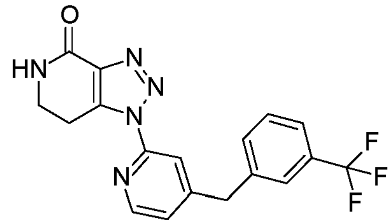 Example 42 |
| 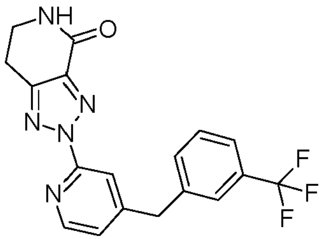 Example 43 | 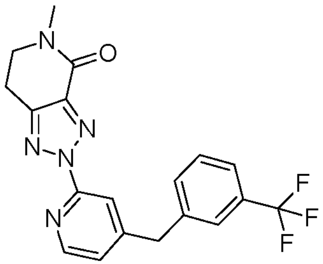 Example 44 | 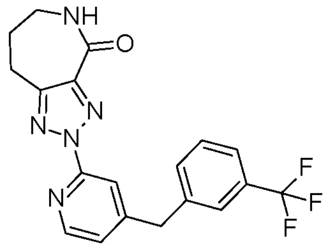 Example 45 |
| 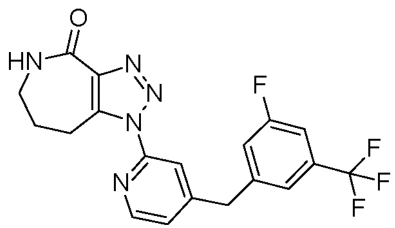 Example 46 | 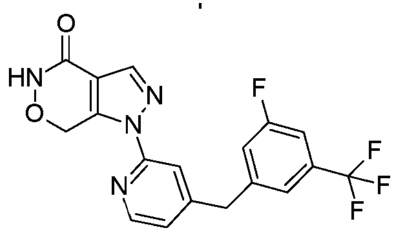 Example 47 | 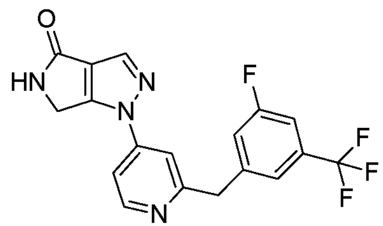 Example 48 |

(continued)

| | | |
|---|---|---|
| 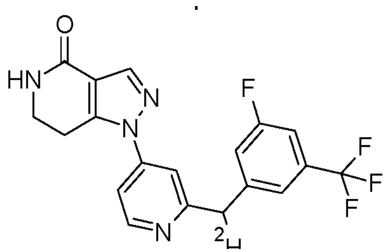 Example 49 | 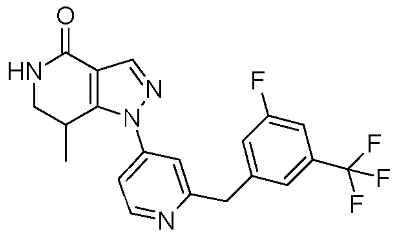 Example 50 | 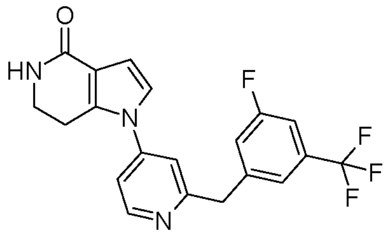 Example 51 |
| 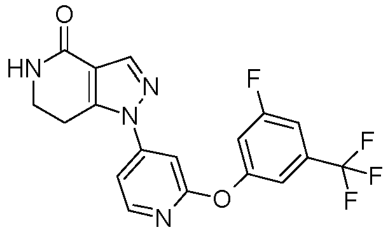 Example 52 | 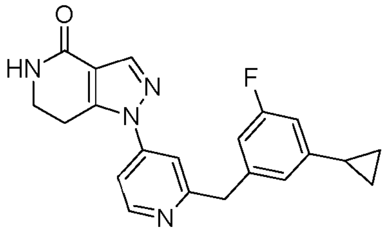 Example 53 | 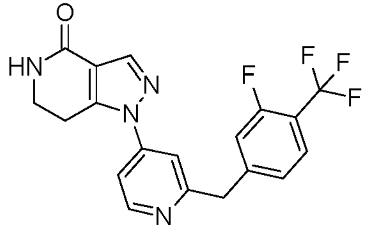 Example 54 |
| 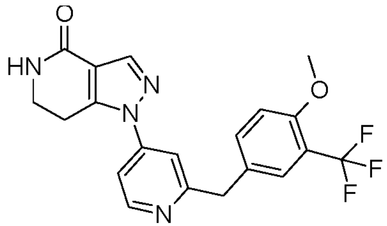 Example 55 | 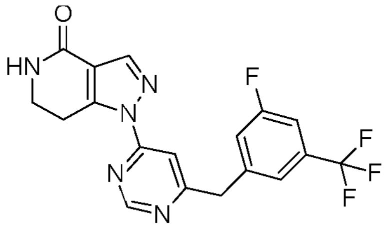 Example 56 | 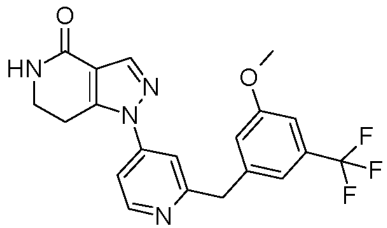 Example 57 |
| 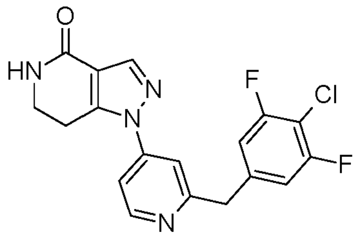 Example 58 | 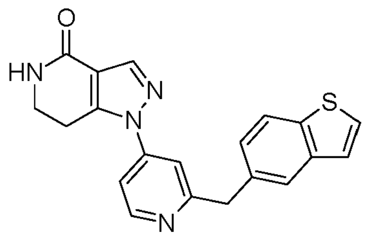 Example 59 | 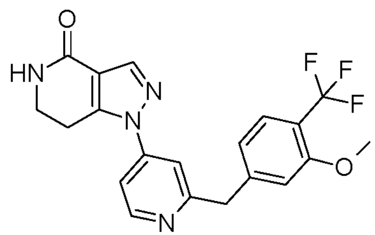 Example 60 |

(continued)

| | | |
|---|---|---|
| 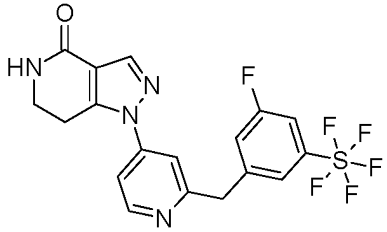 Example 61 | 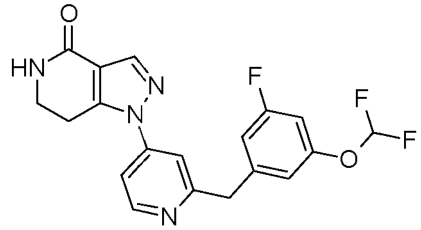 Example 62 | 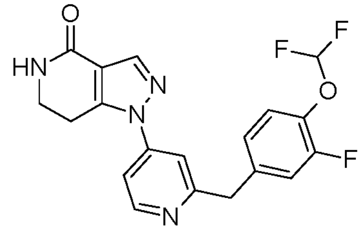 Example 63 |
| 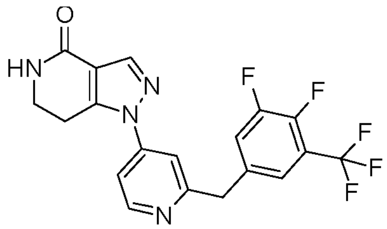 Example 64 | 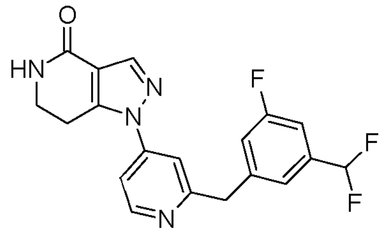 Example 65 | 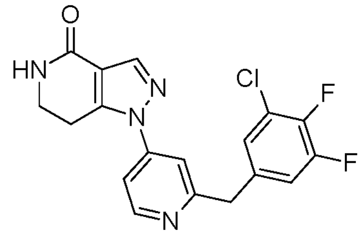 Example 66 |
| 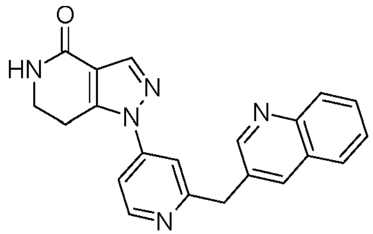 Example 67 | 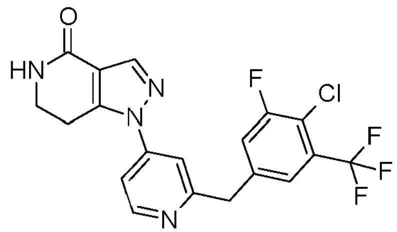 Example 68 | 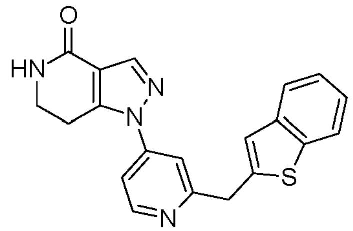 Example 69 |
| 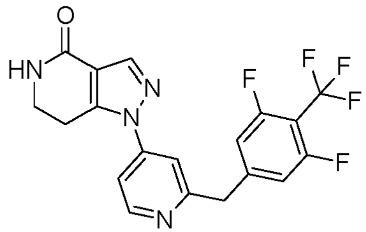 Example 70 | 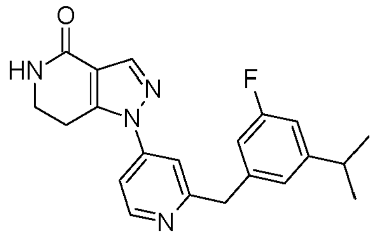 Example 71 | 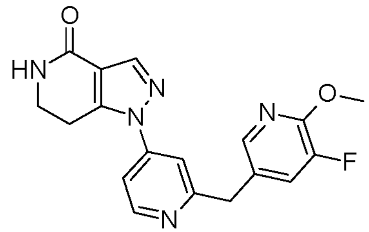 Example 72 |

(continued)

| | | |
|---|---|---|
| 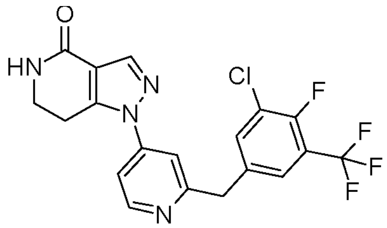 Example 73 | 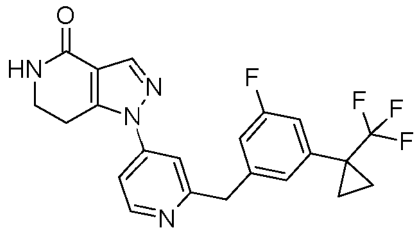 Example 74 | 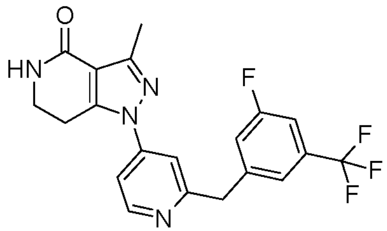 Example 75 |
| 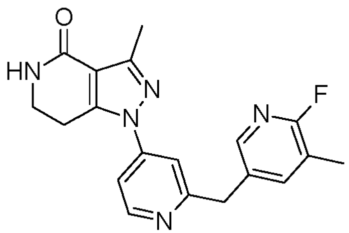 Example 76 | 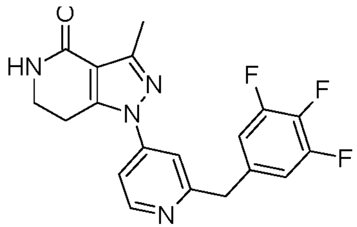 Example 77 | 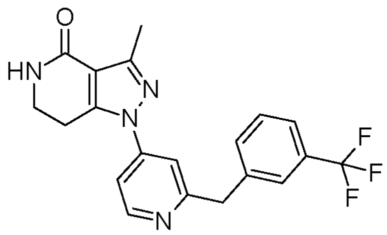 Example 78 |
| 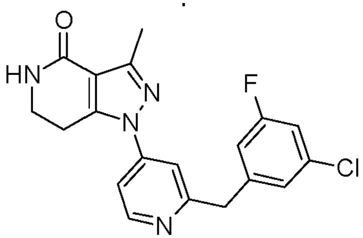 Example 79 | 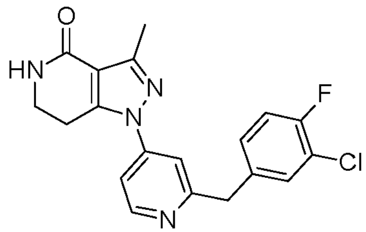 Example 80 | 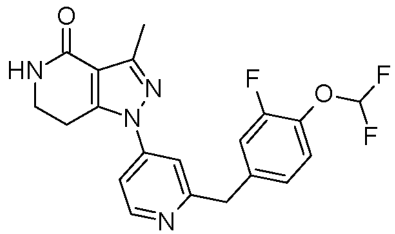 Example 81 |
| 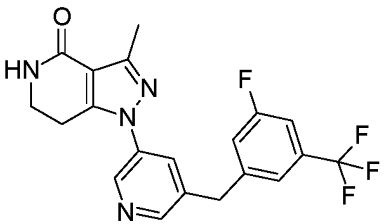 Example 82 | 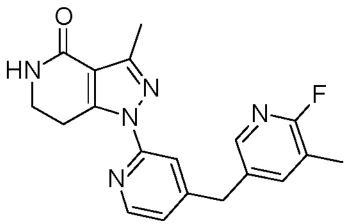 Example 83 | 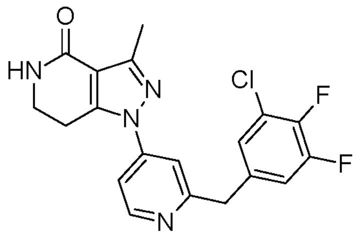 Example 84 |

(continued)

| | | |
|---|---|---|
| 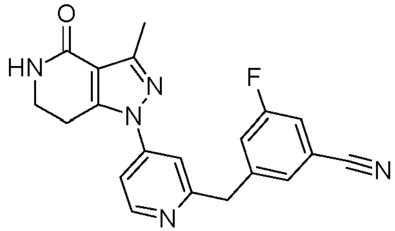 Example 85 | 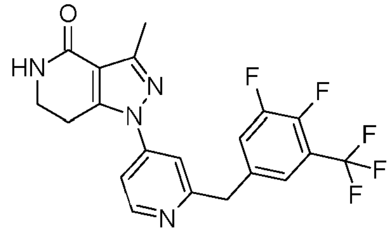 Example 86 | 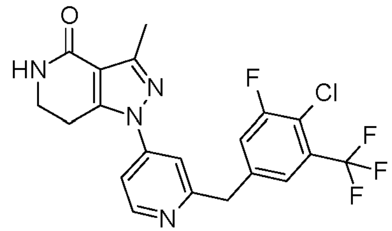 Example 87 |
| 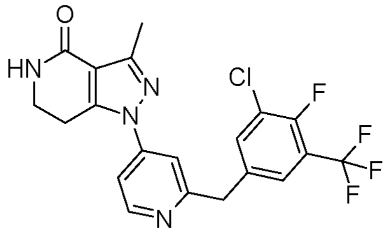 Example 88 | 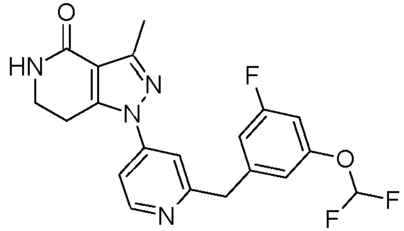 Example 89 | 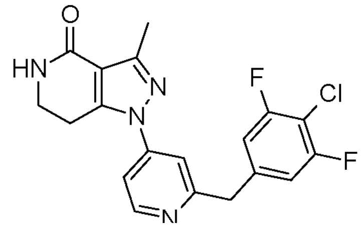 Example 90 |
| 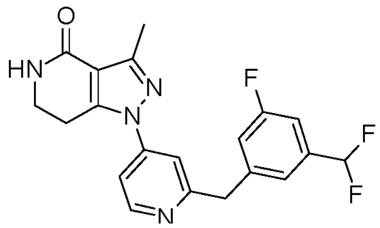 Example 91 | 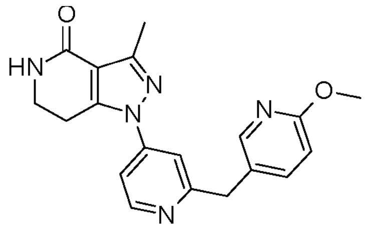 Example 92 | 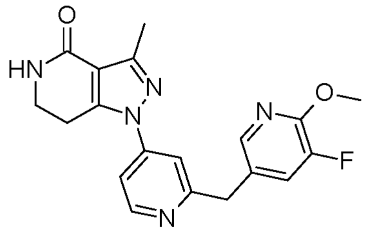 Example 93 |
| 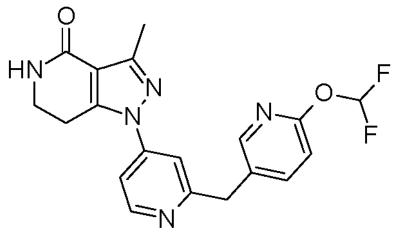 Example 94 | 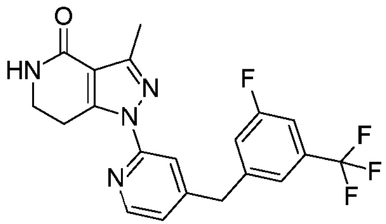 Example 95 | 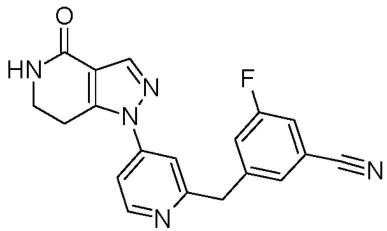 Example 96 |

(continued)

| | | |
|---|---|---|
| 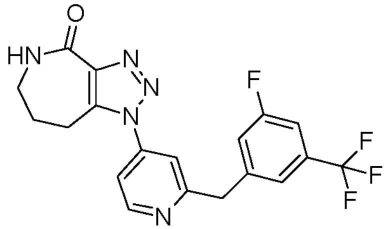 Example 97 | 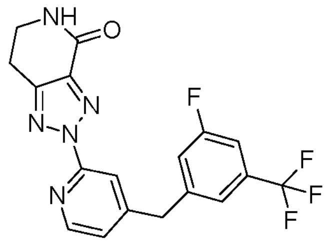 Example 98 | 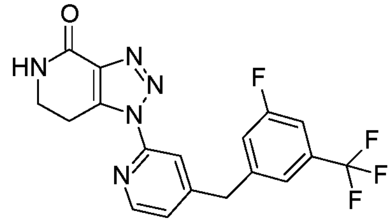 Example 99 |
| 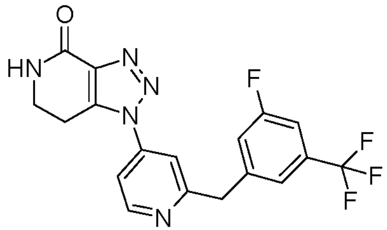 Example 100 | 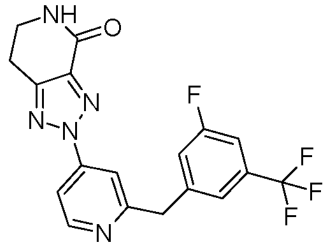 Example 101 | 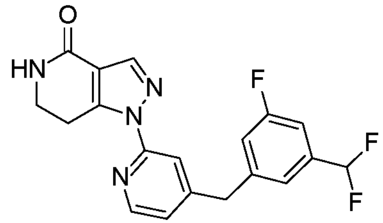 Example 102 |
| 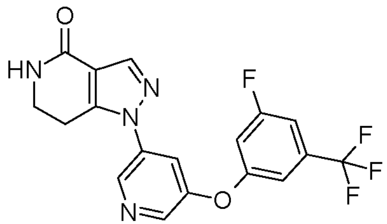 Example 103 | 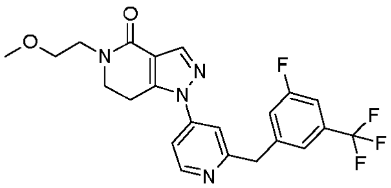 Example 104 | 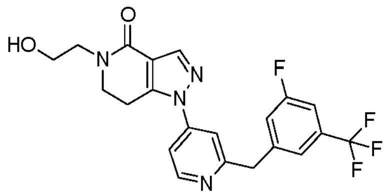 Example 105 |
| 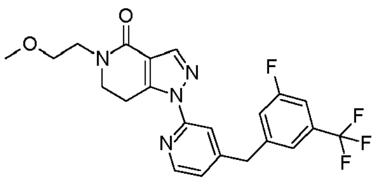 Example 106 | 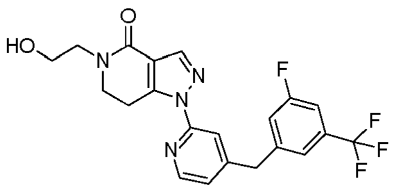 Example 107 | 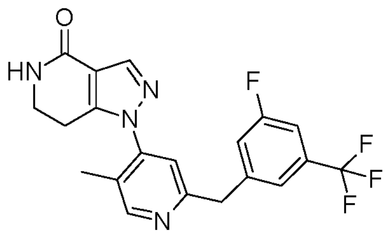 Example 108 |

(continued)

| | | |
|---|---|---|
| 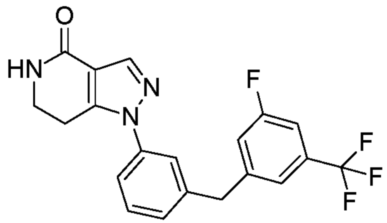 Example 109 | 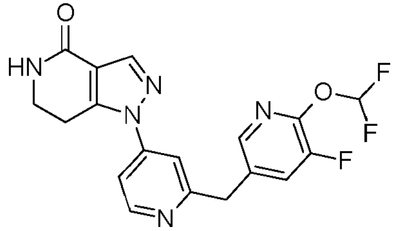 Example 110 | 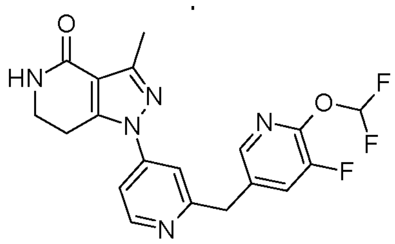 Example 111 |
| 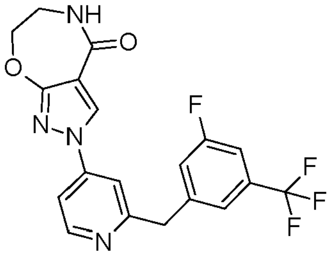 Example 112 | 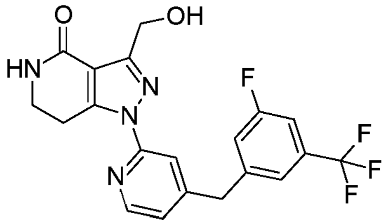 Example 113 | 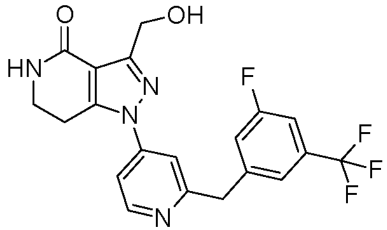 Example 114 |
| 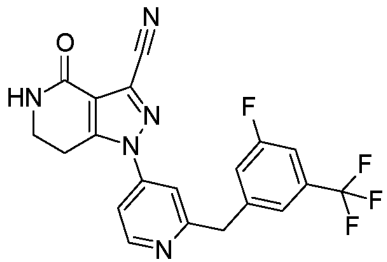 Example 115 | 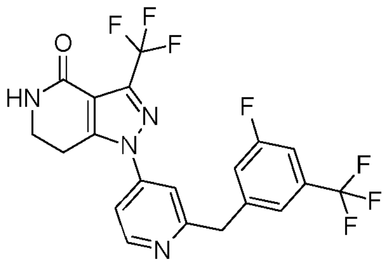 Example 116 | 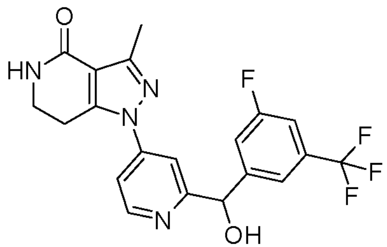 Example 117 |
| 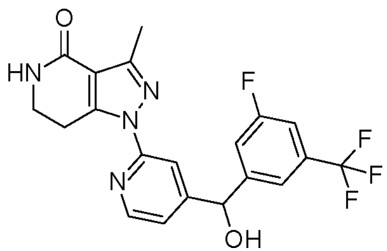 Example 118 | | |

**METHODS FOR THE PREPARATION OF COMPOUNDS OF THE FORMULA (1), (1'), (1") (1a), (1b)**

Compounds of Formula (1) (1'), (1a), (1b) can be prepared in accordance with synthetic methods well known to the skilled person. Provided is a process for the preparation of a compound as defined in Formula (1) above. Compounds of Formula (1) and associated intermediates can be prepared according to the procedures and schemes described herein. Disclosed intermediates may be applicable to the synthesis of one or more compounds of Formula (1). Procedures described herein may be applicable to the synthesis of one or more intermediates or compounds of Formula (1). Compounds of Formula (1) may be prepared by reaction of another compound of Formula (1). Intermediates may be used in subsequent synthetic steps without isolation or full characterisation per se. Certain compounds of the invention may be prepared according to the below general scheme:

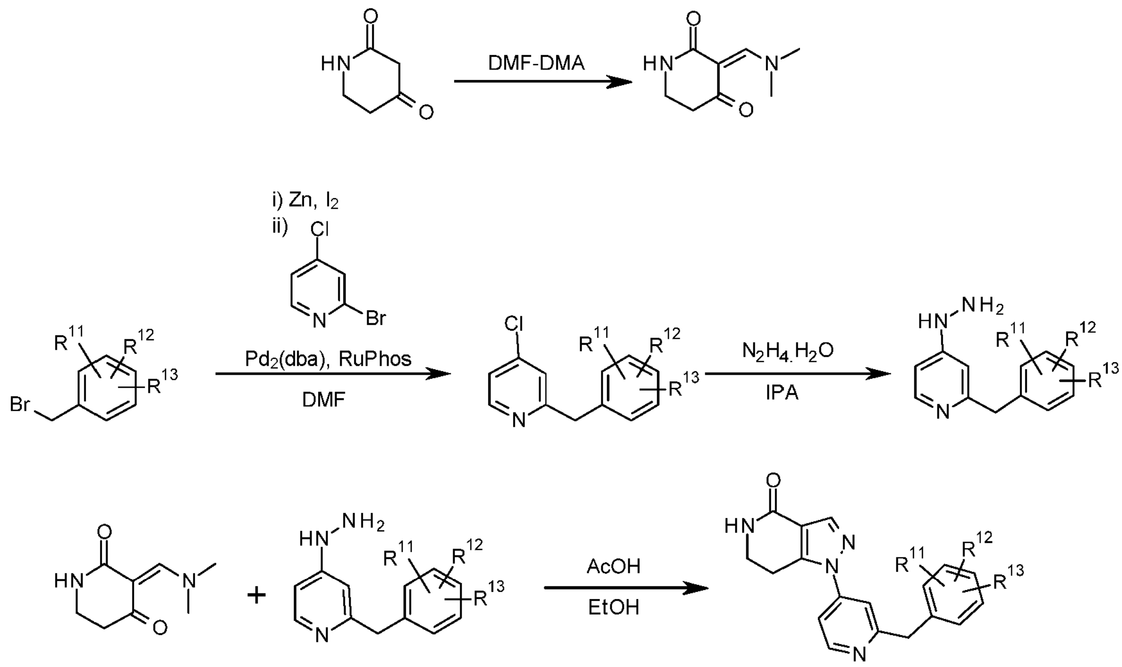


Certain compounds of the invention may be prepared according to the below general scheme:

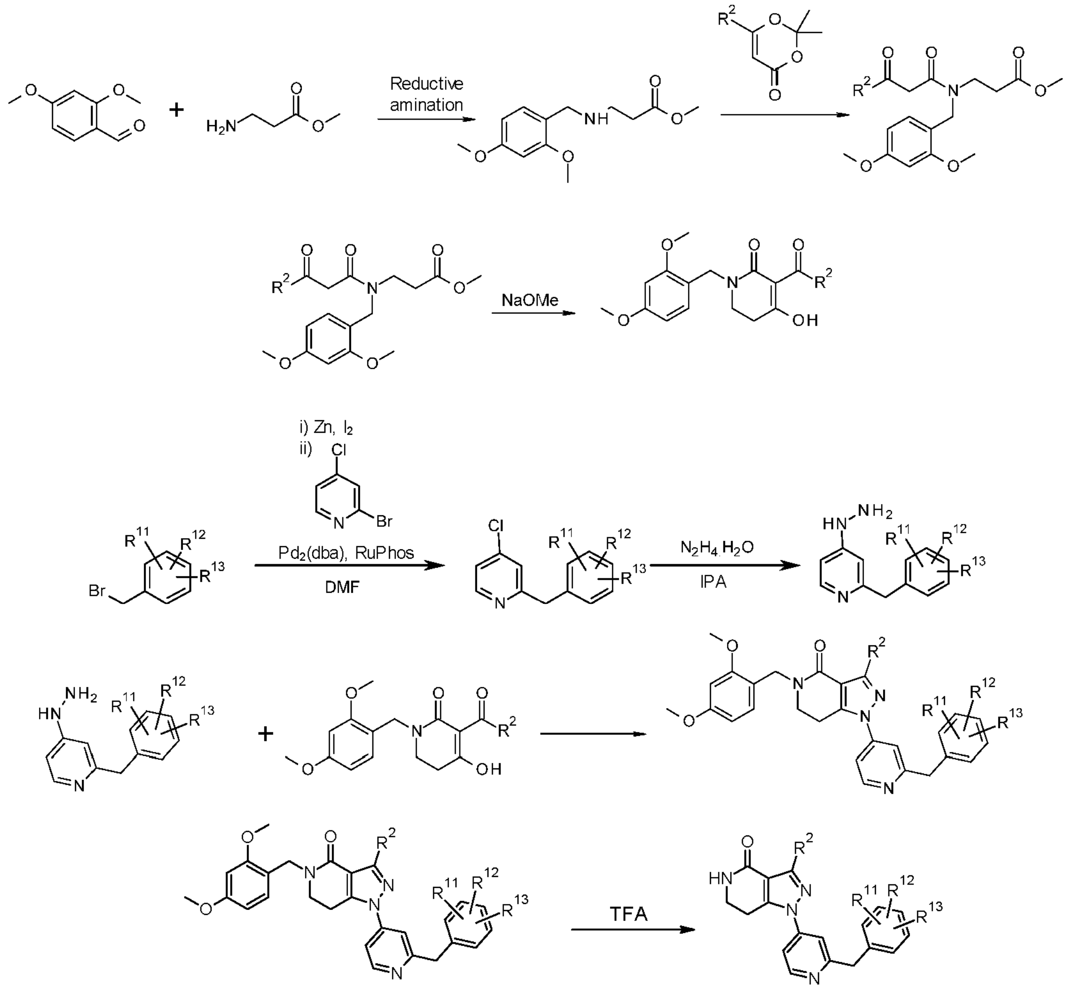


Where intermediates are commercially available they are identified by their chemical abstracts service (CAS) reference number in Table 3, where not commercially available the synthesis of the intermediates using standard transformations is detailed herein. Commercial reagents were utilized without further purification.

General procedures

Room temperature (RT) refers to approximately 20-27 °C. $^1$H NMR spectra were typically recorded at 400 MHz at ambient temperature unless otherwise specified. Chemical shift values are expressed in parts per million (ppm), i.e. ($\delta$)-values. Standard abbreviations, or their combinations, are used for the multiplicity of the NMR signals, for example: s=singlet, br=broad, d=doublet, t=triplet, q=quartet, quin=quintet or p=pentet, h=heptet, dd=doublet of doublets, dt=doublet of triplets, m=multiplet. Coupling constants are listed as J values, measured in Hz. NMR and mass spectroscopy results were corrected to account for background peaks. Chromatography refers to column chromatography performed using silica or C18 silica and executed under positive pressure (flash chromatography) conditions.

LCMS methods

LCMS experiments were carried out using electrospray conditions under the conditions below (Solvents: A1 = 0.1% TFA in $H_2O$:MeCN (95:5); A2 = 5 mM ammonium acetate in $H_2O$; A3 = 2.5 L $H_2O$ + 2.5 mL 28% ammonia in $H_2O$ solution; A4 = 0.1% $HCO_2H$ in $H_2O$:MeCN (95:5); A5 = 10 mM $NH_4HCO_3$ in $H_2O$; A6 = 0.2% of 28% ammonia solution in $H_2O$; A7 = 0.1% TFA in $H_2O$; A8 = 5 mM $NH_4HCO_3$ in $H_2O$; A9 = 10 mM ammonium acetate in $H_2O$; B1 = 0.1% TFA in MeCN; B2 = MeCN; B3 = 2.5 L MeCN + 135 mL $H_2O$ + 2.5 mL 28% ammonia in $H_2O$ solution.) LCMS data are given in the format: Mass ion, electrospray mode (positive or negative), retention time (experimental text and Table 2); Mass ion, electrospray mode (positive or negative), retention time, approximate purity (Table 3).

**Method 1.** Instruments: Hewlett Packard 1100 with G1315A DAD, Micromass ZQ; Column: Phenomenex Gemini-NX C18, 3 micron, 2.0 x 30 mm; Gradient [time (min)/solvent B3 in A3 (%)]: 0.00/2, 0.10/2, 8.40/95, 10.00/95;

Injection volume 1 μL; UV detection 230 to 400 nM; Column temperature 45 °C; Flow rate 1.5 mL/min.

**Method 2.** Instruments: Agilent Technologies 1290 Infinity II Series LC, 6125 Quadrupole MSD SL; Column: Waters XBridgeC8 3.5 micron, 4.6 x 50 mm; Gradient [time (min)/solvent B1 in A1 (%)]: 0.0/5, 2.5/95,4.0/95,4.5/5,6.0/5; Injection volume 1 μL; UV detection 210 to 400 nM; Column temperature 25 °C; 1.5 mL/min.

**Method 3.** Instruments: Agilent Technologies 1290 Infinity II Series LC, 6125 Quadrupole MSD SL; Column: Zorbax XDB C18, 5 micron; Gradient [time (min)/solvent B2 in A4 (%)]: 0.00/5, 2.50/95, 4.00/95, 4.50/5, 6.00/5; Injection volume 1 μL; UV detection 210-400 nm; Column temperature 25 °C; Flow rate 1.5 mL/min.

**Method 4.** Instruments: Agilent Technologies 1260 LC with Chemstation software, Diode Array Detector, Agilent 6120 Quadrupole MS with APCI and ES Source; Column: Phenomenex Gemini-NX C18, 3 micron, 2 x 30 mm; Gradient [time (min)/solvent B3 in A3 (%)]: 0.00/5, 2.00/95, 2.50/95, 2.60/5, 3.00/5; Injection volume 0.5 μL; UV detection 190-400 nm; column temperature 40 °C; Flow rate 1.5 mL/min.

**Method 5.** Instruments: Waters Acquity UPLC, Waters 3100 PDA Detector, SQD; Column: Acquity BEH C-18, 1.7 micron, 2.1 x 100 mm; Gradient [time (min)/solvent B2 in A2 (%)]: 0.00/2, 2.00/2, 7.00/50, 8.50/80, 9.50/2, 10.0/2; Injection volume 1μL; Detection wavelength 214 nm; Column temperature 30 °C; Flow rate 0.3 mL per min.

**Method 6.** Instruments: Agilent Technologies 1290 Infinity II Series LC, 6125 Quadrupole MSD SL; Column: Waters XBridgeC8 3.5 micron, 4.6 x 50 mm; Gradient [time (min)/solvent B2 in A5 (%)]: 0.0/10, 4.0/95, 5.0/95, 5.5/10, 7.0/10.; Injection volume 1 μL; UV detection 210 to 400 nM; Column temperature 25 °C; Flow rate 1.2 mL/min.

**Method 7.** Instruments: Agilent Technologies 1290 Infinity II Series LC, 6125 Quadrupole MSD SL; Column: Zorbax eclipse plus C18, 1.8 micron, 2.1 x 50mm; Gradient [time (min)/solvent B2 in A4 (%)]: 0.0/5, 0.25/5, 2.5/100, 3.0/100, 3.1/5, 4.0/5; Injection volume 1 μL; UV detection 210-400 nm; Column temperature 25 °C; Flow rate 0.8 mL/min.

**Method 8.** Instruments: Waters Acquity UPLC, Waters 3100 PDA Detector, SQD; Column: Acquity HSS-T3, 1.8 micron, 2.1 x 100 mm; Gradient [time (min)/solvent B2 in A7 (%)]: 0.0/10, 1.00/10, 2.00/15, 4.50/55, 6.00/90, 8.00/90, 9.00/10, 10.00/10; Injection volume 1μL; Detection wavelength 214 nm; Column temperature 30 °C; Flow rate 0.3 mL per min. **Method 9.** Instruments: Waters Acquity UPLC, Waters 3100 PDA Detector, SQD; Column: Acquity BEH C-18, 1.7 micron, 2.1 x 100 mm; Gradient [time (min)/solvent B2 in A2 (%)]: 0.00/5, 0.25/5, 1.50/35, 2.50/95, 3.20/95 3.60/5, 4.00/5; Injection volume 1 μL; Detection wavelength 214 nm; Column temperature 35 °C; Flow rate 0.6 mL per min to 3.20 min then 0.8 mL per min.

**Method 10.** Instruments: Agilent Technologies 1290 Infinity II Series LC, 6125 Quadrupole MSD SL; Column: Zorbax extend C18, 5 micron, 4.6 x 50 mm; Gradient [time (min)/solvent B2 in A9 (%)]: 0.0/10, 4.0/95, 5.0/95, 5.5/5, 6.0/5; Injection volume 1 μL; UV detection 210-400 nm; Column temperature 25 °C; Flow rate 1.2 mL/min.

**Method 11.** Instruments: Agilent Technologies 1290 Infinity II Series LC, 6125 Quadrupole MSD SL; Column: Acquity BEH C18, 1.7 micron, 2.1 x 50 mm; Gradient [time (min)/solvent B2 in A9 (%)]: 0.0/5, 0.25/5, 2.5/100, 3.0/100, 3.1/5, 4.0/5; Injection volume 1 μL; UV detection 210-400 nm; Column temperature 25 °C; Flow rate 0.8 mL/min.

**Method 12.** Instruments: Agilent Technologies 1290 Infinity II Series LC, 6125 Quadrupole MSD SL; Column: Atlantis dC18, 5 micron, 4.6 x 50 mm; Gradient [time (min)/solvent B2 in A4 (%)]: 0.0/5, 2.5/95, 4.0/95, 4.5/5, 6.0/5; Injection volume 1 μL; UV detection 210-400 nm; Column temperature 25 °C; Flow rate 1.5 mL/min.

GCMS methods

GCMS data are given in the format: Mass ion, electrospray mode (positive or negative), retention time.

**Method 1.** Instrument: Agilent GCMS 7890B; Column: HP-5ms UI (30m x 250μm x 0.25μm); Inlet temp: 250 °C; Split ratio: 75:1; Oven temp: 50 °C, hold time 3 min; Ramp 1: 40 °C/min to 300 °C, hold time 2 min; Detector temperature: 310 °C; Column flow: 2 mL/min; Air flow: 300 mL/min; $H_2$ flow: 40 mL/min; Make up flow (He): 25 mL/min; Source temp: 230 °C.

**Method 2.** Instrument: Agilent GCMS 7890B; Column: HP-5ms UI (30m x 250μm x 0.25μm); Inlet temp: 250 °C; Split ratio: 75:1; Oven temp: 120 °C, hold time 1 min; Ramp 1: 40 °C/min to 300 °C, hold time 4 min; Detector temperature: 310 °C; Column flow: 2 mL/min; Air flow: 300 mL/min; $H_2$ flow: 40 mL/min; Make up flow (He): 25 mL/min; Source temp: 230 °C.

MS methods

**Method 1.** Data acquired on either a Waters QDA or Waters SQD instrument after a 4 - 6 minute run through a UPLC column using buffer.

Prep HPLC methods

See LCMS methods section for solvent conditions.

**Method 1.** Instruments: Agilent Technologies 1260 Infinity II Series LC / 6125 Quadrupole MSD; Column: Waters

XBridge C8 5 micron 19 x 150 mm; Gradient [time (min)/solvent B2 in A5 (%)]: 0.0/10, 15/95, 18/95, 19/10, 21/10.

**Method 2.** Instruments: Gilson Semi Preparative HPLC System - 321 Pump/171 Diode Array Detector/GX-271 Liquid Handler; Column: Phenomenex Gemini-NX C18 5 micron 30 x 100 mm; Gradient 12.5 min, solvent B2 in A6 (%) varies on individual run basis (see exemplified procedures for details).

**Method 3.** Instruments: Waters 2767 Auto purification; Column: Reprosil Gold C18 5 micron 19 x 250 mm; Gradient 15 min, solvent B2 in A2 (%) varies on individual run basis (see exemplified procedures for details).

**Method 4.** Instruments: Agilent Technologies 1260 Infinity II Series LC / 6125 Quadrupole MSD; Column: Waters XBridge C8 5 micron 19 x 150 mm; Gradient [time (min)/solvent B2 in A7 (%)]: 0.0/10, 15/95, 18/95, 19/10, 21/10.

**Method 5.** Instruments: Waters 2767 Auto purification; Column: Xtimate hexyl phenyl 10 micron 19 x 250 mm; Gradient 18 min, solvent B2 in A8 (%) varies on individual run basis (see exemplified procedures for details).

**Method 6.** Instruments: Waters 2767 Auto purification; Column: X Select C18 10 micron 19 x 250 mm; Gradient 20 min, solvent B2 in A8 (%) varies on individual run basis (see exemplified procedures for details).

Chiral SFC methods

**Method 1.** Instruments: PIC Solution PIC-100, PIC-150, PIC-175 and PIC-400; Column: Lux A1 5 micron, 21.2 x 250 mm; Co-solvent 0.5% iso-propyl amine in MeOH; Column temperature 35 °C; 20 mL/min.

**Method 2.** Instruments: Sepiatec Prep SFC 100 with Prep SFC 100 control software and UV/Vis detector; Column: Lux C1 5 micron, 21.2 x 250 mm; Co-solvent 0.2% $NH_3$ in IPA; Column temperature 40 °C; 50 mL/min.

**Method 3.** Instruments: PIC Solution PIC 10-20 and PIC-10; Column: Lux A1 3 micron, 2 x 50 mm; Co-solvent 0.5% iso-propyl amine in MeOH; Column temperature 35 °C; 3 mL/min.

**Method 4.** Instruments: Waters Acquity UPC2 with Masslynx software, PDA detector and a QDa mass detector; Column: Lux C1 3 micron, 2 x 50 mm; Co-solvent IPA; Column temperature 45 °C; 1.5 mL/min.

**Method 5.** Instruments: PIC Solution PIC-100, PIC-150, PIC-175 and PIC-400; Column: Lux A1 5 micron, 21.2 x 250 mm; Co-solvent MeOH/MeCN (1:1); Column temperature 35 °C; 30 mL/min.

**Method 6.** Instruments: PIC Solution PIC 10-20 and PIC-10; Column: Lux A1 3 micron, 2 x 50 mm; Co-solvent MeOH/MeCN (1:1); Column temperature 35 °C; 3 mL/min.

**Method 7.** Instruments: PIC Solution PIC-100, PIC-150, PIC-175 and PIC-400; Column: Chiralpak AS-H, 30 x 250 mm; Co-solvent 0.5% iso-propyl amine in MeOH; Column temperature 35 °C; 30 mL/min.

**Abbreviations**

| | |
|---|---|
| aq = | aqueous |
| Boc = | tert-butoxycarbonyl |
| DAST = | (diethylamino)sulfur trifluoride |
| Davis reagent = | 2-(phenylsulfonyl)-3-phenyl-oxaziridine |
| dba = | dibenzylideneacetone |
| DCM = | dichloromethane |
| Dess-Martin = | 1,1,1-tris(acetyloxy)-1,1-dihydro-1,2-benziodoxol-3-(1*H*)-one |
| DIPEA = | *N*,*N*-diisopropylethylamine |
| DMA = | *N*,*N*-Dimethylacetamide |
| DMAP = | 4-(dimethylamino)pyridine |
| DME = | 1,2-dimethoxyethane |
| DMF = | *N*,*N*-dimethylformamide |
| DMF-DMA = | *N*,*N*-dimethylformamide dimethyl acetal |
| DMS = | dimethyl sulfide |
| DMSO = | dimethylsulfoxide |
| dppf = | 1,1'-ferrocenediyl-bis(diphenylphosphine) |
| EDCI = | *N*-(3-dimethylaminopropyl)-*N'*-ethylcarbodiimide hydrochloride |
| ES = | electrospray |
| EtOAc = | ethyl acetate |
| EtOH = | ethanol |
| h = | hour(s) |
| HATU = | *N*-[(Dimethylamino)-1H-1,2,3-triazolo-[4,5-b]pyridin-1-ylmethylene]-N-methylmethanaminium hexafluorophosphate *N*-oxide |
| HMDS = | hexamethyldisilazane |

| | |
|---|---|
| IPA = | *i*-propyl alcohol |
| L = | litre |
| LC = | liquid chromatography |
| LCMS = | liquid chromatography mass spectrometry |
| $LiAlH_4$ = | lithium aluminum hydride |
| MeCN = | acetonitrile |
| MeOH = | methanol |
| min = | minute(s) |
| MS = | mass spectrometry |
| NMP = | 1-methyl-2-pyrrolidinone |
| NMR = | nuclear magnetic resonance |
| MTBE = | methyl *tert*-butyl ether |
| NIS = | *N*-iodosuccinimide |
| Pet-ether = | petroleum ether |
| pin = | pinacol |
| RT = | room temperature |
| RuPhos = | 2-dicyclohexylphosphino-2',6'-di*i*sopropoxybiphenyl |
| SFC = | supercritical fluid chromatography |
| SPhos = | 2-dicyclohexylphosphino-2',6'-dimethoxybiphenyl |
| TFA = | trifluoroacetic acid |
| THF = | tetrahydrofuran |
| TMSI = | iodotrimethylsilane |
| TrixiePhos = | *rac*-2-(di-*tert*-butylphosphino)-1,1'-binaphthyl |
| Ts = | *para*-toluenesulfonyl |
| XPhos = | 2-dicyclohexylphosphino-2',4',6'-tri*iso*propylbiphenyl |

Prefixes *n-, s-, i-, t-* and *tert-* have their usual meanings: normal, secondary, iso, and tertiary.

## SYNTHESIS OF INTERMEDIATES

### Preparation of substituted (dimethylamino)methylene intermediates

**Typical procedure 1, exemplified by the preparation of Intermediate 1, (*E*)-3-((dimethylamino)methylene)piperidine-2,4-dione**

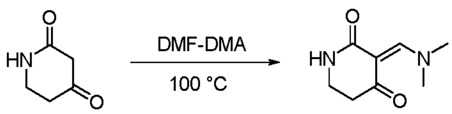


A solution of piperidine-2,4-dione (10 g, 88.5 mmol) in DMF-DMA (100 mL) was stirred at 100 °C for 3 h and then at RT for 15 h. Precipitated solid was filtered, rinsed with $Et_2O$ (2x30 mL) and dried *in vacuo* to afford (*E*)-3-((dimethylamino)methylene)piperidine-2,4-dione as a brown solid (5 g, 33%). **Data in table 2.**

**Intermediate 2, 2-((dimethylamino)methylene)cyclohexane-1,3-dione**

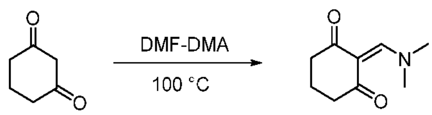


The title compound (1.3 g, 88%) was prepared from cyclohexane-1,3-dione (1 g, 8.8 mmol) in DMF-DMA (10 mL) heated at 100 °C for 3 h using the method of Intermediate 1. **Data in table 2.**

**Intermediate 17, (*E*)-3-((dimethylamino)methylene)-6-methylpiperidine-2,4-dione**

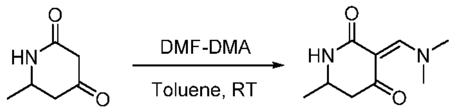


The title compound (390 mg, 91%) was prepared from 6-methylpiperidine-2,4-dione (300 mg, 2.36 mmol) in DMF-DMA (0.5 mL) and toluene (5 mL) stirred at RT for 2 h using the method of Intermediate 1. **Data in table 2.**

**Intermediate 20, (*E*)-3-((dimethylamino)methylene)-5-methylpiperidine-2,4-dione**

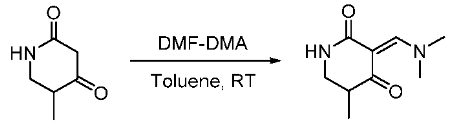


The title compound (257 mg, 90%) was prepared from 5-methylpiperidine-2,4-dione (200 mg, 1.56 mmol) in DMF-DMA (0.3 mL) and toluene (2 mL) stirred at RT for 2 h using the method of Intermediate 1. **Data in table 2.**

**Typical procedure 2, exemplified by the preparation of Intermediate 19, ethyl (*Z*)-4-(*tert*-butoxy)-2-((dimethylamino)methylene)-3-oxobutanoate**

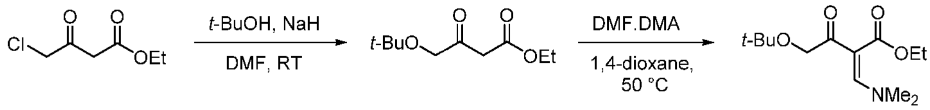


Step 1. Ethyl 4-chloro-3-oxobutanoate (2.00 g, 12.2 mmol) and *t*-BuOH (1.80 g, 24.3 mmol) were added to a suspension of sodium hydride (60% in mineral oil, 1.46 g, 36.5 mmol) in DMF (5 mL) and the reaction mixture was stirred at RT for 16 h. The reaction mixture was quenched with 1N HCl (20 mL) and the aqueous layer was extracted with EtOAc (3x50 mL). The organic layers were combined, dried ($Na_2SO_4$) and the solvent removed *in vacuo* to afford ethyl 4-(*tert*-butoxy)-3-oxobutanoate as a yellow liquid (1.60 g, 65%).

**$^1$H NMR:** (400 MHz, $CDCl_3$) δ: 4.14 (q, *J* = 6.8 Hz, 2H), 3.96 (s, 2H), 3.49 (s, 2H), 1.26-1.20 (m, 3H), 1.14 (s, 9H).

Step 2. DMF-DMA (1.27 mL) was added dropwise to a solution of ethyl 4-(*tert*-butoxy)-3-oxobutanoate (1.60 g, 7.91 mmol) in 1,4-dioxane (10 mL). The reaction mixture was heated at 50 °C for 2 h. The reaction mixture was concentrated *in vacuo* to afford ethyl (*Z*)-4-(*tert*-butoxy)-2-((dimethylamino)methylene)-3-oxobutanoate as a brown liquid (1.60 g, 79%). **Data in table 2.**

**<u>Preparation of substituted dihydropyridinone and equivalent intermediates</u>**

**Typical procedure 3, exemplified by the preparation of Intermediate 35, 3-acetyl-1-(2,4-dimethoxybenzyl)-4-hydroxy-5,6-dihydropyridin-2(1*H*)-one**

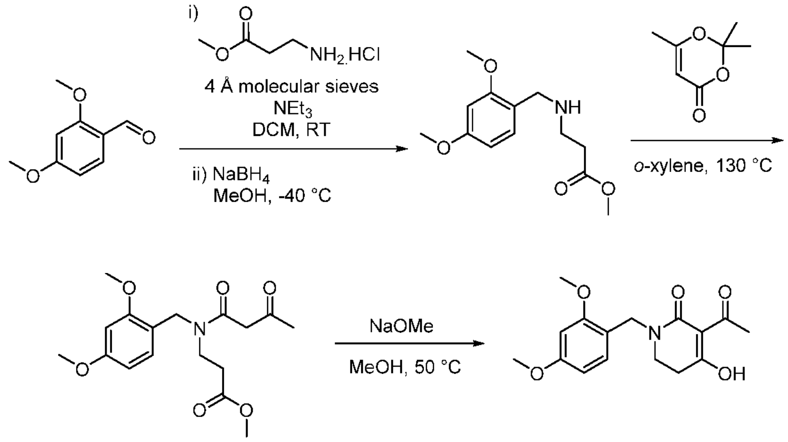


Step 1. 4 Å Molecular sieves (10 g) were added to a suspension of 2,4-dimethoxybenzaldehyde (5 g, 30.1 mmol) and methyl 3-aminopropanoate hydrochloride (5.38 g, 38.7 mmol) in DCM (100 mL) at RT followed by the addition of triethyl amine (15 mL, 107 mmol) and the reaction mixture was stirred at RT for 15 h. The reaction mixture was filtered through celite and washed with DCM (100 mL). The filtrate was washed with 10% $NaHCO_3$ solution (100 mL) and brine (100 mL). The organic layer was separated, dried ($Na_2SO_4$) and the solvent removed *in vacuo.* The residue was dissolved MeOH (50 mL), cooled to -40 °C and $NaBH_4$ (1.71 g, 45.2 mmol) was added portion-wise and the reaction mixture was stirred at -40 °C for 1 h. The solvent was removed *in vacuo* and the residue obtained was dissolved in EtOAc (200 mL) and washed with $H_2O$ (200 mL). The organic layer was separated, dried ($Na_2SO_4$) and the solvent removed *in vacuo* to afford methyl 3-((2,4-dimethoxybenzyl)amino)propanoate as a pale yellow gum (6.8 g, 89%).

**LCMS (Method 3):** m/z 254.1 (ES+), at 1.02 min.

**$^1$H NMR:** (400 MHz, $CDCl_3$) δ: 7.15 (d, *J* = 8.0 Hz, 1H), 6.48-6.44 (m, 2H), 3.84 (s, 3H), 3.82 (s, 3H), 3.75 (s, 2H), 3.70 (s, 3H), 2.87 (t, *J* = 6.4 Hz, 2H), 2.55 (t, *J* = 6.8 Hz, 2H). 1 exchangeable proton not observed.

Step 2. A solution of methyl 3-((2,4-dimethoxybenzyl)amino)propanoate (6.8 g, 26.8 mmol) and 2,2,6-trimethyl-4*H*-1,3-dioxin-4-one (7.6 g, 53.7 mmol) in o-xylene (100 mL) was stirred at 130 °C for 4 h. The solvent was removed *in vacuo* and the residue was purified by gradient flash column chromatography eluting with 0-50% EtOAc in pet-ether to afford methyl 3-(*N*-(2,4-dimethoxybenzyl)-3-oxobutanamido)propanoate as a yellow gum (6.0 g, 66%).

**LCMS (Method 7):** m/z 338.2 (ES+), at 1.82 min.

**$^1$H NMR:** (400 MHz, DMSO-$d_6$) δ: 7.06 (d, *J* = 11.2 Hz, 1H), 6.59-6.47 (m, 2H), 4.42-4.34 (m, 2H), 3.79-3.75 (m, 9H), 3.64 (s, 2H), 3.58-3.56 (m, 4H), 2.12 (s, 3H).

Step 3. Sodium methoxide (0.6 g, 11.3 mmol) was added to a stirred solution of methyl 3-(*N*-(2,4-dimethoxybenzyl)-3-oxobutanamido)propanoate (2.5 g, 7.42 mmol) in MeOH (50 mL) and the reaction mixture was heated at 50 °C for 4 h. The solvent was removed *in vacuo* and the residue was dissolved in EtOAc (50 mL) and washed with water (50 mL). The organic layer was separated, washed with brine solution (50 mL), dried ($Na_2SO_4$) and the solvent removed *in vacuo.* The residue was purified by gradient flash column chromatography eluting with 0-40 % EtOAc in pet-ether to afford 3-acetyl-1-(2,4-dimethoxybenzyl)-4-hydroxy-5,6-dihydropyridin-2(1*H*)-one as an off-white solid (450 mg, 20%). **Data in table 2.**

**Typical procedure 4, exemplified by the preparation of Intermediate 44, *tert*-butyl 3-(2-methoxyacetyl)-2,4-dioxopiperidine-1-carboxylate**

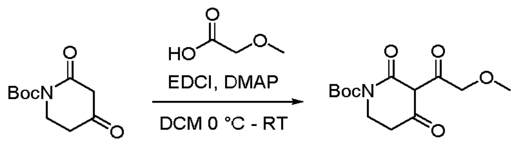


*N*-(3-Dimethylaminopropyl)-*N'*-ethylcarbodiimide hydrochloride (0.180 g, 0.94 mmol) and DMAP (0.126 g, 1.03 mmol) were added to a stirred solution of *tert*-butyl 2,4-dioxopiperidine-1-carboxylate (0.2 g, 0.94 mmol) and 2-methoxyacetic acid (0.084 g, 0.94 mmol) in DCM (10 mL) at 0 °C and the reaction mixture was stirred at RT for 2 h. The reaction mixture was partitioned between water (30 mL) and DCM (2x30 mL). The combined organic layers were washed with brine solution (30 mL), dried ($Na_2SO_4$) and the solvent removed *in vacuo.* The residue was purified by

gradient flash column chromatography eluting with 0-20% EtOAc in pet-ether to afford *tert*-butyl 3-(2-methoxyacetyl)-2,4-dioxopiperidine-1-carboxylate as a yellow liquid (0.2 g, 75%). **Data in table 2.**

**Preparation of substituted fluoropyridine intermediates**

**Typical procedure 5, exemplified by the preparation of Intermediate 3, 4-fluoro-2-(3-fluoro-5-(trifluoromethyl)benzyl)pyridine**

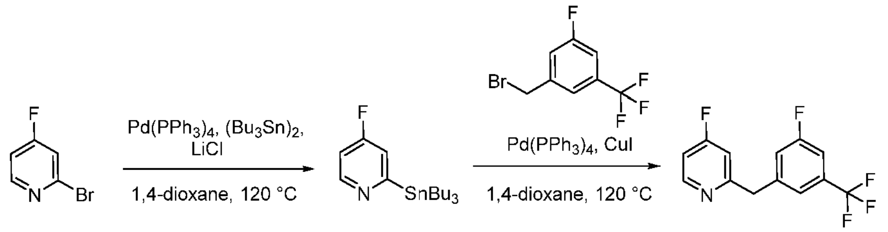


Step 1. A solution of 2-bromo-4-fluoropyridine (4.0 g, 22.7 mmol) in 1,4-dioxane (60 mL) was degassed with argon for 10 min and *bis*(tributyltin) (17.3 mL, 34.0 mmol), LiCl (2.88 g, 68.1 mmol) and $Pd(PPh_3)_4$ (1.31 g, 1.13 mmol) were added. The reaction mixture was heated at 120 °C for 16 h. The reaction was quenched with water (100 mL) and the aqueous layer was extracted with EtOAc (2x100 mL). The organic layers were combined, dried ($Na_2SO_4$) and the solvent removed *in vacuo* to afford 4-fluoro-2-(tributylstannyl)pyridine as a yellow liquid (14.3 g crude). The crude product was used in the next step without further purification. **MS (Method 1):** m/z 388 (ES+)

Step 2. 1-(Bromomethyl)-3-fluoro-5-(trifluoromethyl)benzene (1.33 g, 5.18 mmol) was added to a solution of 4-fluoro-2-(tributylstannyl)pyridine (14.3 g, 5.18 mmol) in 1,4-dioxane (30 mL). The reaction mixture was degassed with argon for 10 min and CuI (98 mg, 0.51 mmol), $Pd(PPh_3)_4$ (299 mg, 0.26 mmol) were added. The reaction mixture was heated at 120 °C for 16 h. The reaction was quenched with water (30 mL) and the aqueous layer was extracted EtOAc (2x100 mL). The combined organic layers were washed with brine, dried ($Na_2SO_4$) and the solvent removed *in vacuo.* The residue was purified by gradient flash column chromatography eluting with 5-10% EtOAc in hexane to afford 4-fluoro-2-(3-fluoro-5-(trifluoromethyl)benzyl)pyridine as a light yellow liquid (400 mg, 6.4% over two steps). **Data in table 2.**

**Typical procedure 6, exemplified by the preparation of Intermediate 4, 2-fluoro-4-(3-(trifluoromethyl)benzyl)pyridine**

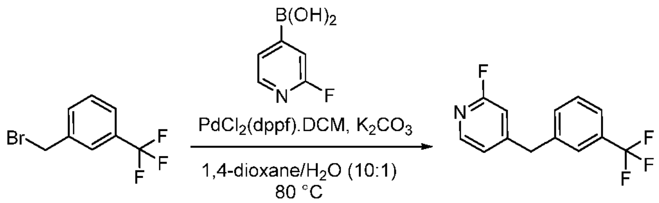


1-(Bromomethyl)-3-(trifluoromethyl)benzene (0.14 mL, 0.88 mmol) was added to a suspension of 2-fluoropyridine-4-boronic acid (150 mg, 1.06 mmol), $K_2CO_3$ (146 mg, 1.06 mmol) and $PdCl_2$(dppf).DCM (129 mg, 0.18 mmol) in 1,4-dioxane (4 mL)/water (0.4 mL) and the reaction mixture heated at 80 °C for 2 hours. The reaction mixture was partitioned between water (6 mL) and EtOAc (6 mL) and the organic layer removed. The aqueous layer was extracted with EtOAc (2x6 mL), the combined organic layers dried (phase separator) and the solvent removed *in vacuo.* The residue was purified by gradient flash column chromatography eluting with 0-50% EtOAc in *i*-hexane to afford 2-fluoro-4-(3-(trifluoromethyl)benzyl)pyridine as a yellow liquid (167 mg, 74%). **Data in table 2.**

**Intermediate 5, 2-fluoro-4-(3-fluoro-5-(trifluoromethyl)benzyl)pyridine**

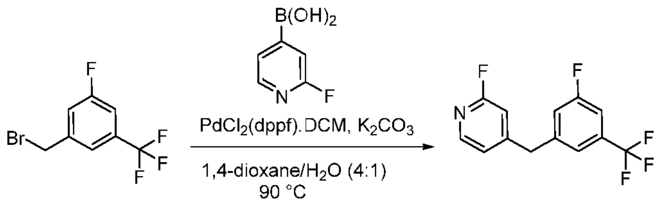


The title compound (1.8 g, 86%) was prepared from (2-fluoropyridin-4-yl)boronic acid (1.3 g, 7.77 mmol), 1-(bromomethyl)-3-fluoro-5-(trifluoromethyl)benzene (1.3 g, 9.30 mmol), $PdCl_2$(dppf).DCM (284 mg, 0.38 mmol) and $K_2CO_3$ (3.2 g, 22.3 mmol) in 1,4-dioxane (20 mL)/water (5 mL) heated at 90 °C for 1 h using the method of Intermediate 4. The title compound was isolated as a brown oil by partitioning between EtOAc (100 mL) and water (70 mL). The organic layer was separated, washed with brine (50 mL), dried ($Na_2SO_4$) and the solvent removed *in vacuo.* The residue was purified by gradient flash column chromatography eluting with 0-30% EtOAc in pet-ether. **Data in table 2.**

**Intermediate 18, 2-chloro-4-(3-fluoro-5-(trifluoromethyl)benzyl)pyridine**

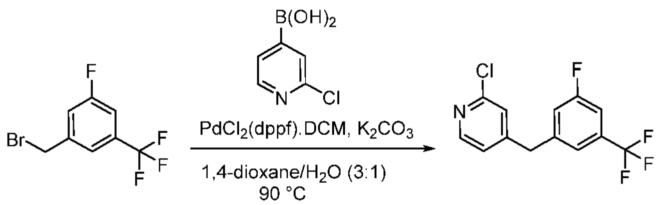


The title compound (3.00 g, 88%) was prepared from (2-chloropyridin-4-yl)boronic acid (1.84 g, 11.7 mmol), 1-(bromomethyl)-3-fluoro-5-(trifluoromethyl)benzene (3.00 g, 11.7 mmol), $PdCl_2$(dppf).DCM (476 mg, 0.58 mmol) and $K_2CO_3$ (4.84 g, 35.0 mmol) in 1,4-dioxane (33.7 mL)/water (11.3 mL) heated at 90 °C for 2 h using the method of Intermediate 4. The title compound was isolated as a colourless oil by partitioning between EtOAc (50 mL) and water (50 mL). The organic layer was separated and the aqueous layer extracted with EtOAc (2x50 mL). The combined organic layers were dried ($Na_2SO_4$) and the solvent removed in *vacuo.* The residue was purified by gradient flash column chromatography eluting with 10-12% EtOAc in hexane. **Data in table 2.**

**Preparation of substituted hydrazineyl intermediates**

**Typical procedure 7, exemplified by the preparation of Intermediate 6, 2-(3-fluoro-5-(trifluoromethyl)benzyl)-4-hydrazineylpyridine**

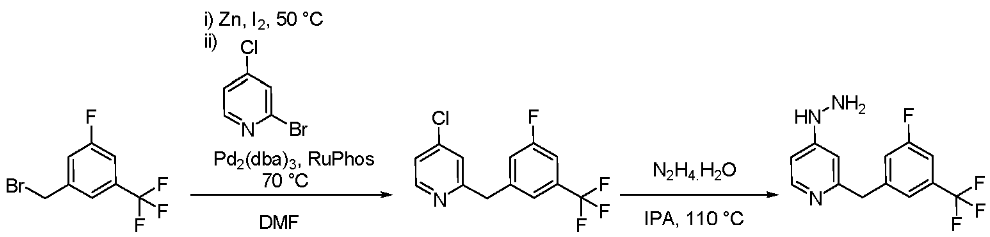


Step 1. A pinch of iodine was added to a stirred solution of activated zinc (35 g, 583 mmol) in DMF (300 mL) and the solution heated at 50 °C for 5 min followed by the addition of 1-(bromomethyl)-3-fluoro-5-(trifluoromethyl)benzene (32 g, 124 mmol) in DMF (50 mL). The reaction mixture was heated at 50 °C for 1 h and then allowed to cool to RT. The residual zinc was allowed to settle and the supernatant pale green DMF layer was transferred *via* cannula to a degassed suspension of 2-bromo-4-chloropyridine (16 g, 83.3 mmol) and RuPhos (2.3 g, 4.99 mmol) in DMF (50 mL) followed by the addition of tris(dibenzylideneacetone)dipalladium(0) (3.8 g, 4.16 mmol). The reaction mixture was heated at 70 °C for 16 h and then filtered through Celite and washed with EtOAc (600 mL). The filtrate was washed with brine (3x300 mL). The organic layer was separated, dried ($Na_2SO_4$) and the solvent removed *in vacuo.* The residue was purified by gradient flash

column chromatography eluting with 0-5% EtOAc in pet-ether to afford 4-chloro-2-(3-fluoro-5-(trifluoromethyl)benzyl) pyridine as a yellow semi solid (8 g, 33%).

**LCMS (Method 3):** m/z 290.1 (ES+), at 2.65 min.

**$^1$H NMR:** (400 MHz, DMSO-*d*$_6$) δ: 8.49 (d, *J* = 5.2 Hz, 1H), 7.83-7.79 (m, 1H), 7.61-7.41 (m, 4H), 4.23 (s, 2H).

Step 2. Hydrazine hydrate (20 g, 415 mmol) was added to a stirred solution of 4-chloro-2-(3-fluoro-5-(trifluoromethyl)benzyl)pyridine (8 g, 27.68 mmol) in IPA (100 mL) in a sealed tube and the reaction mixture was heated at 110 °C for 72 h. The solvent was removed *in vacuo* and the residue partitioned between water (200 mL) and EtOAc (200 mL). The organic layer was separated, washed with brine (200 mL), dried ($Na_2SO_4$) and the solvent removed *in vacuo* to afford 2-(3-fluoro-5-(trifluoromethyl)benzyl)-4-hydrazineylpyridine as a yellow gum (5 g, 63%). **Data in table 2.**

**Intermediate 7, 2-hydrazineyl-4-(3-(trifluoromethyl)benzyl)pyridine**

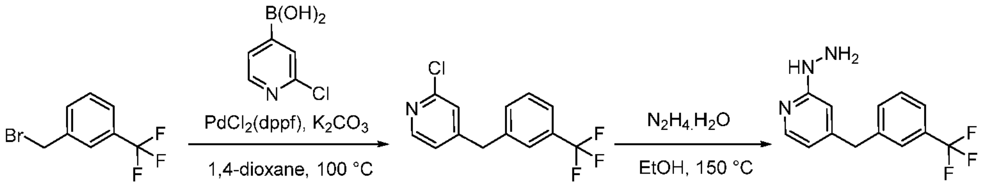


The title compound (486 mg, 80%) was prepared in two steps from (2-chloropyridin-4-yl)boronic acid (3.6 g, 23.2 mmol), 1-(bromomethyl)-3-(trifluoromethyl)benzene (5 g, 21.09 mmol), $PdCl_2$(dppf).DCM (3.4 g, 4.21 mmol, 20 mol%) and $K_2CO_3$ (3.4 g, 25.3 mmol) in 1,4-dioxane (40 mL) heated at 100 °C for 1 h; and step 1 product (500 mg, 1.84 mmol), hydrazine hydrate (0.45 mL, 9.20 mmol) in EtOH (4 mL) heated at 150 °C for 12 h using the methods of Intermediate 4 and Intermediate 6, step 2. After completion of step 2, the title compound was isolated as an orange oil by partitioning between EtOAc (6 mL) and water (6 mL). The aqueous layer was washed with EtOAc (2x6 mL), dried (phase separator) and the solvent removed *in vacuo.* **Data in table 2.**

**Intermediate 39, 4-(3-(difluoromethyl)-5-fluorobenzyl)-2-hydrazineylpyridine**

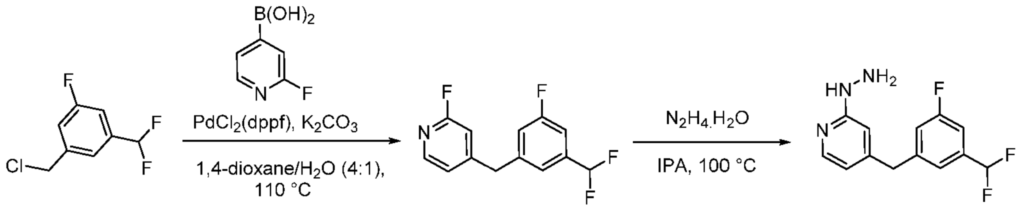


The title compound (110 mg, crude) was prepared in two steps from (2-fluoropyridin-4-yl)boronic acid (145 mg, 1.03 mmol), 1-(chloromethyl)-3-(difluoromethyl)-5-fluorobenzene (Intermediate 26, 200 mg, 1.03 mmol), $PdCl_2$(dppf).DCM (84 mg, 0.103 mmol) and $K_2CO_3$ (426 mg, 3.09 mmol) in 1,4-dioxane (8 mL)/water (2 mL) heated at 110 °C for 16 h; and hydrazine hydrate (0.5 mL, 9.77 mmol) in IPA (10 mL) heated at 100 °C for 48 h using the methods of Intermediate 4 and Intermediate 6, step 2. After completion of step 2, the title compound was isolated as a yellow gum by partitioning between EtOAc (10 mL) and water (10 mL). The aqueous layer was extracted with EtOAc (10 mL). The combined organic layers were washed with brine solution (10 mL), dried ($Na_2SO_4$) and the solvent removed in *vacuo.* The crude product was used in the next step without further purification. **Data in table 2.**

**Intermediate 8, 4-(3-fluoro-5-(trifluoromethyl)benzyl)-2-hydrazineylpyridine**

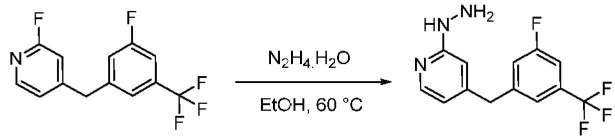


The title compound (350 mg, 100%) was prepared from 2-fluoro-4-(3-fluoro-5-(trifluoromethyl)benzyl)pyridine (Intermediate 5, 300 mg, 1.17 mmol) and hydrazine hydrate (0.17 mL, 3.52 mmol) in EtOH (10 mL) heated at 60 °C for 16 h using the method of Intermediate 6, step 2. The title compound was isolated as a brown oil by partitioning between EtOAc (50 mL) and water (50 mL). The organic layer was separated, washed with brine (50 mL), dried ($Na_2SO_4$) and the solvent removed *in vacuo.* **Data in table 2.**

**Typical procedure 8, exemplified by the preparation of Intermediate 9, 5-(3-fluoro-5-(trifluoromethyl)benzyl)-3-hydrazineylpyridazine**

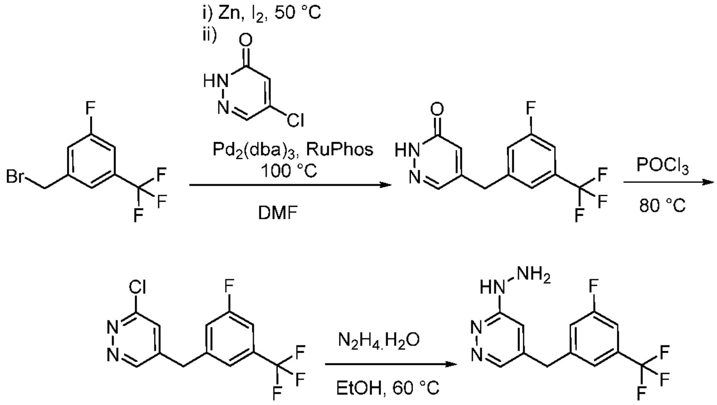


Step 1. A pinch of iodine was added to a stirred solution of activated zinc (1.49 g, 23.0 mmol) in dry DMF (30 mL) and heated to 50 °C for 5 min followed by the addition of 1-(bromomethyl)-3-fluoro-5-(trifluoromethyl)benzene (1.19 g, 4.61 mmol) in DMF (10 mL). The reaction mixture was heated at 50 °C for 1 h and then allowed to cool to RT. The residual zinc was allowed to settle and the supernatant pale green DMF layer was transferred *via* cannula to a degassed suspension of 5-chloropyridazin-3(2*H*)-one (200 mg, 1.53 mmol), RuPhos (70 mg, 0.15 mmol) and tris(dibenzylideneacetone)dipalladium(0) (140 mg, 0.15 mmol) in DMF (10 mL) and the reaction mixture was heated to 100 °C for 16 h and then filtered through Celite and washed with EtOAc (2x50 mL). The filtrate was washed with water (2x70 mL). The organic layer was separated, dried ($Na_2SO_4$) and the solvent removed *in vacuo.* The residue was purified by gradient flash chromatography eluting with 0-20% EtOAc in pet-ether to afford 5-(3-fluoro-5-(trifluoromethyl)benzyl)pyridazin-3(2H)-one as a pale yellow liquid (95 mg, 21%).

**LCMS (Method 3):** m/z 273.1 (ES+), at 1.77 min.

**$^1$H NMR:** (400 MHz, DMSO-$d_6$) δ: 12.94 (s, 1H), 7.86 (d, *J* = 2.8 Hz, 1H), 7.63-7.57 (m, 3H), 6.71 (s, 1H), 3.96 (s, 2H).

Step 2. A suspension of 5-(3-fluoro-5-(trifluoromethyl)benzyl)pyridazin-3(2*H*)-one (90 mg) in $POCl_3$ (2 mL) was heated at 80 °C for 1 h. The reaction mixture was quenched with ice-water (30 mL) and extracted with EtOAc (30 mL). Organic layer was separated, washed with brine (20 mL), dried ($Na_2SO_4$) and the solvent removed *in vacuo* to afford 3-chloro-5-(3-fluoro-5-(trifluoromethyl)benzyl)pyridazine as a brown liquid (120 mg, crude). The crude product was used in the next step without further purification.

**LCMS (Method 3):** m/z 291.0 (ES+), at 2.26 min.

Step 3. Hydrazine hydrate (0.2 ml, 0.68 mmol) was added to a stirred solution of 3-chloro-5-(3-fluoro-5-(trifluoromethyl)benzyl)pyridazine (100 mg) in ethanol (20 mL) and the reaction mixture was heated at 80 °C for 16 h. The solvent was removed *in vacuo* and the residue partitioned between water (50 mL) and EtOAc (50 mL). The organic layer was separated, washed with brine (20 mL), dried ($Na_2SO_4$) and the solvent removed *in vacuo* to afford 5-(3-fluoro-5-(trifluoromethyl)benzyl)-3-hydrazineylpyridazine as a pale yellow liquid (90 mg, crude). The crude product was used in the next step without further purification. **Data in table 2.**

**Typical procedure 9, exemplified by the preparation of Intermediate 42, *tert*-butyl 2-(3-(3-fluoro-5-(trifluoromethyl)benzyl)phenyl)hydrazine-1-carboxylate**

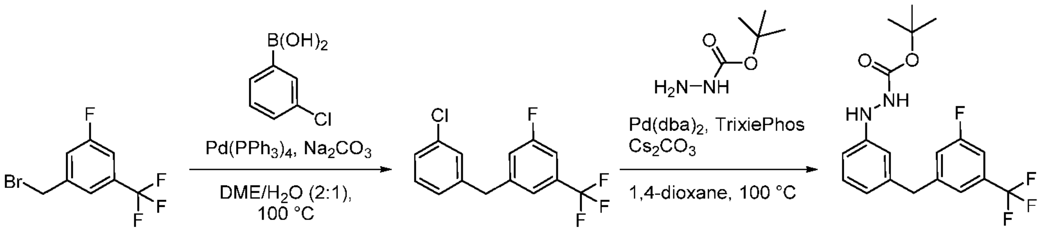


Step 1. (3-Chlorophenyl)boronic acid (188 mg, 1.2 mmol), 3-fluoro-5-(trifluoromethyl)benzyl bromide (0.16 mL, 1.00 mmol), $Pd(PPh_3)_4$ (23 mg, 0.02 mmol) and sodium carbonate (223 mg, 2.1 mmol) were dissolved in DME/water (2 mL:1 mL) and the reaction mixture degassed with $N_2$ for 5 min. The reaction mixture was then heated at 100 °C in a microwave for 3 h. The reaction mixture was diluted with water (3 mL) and extracted with DCM (3x3 mL). The combined organic layers were dried (phase separator) and the solvent removed *in vacuo.* The residue was purified by gradient flash column chromatography eluting with 0-100% $Et_2O$ in *i*-hexane to give 1-[(3-chlorophenyl)methyl]-3-fluoro-5-(trifluoromethyl)benzene as a clear oil (303 mg, quant.).

**LCMS (Method 1):** m/z No ionisation observed (ES+), at 6.15 min.

**$^1$H** NMR: (400 MHz, $CDCl_3$) δ: 7.28-7.26 (m, 2H), 7.23 (tq, *J* = 1.7, 0.6 Hz, 1H), 7.20 (ddq, *J* = 2.2, 1.7, 0.6 Hz, 1H), 7.19 (ddd, *J* = 2.3, 1.4, 0.8 Hz, 1H), 7.10 -7.03 (m, 2H), 4.02 (d, *J* = 0.8 Hz, 2H).

Step 2. *tert*-Butyl carbazate (166 mg, 1.26 mmol), $Pd(dba)_2$ (19 mg, 0.03 mmol), *rac*-2-(di-*tert*-butylphosphino)-1,1'-binaphthyl (13 mg, 0.03 mmol) and cesium carbonate (514 mg, 1.57 mmol) were placed in a microwave vial which was evacuated and backfilled with $N_2$ three times. 1-[(3-chlorophenyl)methyl]-3-fluoro-5-(trifluoromethyl)benzene (303 mg, 1.05 mmol) dissolved in 1,4-dioxane (3 mL) was added and the reaction mixture heated at 100 °C in a microwave for 8 h. The reaction mixture was partitioned between EtOAc (3 mL) and water (3 mL) and the organic layer removed. The aqueous layer was extracted with EtOAc (2x3 mL), the combined organic layers dried (phase separator) and the solvent removed *in vacuo.* The residue was purified by gradient flash column chromatography eluting with 0-20% $Et_2O$ in *i*-hexane to give *tert*-butyl 2-(3-(3-fluoro-5-(trifluoromethyl)benzyl)phenyl)hydrazine-1-carboxylate as a clear oil (181 mg, 45%). **Data in table 2.**

**Preparation of 1-substituted 1,5,6,7-tetrahydro-4*H*-pyrazolo[4,3-*c*]pyridin-4-one and 1,5,6,7-tetrahydro-4*H*-pyrrolo[3,2-*c*]pyridin-4-one intermediates**

**Typical procedure 10, exemplified by the preparation of Intermediate 10, 1-(6-chloropyridazin-4-yl)-1,5,6,7-tetrahydro-4*H*-pyrazolo[4,3-*c*]pyridin-4-one**

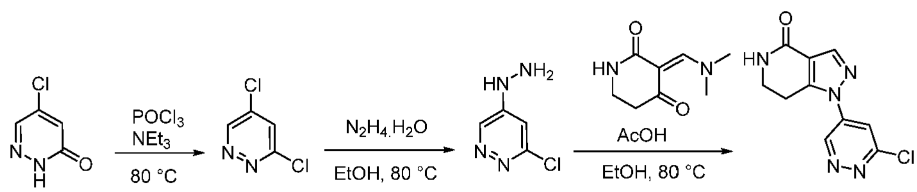


Step 1. $Et_3N$ (2 mL) was added to suspension of 5-chloropyridazin-3(2*H*)-one (5 g, 38.3 mmol) in $POCl_3$ (50 mL) at 0 °C and the reaction mixture heated at 100 °C for 12 h. The solvent was removed *in vacuo* and the residue was purified by gradient flash chromatography eluting with 0-20 % EtOAc in pet-ether to afford 3,5-dichloropyridazine as an off-white solid (2 g, 35%).

**LCMS (Method 3):** m/z 149.0 (ES+), at 1.24 min.

**$^1$H NMR:** (400 MHz, $CDCl_3$) δ: 9.15 (d, *J* = 2.0 Hz, 1H), 7.62 (d, *J* = 2.0 Hz, 1H).

Step 2. Hydrazine hydrate (0.5 mL) was added to a solution of 3,5-dichloropyridazine (2 g, 13.4 mmol) in EtOH (20 mL) and the reaction mixture was heated at 80 °C for 12 h. The solvent was removed *in vacuo* to afford 3-chloro-5-hydrazineylpyridazine (crude). The crude product was used in the next step without further purification.

**LCMS:** Not recorded.

**$^1$H NMR:** Not recorded.

Step 3. AcOH (0.1 mL) was added to a stirred solution of 3-chloro-5-hydrazineylpyridazine (200 mg, 1.39 mmol) and (*E*)-3-((dimethylamino)methylene) piperidine-2,4-dione (Intermediate 1, 278 mg, 1.66 mmol) in EtOH (20 mL) the reaction mixture was heated at 80 °C for 12 h. The solvent was removed *in vacuo* and the residue purified by prep HPLC (Method 1) to afford 1-(6-chloropyridazin-4-yl)-1,5,6,7-tetrahydro-4*H*-pyrazolo[4,3-*c*]pyridin-4-one (60 mg, 17%). **Data in table 2.**

**Intermediate 11, 1-(5-bromopyridin-3-yl)-1,5,6,7-tetrahydro-4*H*-pyrazolo[4,3-*c*]pyridin-4-one**

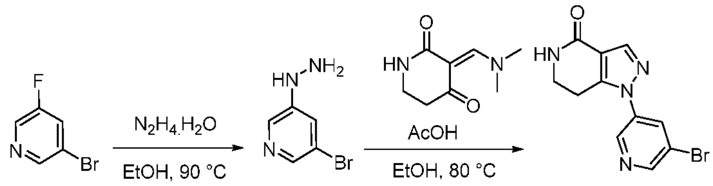


The title compound (200 mg, 98%) was prepared in two steps from 3-bromo-5-fluoropyridine (700 mg, 4.0 mmol) and hydrazine hydrate (1.92 g, 60 mmol) in EtOH (10 mL) heated at 90 °C for 12 h; and (*E*)-3-((dimethylamino)methylene) piperidine-2,4-dione (Intermediate 1, 402 mg, 2.39 mmol) and acetic acid (0.01 mL) in EtOH (20 mL) heated at 80 °C for 15 h using the methods of Intermediate 10, steps 2 and 3. After completion of step 2, the title compound was isolated as a yellow solid by filtration of the precipitated solid which was washed with EtOH (2x10 mL) and dried *in vacuo.* **Data in table 2.**

**Intermediate 40, 1-(5-fluoropyridin-3-yl)-1,5,6,7-tetrahydro-4*H*-pyrazolo[4,3-*c*]pyridin-4-one**

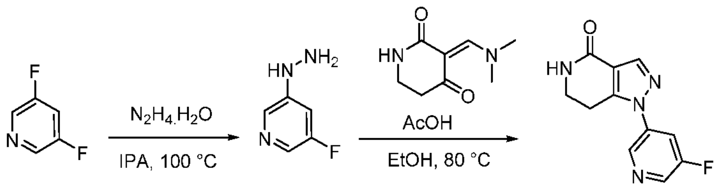


The title compound (370 mg, 37%) was prepared in two steps from 3,5-difluoropyridine (500 mg, 4.34 mmol and hydrazine hydrate (3.29 g, 65.17 mmol) in IPA (50 mL) heated at 100 °C for 12 h; and (*E*)-3-((dimethylamino)methylene) piperidine-2,4-dione (Intermediate 1, 1.37 g, 8.18 mmol) and acetic acid (0.05 mL) in EtOH (50 mL) heated at 80 °C for 16 h using the methods of Intermediate 10, steps 2 and 3. After completion of step 2, the title compound was isolated as a yellow solid by removal of the solvent *in vacuo.* The residue was partitioned between EtOAc (200 mL) and sat. $NaHCO_3$ solution (150 mL). The organic layer was separated, washed with brine solution (2x100 mL), dried ($Na_2SO_4$) and the solvent removed *in vacuo.* The residue was purified by gradient flash chromatography eluting with 0-10% MeOH in DCM. **Data in table 2.**

**Intermediate 12, 1-(4-bromopyridin-2-yl)-1,5,6,7-tetrahydro-4*H*-pyrazolo[4,3-*c*]pyridin-4-one**

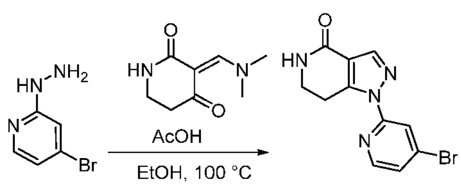


The title compound (410 mg, 78%) was prepared from 4-bromo-2-hydrazineylpyridine (300 mg, 1.78 mmol) and

(*E*)-3-((dimethylamino)methylene) piperidine-2,4-dione (Intermediate 1, 332 mg, 1.78 mmol) in EtOH (5 mL) and AcOH (0.01 mL) heated at 100 °C for 10 h using the method of Intermediate 10, step 3. The title compound was isolated as yellow solid by trituration with $Et_2O$. **Data in table 2.**

**Typical procedure 11, exemplified by the preparation of Intermediate 13, 1-(2-bromopyridin-4-yl)-1,5,6,7-tetrahydro-4*H*-pyrazolo[4,3-*c*]pyridin-4-one**

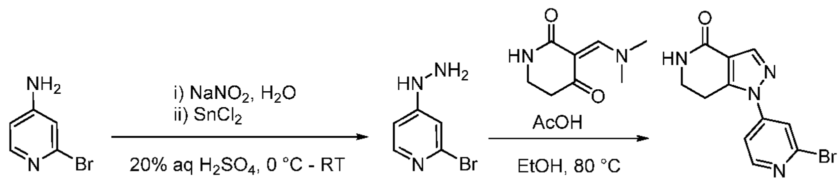


Step 1. $NaNO_2$ (4.7 g, 69.4 mmol) in water (30 mL) was added to a stirred solution of 2-bromopyridin-4-amine (10 g, 57.8 mmol) in 20% aqueous $H_2SO_4$ solution (300 mL) at 0°C and stirred for 1h, followed by the addition of $SnCl_2$ (21.8 g, 116 mmol) in 20% aqueous $H_2SO_4$ solution (100 mL) at 0 °C The reaction mixture was stirred at RT for 1h. The reaction mixture was basified with ammonium hydroxide solution (200 mL) up to pH~8 and the reaction mixture was filtered through Celite. The filtrate was partitioned between water (200 mL) and 10% MeOH in DCM (400 mL). The organic layer was separated, washed with brine (50 mL), dried ($Na_2SO_4$) and the solvent removed *in vacuo* to afford 2-bromo-4-hydrazineylpyridine as a brown solid (7.0 g, 64%).

**LCMS (Method 3):** m/z 188.0 (ES+), at 0.37 min.

**$^1$H NMR:** (400 MHz, DMSO-$d_6$) δ: 7.95 (s, 1H), 7.75 (d, *J* = 6.0 Hz, 1H), 6.81 (s, 1H), 6.59 (d, *J* = 3.6 Hz, 1H), 4.32 (s, 2H).

Step 2. A catalytic amount of acetic acid (0.2 mL) was added to a stirred solution of 2-bromo-4-hydrazineylpyridine (7.0 g, 37.2 mmol) and (*E*)-3-((dimethylamino)methylene)piperidine-2,4-dione (Intermediate 1, 4.3 g, 26.1 mmol) in EtOH (150 mL) and the reaction mixture was heated at 80 °C for 15 h. Precipitated solid was filtered, washed with EtOH (2x50 mL) and pet-ether (2x50 mL) to afford 1-(2-bromopyridin-4-yl)-1,5,6,7-tetrahydro-4*H*-pyrazolo[4,3-*c*]pyridin-4-one as a yellow solid (3.5 g, 32%). **Data in table 2.**

**Intermediate 37, 1-(5-bromopyridin-3-yl)-5-(2,4-dimethoxybenzyl)-3-methyl-1,5,6,7-tetrahydro-4*H*-pyrazolo[4,3-*c*]pyridin-4-one**

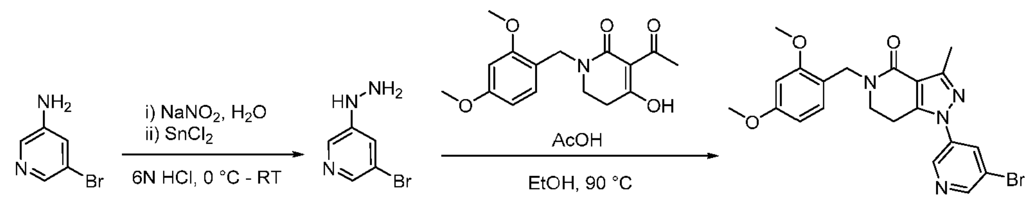


The title compound (400 mg, 38%) was prepared in two steps from 5-bromopyridin-3-amine (1 g, 5.78 mmol) in 6N HCl (10 mL) and $NaNO_2$ (590 mg, 8.67 mmol) in water (20 mL) stirred for 15 min at 0 °C, followed by the addition of $SnCl_2$ (2.1 g, 11.56 mmol) in 6N HCl (20 mL) and stirred at RT for 3 h; and 3-acetyl-1-(2,4-dimethoxybenzyl)-4-hydroxy-5,6-dihydropyridin-2(1H)-one (Intermediate 35, 700 mg, 2.29 mmol) and acetic acid (14 mg, 0.229 mmol) in EtOH (20 mL) heated at 90 °C for 15 h using the methods of Intermediate 13. After completion of step 2, the title compound was isolated as a yellow solid by removal of the solvent *in vacuo.* The residue was purified by gradient flash column chromatography eluting with 0-100% EtOAc in pet-ether **Data in table 2.**

**Typical procedure 12, exemplified by the preparation of Intermediate 14, tert-butyl 1-(2-bromopyridin-4-yl)-4-oxo-1,4,6,7-tetrahydro-5H-pyrazolo[4,3-c]pyridine-5-carboxylate**

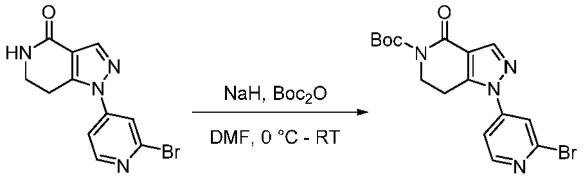


NaH (368 mg, 15.3 mmol) and Boc anhydride (3.34 g, 15.3 mmol) were added to a stirred solution of 1-(2-bromopyridin-4-yl)-1,5,6,7-tetrahydro-4*H*-pyrazolo[4,3-*c*]pyridin-4-one (Intermediate 13, 3 g, 10.2 mmol) in DMF (20 mL) at 0 °C and the reaction mixture was stirred at RT for 12h. The reaction mixture was partitioned between ice-cold water (500 mL) and EtOAc (250 mL). The organic layer was separated, dried ($Na_2SO_4$) and the solvent removed *in vacuo.* The residue was purified by gradient flash column chromatography eluting with 0-80% EtOAc in hexane to afford *tert*-butyl 1-(2-bromopyridin-4-yl)-4-oxo-1,4,6,7-tetrahydro-5*H*-pyrazolo[4,3-c]pyridine-5-carboxylate as a white solid (2.6 g. 64%). **Data in table 2.**

**Typical procedure 13, exemplified by the preparation of Intermediate 21, *tert*-butyl 1-(2-bromopyridin-4-yl)-4-oxo-1,4,6,7-tetrahydro-5*H*-pyrrolo[3,2-*c*]pyridine-5-carboxylate**

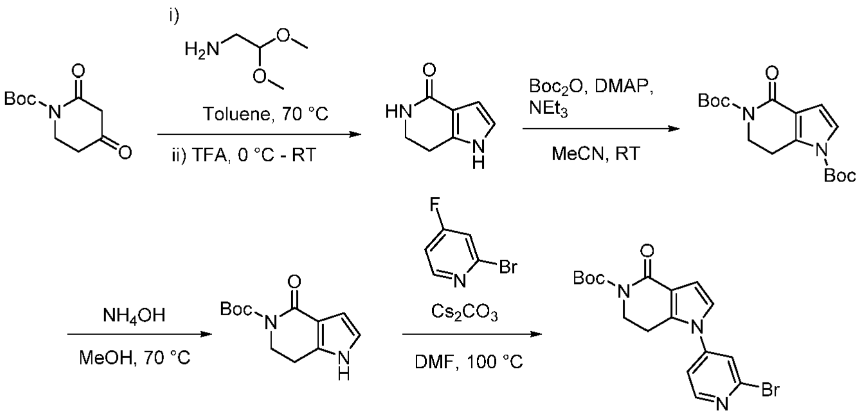


Step 1. Molecular sieves (3 g) were added to a stirred solution of *tert*-butyl 2,4-dioxopiperidine-1-carboxylate (3 g, 14.1 mmol) in toluene (50 mL) followed by the addition of 2,2-dimethoxyethan-1-amine (1.53 mL, 14.1 mmol). The reaction mixture was heated at 70 °C for 1 h. The reaction mixture was diluted with water (50 mL) and extracted with EtOAc (3x50 mL). The combined organic layers were dried ($Na_2SO_4$) and the solvent removed *in vacuo.* The residue was dissolved in DCM (30 mL) and cooled to 0 °C. TFA (30 mL) was added and the reaction mixture was stirred at RT for 2 h. The solvent was removed *in vacuo.* The residue was purified by gradient flash column chromatography eluting with 0-4% MeOH in DCM to afford 1,5,6,7-tetrahydro-4*H*-pyrrolo[3,2-c]pyridin-4-one as an off-white solid.

**LCMS (Method 7):** m/z 137.2 (ES+), at 0.53 min.

Step 2. $NEt_3$ (11.2 mL, 79.3 mmol) and DMAP (0.242 g, 1.98 mmol) were added to a stirred solution of 1,5,6,7-tetrahydro-4*H*-pyrrolo[3,2-c]pyridin-4-one (2.4 g, 13.23 mmol) in MeCN (50 mL) followed by the addition of Boc anhydride (15.9 mL, 69.4 mmol). The reaction mixture was stirred at RT for 16 h. The reaction mixture was diluted with water (50 mL) and extracted with EtOAc (3x50 mL). The combined organic layers were dried ($Na_2SO_4$) and the solvent removed *in vacuo.* The residue was purified by flash column chromatography eluting with 0-25% EtOAc in pet-ether to afford di-*tert*-butyl 4-oxo-6,7-dihydro-1*H*-pyrrolo[3,2-*c*]pyridine-1,5(4*H*)-dicarboxylate as a yellow solid (2.91 g, 65%).

**LCMS (Method 7):** m/z 281.2 (ES+), at 2.56 min.

**$^1$H NMR:** (400 MHz, DMSO-*d*$_6$) δ: 7.25 (d, *J* = 4.8 Hz, 1H), 6.50 (d, *J* = 4.4 Hz, 1H), 3.95 (t, *J* = 8.8 Hz, 2H), 3.25 (t, *J* = 26.8 Hz, 2H), 1.54 (s, 9H), 1.29 (s, 9H).

Step 3. Ammonium hydroxide solution (20 mL) was added to a stirred solution of di-*tert*-butyl 4-oxo-6,7-dihydro-1*H*-pyrrolo[3,2-*c*]pyridine-1,5(4*H*)-dicarboxylate (2.9 g, 8.63 mmol) in MeOH (30 mL) and the reaction mixture was heated at 70 °C for 8 h. The solvent was removed *in vacuo.* The residue was purified by gradient flash column chromatography eluting with 0-40% EtOAc in pet-ether to afford *tert*-butyl 4-oxo-1,4,6,7-tetrahydro-5*H*-pyrrolo[3,2-*c*]pyridine-5-carboxylate as a white solid (0.8 g, 39%).

**LCMS (Method 7):** m/z 235.2 (ES+), at 1.66 min.

**$^1$H NMR:** (400 MHz, DMSO-*d*$_6$) δ: 11.36 (s, 1H), 6.74 (d, *J* = 1.2 Hz, 1H), 6.32 (d, *J* = 2.8 Hz, 1H), 3.91 (t, *J* = 6.4 Hz,

2H), 2.80 (t, *J* = 6.0 Hz, 2H), 1.45 (s, 9H).

Step 4. $Cs_2CO_3$ (1.1 g, 3.409 mmol) was added to a stirred solution of 2-bromo-4-fluoropyridine (0.2 g, 1.13 mmol) and *tert*-butyl 4-oxo-1,4,6,7-tetrahydro-5*H*-pyrrolo[3,2-*c*]pyridine-5-carboxylate (0.268 g, 1.13 mmol) in DMF (10 mL) and the reaction mixture was heated at 100 °C for 2 h. The reaction mixture was diluted with water (50 mL) and extracted with EtOAc (2x50 mL). The combined organic layers were dried ($Na_2SO_4$) and the solvent removed *in vacuo.* The residue was purified by gradient flash column chromatography eluting with 0-40% EtOAc in pet-ether to afford *tert*-butyl 1-(2-bromopyridin-4-yl)-4-oxo-1,4,6,7-tetrahydro-5*H*-pyrrolo[3,2-*c*]pyridine-5-carboxylate as a white solid (0.38 g, 89%). **Data in table 2.**

**Typical procedure 14, exemplified by the preparation of Intermediate 23, *tert*-butyl 1-(6-chloropyrimidin-4-yl)-4-oxo-1,4,6,7-tetrahydro-5*H*-pyrazolo[4,3-*c*]pyridine-5-carboxylate**

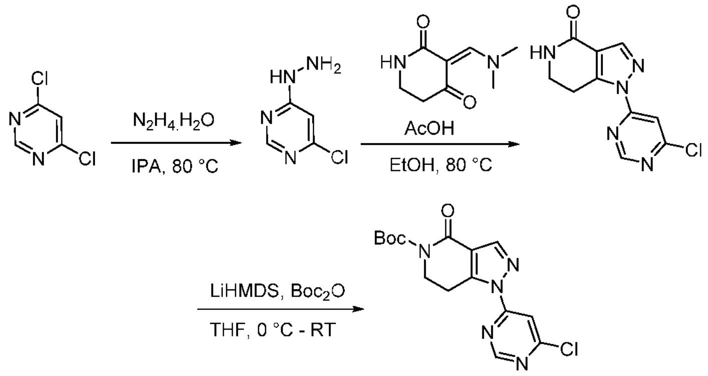


Step 1. Hydrazine hydrate (3 g, 60.39 mmol) was added to a stirred solution of 4,6-dichloropyrimidine (3 g, 20.1 mmol) in IPA (50 mL) and the reaction mixture was heated at 80 °C for 16 h. The solvent was removed *in vacuo* and the residue azeotroped with toluene to afford 4-chloro-6-hydrazineylpyrimidine as a pale yellow solid (2.5 g, 86%).

**LCMS (Method 10):** m/z 145.1 (ES+), at 0.86 min.

**$^1$H NMR:** (400 MHz, DMSO-$d_6$) δ: 8.83 (s, 1H), 8.17 (s, 1H), 6.75 (s, 1H), 4.49 (s, 2H).

Step 2. A catalytic amount of acetic acid (1 mL) was added to a stirred solution of 4-chloro-6-hydrazineylpyrimidine (1 g, 6.94 mmol) and (*E*)-3-((dimethylamino)methylene)piperidine-2,4-dione (Intermediate 1, 1.2 g, 6.94 mmol) in ethanol (30 mL) and the reaction mixture was heated at 80 °C for 16 h. The solvent was removed *in vacuo* and the residue was partitioned between sat. $NaHCO_3$ solution (50 mL) and EtOAc (50 mL). The residue was purified by gradient flash column chromatography eluting with 0-100% EtOAc in pet-ether to afford 1-(6-chloropyrimidin-4-yl)-1,5,6,7-tetrahydro-4*H*-pyrazolo[4,3-c]pyridin-4-one as an off-white solid (300 mg, 17%).

**LCMS (Method 3):** m/z 250.0 (ES+), at 1.41 min.

**$^1$H NMR:** (400 MHz, DMSO-$d_6$) δ: 9.02 (d, *J* = 0.8 Hz, 1H), 8.18 (s, 1H), 8.06 (d, *J* = 0.8 Hz, 1H), 7.61 (s, 1H), 3.50 (d, *J* = 2.0 Hz, 4H).

Step 3. LiHMDS (1M in THF, 0.6 mL, 0.60 mmol) was added to a stirred solution of 1-(6-chloropyrimidin-4-yl)-1,5,6,7-tetrahydro-4*H*-pyrazolo[4,3-c] pyridin-4-one (100 mg, 0.40 mmol) in THF (5 mL) at 0 °C and then stirred at RT for 30 min. Boc anhydride (130 mg, 0.60 mmol) was then added and the reaction mixture was stirred at RT for 16 h. The reaction mixture was quenched with ice water (50 mL) and extracted with EtOAc (50 mL). The organic layer was separated, washed with brine solution (50 mL), dried ($Na_2SO_4$) and the solvent removed *in vacuo.* The residue was purified by gradient flash column chromatography eluting with 0-50 % EtOAc in pet-ether to afford *tert*-butyl 1-(6-chloropyrimidin-4-yl)-4-oxo-1,4,6,7-tetrahydro-5*H*-pyrazolo[4,3-*c*]pyridine-5-carboxylate as a yellow solid (60 mg, 42%). **Data in table 2.**

**Typical procedure 15, exemplified by the preparation of Intermediate 36, 1-(2-bromopyridin-4-yl)-5-(2,4-dimethoxybenzyl)-3-methyl-1,5,6,7-tetrahydro-4*H*-pyrazolo[4,3-*c*]pyridin-4-one**

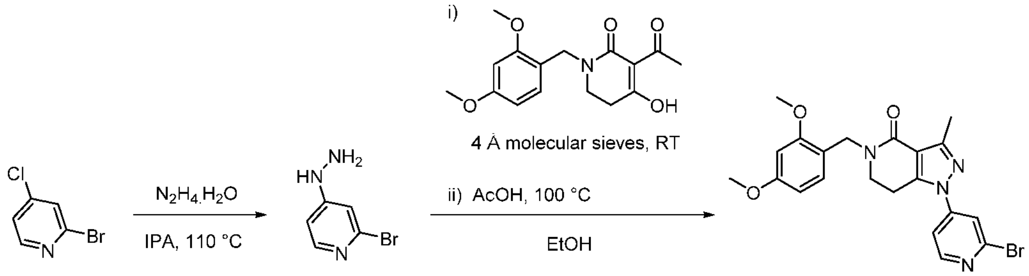


Step 1. Hydrazine hydrate (26 g, 521 mmol) was added to a stirred solution of 2-bromo-4-chloropyridine (20 g, 104 mmol) in IPA (100 mL) and the reaction mixture was heated at 110 °C for 16 h. The solvent was removed *in vacuo* and the residue was dissolved in EtOAc (500 mL) and washed with water (500 mL). The organic layer was separated, washed with brine solution (500 mL), dried ($Na_2SO_4$) and the solvent removed *in vacuo* to afford 2-bromo-4-hydrazineylpyridine as a white solid (15 g, 77%).

**LCMS (Method 10):** m/z 188.2 (ES+), at 1.06 min.

**$^1$H NMR:** (400 MHz, DMSO-$d_6$) δ: 7.95-7.93 (m, 1H), 7.74 (d, *J* = 7.2 Hz, 1H), 6.80 (d, *J* = 6.4 Hz, 1H), 6.75 (s, 1H), 4.32 (s, 2H).

Step 2. 4 Å Molecular sieves were added to a stirred solution of 2-bromo-4-hydrazineylpyridine (5 g, 26.6 mmol) and 3-acetyl-1-(2,4-dimethoxybenzyl)-4-hydroxy-5,6-dihydropyridin-2(1*H*)-one (Intermediate 35, 9.7 g, 31.9 mmol) in EtOH (100 mL) and the reaction mixture was stirred at RT for 10 h. Acetic acid (1.6 mL, 26.6 mmol) was then added and the reaction mixture was heated at 100 °C for 16 h. The reaction mixture was filtered through celite, washed with EtOAc (2x50 mL) and the solvent removed *in vacuo.* The residue was dissolved in EtOAc (300 mL) and washed with 10% $NaHCO_3$ solution (100 mL). The organic layer was separated, dried ($Na_2SO_4$) and the solvent removed *in vacuo.* The residue was purified by gradient flash column chromatography eluting with 0-50 % EtOAc in pet-ether to afford 1-(2-bromopyridin-4-yl)-5-(2,4-dimethoxybenzyl)-3-methyl-1,5,6,7-tetrahydro-4*H*-pyrazolo[4,3-c]pyridin-4-one as a white solid (4.1 g, 28%). **Data in table 2.**

**Intermediate 38, 1-(4-bromopyridin-2-yl)-5-(2,4-dimethoxybenzyl)-3-methyl-1,5,6,7-tetrahydro-4*H*-pyrazolo[4,3-*c*]pyridin-4-one**

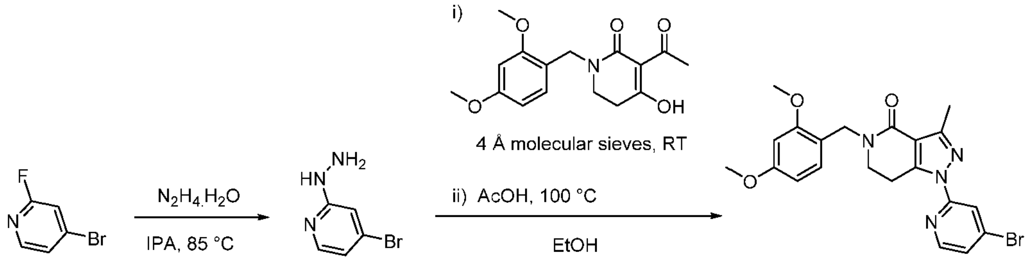


The title compound (700 mg, 25%) was prepared in two steps from 4-bromo-2-fluoropyridine (2 g, 11.4 mmol) and hydrazine hydrate (5.6 g, 114 mmol) in IPA (20 mL) heated at 85 °C for 12 h; and 4 Å Molecular sieves (1 g) and 3-acetyl-1-(2,4-dimethoxybenzyl)-4-hydroxy-5,6-dihydropyridin-2(1*H*)-one (Intermediate 35, 1.6 g, 5.31 mmol) in EtOH (20 mL) stirred at RT for 12 h, followed by addition of acetic acid (0.1 mL) heated at 90 °C for 12 h using the methods of Intermediate 36. After completion of step 2, the title compound was isolated as a yellow solid by filtration, rinsing with EtOAc (2x50 mL) and removal of the solvent *in vacuo.* The residue was dissolved in EtOAc (50 mL) and washed with 10% $NaHCO_3$ solution (50 mL). The organic layer was separated, washed with brine solution (20 mL), dried ($Na_2SO_4$) and the solvent removed *in vacuo.* The residue was purified by gradient flash column chromatography eluting with 0-70 % EtOAc in pet-ether. **Data in table 2.**

**Typical procedure 16, exemplified by the preparation of Intermediate 41, *tert*-butyl 1-(2-bromo-5-methylpyridin-4-yl)-4-oxo-1,4,6,7-tetrahydro-5*H*-pyrazolo[4,3-*c*]pyridine-5-carboxylate**

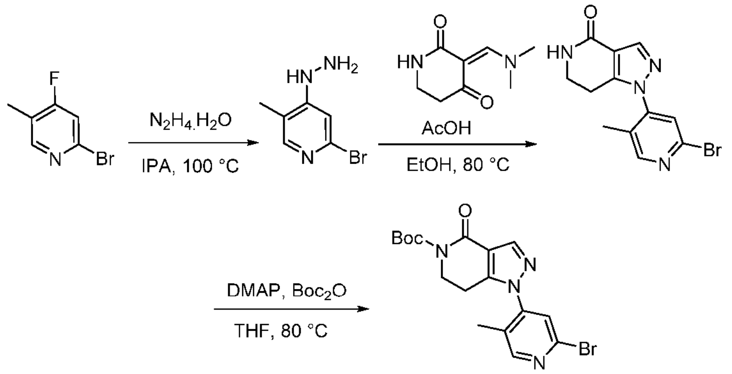


Step 1. Hydrazine hydrate (0.16 mL, 3.16 mmol) was added to a stirred solution of 2-bromo-4-fluoro-5-methylpyridine (200 mg, 1.05 mmol) in IPA (10 mL) at RT and the reaction mixture was heated at 100 °C for 16 h. The solvent was removed *in vacuo.* The residue was triturated with 10% $NaHCO_3$ solution (5 mL) and the solids filtered, washed with water (10 mL) and dried *in vacuo* to afford 2-bromo-4-hydrazineyl-5-methylpyridine as an off-white solid (130 mg, 61%).

**LCMS (Method 2):** m/z 201.7 (ES+), at 1.07 min.

**$^1$H NMR:** (300 MHz, DMSO-$d_6$) δ: 7.60 (s, 1H), 7.50 (s, 1H), 6.99 (s, 1H), 4.29 (s, 2H), 1.92 (s, 3H).

Step 2. (*E*)-3-((Dimethylamino)methylene)piperidine-2,4-dione (Intermediate 1, 162 mg, 0.97 mmol) and acetic acid (19 mg, 0.32 mmol) were added to a solution of 2-bromo-4-hydrazineyl-5-methylpyridine (130 mg, 0.64 mmol) in EtOH (10 mL) at RT. The reaction mixture was heated at 80 °C for 16 h. The reaction mixture was partitioned between 10% $NaHCO_3$ solution (15 mL) and EtOAc (15 mL). The organic layer was separated, dried ($Na_2SO_4$) and the solvent removed *in vacuo* to afford 1-(2-bromo-5-methylpyridin-4-yl)-1,5,6,7-tetrahydro-4*H*-pyrazolo[4,3-c]pyridin-4-one as a yellow gum (100 mg, 51%).

**LCMS (Method 10):** m/z 307.0 (ES+), at 1.12 min.

**$^1$H NMR:** (400 MHz, DMSO-$d_6$) δ: 8.52 (s, 1H), 8.03 (s, 1H), 7.85 (s, 1H), 7.45 (s, 1H), 3.42-3.41 (m, 2H), 2.89 (t, *J* = 9.2 Hz, 2H), 2.27 (s, 3H).

Step 3. DMAP (7.96 mg, 0.07 mmol) and Boc anhydride (0.09 mL, 0.39 mmol) were added to a solution of 1-(2-bromo-5-methylpyridin-4-yl)-1,5,6,7-tetrahydro-4*H*-pyrazolo[4,3-*c*]pyridin-4-one (100 mg, 0.33 mmol) in THF (10 mL) at RT and the reaction mixture was heated at 80 °C for 15 h. The solvent was removed *in vacuo.* The residue was purified by gradient flash column chromatography eluting with 0-60% EtOAc in pet-ether to afford *tert*-butyl 1-(2-bromo-5-methylpyridin-4-yl)-4-oxo-1,4,6,7-tetrahydro-5*H*-pyrazolo[4,3-*c*]pyridine-5-carboxylate as a white solid (100 mg, 75%). **Data in table 2.**

**Typical procedure 17, exemplified by the preparation of Intermediate 45, 1-(2-(3-fluoro-5-(trifluoromethyl)benzyl)pyridin-4-yl)-3-iodo-1,5,6,7-tetrahydro-4*H*-pyrazolo[4,3-*c*]pyridin-4-one**

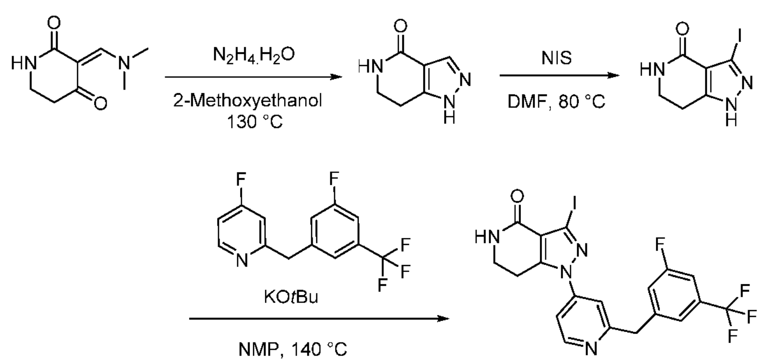


Step 1. Hydrazine hydrate (297 mg, 5.95 mmol) was added to a stirred solution of (*E*)-3-((dimethylamino) methylene)piperidine-2,4-dione (Intermediate 1, 500 mg, 2.97 mmol) in 2-methoxyethanol (10 mL) and the reaction mixture was heated at 130 °C for 12 h. The solvent was removed *in vacuo.* The residue was purified by gradient flash column chromatography eluting with 0-15% MeOH in DCM to afford (1,5,6,7-tetrahydro-4*H*-pyrazolo[4,3-*c*]pyridin-4-one as a brown solid (300 mg, 73%).

**LCMS (Method 6):** m/z 138.2 (ES+), at 0.63 min.

**$^1$H NMR:** (400 MHz, DMSO-*d*$_6$) δ: 7.92 (s, 1H), 7.28 (s, 1H), 3.39-3.35 (m, 2H), 2.81-2.77 (m, 2H). 1 exchangeable proton not observed.

Step 2. NIS (980 mg, 4.37 mmol) was added to a stirred solution of (1,5,6,7-tetrahydro-4*H*-pyrazolo[4,3-*c*]pyridin-4-one (300 mg, 2.18 mmol) in DMF (10 mL) at RT and the reaction mixture was heated at 80 °C for 12 h. The solvent was removed *in vacuo.* The residue was purified by gradient flash column chromatography eluting with 0-15% MeOH in DCM to afford 3-iodo-1,5,6,7-tetrahydro-4*H*-pyrazolo[4,3-*c*]pyridin-4-one as an off-white solid (290 mg, 51%).

**LCMS (Method 3):** m/z 263.9 (ES+), at 0.61 min.

**$^1$H NMR:** (400 MHz, DMSO-*d*$_6$) δ: 13.41 (s, 1H), 7.30 (s, 1H), 2.84-2.63 (m, 4H).

Step 3. KO*t*-Bu (246 mg, 2.20 mmol) was added to a stirred solution of 3-iodo-1,5,6,7-tetrahydro-4*H*-pyrazolo[4,3-*c*]pyridin-4-one (290 mg, 1.10 mmol) in NMP (10 mL) followed by the addition of 4-fluoro-2-(3-fluoro-5-(trifluoromethyl)benzyl)pyridine (Intermediate 3, 362 mg, 1.32 mmol). The reaction mixture was heated in a microwave at 140 °C for 1 h. The reaction mixture was diluted with water (20 mL) and extracted with EtOAc (2x50 mL). The combined organic layers were washed with brine solution (20 mL), dried ($Na_2SO_4$) and the solvent removed *in vacuo.* The residue was purified by gradient flash column chromatography eluting with 0-15% MeOH in DCM to afford 1-(2-(3-fluoro-5-(trifluoromethyl)benzyl)pyridin-4-yl)-3-iodo-1,5,6,7-tetrahydro-4*H*-pyrazolo[4,3-*c*]pyridin-4-one as a white solid (150 mg, 26%). **Data in table 2.**

## Preparation of substituted triazole and pyrazole intermediates

### Typical procedure 18, exemplified by the preparation of Intermediate 15, 1,5,6,7-tetrahydro-4*H*-benzo[*d*][1,2,3]triazol-4-one

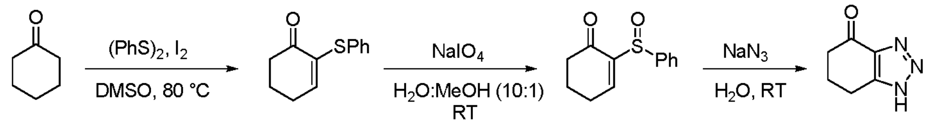


Step 1. Iodine (0.26 g, 1 mmol) was added to a stirred mixture of cyclohexanone (2 g, 20 mmol) and 1,2-diphenyldisulfane (1.7 g, 80 mmol) in DMSO (12 mL) and the reaction mixture was heated at 80 °C for 12 h. The reaction was quenched by the addition of water (100 mL) and the aqueous layer was extracted with EtOAc (3x100 mL). The organic layers were combined, dried ($Na_2SO_4$) and the solvent removed *in vacuo* to afford 2-(phenylthio)cyclohex-2-en-1-one as a yellow liquid (4.5 g crude). The crude product was used in the next step without further purification.

**MS (Method 1):** m/z 205 (ES+).

Step 2. Sodium periodate (9.39 g, 40 mmol) was added to a stirred solution of 2-(phenylthio)cyclohex-2-en-1-one (4.5 g, 22 mmol) in MeOH (1.2 mL) and $H_2O$ (12 mL) and the reaction mixture was stirred at RT for 16 h. The reaction was quenched by the addition of water (100 mL) and the aqueous layer was extracted with EtOAc (3x100 mL). The organic layers were combined, dried ($Na_2SO_4$) and the solvent removed *in vacuo.* The residue was purified by gradient flash column chromatography eluting with 30-35% EtOAc in hexane to afford 2-(phenylsulfinyl) cyclohex-2-en-1-one as an orange gum (2.1 g, 43%).

**MS (Method 1):** m/z 221 (ES+).

Step 3. Sodium azide (324 mg, 4 mmol) was added to a stirred solution of 2-(phenylsulfinyl)cyclohex-2-en-1-one (1 g, 4 mmol) in $H_2O$ (17 mL) and the reaction mixture was stirred at RT for 16 h. The reaction mixture was acidified to pH 2 using 1 N HCl (19 mL) and water (100 mL) was added. The aqueous layer was extracted with EtOAc (3x100 mL). The organic layers were combined, dried ($Na_2SO_4$) and the solvent removed *in vacuo* to afford 1,5,6,7-tetrahydro-4*H*-benzo[*d*][1,2,3]triazol-4-one as an off white solid (220 mg, 35%). **Data in table 2.**

### Typical procedure 19, exemplified by the preparation of Intermediate 16, 1,5,6,7-tetrahydro-4*H*-[1,2,3]triazolo[4,5-*c*]pyridin-4-one

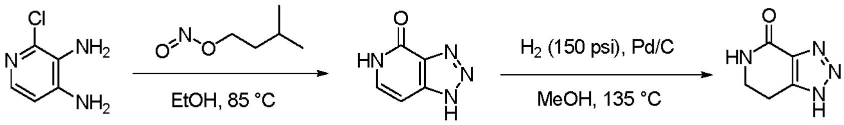


Step 1. *iso*-Amyl nitrite (1.41 mL, 10.48 mmol) was added to a solution of 2-chloropyridine-3,4-diamine (300 mg, 2.09 mmol) in EtOH (5 mL). The reaction mixture was heated at 85 °C for 16 h. The solvent was removed *in vacuo* to afford 1,5-dihydro-4*H*-[1,2,3]triazolo[4,5-c]pyridin-4-one as a brown solid (310 mg crude). The crude product was used in the next step without further purification.

**MS (Method 1):** m/z 134.9 (ES-)

**$^1$H NMR:** (400 MHz, DMSO-$d_6$) δ: 11.46 (bs, 1H), 7.30-7.21 (m, 1H), 6.65-6.58 (m, 1H). 1 exchangeable proton not observed.

Step 2. 10% Pd/C (50% w/w, 400 mg) was slowly added to a mixture of 1,5-dihydro-4*H*-[1,2,3]triazolo[4,5-*c*]pyridin-4-one (300 mg, 2.20 mmol) in MeOH (35 mL) and the reaction mixture was heated at 135 °C in a $H_2$ atmosphere (150 psi) for 16 h. The reaction mixture was filtered through Celite which was washed with MeOH (25 mL). The solvent was removed *in vacuo* to afford 1,5,6,7-tetrahydro-4*H*-[1,2,3]triazolo[4,5-c]pyridin-4-one as an off white solid (200 mg, crude). **Data in table 2.**

**Typical procedure 20, exemplified by the preparation of Intermediate 43, ethyl 5-(2-((*tert*-butoxycarbonyl)amino)ethoxy)-1*H*-pyrazole-4-carboxylate**

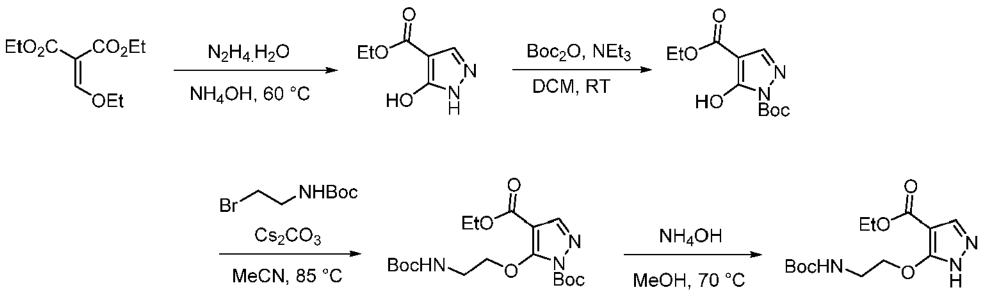


Step 1. Hydrazine hydrate (2.5 g, 48 mmol) was added to a suspension of diethyl 2-(ethoxymethylene)malonate (8.5 g, 40 mmol) in 25% aqueous ammonium hydroxide solution (11 mL, 320 mmol) at RT and the reaction mixture was heated at 60 °C for 4 h. The reaction mixture was diluted with water (200 mL), adjusted pH~7 with 1.5 N HCl (30 mL) and extracted with EtOAc (500 mL). The organic layer was separated, washed with brine solution (100 mL), dried ($Na_2SO_4$) and the solvent removed *in vacuo* to afford ethyl 5-hydroxy-1*H*-pyrazole-4-carboxylate as an off-white solid (3 g, 48%).

**LCMS (Method 7):** m/z 157.2 (ES+), at 0.43 min.

**$^1$H NMR:** (400 MHz, DMSO-$d_6$) δ: 12.22 (s, 1H), 10.19 (s, 1H), 7.85 (s, 1H), 4.14 (q, *J* = 7.2 Hz, 2H), 1.23 (t, *J* = 6.8 Hz, 3H).

Step 2. $NEt_3$ (1 g, 10.25 mmol) was added to a stirred solution of ethyl 5-hydroxy-1*H*-pyrazole-4-carboxylate (1.6 g, 10.3 mmol) in DCM (30 mL) followed by the addition of Boc anhydride (2.23 g, 10.25 mmol) and the reaction mixture was stirred at RT for 4 h. The solvent was removed *in vacuo* and the residue was triturated with $Et_2O$ (2x30 mL), and dried *in vacuo* to afford 1-(*tert*-butyl) 4-ethyl 5-hydroxy-1*H*-pyrazole-1,4-dicarboxylate as a yellow solid (1.9 g, 73%).

**LCMS (Method 7):** m/z 201.0 (ES+), at 1.85 min.

**$^1$H NMR:** (400 MHz, DMSO-$d_6$) δ: 11.69 (s, 1H), 8.40 (s, 1H), 4.20 (q, *J* = 6.8 Hz, 2H), 1.56 (s, 9H), 1.26 (t, *J* = 6.8 Hz, 3H).

Step 3. $Cs_2CO_3$ (127 mg, 0.39 mmol) was added to a stirred solution of 1-(*tert*-butyl) 4-ethyl 5-hydroxy-1*H*-pyrazole-1,4-dicarboxylate (100 mg, 0.39 mmol) in MeCN (2 mL) followed by the addition of *tert*-butyl (2-bromoethyl) carbamate (87 mg, 0.39 mmol) at RT and the reaction mixture was heated at 85 °C for 4 h. The reaction mixture was diluted with water (20 mL) and extracted with EtOAc (40 mL). The organic layer was separated, washed with brine solution (20 mL), dried ($Na_2SO_4$) and the solvent removed *in vacuo*. The residue was purified by gradient flash column chromatography eluting with 0-40% EtOAc in pet-ether to afford 1-(*tert*-butyl) 4-ethyl 5-(2-((tert-butoxycarbonyl)amino)ethoxy)-1*H*-pyrazole-1,4-dicarboxylate as a white solid (50 mg, 32%).

**LCMS (Method 7):** m/z 300.1 (ES+), at 2.33 min.

**$^1$H NMR:** (400 MHz, $CDCl_3$) δ: 8.37 (s, 1H), 5.13 (s, 1H), 4.45 (t, *J* = 4.8 Hz, 2H), 4.36-4.31 (m, 2H), 3.59 (d, *J* = 1.2

Hz, 2H), 1.57 (s, 9H), 1.49 (s, 9H), 1.39 (t, *J* = 7.2 Hz, 3H).

Step 4. $NH_4OH$ solution (2 mL) was added to a stirred solution of 1-(*tert*-butyl) 4-ethyl 5-(2-((*tert*-butoxycarbonyl) amino)ethoxy)-1*H*-pyrazole-1,4-dicarboxylate (50 mg, 0.13 mmol) in MeOH (2 mL) and the reaction mixture was heated at 70 °C for 3 h. The reaction mixture was diluted with water (20 mL) and extracted with EtOAc (40 mL). The organic layer was separated, washed with brine solution (20 mL), dried ($Na_2SO_4$) and the solvent removed *in vacuo* to afford ethyl 5-(2-((*tert*-butoxycarbonyl)amino)ethoxy)-1*H*-pyrazole-4-carboxylate as a white solid (35 mg, 93%). **Data in table 2.**

**Preparation of substituted benzyl halide intermediates**

**Typical procedure 21, exemplified by the preparation of Intermediate 22, 1-(chloromethyl)-3-cyclopropyl-5-fluorobenzene**

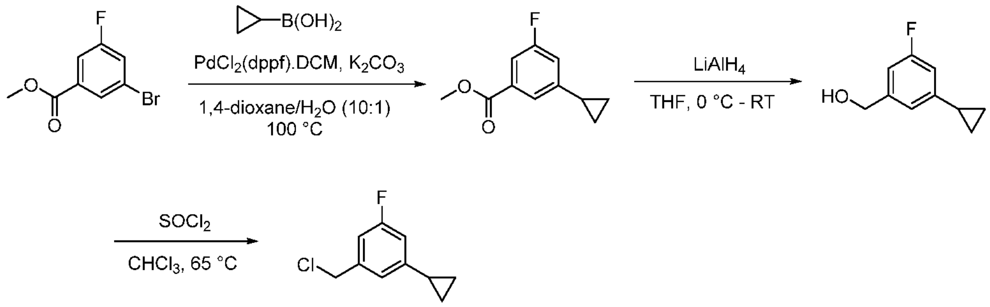


Step 1. $K_2CO_3$ (1.0 g, 12.87 mmol) was added to a stirred solution of methyl 3-bromo-5-fluorobenzoate (1.0 g, 4.29 mmol) and cyclopropylboronic acid (401 mg, 4.72 mmol) in 1,4-dioxane (10 mL) and water (1 mL). The reaction mixture was degassed with $N_2$ for 20 min then Pd(dppf)$_2$Cl$_2$.DCM complex (350 mg, 0.429 mmol) was added. The reaction mixture was heated at 100 °C for 12 h. The reaction mixture was partitioned between water (200 mL) and EtOAc (80 mL). The organic layer was separated, dried ($Na_2SO_4$) and the solvent removed *in vacuo.* The residue was purified by flash column chromatography eluting with 0-50% EtOAc in hexane to afford methyl 3-cyclopropyl-5-fluorobenzoate as a yellow liquid (750 mg, 90%).

**GCMS (Method 2):** m/z 194.0 (ES+), at 3.43 min.

**$^1$H NMR:** (400 MHz, DMSO-$d_6$) δ: 7.54 (t, *J* = 1.6 Hz, 1H), 7.46-7.45 (m, 1H), 7.24-7.23 (m, 1H), 3.86 (s, 3H), 2.11-2.09 (m, 1H), 1.05-1.04 (m, 2H), 0.79-0.78 (m, 2H).

Step 2. $LiAlH_4$ (1.0 M in THF, 7.72 mL, 7.72 mmol) was added to a stirred solution of methyl 3-cyclopropyl-5-fluorobenzoate (1 g, 5.15 mmol) in THF (10 mL) at 0 °C and the reaction mixture was stirred at RT for 3 h. The reaction mixture was neutralized with 1.5 N HCl (25 mL) to pH~7. The reaction mixture was partitioned between water (50 mL) and EtOAc (50 mL). The organic layer was separated, dried ($Na_2SO_4$) and the solvent removed *in vacuo* to afford (3-cyclopropyl-5-fluorophenyl) methanol as a colourless liquid (810 mg, 94%).

**GCMS (Method 1):** m/z 166.1 (ES+), at 7.15 min.

**$^1$H NMR:** (400 MHz, DMSO-$d_6$) δ: 6.88-6.87 (m, 2H), 6.76-6.75 (m, 1H), 5.25 (t, *J* = 5.6 Hz, 1H), 4.46-4.45 (m, 2H), 1.92 (d, *J* = 5.6 Hz, 1H), 0.98-0.97 (m, 2H), 0.71-0.70 (m, 2H).

Step 3. Thionyl chloride (1.3 mL, 18.07 mmol) was added to a stirred solution of (3-cyclopropyl-5-fluorophenyl) methanol (300 mg, 1.80 mmol) in chloroform (10 mL) at RT and the reaction mixture was heated at 65 °C for 12 h. The reaction mixture was basified with 10% $NaHCO_3$ solution (20 mL) to pH~7 and partitioned between water (50 mL) and EtOAc (50 mL). The organic layer was separated, dried ($Na_2SO_4$) and the solvent removed *in vacuo* to afford 1-(chloromethyl)-3-cyclopropyl-5-fluorobenzene as a brown liquid (300 mg, crude). The crude product was used in the next step without further purification. **Data in table 2.**

**Intermediate 31, 1-chloro-5-(chloromethyl)-2-fluoro-3-(trifluoromethyl)benzene**

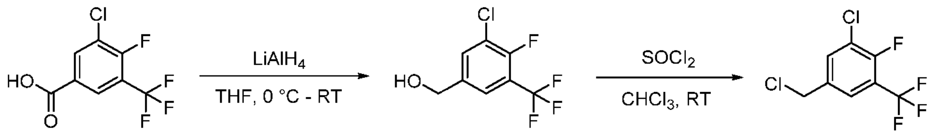


The title compound (70 mg, crude) was prepared in two steps from 3-chloro-4-fluoro-5-(trifluoromethyl)benzoic acid (0.5 g, 2.06 mmol) and $LiAlH_4$ (2 M in THF, 1.5 mL, 3.1 mmol) in THF (10 mL) stirred at RT for 1 h; and thionyl chloride (0.03 mL, 0.43 mmol) in chloroform (5 mL) stirred at RT for 1 h using the methods of Intermediate 22, steps 2 and 3. After completion of step 2, the title compound was isolated as an off-white solid by quenching with 10% $NaHCO_3$ solution (50 mL) and then extraction with DCM (50 mL). The organic layer was separated, dried ($Na_2SO_4$) and the solvent removed *in vacuo.* **Data in table 2.**

**Typical procedure 22, exemplified by the preparation of Intermediate 24, 4-(chloromethyl)-1-(difluoromethoxy)-2-fluorobenzene**

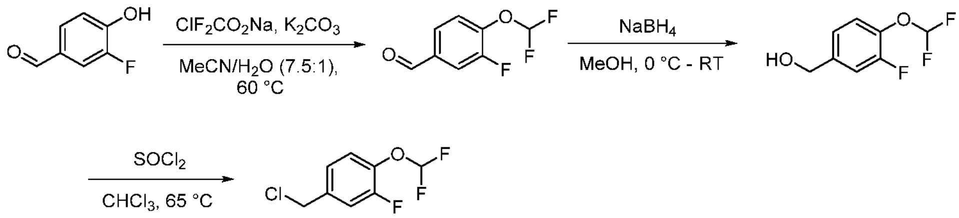


Step 1. $K_2CO_3$ (2.95 g, 21.42 mmol) was added to a stirred solution of 3-fluoro-4-hydroxybenzaldehyde (1 g, 7.14 mmol) in MeCN:$H_2O$ (15 mL:2 mL) at RT, followed by the addition of sodium 2-chloro-2,2-difluoroacetate (1.2 g, 8.57 mmol) and the reaction mixture was heated at 60 °C for 12h. The solvent was removed *in vacuo* and the residue was partitioned between water (20 mL) and EtOAc (50 mL). The organic layer was separated, washed with brine solution (20 mL), dried ($Na_2SO_4$) and the solvent removed *in vacuo.* The residue was purified by gradient flash column chromatography eluting with 0-100% EtOAc in pet-ether to afford 4-(difluoromethoxy)-3-fluorobenzaldehyde as a colourless liquid (400 mg, 30%).

**GCMS (Method 2):** m/z 190.0 (ES+), at 2.19 min.

**$^1$H NMR:** (400 MHz, DMSO-$d_6$) $\delta$: 9.97 (d, *J* = 2.0 Hz, 1H), 7.93-7.85 (m, 2H), 7.64-7.60 (m, 1H), 7.45-7.26 (m, 1H).

Step 2. $NaBH_4$ (160 mg, 4.21 mmol) was added slowly to a suspension of 4-(difluoromethoxy)-3-fluorobenzaldehyde (400 mg, 2.10 mmol) in MeOH (10 mL) at 0 °C and the reaction mixture was stirred at RT for 3 h. The reaction mixture was quenched with ice water (10 mL), the organic layer was separated and the solvent removed *in vacuo.* The residue was partitioned between water (30 mL) and EtOAc (50 mL). The organic layer was separated, washed with brine solution (20 mL), dried ($Na_2SO_4$) and the solvent removed *in vacuo.* The residue was purified by gradient flash column chromatography eluting with 0-100% EtOAc in pet-ether gradient to afford (4-(difluoromethoxy)-3-fluorophenyl)methanol as a colourless liquid (260 mg, 64%).

**GCMS (Method 2):** m/z 192.0 (ES+), at 2.63 min.

**$^1$H NMR:** (400 MHz, DMSO-$d_6$) $\delta$: 7.34-7.29 (m, 2H), 7.21-7.03 (m, 2H), 5.37-5.34 (m, 1H), 4.50 (d, *J* = 4.4 Hz, 2H).

Step 3. $SOCl_2$ (798 mg, 6.77 mmol) was added to a stirred solution of (4-(difluoromethoxy)-3-fluorophenyl) methanol (260 mg, 1.35 mmol) in $CHCl_3$ (15 mL) and the reaction mixture was heated at 65 °C for 12 h. The reaction mixture was partitioned between sat. $NaHCO_3$ solution (50 mL) and DCM (50 mL). The organic layer was separated, and the aqueous layer was extracted with DCM (50 mL). The combined organic layers were dried ($Na_2SO_4$) and the solvent removed *in vacuo* to afford 4-(chloromethyl)-1-(difluoromethoxy)-2-fluorobenzene as a colourless liquid (210 mg, crude). The crude product was used in the next step without further purification. **Data in table 2.**

**Typical procedure 23, exemplified by the preparation of Intermediate 25, 5-(bromomethyl)-1,2-difluoro-3-(trifluoromethyl)benzene**

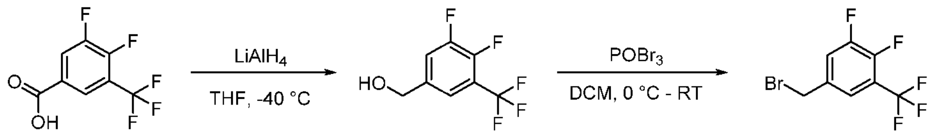


Step 1. $LiAlH_4$ (2 M in THF, 1.6 mL, 3.32 mmol) was slowly added dropwise to a suspension of 3,4-difluoro-5-(trifluoromethyl)benzoic acid (250 mg, 1.11 mmol) in THF (20 mL) at -40 °C and the reaction mixture was stirred at -40 °C for 3 h. The reaction mixture was quenched with sat. $NH_4Cl$ solution (50 mL) and the aqueous layer was extracted with EtOAc (2x100 mL). The organic layer was separated, washed with brine solution (100 mL), dried ($Na_2SO_4$) and the solvent removed *in vacuo.* The residue was purified by gradient flash column chromatography eluting with 0-60% EtOAc in petether to afford (3,4-difluoro-5-(trifluoromethyl)phenyl)methanol as a colourless liquid (130 mg, 56%).
**GCMS (Method 1):** m/z 212.0 (ES+), at 2.19 min.
**$^1$H NMR:** (400 MHz, DMSO-$d_6$) δ: 7.75-7.71 (m, 1H), 7.56-7.41 (m, 1H), 5.58 (t, *J* = 5.6 Hz, 1H), 4.60 (d, *J* = 5.6 Hz, 2H).
Step 2. $POBr_3$ (526 mg, 1.81 mmol) was added dropwise to a suspension of (3,4-difluoro-5-(trifluoromethyl) phenyl)methanol (130 mg, 0.613 mmol) in DCM (15 mL) at 0 °C and the reaction mixture was stirred at RT for 12 h. The reaction mixture was diluted with ice cold water (50 mL) and neutralised with sat. $NaHCO_3$ solution (40 mL) up to pH~7. The organic layer was separated and the aqueous layer extracted with DCM (2x50 mL). The combined organic layers were dried ($Na_2SO_4$) and the solvent removed *in vacuo* to afford 5-(bromomethyl)-1,2-difluoro-3-(trifluoromethyl)benzene as a brown gum (140 mg, crude). The crude product was used in the next step without further purification. **Data in table 2.**

**Typical procedure 24, exemplified by the preparation of Intermediate 26, 1-(chloromethyl)-3-(difluoromethyl)-5-fluorobenzene**

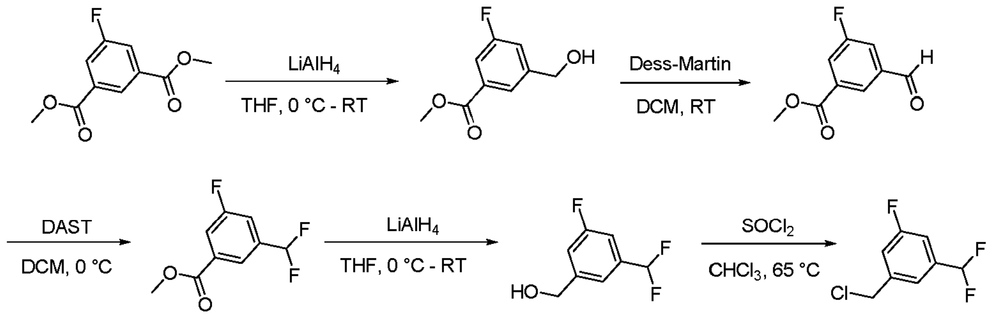


Step 1. $LiAlH_4$ (1.0 M in THF, 7.0 mL, 7.0 mmol) was added to a stirred solution of dimethyl 5-fluoroisophthalate (3 g, 14.1 mmol) in THF (10 mL), at 0 °C and the reaction mixture was stirred at RT for 3h. The reaction mixture was neutralized with 1.5 N HCl (50 mL) up to pH~7, and the reaction mixture was partitioned between water (100 mL) and EtOAc (50 mL). The organic layer was separated, dried ($Na_2SO_4$) and the solvent removed *in vacuo* to afford methyl 3-fluoro-5-(hydroxymethyl)benzoate as a colourless liquid (1.12 g. 43%).
**GCMS (Method 1):** m/z 184.0 (ES+), at 7.34 min.
**$^1$H NMR:** (400 MHz, DMSO-$d_6$) δ: 7.79 (s, 1H), 7.55 (d, *J* = 12.8 Hz, 1H), 7.43 (d, *J* = 12.8 Hz, 1H), 5.49 (t, *J* = 7.6 Hz, 1H), 4.58 (d, *J* = 7.6 Hz, 2H), 3.87 (d, *J* = 2.4 Hz, 3H).
Step 2. Dess-Martin periodinane (2.3 g, 5.54 mmol) was added to a solution of methyl 3-fluoro-5-(hydroxymethyl) benzoate (510 mg, 2.77 mmol) in DCM (10 mL) and the reaction mixture was stirred at RT for 2h. The reaction mixture was filtered and the filtrate was concentrated *in vacuo.* The residue was purified by gradient flash column chromatography eluting with 0-30% EtOAc in hexane to afford methyl 3-fluoro-5-formylbenzoate as a white solid (410 mg, 81%).
**GCMS (Method 1):** m/z 182.0 (ES+), at 6.76 min.
**$^1$H NMR:** (400 MHz, DMSO-$d_6$) δ: 10.08 (d, *J* = 2.4 Hz, 1H), 8.33 (d, *J* = 1.6 Hz, 1H), 8.05-8.04 (m, 2H), 3.92 (s, 3H).
Step 3. DAST (0.44 mL, 3.37 mmol) was added to a solution of methyl 3-fluoro-5-formylbenzoate (410 mg, 2.25 mmol) at 0 °C and the reaction mixture was stirred at RT for 2h. The reaction mixture was neutralized with 10% $NaHCO_3$ (20 mL) up to pH~7, and reaction mixture was partitioned between water (100 mL) and DCM (50 mL). The organic layer was separated, dried ($Na_2SO_4$) and the solvent removed *in vacuo.* The residue was purified by gradient flash column chromatography eluting with 0-30% EtOAc in hexane to afford methyl 3-(difluoromethyl)-5-fluorobenzoate as a colourless

liquid (400 mg, 87%).

**GCMS (Method 2):** m/z 204.0 (ES+), at 2.36 min.

**$^1$H NMR:** (400 MHz, DMSO-$d_6$) δ: 7.99 (s, 1H), 7.89 (d, *J* = 11.2 Hz, 1H), 7.80 (d, *J* = 11.2 Hz, 1H), 7.35-6.98 (m, 1H), 3.91 (s, 3H).

Step 4. $LiAlH_4$ (2.0 M in THF, 0.45 mL, 0.90 mmol) was added to a solution of methyl 3-(difluoromethyl)-5-fluorobenzoate (390 mg, 1.81 mmol) in THF (10 mL) at 0 °C and the reaction mixture was stirred at RT for 1h. The reaction mixture was neutralized with 1.5 N HCl (50 mL) up to pH~7 and then partitioned between water (100 mL) and EtOAc (50 mL). The organic layer was separated, dried ($Na_2SO_4$) and the solvent removed *in vacuo* to afford (3-(difluoromethyl)-5-fluorophenyl) methanol as a colourless liquid (230 mg, 72%).

**GCMS (Method 2):** m/z 176.0 (ES+), at 6.36 min.

**$^1$H NMR:** (400 MHz, DMSO-$d_6$) δ: 7.39 (s, 1H), 7.32-7.29 (m, 3H), 5.46 (d, *J* = 6.4 Hz, 1H), 4.57 (t, *J* = 6.4 Hz, 2H).

Step 5. Thionyl chloride (3 mL, 43.2 mmol) was added to a solution of (3-(difluoromethyl)-5-fluorophenyl) methanol (170 mg, 0.96 mmol) in chloroform (10 mL) at RT and the reaction mixture was heated at 65 °C for 12h. The reaction mixture was neutralized with 10% $NaHCO_3$ (20 mL) up to pH~7, then partitioned between water (50 mL) and EtOAc (50 mL). The organic layer was separated, dried ($Na_2SO_4$) and the solvent removed *in vacuo* to afford 1-(chloromethyl)-3-(difluoromethyl)-5-fluorobenzene as a colourless liquid (170 mg, crude). The crude product was used in the next step without further purification. **Data in table 2.**

**Typical procedure 25, exemplified by the preparation of Intermediate 27, 1-chloro-5-(chloromethyl)-2,3-difluorobenzene**

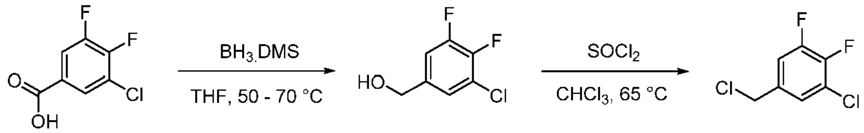


Step 1. Borane dimethyl sulfide complex (0.58 mL, 6.25 mmol) was added to a solution of 3-chloro-4,5-difluorobenzoic acid (400 mg, 2.08 mmol) in THF (6 mL) at 50 °C and the reaction mixture was heated at 70 °C for 2 h. The reaction mixture was cooled to 0 °C, quenched with methanol and stirred at RT for 12 h. The solvent was removed *in vacuo.* The residue was purified by gradient flash column chromatography eluting with 0-30% EtOAc in hexane to afford (3-chloro-4,5-difluorophenyl)methanol as a pink liquid (237 mg, 63%).

Step 2. Thionyl chloride (1.8 mL, 25.2 mmol) was added to a solution of (3-chloro-4,5-difluorophenyl)methanol (100 mg, 0.56 mmol) in chloroform (5 mL) and the reaction mixture was heated at 65 °C for 12 h. The reaction mixture was neutralized with 10% $NaHCO_3$ solution (20 mL) up to pH~7, and then partitioned between water (50 mL) and DCM (50 mL). The organic layer was separated, dried ($Na_2SO_4$) and the solvent removed *in vacuo* to afford 1-chloro-5-(chloromethyl)-2,3-difluorobenzene as a colourless liquid (90 mg, crude). The crude product was used in the next step without further purification. **Data in table 2.**

**Intermediate 28, 5-(chloromethyl)-1,3-difluoro-2-(trifluoromethyl)benzene**

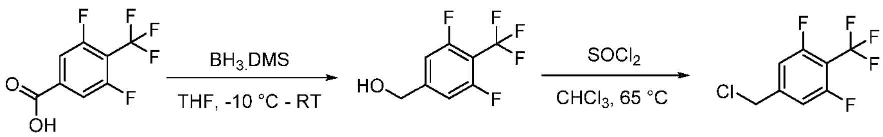


The title compound (128 mg, 25%) was prepared in two steps from 3,5-difluoro-4-(trifluoromethyl)benzoic acid (500 mg, 2.21 mmol) and borane dimethyl sulfide complex (1 M in THF, 11.1 mL, 11.1 mmol) in THF (50 mL) stirred at RT for 12 h; and thionyl chloride (130 μL, 1.76 mmol) in chloroform (10 mL) heated at 70 °C for 12 h using the methods of Intermediate 27. After completion of step 2, the title compound was isolated as a brown oil by neutralization with 10% $NaHCO_3$ solution (20 mL) up to pH~7, and then partitioning between water (50 mL) and DCM (50 mL). The organic layer was separated, dried ($Na_2SO_4$) and the solvent removed *in vacuo.* **Data in table 2.**

**Intermediate 34, 5-(chloromethyl)-2-(difluoromethoxy)pyridine**

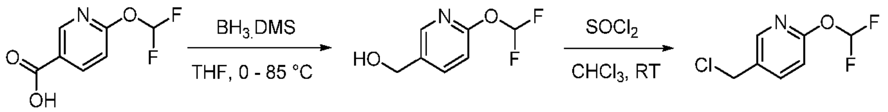


The title compound (140 mg, 54%) was prepared in two steps from 6-(difluoromethoxy)nicotinic acid (250 mg, 1.32 mmol) and borane dimethyl sulfide complex (2 M in THF, 3.3 mL, 6.6 mmol) in THF (2 mL) heated at 85 °C for 16 h; and thionyl chloride (0.2 mL, 2.4 mmol) in chloroform (2 mL) stirred at RT for 3 h using the methods of Intermediate 27. After completion of step 2, the title compound was isolated as a colourless liquid by quenching with ice cold water (50 mL) and extraction with DCM (2x50 mL). The combined organic layers were washed with brine solution (100 mL), dried ($Na_2SO_4$) and the solvent removed *in vacuo.* **Data in table 2.**

**Typical procedure 26, exemplified by the preparation of Intermediate 29, 1-(chloromethyl)-3-fluoro-5-isopropylbenzene**

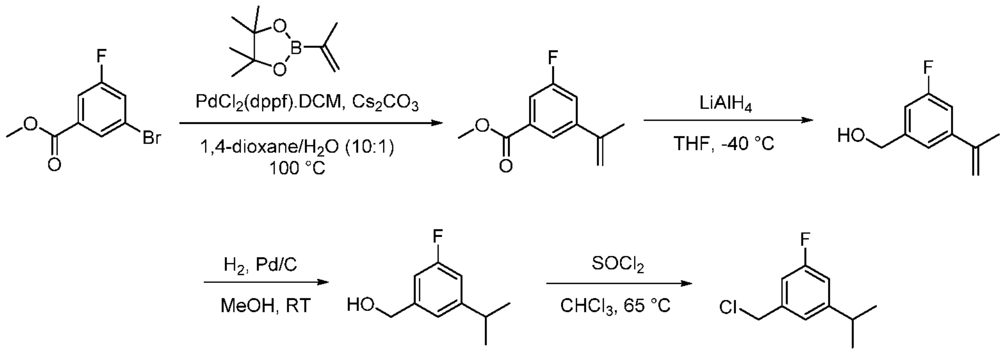


Step 1. In a sealed tube, $Cs_2CO_3$ (1.39 g, 4.29 mmol), Pd(dppf)$Cl_2$.DCM (175 mg, 0.214 mmol) and 4,4,5,5-tetramethyl-2-(prop-1-en-2-yl)-1,3,2-dioxaborolane (541 mg, 3.21 mmol) were added to a degassed suspension of methyl 3-bromo-5-fluorobenzoate (500 mg, 2.145 mmol) in 1,4-dioxane:$H_2O$ (10 mL:1 mL) and the reaction mixture was heated at 100 °C for 16 h. The reaction mixture was partitioned between water (100 mL) and EtOAc (100 mL). The organic layer was removed and the aqueous layer was extracted with EtOAc (100 mL). The combined organic layers were washed with brine solution (50 mL), dried ($Na_2SO_4$) and the solvent removed *in vacuo.* The residue was purified by gradient flash column chromatography eluting with 0-20% EtOAc in pet-ether to afford methyl 3-fluoro-5-(prop-1-en-2-yl)benzoate as a colourless liquid (380 mg, 91%).

**GCMS (Method 1):** m/z 194.0 (ES+), at 6.89 min.

**$^1$H NMR:** (300 MHz, DMSO-$d_6$) δ: 7.88 (s, 1H), 7.71-7.61 (m, 2H), 5.60 (s, 1H), 5.27 (s, 1H), 3.89 (s, 3H), 2.14 (s, 3H).

Step 2. $LiAlH_4$ (1 M in THF, 2.93 mL, 2.93 mmol) was added dropwise to a suspension of methyl 3-fluoro-5-(prop-1-en-2-yl)benzoate (380 mg, 1.95 mmol) in THF (20 mL) at -40 °C and the reaction mixture was stirred at -40 °C for 3 h. The reaction mixture was quenched with sat. $NH_4Cl$ solution (100 mL) and extracted with EtOAc (2x150 mL). The combined organic layers were washed with brine solution (100mL), dried ($Na_2SO_4$) and the solvent removed *in vacuo.* The residue was purified by gradient flash column chromatography eluting with 0-60% EtOAc in pet-ether to afford (3-fluoro-5-(prop-1-en-2-yl)phenyl) methanol as a colourless liquid (250 mg, 77%).

**GCMS (Method 1):** m/z 166.0 (ES+), at 6.94 min.

**$^1$H NMR:** (400 MHz, DMSO-$d_6$) δ: 7.29 (s, 1H), 7.20-7.16 (m, 1H), 7.07-7.04 (m, 1H), 5.49 (s, 1H), 5.34 (t, $J$ = 5.6 Hz, 1H), 5.16 (s, 1H), 4.52 (d, $J$ = 6.0 Hz, 2H), 2.10 (s, 3H).

Step 3. Pd/C (100 mg) was added to a solution of (3-fluoro-5-(prop-1-en-2-yl)phenyl)methanol (250 mg, 1.50 mmol) in MeOH (25 mL) and the reaction mixture was stirred under a hydrogen atmosphere at RT for 4 h. The reaction mixture was filtered through a celite pad, washed with MeOH (2x20 mL) and the filtrate was concentrated *in vacuo* to afford (3-fluoro-5-isopropylphenyl)methanol as a colourless liquid (230 mg, 91%).

**$^1$H NMR:** (400 MHz, DMSO-$d_6$) δ: 7.03 (s, 1H), 6.94-6.91 (m, 2H), 5.28 (t, $J$ = 6.0 Hz, 1H), 4.48 (d, $J$ = 5.6 Hz, 2H),

2.91-2.88 (m, 1H), 1.20-1.18 (m, 6H).

Step 4. Thionyl chloride (500 μL, 6.84 mmol) was added to a suspension of (3-fluoro-5-isopropylphenyl) methanol (230 mg, 1.36 mmol) in chloroform (10 mL) at 0 °C and the reaction mixture was stirred at RT for 16 h. The reaction mixture was partitioned between 10% $NaHCO_3$ solution (100 mL) and EtOAc (100 mL). The organic layer was removed and the aqueous layer was extracted with EtOAc (100 mL). The combined organic layers were washed with brine solution (50 mL), dried ($Na_2SO_4$) and the solvent removed *in vacuo* to afford 1-(chloromethyl)-3-fluoro-5-isopropylbenzene as a yellow liquid (204 mg, 80%). **Data in table 2.**

**Typical procedure 27, exemplified by the preparation of Intermediate 30, 1-(chloromethyl)-3-fluoro-5-(1-(trifluoromethyl)cyclopropyl)benzene**

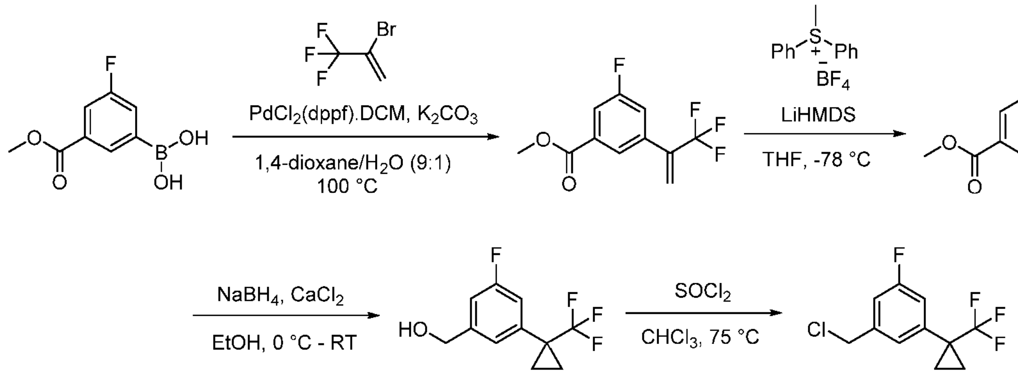


Step 1. $K_2CO_3$ (2.36 g, 17.14 mmol) was added to a solution of 2-bromo-3,3,3-trifluoroprop-1-ene (1.5 g, 8.57 mmol) and (3-fluoro-5-(methoxycarbonyl)phenyl)boronic acid (1.86 g, 9.43 mmol) in 1,4-dioxane:water (18 mL:2 mL). The reaction mixture was degassed with $N_2$ for 10 minutes, followed by the addition of $Pd(dppf)_2Cl_2$.DCM complex (351 mg, 0.43 mmol). The reaction mixture was heated at 100 °C for 16 h. The reaction mixture was filtered through celite pad, washed with DCM (100 mL) and the filtrate was concentrated *in vacuo.* The residue was purified by gradient flash column chromatography eluting with 0-15% EtOAc in pet-ether to afford methyl 3-fluoro-5-(3,3,3-trifluoroprop-1-en-2-yl)benzoate as a colourless liquid (1.2 g, 57%).

**GCMS (Method 2):** m/z 248.0 (ES+), at 2.67 min.

**$^1$H NMR:** (400 MHz, $CDCl_3$) δ: 7.96 (s, 1H), 7.79-7.76 (m, 1H), 7.41-7.37 (m, 1H), 6.10-6.09 (m, 1H), 5.91-5.90 (m, 1H), 3.97 (s, 3H).

Step 2. Diphenyl(methyl)sulfonium tetrafluoroborate (1.39 g, 4.84 mmol) and lithium bis(hexamethyldisilazide) solution (1 M in THF, 9.67 mL, 9.67 mmol) were added sequentially to a solution of methyl 3-fluoro-5-(3,3,3-trifluoroprop-1-en-2-yl)benzoate (0.8 g, 3.22 mmol) in THF (50 mL) at -78 °C. The reaction mixture was stirred at -78 °C for 1 h. The reaction mixture was quenched with sat. $NH_4Cl$ solution (100 mL) and the aqueous layer extracted with EtOAc (2x100 mL). The combined organic layers were dried ($Na_2SO_4$) and the solvent removed *in vacuo.* The residue was purified by gradient flash column chromatography eluting with 0-10 % EtOAc in pet-ether to afford methyl 3-fluoro-5-(1-(trifluoromethyl) cyclopropyl)benzoate as a brown liquid (180 mg, 7%).

**GCMS (Method 2):** m/z 262.0 (ES+), at 3.07 min.

Step 3. $NaBH_4$ (0.121 g, 3.2 mmol) was added portion wise, followed by the addition of a solution of $CaCl_2$ (0.711 g, 6.41 mmol) in EtOH (5mL) drop wise to a solution of methyl 3-fluoro-5-(1-(trifluoromethyl)cyclopropyl)benzoate (0.28 g, 1.06 mmol) in EtOH (5 mL) at 0 °C. The reaction mixture was stirred at RT for 20 h. The reaction mixture was quenched with ice cold water (50 mL), and the aqueous layer extracted with EtOAc (2x100 mL). The combined organic layers were dried ($Na_2SO_4$) and the solvent removed *in vacuo* to afford (3-fluoro-5-(1-(trifluoromethyl)cyclopropyl)phenyl)methanol as a colourless liquid (130 mg, 52%).

**LCMS (Method 3):** m/z No ionisation oberved (ES+), at 2.13 min.

**$^1$H NMR:** (300 MHz, DMSO-$d_6$) δ: 7.28 (s, 1H), 7.16-7.12 (m, 2H), 5.38 (t, *J* = 8.0 Hz, 1H), 4.52 (d, *J* = 8.0 Hz, 2H), 1.35 (t, *J* = 8.0 Hz, 2H), 1.24-1.15 (m, 2H).

Step 4. Thionyl chloride (1.2 mL, 16.6 mmol) was added dropwise to a solution of (3-fluoro-5-(1-(trifluoromethyl) cyclopropyl)phenyl)methanol (0.13 g, 0.55 mmol) in $CHCl_3$ (20 mL), at 0 °C and the reaction mixture was heated at 75 °C for 48 h. The solvent was removed *in vacuo* to afford 1-(chloromethyl)-3-fluoro-5-(1-(trifluoromethyl)cyclopropyl)benzene as a brown liquid (150 mg, crude). The crude product was used in the next step without further purification. **Data in table 2.**

**Intermediate 32, 5-(chloromethyl)-3-fluoro-2-methoxypyridine**

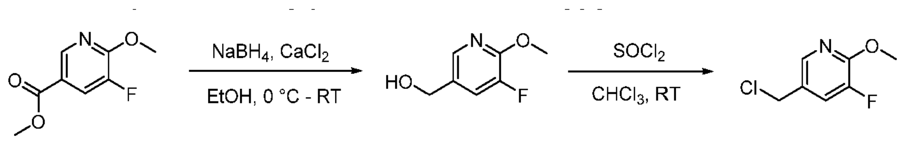


The title compound (294 mg, 62%) was prepared in two steps from methyl 5-fluoro-6-methoxynicotinate (0.5 g, 2.6 mmol) and $NaBH_4$ (0.15 g, 4.04 mmol) in EtOH (5 mL) and $CaCl_2$ (0.44 g, 4.04 mmol) in EtOH (5 mL) stirred at RT for 1 h; and thionyl chloride (1.13 g, 9.54 mmol) in chloroform (10 mL) stirred at RT for 1 h using the methods of Intermediate 30, steps 3 and 4. After completion of step 2, the title compound was isolated as a colourless gum by quenching the reaction mixture with 10 % $NaHCO_3$ solution (50 mL) and extraction with DCM (50 mL). The organic layer was separated, dried ($Na_2SO_4$) and the solvent removed *in vacuo.* The residue was purified by gradient flash column chromatography eluting with 0-10% EtOAc in pet-ether gradient. **Data in table 2.**

**Intermediate 33, 5-(chloromethyl)-2-methoxypyridine**

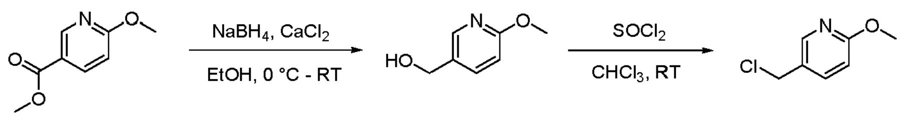


The title compound (180 mg, 75%) was prepared in two steps from methyl 6-methoxynicotinate (0.5 g, 2.99 mmol), $NaBH_4$ (0.37 g, 8.97 mmol) and $CaCl_2$ (0.49 g 4.48 mmol) in EtOH (30 mL) stirred at RT for 16 h; and thionyl chloride (0.52 mL, 7.18 mmol) in chloroform (20 mL) stirred at RT for 2 h using the methods of Intermediate 30, steps 3 and 4. After completion of step 2, the title compound was isolated as a colourless liquid by quenching the reaction mixture with 10% $NaHCO_3$ solution (50 mL) and extraction with DCM (2x50 mL). The combined organic layers washed with brine solution (50 mL), dried ($Na_2SO_4$) and the solvent removed *in vacuo.* **Data in table 2.**

**SYNTHESIS OF EXAMPLES**

**Typical procedures for the preparation of examples, as exemplified by the preparation of the below examples in Procedures 1 - 27.**

**Procedure 1:**

**Example 1, 1-(2-(3-fluoro-5-(trifluoromethyl)benzyl)pyridin-4-yl)-1,5,6,7-tetrahydro-4*H*-pyrazolo[4,3-*c*]pyridin-4-one**

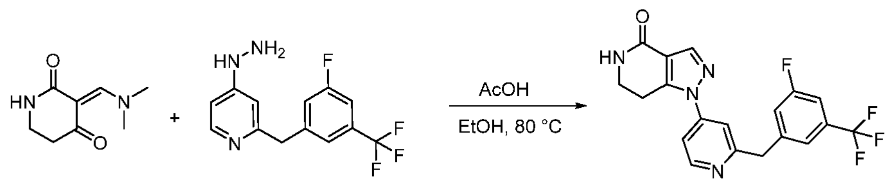


A catalytic amount of acetic acid (1 mL) was added to a solution of 2-(3-fluoro-5-(trifluoromethyl)benzyl)-4-hydrazineylpyridine (Intermediate 6, 5 g, 17.5 mmol) in EtOH (250 mL) at RT, followed by the addition of (*E*)-3-((dimethylamino)methylene)piperidine-2,4-dione (Intermediate 1, 3.5 g, 21.1 mmol) and the reaction mixture was heated at 80 °C for 16 h. The solvent was removed *in vacuo* and the residue was partitioned between EtOAc (100 mL) and sat. $NaHCO_3$ solution (100 mL). The organic layer was separated, dried ($Na_2SO_4$) and the solvent removed *in vacuo.* The residue was purified by gradient flash chromatography eluting with 0-10% MeOH in DCM to afford 1-(2-(3-fluoro-5-(trifluoromethyl)benzyl)pyridin-4-yl)-1,5,6,7-tetrahydro-4*H*-pyrazolo[4,3-c]pyridin-4-one as an off-white solid (2.0 g, 30%). **Data in table 3.**

**Procedure 2:**

**Example 3, 5-methyl-1-(4-(3-(trifluoromethyl)benzyl)pyridin-2-yl)-1,5,6,7-tetrahydro-4*H*-pyrazolo[4,3-*c*]pyridin-4-one**

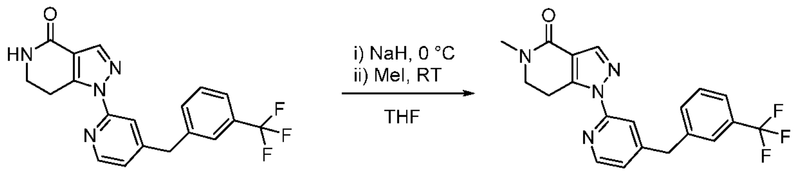


NaH (60% in mineral oil, 21 mg, 0.53 mmol) was added to a solution of 1-(4-(3-(trifluoromethyl)benzyl)pyridin-2-yl)-1,5,6,7-tetrahydro-4*H*-pyrazolo[4,3-*c*]pyridin-4-one (Example 2, 100 mg, 0.26 mmol) in THF (4 mL) at 0 °C. After stirring for 30 min, MeI (0.03 mL, 0.53 mmol) in THF (1 mL) was slowly added and the reaction mixture stirred at RT for 1 h. The reaction mixture was quenched with water (15 mL) and the aqueous layer was extracted with EtOAc (3x15 mL). The combined organic layers were dried ($Na_2SO_4$) and the solvent removed *in vacuo.* The residue was purified by gradient flash column chromatography eluting with 27-29% EtOAc in hexane to afford 5-methyl-1-(4-(3-(trifluoromethyl)benzyl)pyridin-2-yl)-1,5,6,7-tetrahydro-4*H*-pyrazolo[4,3-*c*]pyridin-4-one as a colourless gel (43 mg, 43%). **Data in table 3.**

**Procedure 3:**

**Example 6, 1-(4-((6-fluoro-5-methylpyridin-3-yl)methyl)pyridin-2-yl)-1,5,6,7-tetrahydro-4*H*-pyrazolo[4,3-*c*]pyridin-4-one**

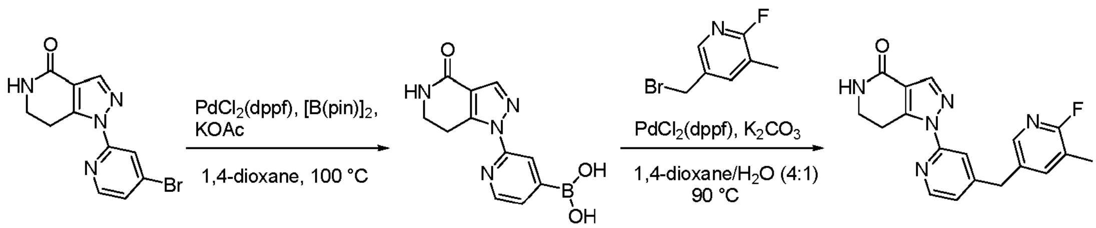


Step 1. bis(Pinacolato)diboron (260 mg, 1.02 mmol) was added to a solution of 1-(4-bromopyridin-2-yl)-1,5,6,7-tetrahydro-4*H*-pyrazolo[4,3-c]pyridin-4-one (Intermediate 12, 200 mg, 0.68 mmol) in 1,4-dioxane (8 mL), the reaction mixture was degassed under argon for 25 min and then KOAc (201 mg, 2.05 mmol) and $PdCl_2$(dppf).DCM (28 mg, 0.03 mmol) were added. The reaction mixture was heated at 100 °C for 16 h. The reaction mixture was filtered through Celite and washed with 5% MeOH in DCM (100mL). The solvent was removed *in vacuo* and the residue was triturated with MTBE (20 mL) to afford (2-(4-oxo-4,5,6,7-tetrahydro-1*H*-pyrazolo[4,3-*c*]pyridin-1-yl)pyridin-4-yl)boronic acid as a brown solid (415 mg, crude). The crude product was used in the next step without further purification.

**MS (Method 1):** m/z 259.0 (ES+).

Step 2. 5-(Bromomethyl)-2-fluoro-3-methylpyridine (200 mg, 0.98 mmol) and $K_2CO_3$ (407 mg, 2.95 mmol) were added to a solution of (2-(4-oxo-4,5,6,7-tetrahydro-1*H*-pyrazolo[4,3-c]pyridin-1-yl)pyridin-4-yl)boronic acid (432 mg, 1.67 mmol) in 1,4-dioxane:$H_2O$ (4:1). After degassing with argon for 25 min, $PdCl_2$(dppf).DCM (40 mg, 0.04 mmol) was added and the reaction mixture was heated at 90 °C for 1 h. The reaction mixture was quenched by the addition of water (100 mL) and the aqueous layer was extracted with EtOAc (3x50 mL). The organic layers were combined, dried ($Na_2SO_4$) and the solvent removed *in vacuo.* The residue was triturated with $Et_2O$, filtered and washed with 2% MeOH in DCM to afford 1-(4-((6-fluoro-5-methylpyridin-3-yl)methyl)pyridin-2-yl)-1,5,6,7-tetrahydro-4*H*-pyrazolo[4,3-*c*]pyridin-4-one as an off white solid (180 mg, 54%). **Data in table 3.**

**Procedure 4:**

**Example 8, 2-(4-(3-(trifluoromethyl)benzyl)pyridin-2-yl)-2,5,6,7-tetrahydro-4*H*-pyrazolo[4,3-*c*]pyridin-4-one**

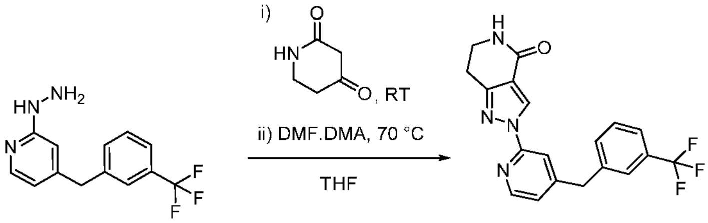


2-Hydrazineyl-4-(3-(trifluoromethyl)benzyl)pyridine (Intermediate 7, 54 mg, 0.2 mmol) in THF (1 mL) was added to a suspension of piperidine-2,4-dione (22 mg, 0.2 mmol) in THF (1 mL) and the reaction mixture stirred at RT for 2 h. DMF-DMA (0.08 mL, 0.6 mmol) was added and the reaction mixture heated at 70 °C for 16 h. The solvent was removed *in vacuo* and the residue was purified by gradient flash chromatography eluting with 0-10% MeOH in DCM. The residue was further purified by prep HPLC (Method 2 - 40-70% gradient) to afford 2-(4-(3-(trifluoromethyl)benzyl)pyridin-2-yl)-2,5,6,7-tetrahydro-4*H*-pyrazolo[4,3-*c*]pyridin-4-one as a white solid (13 mg, 18%). **Data in table 3.**

<u>**Procedure 5:**</u>

**Example 10, 1-(4-(3-fluoro-5-(trifluoromethyl)benzyl)pyridin-2-yl)-5,6,7,8-tetrahydropyrazolo[4,3-*c*]azepin-4(1*H*)-one**

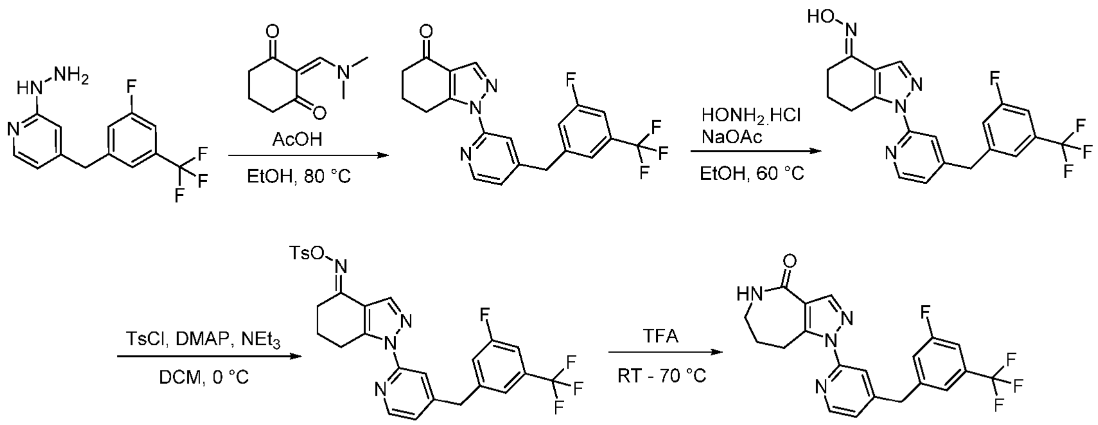


Step 1. 2-((Dimethylamino)methylene)cyclohexane-1,3-dione (Intermediate 2, 3.37 g, 20.2 mmol) and acetic acid (2.7 mL) were added to a suspension of 4-(3-fluoro-5-(trifluoromethyl)benzyl)-2-hydrazineylpyridine (Intermediate 8, 5.82 g, 20.4 mmol) in EtOH (70 mL) and the reaction mixture heated at 80 °C for 3 h. The solvent was removed *in vacuo.* The residue was purified by gradient flash column chromatography eluting with 12-100 % EtOAc in i-hexane and the solvent removed *in vacuo* to afford 1-(4-(3-fluoro-5-(trifluoromethyl)benzyl)pyridin-2-yl)-1,5,6,7-tetrahydro-4*H*-indazol-4-one as a dark orange solid (5.55 g, 71% yield).

**LCMS (Method 4):** m/z 390.4 (ES+), at 1.63 min.

**$^1$H NMR:** (400 MHz, DMSO-$d_6$) δ: 8.46 (dd, *J* = 5.1, 0.7 Hz, 1H), 8.09 (s, 1H), 7.88 (dd, *J* = 1.6, 0.8 Hz, 1H), 7.66 (dt, *J* = 1.6, 0.8 Hz, 1H), 7.63-7.54 (m, 2H), 7.40 (dd, *J* = 5.1, 1.5 Hz, 1H), 4.24 (s, 2H), 3.45-3.36 (m, 2H), 2.49-2.37 (m, 2H), 2.08 (p, *J* = 6.2 Hz, 2H).

Step 2. Hydroxylamine hydrochloride (444 mg, 6.39 mmol) and sodium acetate (524 mg, 6.39 mmol) were added to a suspension of 1-(4-(3-fluoro-5-(trifluoromethyl)benzyl)pyridin-2-yl)-1,5,6,7-tetrahydro-4*H*-indazol-4-one (500 mg, 1.28 mmol) in EtOH (30 mL) and the reaction mixture was heated at 60 °C for 3 h. The reaction mixture was partitioned between water (20 mL) and EtOAc (20 mL) and the organic layer removed. The aqueous layer was extracted with EtOAc (2x20 mL), the combined organic layers were dried (phase separator) and the solvent removed *in vacuo* to afford 1-(4-(3-fluoro-5-(trifluoromethyl)benzyl)pyridin-2-yl)-1,5,6,7-tetrahydro-4*H*-indazol-4-one oxime as a beige semi-solid (497 mg, 96%, mixture of oxime isomers).

**LCMS (Method 4):** m/z 405.4 (ES+), at 1.59 min.

**$^1$H NMR:** (400 MHz, DMSO-$d_6$) δ: 10.74 (s, 0.8H), 10.68 (s, 0.2H), 8.45-8.36 (m, 1H), 8.30 (s, 1H), 7.88 (dd, *J* = 1.6, 0.8 Hz, 0.9H), 7.82 (d, *J* = 1.6 Hz, 0.1H), 7.65 (s, 1H), 7.63-7.52 (m, 1.8H), 7.51-7.43 (m, 0.2H), 7.34 (dd, *J* = 5.1, 1.5 Hz, 0.7H), 7.31 (d, *J* = 5.3 Hz, 0.3H), 4.22 (d, *J* = 3.2 Hz, 2H), 3.24 (t, *J* = 6.1 Hz, 2H), 2.46-2.38 (m, 2H), 1.96-1.87 (m, 2H).

Step 3. DMAP (10.0 mg, 0.08 mmol) was added to a suspension of 1-(4-(3-fluoro-5-(trifluoromethyl)benzyl) pyridin-2-yl)-1,5,6,7-tetrahydro-4*H*-indazol-4-one oxime (487 mg, 1.20 mmol) and triethyl amine (0.5 mL, 3.55 mmol) in DCM (20 mL), and the reaction mixture cooled to 0 °C. To this was added *para*-toluenesulfonyl chloride (273 mg, 1.43 mmol) and the reaction mixture stirred for 2 h at 0 °C. The residue was partitioned between DCM (10 mL) and water (10 mL) and the aqueous layer removed. The organic layer was washed with water (2x10 mL), dried (phase separator) and the solvent removed *in vacuo.* The residue was purified by gradient flash column chromatography eluting with 10-60% EtOAc in *i*-hexane to afford 1-(4-(3-fluoro-5-(trifluoromethyl)benzyl)pyridin-2-yl)-1,5,6,7-tetrahydro-4*H*-indazol-4-one *O*-tosyl oxime as a beige semi-solid (428 mg, 64%).

**LCMS (Method 4):** m/z 599.3 (ES+), at 1.99 min.

**$^1$H NMR:** (400 MHz, DMSO-$d_6$) δ: 8.44 (dd, *J* = 5.1, 0.7 Hz, 0.9H), 8.41 (dd, *J* = 5.1, 0.7 Hz, 0.1H), 8.18 (s, 1H), 7.93-7.82 (m, 3H), 7.69-7.64 (m, 1H), 7.64-7.53 (m, 2H), 7.52-7.46 (m, 2H), 7.40 (dd, *J* = 5.1, 1.5 Hz, 0.9H), 7.35 (dd, *J* = 5.1, 1.5 Hz, 0.1H), 4.25 (s, 1.8H), 4.22 (s, 0.2H), 3.25 (t, *J* = 6.2 Hz, 2H), 2.49-2.44 (m, 2H), 2.42 (s, 3H), 1.96-1.81 (m, 2H).

Step 4. A suspension of 1-(4-(3-fluoro-5-(trifluoromethyl)benzyl)pyridin-2-yl)-1,5,6,7-tetrahydro-4*H*-indazol-4-one O-tosyl oxime (428 mg, 0.77 mmol) in TFA (20 mL) was stirred at RT for 1 h. The reaction mixture was then heated at 70 °C for 16 h. The residue was partitioned between ice and DCM (10 mL) and the organic layer separated. The aqueous layer was washed with DCM (2x10 mL), the combined organic layers dried (phase separator) and the solvent removed *in vacuo.* The residue was purified by gradient flash column chromatography eluting with 2-7% MeOH in DCM to afford 1-(4-(3-fluoro-5-(trifluoromethyl)benzyl)pyridin-2-yl)-5,6,7,8-tetrahydropyrazolo[4,3-c]azepin-4(1*H*)-one as a white solid (218 mg, 70%). **Data in table 3.**

<u>**Procedure 6:**</u>

**Example 13, 1-(4-(3-(trifluoromethyl)benzyl)pyridin-2-yl)-6,7-dihydro-1*H*-pyrazolo[4,3-*f*][1,4]oxazepin-4(5H)-one**

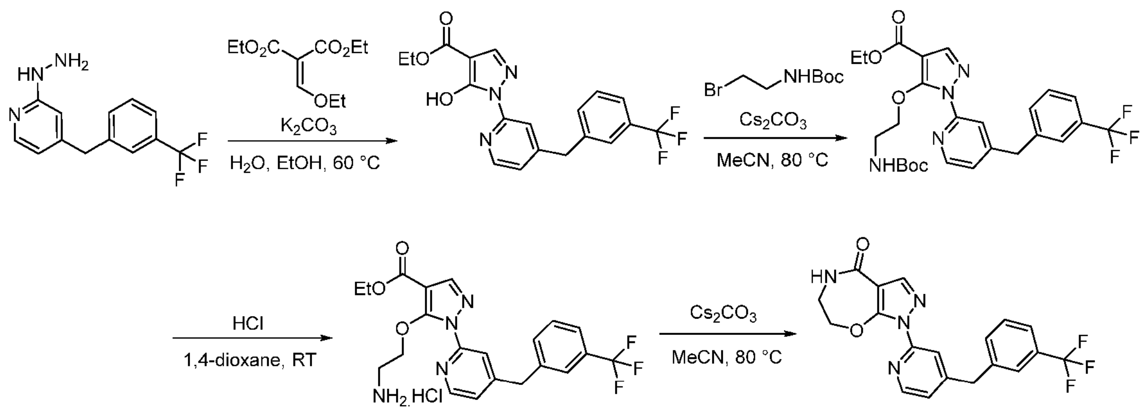


Step 1. $K_2CO_3$ (5.16 g, 37.45 mmol) was added to a solution 2-hydrazineyl-4-(3-(trifluoromethyl)benzyl)pyridine (Intermediate 7, 4 g, 14.98 mmol) and diethyl 2-(ethoxymethylene)malonate (8.08 g, 37.45 mmol) in EtOH:$H_2O$ (85 mL:15 mL) and the reaction mixture heated at 60 °C for 16 h. The reaction mixture was partitioned between water (100 mL) and EtOAc (100 mL). The organic layer was separated, dried ($Na_2SO_4$) and the solvent removed *in vacuo.* The residue was purified by gradient flash chromatography eluting with 0-5% MeOH in DCM to afford ethyl 5-hydroxy-1-(4-(3-(trifluoromethyl)benzyl)pyridin-2-yl)-1*H*-pyrazole-4-carboxylate as a yellow solid (1.5 g, 25%). **LCMS (Method 3):** m/z 392.0 (ES+), at 2.55 min.

**$^1$H NMR:** (400 MHz, DMSO-$d_6$) δ: 8.20 (d, *J* = 4.8 Hz, 2H), 7.63-7.57 (m, 4H), 7.42 (s, 1H), 6.88 (d, *J* = 6.3 Hz, 1H), 4.05-3.96 (m, 4H), 1.17 (t, *J* = 7.1 Hz, 3H). 1 exchangeable proton not observed.

Step 2. $K_2CO_3$ (0.79 g, 5.75 mmol) was added to a solution of ethyl 5-hydroxy-1-(4-(3-(trifluoromethyl)benzyl) pyridin-2-yl)-1*H*-pyrazole-4-carboxylate (1.5 g, 3.83 mmol) and *tert*-butyl (2-bromoethyl)carbamate (1.02 g, 4.59 mmol) in MeCN (30 mL) and the reaction mixture was heated at 80 °C for 16 h. The reaction mixture was partitioned between water (50 mL) and EtOAc (50 mL). The organic layer was separated, dried ($Na_2SO_4$) and the solvent removed *in vacuo* to afford

ethyl 5-(2-((*tert*-butoxycarbonyl)amino)ethoxy)-1-(4-(3-(trifluoromethyl)benzyl)pyridin-2-yl)-1*H*-pyrazole-4-carboxylate as a colourless liquid (500 mg, crude).

**LCMS (Method 3):** m/z 535.0 (ES+), at 3.03 min.

Step 3. HCl solution (4 M in 1,4-dioxane, 0.4 mL) was added to a solution of ethyl 5-(2-((*tert*-butoxycarbonyl) amino)ethoxy)-1-(4-(3-(trifluoromethyl)benzyl)pyridin-2-yl)-1*H*-pyrazole-4-carboxylate (200 mg, 0.374 mmol in 1,4-dioxane (2 mL) at 0 °C and the reaction mixture stirred at RT for 1h. The solvent was removed *in vacuo* to afford ethyl 5-(2-aminoethoxy)-1-(4-(3-(trifluoromethyl)benzyl)pyridin-2-yl)-1*H*-pyrazole-4-carboxylate hydrochloride as a white solid (250 mg, crude).

**LCMS (Method 3):** m/z 435.3 (ES+), at 1.85 min.

Step 4. $Cs_2CO_3$ (169 mg, 0.518 mmol) was added to a solution of ethyl 5-(2-aminoethoxy)-1-(4-(3-(trifluoromethyl)benzyl)pyridin-2-yl)-1*H*-pyrazole-4-carboxylate hydrochloride (150 mg) in MeCN (1.5 mL) and the reaction mixture was heated at 80 °C for 16 h. The reaction mixture was partitioned between water (50 mL) and EtOAc (50 mL). The organic layer was separated, dried ($Na_2SO_4$) and the solvent removed *in vacuo.* The residue was purified by prep HPLC (Method 1) to afford 1-(4-(3-(trifluoromethyl)benzyl)pyridin-2-yl)-6,7-dihydro-1*H*-pyrazolo[4,3-*f*][1,4]oxazepin-4(5*H*)-one as an off-white solid (39 mg, 29%). **Data in table 3.**

**Procedure 7:**

**Example 16, 1-(5-(3-fluoro-5-(trifluoromethyl)benzyl)pyridin-3-yl)-1,5,6,7-tetrahydro-4*H*-pyrazolo[4,3-*c*]pyridin-4-one**

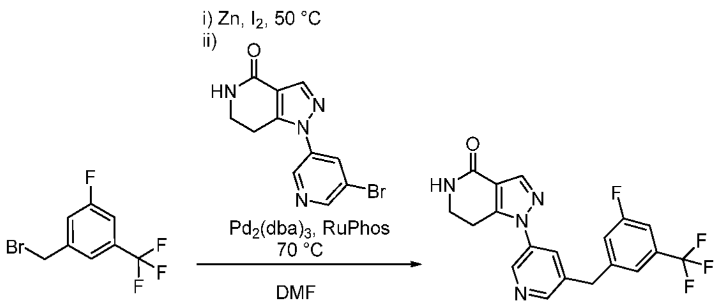


A pinch of iodine was added to a solution of activated zinc (665 mg, 10.2 mmol) in DMF (10 mL) and heated to 50 °C for 5 min followed by the addition of 1-(bromomethyl)-3-fluoro-5-(trifluoromethyl)benzene (526 mg, 2.04 mmol) in DMF (5 mL). The reaction mixture was heated at 50 °C for 1 h and then allowed to cool to RT. The supernatant DMF layer was transferred to a degassed suspension of 1-(5-bromopyridin-3-yl)-1,5,6,7-tetrahydro-4*H*-pyrazolo[4,3-c]pyridin-4-one (Intermediate 11, 200 mg, 0.68 mmol) and RuPhos (63 mg, 0.13 mmol) in DMF (10 mL) followed by the addition of tris(dibenzylideneacetone)dipalladium(0) (63 mg, 0.06 mmol). The reaction mixture was heated to 70 °C for 16 h and then filtered through Celite and washed with EtOAc (2x50 mL). The filtrate was washed with water (2x50 mL). The organic layer was separated, dried ($Na_2SO_4$) and the solvent removed *in vacuo.* The residue was purified by gradient flash chromatography eluting with 0-100% EtOAc in pet-ether to afford 1-(5-(3-fluoro-5-(trifluoromethyl)benzyl)pyridin-3-yl)-1,5,6,7-tetrahydro-4*H*-pyrazolo[4,3-*c*]pyridin-4-one as an off-white solid (170 mg, 64%). **Data in table 3.**

**Procedure 8:**

**Example 18, 1-(4-(3-fluoro-5-(trifluoromethyl)phenoxy)pyridin-2-yl)-1,5,6,7-tetrahydro-4*H*-pyrazolo[4,3-*c*]pyridin-4-one**

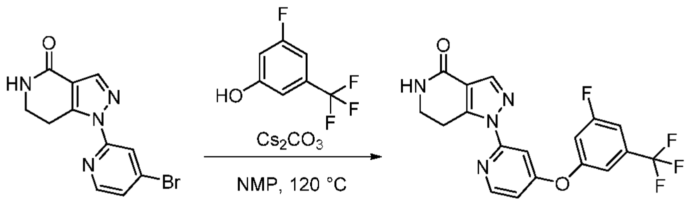


$Cs_2CO_3$ (1.08 g, 3.33 mmol) was added to a solution of 3-fluoro-5-(trifluoromethyl)phenol (200 mg, 1.11 mmol) in NMP (5 mL), the reaction mixture stirred at RT for 10 min followed by the addition of 1-(4-bromopyridin-2-yl)-1,5,6,7-tetrahydro-4*H*-pyrazolo[4,3-*c*]pyridin-4-one (Intermediate 12, 259 mg, 0.88 mmol). The reaction mixture was heated at 120 °C for 16 h. The reaction mixture was poured into water (30 mL) and the precipitate which formed filtered, washed with water (3x20 mL) and dried *in vacuo.* The residue was purified by prep HPLC (Method 3 - 100% isocratic gradient) to afford 1-(4-(3-fluoro-5-(trifluoromethyl)phenoxy)pyridin-2-yl)-1,5,6,7-tetrahydro-4*H*-pyrazolo[4,3-*c*]pyridin-4-one as a white solid (12 mg, 12%). **Data in table 3.**

**Procedure 9:**

**Example 19, 1-(2-(4-chlorobenzyl)pyridin-4-yl)-1,5,6,7-tetrahydro-4*H*-pyrazolo[4,3-c]pyridin-4-one**

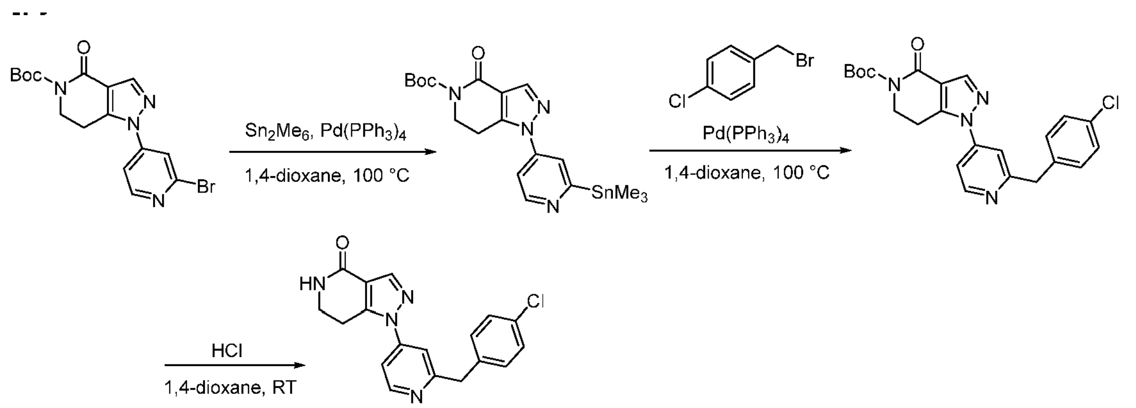


Step 1. Hexamethylditin (1.24 g, 3.81 mmol) was added to a solution of *tert*-butyl 1-(2-bromopyridin-4-yl)-4-oxo-1,4,6,7-tetrahydro-5*H*-pyrazolo[4,3-*c*]pyridine-5-carboxylate (Intermediate 14, 1 g, 2.54 mmol) in 1,4-dioxane (20 mL) and followed by the addition of $Pd(PPh_3)_4$ (293 mg, 0.25 mmol) and the reaction mixture was heated at 100 °C for 2 h. The solvent was removed *in vacuo* afford *tert*-butyl 4-oxo-1-(2-(trimethylstannyl)pyridin-4-yl)-1,4,6,7-tetrahydro-5*H*-pyrazolo[4,3-*c*]pyridine-5-carboxylate as a yellow gum (1.5 g, crude). **LCMS (Method 3):** m/z 479.1 (ES+), at 1.47 min.

Step 2. 1-(Bromomethyl)-4-chlorobenzene (36 mg, 0.18 mmol) was added to a solution of *tert*-butyl 4-oxo-1-(2-(trimethylstannyl)pyridin-4-yl)-1,4,6,7-tetrahydro-5*H*-pyrazolo[4,3-c]pyridine-5-carboxylate (60 mg) in 1,4-dioxane (10 mL) followed by the addition of $Pd(PPh_3)_4$ (14 mg, 0.01 mmol) under argon, then the reaction mixture was heated at 100 °C for 12 h. The solvent was removed *in vacuo* and the residue was purified by gradient flash chromatography eluting with 0-50% EtOAc in pet-ether to afford *tert*-butyl 1-(2-(4-chlorobenzyl)pyridin-4-yl)-4-oxo-1,4,6,7-tetrahydro-5*H*-pyrazolo[4,3-*c*]pyridine-5-carboxylate as a colourless liquid (40 mg, 72%).

**LCMS (Method 3):** m/z 439.2 (ES+), at 2.40 min.

Step 3. HCl solution (4 M in 1,4-dioxane, 5 mL) was added to a solution of *tert*-butyl 1-(2-(4-chlorobenzyl) pyridin-4-yl)-4-oxo-1,4,6,7-tetrahydro-5*H*-pyrazolo[4,3-*c*]pyridine-5-carboxylate (40 mg, 0.09 mmol) in 1,4-dioxane (5 mL) at RTand the reaction mixture was stirred at RT for 2h. The solvent was removed *in vacuo* and the residue was purified by prep HPLC (Method 4). The solvent was removed *in vacuo* and the residue was partitioned between 10% $NaHCO_3$ solution (5 mL) and EtOAc (10 mL). The organic layer was separated, washed with saturated brine solution (5 mL), dried ($Na_2SO_4$) and the solvent removed *in vacuo* to afford 1-(2-(4-chlorobenzyl)pyridin-4-yl)-1,5,6,7-tetrahydro-4*H*-pyrazolo [4,3-*c*]pyridin-4-one as a white solid (6 mg, 20%). **Data in table 3.**

**Procedure 10:**

**Example 33, 3-((4-(4-oxo-4,5,6,7-tetrahydro-1*H*-pyrazolo[4,3-*c*]pyridin-1-yl)pyridin-2-yl)methyl)-5-(trifluoromethyl)benzonitrile**

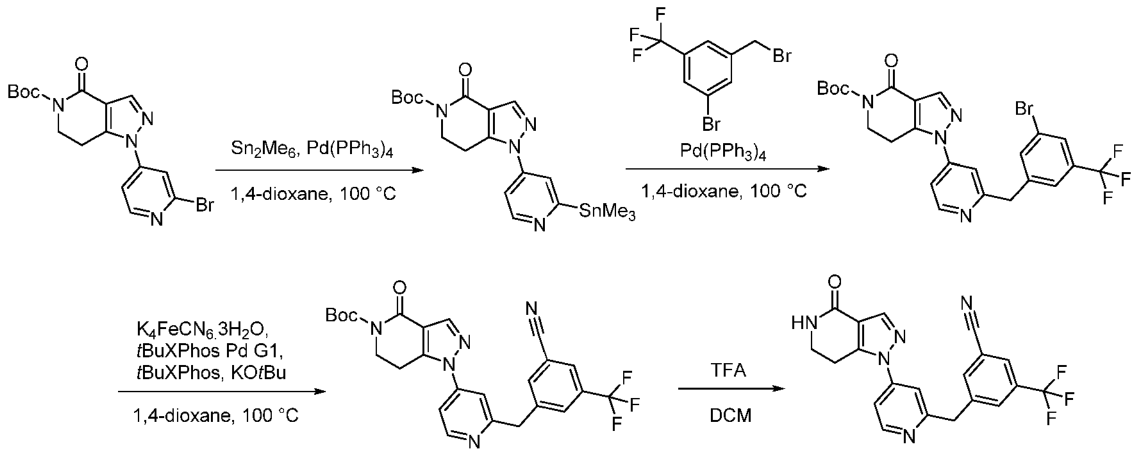


Steps 1 and 2. *tert*-Butyl 1-(2-(3-bromo-5-(trifluoromethyl)benzyl)pyridin-4-yl)-4-oxo-1,4,6,7-tetrahydro-5*H*-pyrazolo[4,3-*c*]pyridine-5-carboxylate (150 mg, 54%) was prepared from *tert*-butyl 1-(2-bromopyridin-4-yl)-4-oxo-1,4,6,7-tetrahydro-5*H*-pyrazolo[4,3-*c*]pyridine-5-carboxylate (Intermediate 14, 200 mg, 0.50 mmol) and 1-bromo-3-(bromomethyl)-5-(trifluoromethyl)benzene (322 mg, 1.01 mmol) using the methods of Procedure 9, steps 1 and 2.

**LCMS (Method 2):** m/z 450.9 (ES+), at 2.63 min.

**$^1$H NMR:** (400 MHz, DMSO-$d_6$) δ: 8.68 (d, *J* = 5.2 Hz, 1H), 8.22 (s, 1H), 7.92 (s, 1H), 7.84 (s, 1H), 7.78 (s, 1H), 7.74-7.61 (m, 1H), 7.54-7.52 (m, 1H), 4.32 (s, 2H), 4.05-4.01 (m, 2H), 3.32-3.29 (m, 2H), 1.49 (s, 9H).

Step 3. 1 M KOAc in $H_2O$ (8.8 mg, 0.09 mmol) was added to a solution of *tert*-butyl 1-(2-(3-bromo-5-(trifluoromethyl)benzyl)pyridin-4-yl)-4-oxo-1,4,6,7-tetrahydro-5*H*-pyrazolo[4,3-c]pyridine-5-carboxylate (100 mg, 0.18 mmol) and $K_4FeCN_6.3H_2O$ (114 mg, 0.27 mmol) in 1,4-dioxane (5 mL) in a sealed tube, and degassed with argon for 15 minutes. *t*-ButylXPhos (7.6 mg, 0.018 mmol) and *t*-butylXPhos Pd G1 (12.3 mg, 0.018 mmol) were then added and the reaction mixture degassed with argon for another 5 minutes. The reaction mixture was heated at 100 °C for 3 h. The reaction mixture was partitioned between $H_2O$ (10 mL) and EtOAc (15 mL). The organic layer was separated, dried ($Na_2SO_4$) and the solvent removed *in vacuo*. The residue was purified by gradient flash column chromatography eluting with 0-100% EtOAc in pet-ether to afford *tert*-butyl 1-(2-(3-cyano-5-(trifluoromethyl)benzyl)pyridin-4-yl)-4-oxo-1,4,6,7-tetrahydro-*5*H-pyrazolo[4,3-*c*]pyridine-5-carboxylate as a yellow solid (80 mg, 88%).

**LCMS (Method 2):** m/z 498.0 (ES+), at 2.47 min.

**$^1$H NMR:** (400 MHz, DMSO-$d_6$) δ: 8.67 (d, *J* = 5.6 Hz, 1H), 8.21 (d, *J* = 6.0 Hz, 1H), 8.17 (s, 1H), 7.74 (d, *J* = 2.0 Hz, 1H), 7.65-7.61 (m, 1H), 7.58-7.53 (m, 2H), 4.77 (s, 2H), 4.39-4.31 (m, 2H), 4.02-3.99 (m, 2H), 1.49 (s, 9H).

Step 4. 25% TFA in DCM (1.5 mL) was added to a solution of *tert*-butyl 1-(2-(3-cyano-5-(trifluoromethyl)benzyl)pyridin-4-yl)-4-oxo-1,4,6,7-tetrahydro-5*H*-pyrazolo[4,3-*c*]pyridine-5-carboxylate (80 mg, 0.16 mmol) in DCM (1.5 mL) and the reaction mixture was stirred at RT for 2 h. The solvent was removed *in vacuo* and the residue was purified by prep HPLC (Method 4). The solvent was removed *in vacuo* and the residue was partitioned between 10% $NaHCO_3$ solution (20 mL) and EtOAc (20 mL). The organic layer was separated, dried ($Na_2SO_4$) and the solvent removed *in vacuo* to afford 3-((4-(4-oxo-4,5,6,7-tetrahydro-1*H*-pyrazolo[4,3-*c*]pyridin-1-yl)pyridin-2-yl)methyl)-5-(trifluoromethyl)benzonitrile as a white solid (12 mg, 15%). **Data in table 3.**

**Procedure 11:**

**Example 35, 1-(2-(3-fluoro-5-(trifluoromethyl)benzoyl)pyridin-4-yl)-1,5,6,7-tetrahydro-4*H*-pyrazolo[4,3-*c*]pyridin-4-one**

**Example 36, 1-(2-((3-fluoro-5-(trifluoromethyl)phenyl)(hydroxy)methyl)pyridin-4-yl)-1,5,6,7-tetrahydro-4*H*-pyrazolo[4,3-*c*]pyridin-4-one**

**Example 37, 1-(2-(fluoro(3-fluoro-5-(trifluoromethyl)phenyl)methyl)pyridin-4-yl)-1,5,6,7-tetrahydro-4*H*-pyrazolo[4,3-*c*]pyridin-4-one**

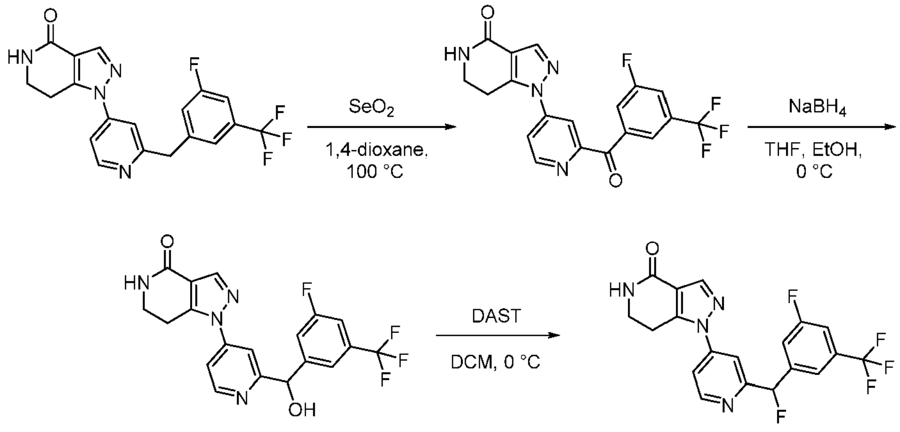


Step 1. Selenium dioxide (113 mg, 1.02 mmol) was added to a solution of 1-(2-(3-fluoro-5-(trifluoromethyl)benzyl)pyridin-4-yl)-1,5,6,7-tetrahydro-4*H*-pyrazolo[4,3-*c*]pyridin-4-one (Example 1, 600 mg, 1.53 mmol) in 1,4-dioxane (15 mL) and the reaction mixture was heated at 100 °C for 3 h. The solvent was removed *in vacuo* and the residue was purified by gradient flash column chromatography eluting with 0-100% EtOAc in pet-ether to afford 1-(2-(3-fluoro-5-(trifluoromethyl)benzoyl)pyridin-4-yl)-1,5,6,7-tetrahydro-4*H*-pyrazolo[4,3-*c*]pyridin-4-one as an off-white solid (Example 35, 400 mg, 64%). **Data in table 3.**

Step 2. Sodium borohydride (28 mg, 0.742 mmol) was added to a solution of 1-(2-(3-fluoro-5-(trifluoromethyl)benzoyl)pyridin-4-yl)-1,5,6,7-tetrahydro-4*H*-pyrazolo[4,3-*c*]pyridin-4-one (200 mg, 0.495 mmol) in EtOH:THF (1 mL:10 mL) at 0 °C and the reaction mixture was stirred at RT for 3 h. The reaction mixture was partitioned between EtOAc (20 mL) and water (20 mL). The organic layer was separated, dried ($Na_2SO_4$) and the solvent removed *in vacuo.* The residue was purified by gradient flash column chromatography eluting with 0-100% EtOAc in pet-ether to afford 1-(2-((3-fluoro-5-(trifluoromethyl)phenyl)(hydroxy)methyl)pyridin-4-yl)-1,5,6,7-tetrahydro-4*H*-pyrazolo[4,3-*c*]pyridin-4-one as a white solid (Example 36, 135 mg, 68%). **Data in table 3.**

Step 3. DAST (22 mg, 0.13 mmol) was added to a solution of 1-(2-((3-fluoro-5-(trifluoromethyl)phenyl)(hydroxy)methyl)pyridin-4-yl)-1,5,6,7-tetrahydro-4*H*-pyrazolo[4,3-c]pyridin-4-one (50 mg, 0.12 mmol) in DCM (10 mL) at 0 °C and the reaction mixture was stirred at RT for 16 h. The reaction mixture was partitioned between $NaHCO_3$ solution (10 mL) and DCM (10 mL). The organic layer was separated, dried ($Na_2SO_4$) and the solvent removed *in vacuo.* The residue was purified by gradient flash column chromatography eluting with 0-100% EtOAc in pet-ether to afford 1-(2-(fluoro(3-fluoro-5-(trifluoromethyl)phenyl)methyl)pyridin-4-yl)-1,5,6,7-tetrahydro-4*H*-pyrazolo[4,3-c]pyridin-4-one as a white solid (Example 37, 13 mg, 26%). **Data in table 3.**

**Procedure 12:**

**Example 38, 1-(2-(difluoro(3-fluoro-5-(trifluoromethyl)phenyl)methyl)pyridin-4-yl)-1,5,6,7-tetrahydro-4*H*-pyrazolo[4,3-c]pyridin-4-one**

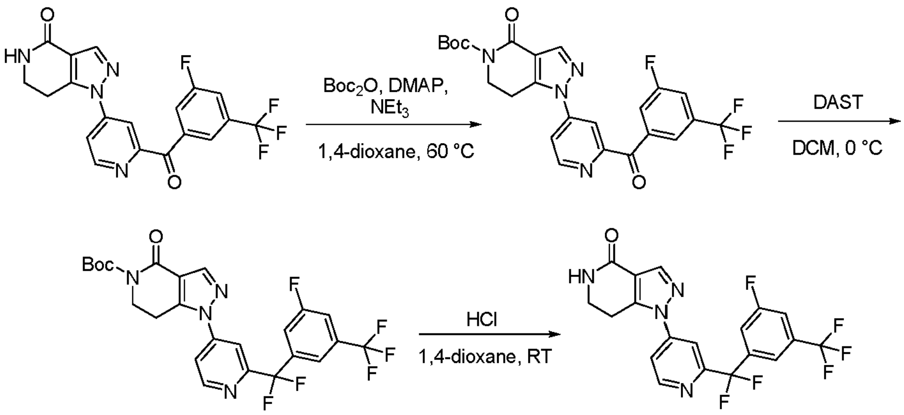


Step 1. $NEt_3$ (0.2 ml, 1.48 mmol), DMAP (12 mg, 0.09 mmol) and Boc anhydride (128 mg, 0.15 mmol) were added to a solution of 1-(2-(3-fluoro-5-(trifluoromethyl)benzoyl)pyridin-4-yl)-1,5,6,7-tetrahydro-4*H*-pyrazolo[4,3-*c*]pyridin-4-one (Example 35, 200 mg, 0.49 mmol) in 1,4-dioxane (10 mL) and the reaction mixture was heated at 60 °C for 16 h. The solvent was removed *in vacuo* and the residue was purified by gradient flash column chromatography eluting with 0-80% EtOAc in pet-ether to afford *tert*-butyl 1-(2-(3-fluoro-5-(trifluoromethyl)benzoyl)pyridin-4-yl)-4-oxo-1,4,6,7-tetrahydro-5*H*-pyrazolo [4,3-*c*]pyridine-5-carboxylate as an off-white solid (100 mg, 40%).

**LCMS (Method 2):** m/z 505.0 (ES+), at 2.89 min.

**$^1$H NMR:** (400 MHz, DMSO-$d_6$) δ: 8.94-8.93 (m, 1H), 8.32 (d, *J* = 1.6 Hz, 2H), 8.21-8.21 (m, 2H), 8.10-8.00 (m, 1H), 8.00-7.98 (m, 1H), 4.06-4.03 (m, 2H), 3.42-3.39 (m, 2H), 1.49 (d, *J* = 8.0 Hz, 9H).

Step 2. DAST (70 mg, 0.42 mmol) was added to a solution of *tert*-butyl 1-(2-(3-fluoro-5-(trifluoromethyl)benzoyl) pyridin-4-yl)-4-oxo-1,4,6,7-tetrahydro-5*H*-pyrazolo[4,3-*c*]pyridine-5-carboxylate (100 mg, 0.19 mmol) in DCM (10 mL) at 0 °C and the reaction mixture was stirred at RT for 16 h. The reaction mixture was partitioned between 10% $NaHCO_3$ solution (10 mL) and EtOAc (10 mL). The organic layer was separated, dried ($Na_2SO_4$) and the solvent removed *in vacuo* to afford *tert*-butyl 1-(2-(difluoro(3-fluoro-5-(trifluoromethyl)phenyl)methyl)pyridin-4-yl)-4-oxo-1,4,6,7-tetrahydro-5*H*-pyrazolo[4,3-*c*]pyridine-5-carboxylate as a yellow gum (70 mg, crude).

**LCMS (Method 7):** m/z 527.0 (ES+), at 2.56 min.

Step 3. *tert*-Butyl 1-(2-(difluoro(3-fluoro-5-(trifluoromethyl)phenyl)methyl)pyridin-4-yl)-4-oxo-1,4,6,7-tetrahydro-5*H*-pyrazolo[4,3-*c*]pyridine-5-carboxylate (70 mg) was dissolved in HCl solution (4 M in 1,4-dioxane, 5 mL) and the reaction mixture stirred at RT for 4 h. The reaction mixture was partitioned between 10% $NaHCO_3$ solution (20 mL) and EtOAc (20 mL). The organic layer was separated, dried ($Na_2SO_4$) the solvent removed *in vacuo.* The residue was purified by prep HPLC (Method 4), The solvent was removed *in vacuo* and the residue was partitioned between 10% $NaHCO_3$ solution (10 mL) and EtOAc (10 mL). The organic layer was separated, dried ($Na_2SO_4$) and the solvent removed *in vacuo* to afford 1-(2-(difluoro(3-fluoro-5-(trifluoromethyl)phenyl)methyl)pyridin-4-yl)-1,5,6,7-tetrahydro-4*H*-pyrazolo[4,3-*c*]pyridin-4-one as a white solid (6 mg, 10%). **Data in table 3.**

**Procedure 13:**

**Example 39, 1-(2-((3-fluoro-5-(trifluoromethyl)phenyl)methyl-*d*$_2$)pyridin-4-yl)-1,5,6,7-tetrahydro-4*H*-pyrazolo [4,3-*c*]pyridin-4-one**

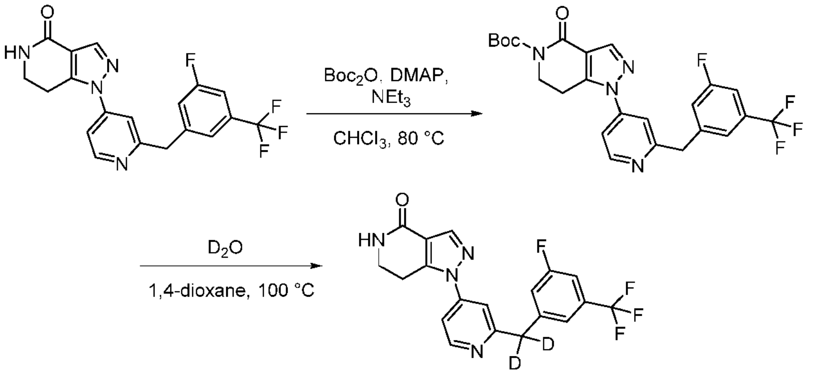


Step 1. $NEt_3$ (0.155 mg, 1.53 mmol), DMAP (12 mg, 0.102 mmol) and Boc anhydride (134 mg, 0.615 mmol) were added to a solution of 1-(2-(3-fluoro-5-(trifluoromethyl)benzyl)pyridin-4-yl)-1,5,6,7-tetrahydro-4*H*-pyrazolo[4,3-*c*]pyridin-4-one (Example 1, 200 mg, 0.512 mmol) in $CHCl_3$ (10 mL) and the reaction mixture was heated at 80 °C for 16 h. The solvent was removed *in vacuo* and the residue was purified by gradient flash column chromatography eluting with 0-50% EtOAc in pet-ether to afford *tert*-butyl 1-(2-(3-fluoro-5-(trifluoromethyl)benzyl)pyridin-4-yl)-4-oxo-1,4,6,7-tetrahydro-5*H*-pyrazolo[4,3-*c*]pyridine-5-carboxylate as an off-white solid (120 mg, 47%).

**LCMS (Method 7):** m/z 491.0 (ES+), at 2.46 min.

$^1$**H NMR:** (400 MHz, DMSO-$d_6$) δ: 8.67 (d, *J* = 7.2 Hz, 1H), 8.21 (s, 1H), 7.71 (s, 1H), 7.61 (s, 1H), 7.56-7.51 (m, 3H), 4.32 (s, 2H), 3.99 (t, *J* = 8.0 Hz, 2H), 3.20 (t, *J* = 6.8 Hz, 2H), 1.48 (s, 9H).

Step 2. $D_2O$ (5 mL) was added to a solution of *tert*-butyl 1-(2-(3-fluoro-5-(trifluoromethyl)benzyl)pyridin-4-yl)-4-oxo-1,4,6,7-tetrahydro-5*H*-pyrazolo[4,3-*c*]pyridine-5-carboxylate (100 mg, 0.20 mmol) in 1,4-dioxane (5 mL) and the reaction mixture was heated at 100 °C for 24 h. After 24 h, the solvent was removed *in vacuo,* the residue dissolved in $D_2O$ (10 mL) and the reaction mixture heated at 100 °C for 24 h. This process was repeated for another 3 times. (Reaction carried out for 5 days). The solvent was removed *in vacuo* and the residue was purified by gradient flash column chromatography eluting with with 0-10% MeOH in DCM to afford 1-(2-((3-fluoro-5-(trifluoromethyl)phenyl)methyl-$d_2$) pyridin-4-yl)-1,5,6,7-tetrahydro-4*H*-pyrazolo[4,3-*c*]pyridin-4-one as a white solid (20 mg, 24%). **Data in table 3.**

**Procedure 14:**

**Example 41, 1-(4-(3-(trifluoromethyl)benzyl)pyridin-2-yl)-5,6,7,8-tetrahydro-[1,2,3]triazolo[4,5-*c*]azepin-4(1*H*)-one**

**Example 45, 2-(4-(3-(trifluoromethyl)benzyl)pyridin-2-yl)-5,6,7,8-tetrahydro-[1,2,3]triazolo[4,5-*c*]azepin-4(2*H*)-one**

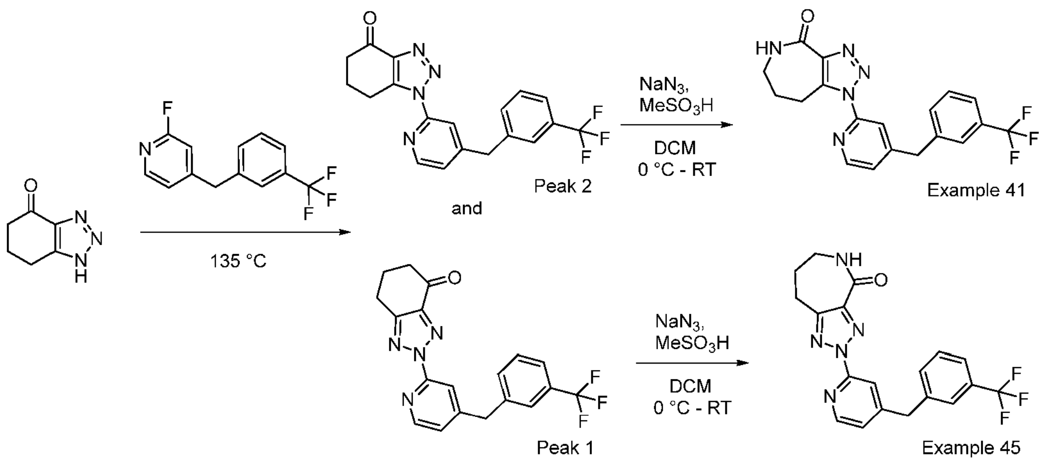


Step 1. 2-Fluoro-4-(3-(trifluoromethyl)benzyl)pyridine (Intermediate 4, 465 mg, 1.82 mmol) was added to 1,5,6,7-tetrahydro-4*H*-benzo[*d*][1,2,3]triazol-4-one (Intermediate 15, 250 mg, 1.82 mmol) and the reaction mixture heated at 135 °C for 16 h. The reaction mixture was dissolved in 5% MeOH/DCM (10 mL) and the solvent removed *in vacuo.* The residue was purified by gradient flash column chromatography eluting with 35-40% EtOAc in hexane to afford 2-(4-(3-(trifluoromethyl)benzyl)pyridin-2-yl)-2,5,6,7-tetrahydro-4*H*-benzo[*d*][1,2,3]triazol-4-one as a white solid (Peak 1, 24 mg, 4%) and 1-(4-(3-(trifluoromethyl)benzyl)pyridin-2-yl)-1,5,6,7-tetrahydro-4*H*-benzo[*d*][1,2,3]triazol-4-one as a white solid (Peak 2, 30 mg, 4%).

Peak 1:

**MS (Method 1):** m/z 373 (ES+).
**$^1$H NMR:** (400 MHz, DMSO-$d_6$) δ: 8.54 (d, *J* = 5.0 Hz, 1H), 8.05 (s, 1H), 7.79 (s, 1H), 7.68 (d, *J* = 7.1 Hz, 1H), 7.63-7.54 (m, 2H), 7.52 (d, *J* = 4.9 Hz, 1H), 4.26 (s, 2H), 3.01 (t, *J* = 6.1 Hz, 2H), 2.66 (t, *J* = 6.2 Hz, 2H), 2.23-2.12 (m, 2H).

Peak 2:

**MS (Method 1):** m/z 373 (ES+).
**$^1$H NMR:** (400 MHz, DMSO-$d_6$) δ: 8.51 (d, *J* = 5.1 Hz, 1H), 8.03 (s, 1H), 7.76 (s, 1H), 7.66 (d, *J* = 7.2 Hz, 1H), 7.63-7.56 (m, 2H), 7.49 (d, *J* = 4.8 Hz, 1H), 4.27 (s, 2H), 3.32 (t, *J* = 6.1 Hz, 2H), 2.57 (t, *J* = 6.3 Hz, 2H), 2.15-2.09 (m, 2H).
Step 2 (Peak 2). $NaN_3$ (62 mg, 0.96 mmol) was slowly added to a solution of 1-(4-(3-(trifluoromethyl)benzyl)pyridin-2-yl)-1,5,6,7-tetrahydro-4*H*-benzo[*d*][1,2,3]triazol-*4*-one (Step 1, Peak 2, 120 mg, 0.32 mmol) in DCM:$MeSO_3H$ (6 mL, 2:1), at 0 °C and the reaction mixture was stirred at RT for 2 h. The reaction mixture was quenched with 2 N aq NaOH solution and the aqueous layer was extracted with EtOAc (3x30 mL). The organic layers were combined, dried ($Na_2SO_4$) and the solvent removed *in vacuo.* The residue was triturated with MTBE to afford 1-(4-(3-(trifluoromethyl)benzyl)pyridin-2-yl)-5,6,7,8-tetrahydro-[1,2,3]triazolo[4,5-c]azepin-4(1*H*)-one) as a white solid (Example 41, 90 mg, 72%). **Data in table 3.**
Step 2 (Peak 1). $NaN_3$ (104 mg, 1.61 mmol) was slowly added to a solution of 2-(4-(3-(trifluoromethyl)benzyl)pyridin-2-yl)-2,5,6,7-tetrahydro-4*H*-benzo[*d*][1,2,3]triazol-4-one (Step 1, Peak 1, 200 mg, 0.53 mmol) in DCM:$MeSO_3H$ (9 mL, 2:1) at 0 °C and the reaction mixture was stirred at RT for 2 h. The reaction mixture was quenched with 2 N aq NaOH solution and the aqueous layer was extracted with EtOAc (3x30 mL). The organic layers were combined, dried ($Na_2SO_4$) and the solvent removed *in vacuo.* The residue was triturated with MTBE to afford 2-(4-(3-(trifluoromethyl)benzyl)pyridin-2-yl)-5,6,7,8-tetrahydro-[1,2,3]triazolo[4,5-*c*]azepin-4(2*H*)-one as a white solid (Example 45, 75 mg, 36%). **Data in table 3.**

**Procedure 15:**

**Example 42, 1-(4-(3-(trifluoromethyl)benzyl)pyridin-2-yl)-1,5,6,7-tetrahydro-4*H*-[1,2,3]triazolo[4,5-*c*]pyridin-4-one**

**Example 43, 2-(4-(3-(trifluoromethyl)benzyl)pyridin-2-yl)-2,5,6,7-tetrahydro-4*H*-[1,2,3]triazolo[4,5-*c*]pyridin-4-one**

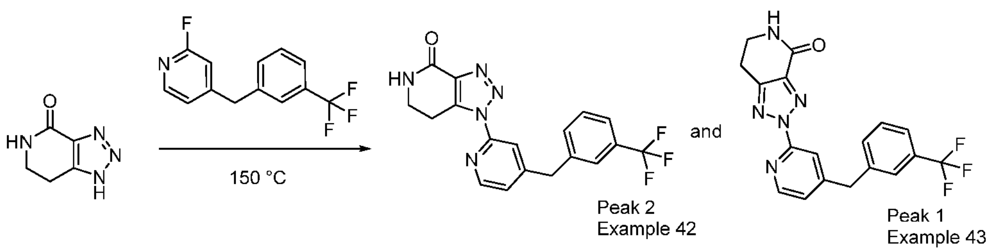


2-Fluoro-4-(3-(trifluoromethyl)benzyl)pyridine (Intermediate 4, 350 mg, 1.36 mmol) was added to 1,5,6,7-tetrahydro-4*H*-[1,2,3]triazolo[4,5-*c*]pyridin-4-one (Intermediate 16, 190 mg, 1.36 mmol) and the reaction mixture heated at 150 °C for 16 h. The reaction mixture was triturated with hexane (10 mL). The residue was purified by prep-HPLC (Method 5 - 35-50% gradient) to afford 1-(4-(3-(trifluoromethyl)benzyl)pyridin-2-yl)-1,5,6,7-tetrahydro-4*H*-[1,2,3]triazolo[4,5-*c*]pyridin-4-one as a white solid (Example 42, Peak 2, 14 mg, 3%) and 2-(4-(3-(trifluoromethyl)benzyl)pyridin-2-yl)-2,5,6,7-tetrahydro-4*H*-[1,2,3]triazolo[4,5-*c*]pyridin-4-one as a white solid (Example 43, Peak 1, 42 mg, 8%). **Data in table 3.**

**Procedure 16:**

**Example 46, 1-(4-(3-fluoro-5-(trifluoromethyl)benzyl)pyridin-2-yl)-5,6,7,8-tetrahydro-[1,2,3]triazolo[4,5-*c*]azepin-4(1*H*)-one**

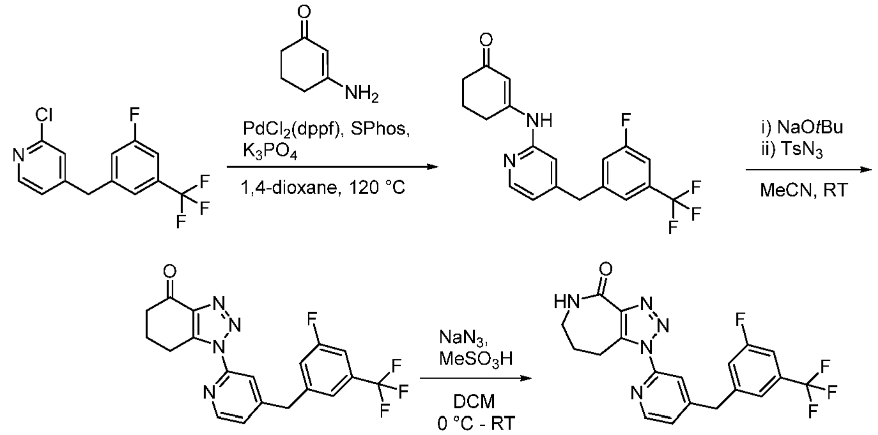


Step 1. 2-Chloro-4-(3-fluoro-5-(trifluoromethyl)benzyl)pyridine (Intermediate 18, 2.61 g, 9.00 mmol) was added to a solution of 3-aminocyclohex-2-en-1-one (1.00 g, 9.00 mmol) in 1,4-dioxane (10 mL). The reaction mixture was degassed with argon for 10 min and $PdCl_2$(dppf).DCM (367 mg, 0.45 mmol), SPhos (369 mg, 0.90 mmol) and $K_3PO_4$ (5.73 g, 27.0 mmol) were sequentially added to the reaction mixture. The reaction mixture was heated at 120 °C for 16 h and then filtered through Celite and washed with EtOAc (300 mL). The solvent was removed *in* vacuo. The residue was purified by gradient flash column chromatography eluting with 3-5% MeOH in DCM to afford 3-((4-(3-fluoro-5-(trifluoromethyl)benzyl)pyridin-2-yl)amino)cyclohex-2-en-1-one as a brown solid (1.50 g, 43%).

**LCMS (Method 9):** m/z 365.0 (ES+), at 2.33 min.

Step 2. A solution of 3-((4-(3-fluoro-5-(trifluoromethyl)benzyl)pyridin-2-yl)amino)cyclohex-2-en-1-one (1.5 g, 4.11 mmol) in MeCN (45 mL) was added dropwise to a suspension of sodium *tert*-butoxide (600 mg, 6.20 mmol) in MeCN (45 mL). After 30 min stirring at RT, a solution of tosyl azide (1.05 g, 6.50 mmol) in MeCN (10 mL) was added dropwise. The reaction mixture was stirred for 2 min at RTand water (60 mL) was added. The aqueous layer was extracted with EtOAc (3 x 50 mL) and the combined organic layers were dried ($Na_2SO_4$) and the solvent removed *in vacuo.* The residue was triturated with $Et_2O$ (30 mL) to afford 1-(4-(3-fluoro-5-(trifluoromethyl)benzyl)pyridin-2-yl)-1,5,6,7-tetrahydro-4*H*-benzo[*d*][1,2,3]triazol-4-one as an off white solid (1.0 g, 63%).

**LCMS (Method 9):** m/z 391.0 (ES+), at 2.33 min.

**$^1$H NMR:** (400 MHz, DMSO-$d_6$) δ: 8.55 (d, *J* = 5.0 Hz, 1H), 8.10 (t, *J* = 4.8 Hz, 1H), 7.75 (s, 1H), 7.65-7.60 (m, 1H), 7.59-7.54 (m, 2H), 4.28 (s, 2H), 2.60-2.51 (m, 4H), 2.20-2.10 (m, 2H).

Step 3. 1-(4-(3-fluoro-5-(trifluoromethyl)benzyl)pyridin-2-yl)-5,6,7,8-tetrahydro-[1,2,3]triazolo[4,5-*c*]azepin-4(1*H*)-one (35 mg, 32%) was prepared from 1-(4-(3-fluoro-5-(trifluoromethyl)benzyl)pyridin-2-yl)-1,5,6,7-tetrahydro-4*H*-benzo[*d*][1,2,3]triazol-4-one (100 mg, 0.26 mmol) and $NaN_3$ (50 mg, 0.78 mmol) using the methods of procedure 14, step 2. The residue was purified by gradient flash column chromatography eluting with 20-30% EtOAc in hexane. **Data in table 3.**

## Procedure 17:

**Example 47, 1-(4-(3-fluoro-5-(trifluoromethyl)benzyl)pyridin-2-yl)-1,7-dihydropyrazolo[4,3-*d*][1,2]oxazin-4(5*H*)-one**

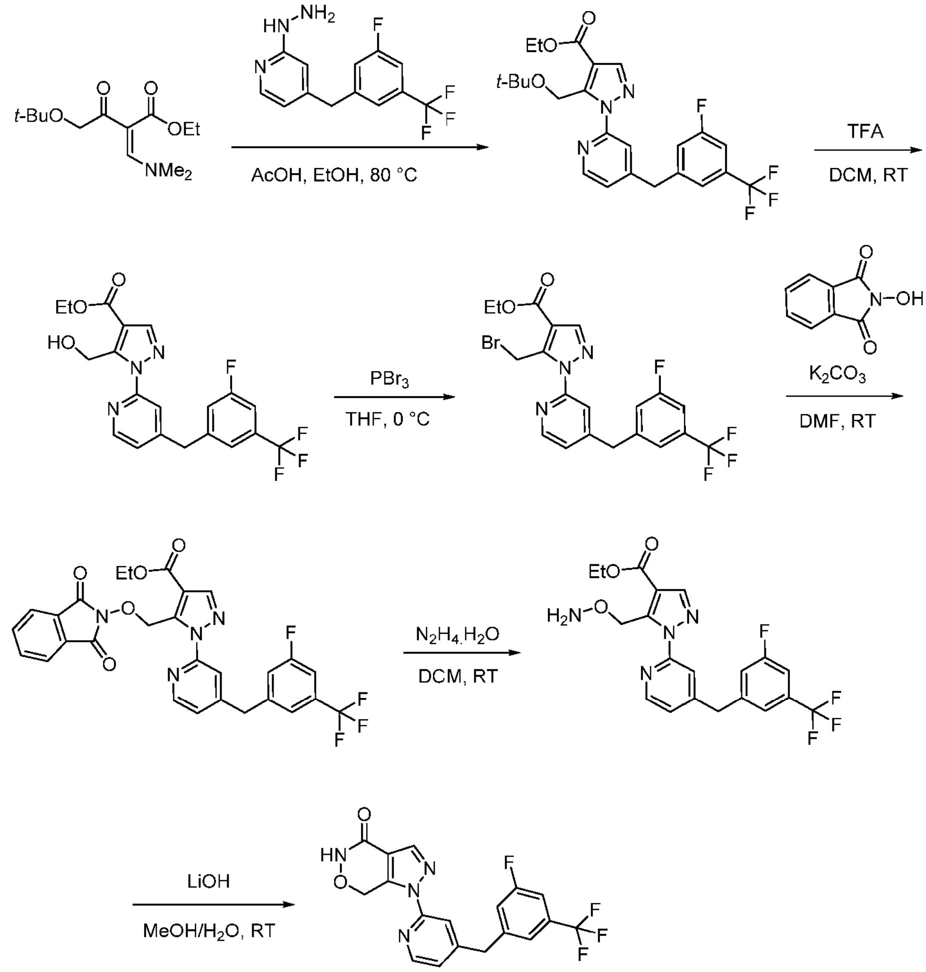


Step 1. A solution of ethyl (*Z*)-4-(*tert*-butoxy)-2-((dimethylamino)methylene)-3-oxobutanoate (Intermediate 19, 500 mg, 1.94 mmol) and 4-(3-fluoro-5-(trifluoromethyl)benzyl)-2-hydrazineylpyridine (Intermediate 8, 554 mg, 1.94 mmol) in EtOH (10 mL) and acetic acid (0.5 mL) was heated at 80 °C for 16 h. The reaction mixture was concentrated *in vacuo* and the residue was purified by gradient flash column chromatography eluting with 15-16% EtOAc in hexane to afford ethyl 5-(*tert*-butoxymethyl)-1-(4-(3-fluoro-5-(trifluoromethyl)benzyl)pyridin-2-yl)-1*H*-pyrazole-4-carboxylate as a light yellow solid (1.30 g, 71%).

**LCMS (Method 9):** m/z 478.4 (ES+), at 2.54 min.

Step 2. TFA (10 mL) was added to a solution of ethyl 5-(*tert*-butoxymethyl)-1-(4-(3-fluoro-5-(trifluoromethyl) benzyl)pyridin-2-yl)-1*H*-pyrazole-4-carboxylate (500 mg, 1.04 mmol) in DCM (10 mL) at 0 °C and the reaction mixture was stirred at RT for 24 h. The reaction mixture was concentrated *in vacuo* to afford ethyl 1-(4-(3-fluoro-5-(trifluoromethyl) benzyl)pyridin-2-yl)-5-(hydroxymethyl)-1*H*-pyrazole-4-carboxylate as a brown semi-solid (400 mg, crude). The crude product was used in the next step without further purification.

**LCMS (Method 9)**: m/z 424.0 (ES+), at 2.39 min.

Step 3. Tribromophosphane (0.27 mL, 2.83 mmol) was added to a solution of ethyl 1-(4-(3-fluoro-5-(trifluoromethyl)benzyl)pyridin-2-yl)-5-(hydroxymethyl)-1*H*-pyrazole-4-carboxylate (400 mg, 0.945 mmol) in THF (15 mL) and the reaction mixture was stirred at 0 °C for 3 h. The reaction was quenched with sat. $NaHCO_3$ solution (5 mL) and the aqueous layer was extracted with EtOAc (3x50 mL). The organic layer was washed with brine, dried ($Na_2SO_4$) and the solvent removed *in vacuo* to afford ethyl 5-(bromomethyl)-1-(4-(3-fluoro-5-(trifluoromethyl)benzyl)pyridin-2-yl)-1*H*-pyrazole-4-carboxylate as a liquid (450 mg, crude). The crude product was used in the next step without further purification.

Step 4. Ethyl 5-(bromomethyl)-1-(4-(3-fluoro-5-(trifluoromethyl)benzyl)pyridin-2-yl)-1*H*-pyrazole-4-carboxylate (450 mg, 0.93 mmol) was added to a solution of 2-hydroxy-2,3-dihydro-1*H*-isoindole-1,3-dione (302 mg, 1.85 mmol) and $K_2CO_3$ (389 mg, 2.78 mmol) in DMF (10 mL) and the reaction mixture was stirred at RT for 16 h. The reaction was quenched with water (10 mL) and the aqueous layer extracted with EtOAc (3x30 mL). The combined organic layers were concentrated *in vacuo.* The residue was purified by gradient flash column chromatography eluting with 30-35% EtOAc in hexane to afford ethyl 5-(((1,3-dioxoisoindolin-2-yl)oxy)methyl)-1-(4-(3-fluoro-5-(trifluoromethyl)benzyl)pyridin-2-yl)-1*H*-pyrazole-4-carboxylate as a solid (180 mg, 34%).

**LCMS (Method 9):** m/z 569.0 (ES+), at 2.44 min.

Step 5. Hydrazine hydrate (220 mg, 4.40 mmol) was added to a solution of ethyl 5-(((1,3-dioxoisoindolin-2-yl)oxy) methyl)-1-(4-(3-fluoro-5-(trifluoromethyl)benzyl)pyridin-2-yl)-1*H*-pyrazole-4-carboxylate (500 mg, 0.88 mmol) in DCM (10 mL) and the reaction mixture stirred at RT for 3 h. The reaction was filtered and the filtrate was concentrated *in vacuo* to afford ethyl 5-((aminooxy)methyl)-1-(4-(3-fluoro-5-(trifluoromethyl)benzyl)pyridin-2-yl)-1*H*-pyrazole-4-carboxylate as semi-solid (280 mg, crude). The crude product was used in the next step without further purification.

**LCMS (Method 9):** m/z 437.4 (ES+), at 2.27 min.

Step 6. LiOH.$H_2O$ (77 mg, 1.83 mmol) and water (0.2 mL) were added to a solution of ethyl 5-((aminooxy) methyl)-1-(4-(3-fluoro-5-(trifluoromethyl)benzyl)pyridin-2-yl)-1*H*-pyrazole-4-carboxylate (250 mg, 0.61 mmol) in MeOH (5 mL) and the reaction mixture was stirred at RT for 16 h. The solvent was removed *in vacuo* and the residue was dissolved in EtOAc (10 mL). This solution was washed with brine (5 mL), dried ($Na_2SO_4$) and the solvent removed *in vacuo.* The residue was purified by gradient flash column chromatography eluting with 30-35% EtOAc in hexane to afford 1-(4-(3-fluoro-5-(trifluoromethyl)benzyl)pyridin-2-yl)-1,7-dihydropyrazolo[4,3-*d*][1,2]oxazin-4(5*H*)-one as a white solid (25 mg, 10%). **Data in table 3.**

<u>**Procedure 18:**</u>

**Example 48, 1-(2-(3-fluoro-5-(trifluoromethyl)benzyl)pyridin-4-yl)-5,6-dihydropyrrolo[3,4-*c*]pyrazol-4(1*H*)-one**

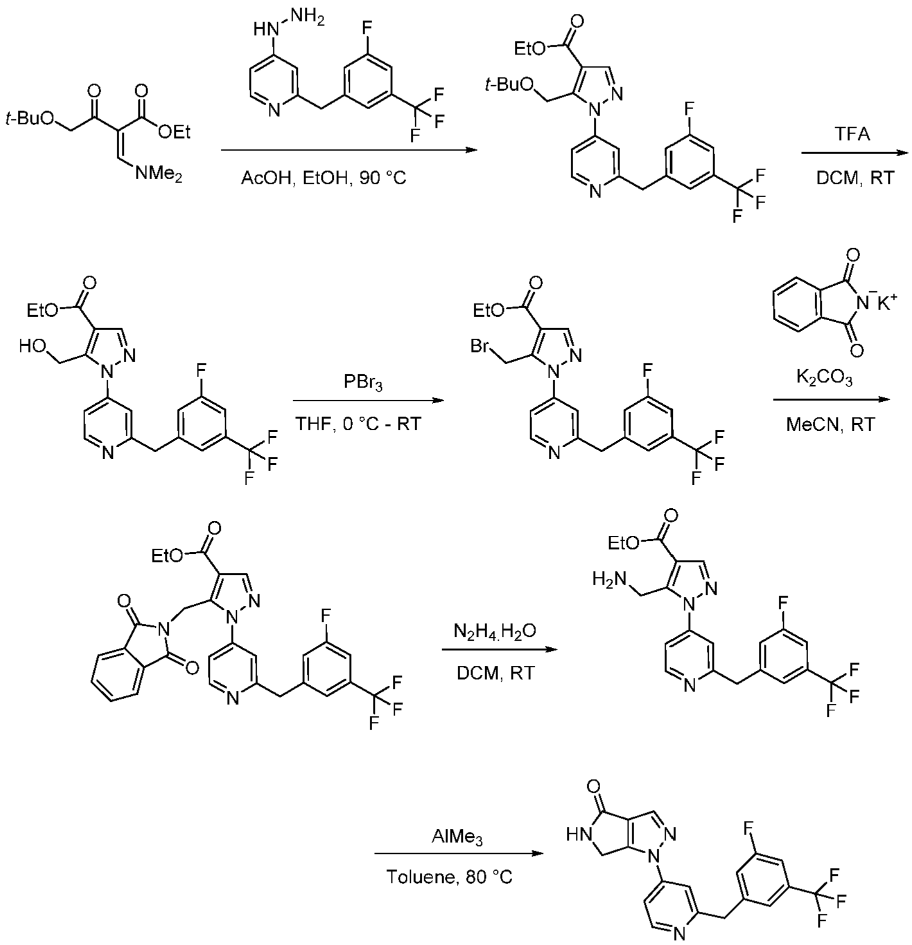


Steps 1, 2 and 3. Ethyl 5-(bromomethyl)-1-(2-(3-fluoro-5-(trifluoromethyl)benzyl)pyridin-4-yl)-1*H*-pyrazole-4-carboxylate (165 mg, crude) was prepared from ethyl (*Z*)-4-(*tert*-butoxy)-2-((dimethylamino)methylene)-3-oxobutanoate (Intermediate 19, 433 mg, 0.56 mmol) and 2-(3-fluoro-5-(trifluoromethyl)benzyl)-4-hydrazineylpyridine (Intermediate 6, 160 mg, 0.56 mmol) using the methods of Procedure 17, steps 1, 2 and 3. The crude product was used in the next step without further purification.

**LCMS (Method 9):** m/z 486.2 (ES+), at 2.46 min.

Step 4. Potassium 1,3-dioxo-2,3-dihydro-1*H*-isoindol-2-ide (63 mg, 0.34 mmol) was added to a solution of ethyl 5-(bromomethyl)-1-(2-(3-fluoro-5-(trifluoromethyl)benzyl)pyridin-4-yl)-1*H*-pyrazole-4-carboxylate (160 mg, 0.34 mmol) in MeCN (4 mL) and the reaction mixture was stirred at RT for 16 h. The reaction was quenched with water (10 mL) and extracted with EtOAc (3x30 mL). The combined organic layers were concentrated *in vacuo* to afford ethyl 5-((1,3-dioxoisoindolin-2-yl)methyl)-1-(2-(3-fluoro-5-(trifluoromethyl)benzyl)pyridin-4-yl)-1*H*-pyrazole-4-carboxylate as a white semi-solid (241 mg, crude). The crude product was used in the next step without further purification.

**LCMS (Method 9):** m/z 552.9 (ES+), at 2.46 min.

Step 5. Ethyl 5-(aminomethyl)-1-(2-(3-fluoro-5-(trifluoromethyl)benzyl)pyridin-4-yl)-1*H*-pyrazole-4-carboxylate (110 mg, crude) was prepared from ethyl 5-((1,3-dioxoisoindolin-2-yl)methyl)-1-(2-(3-fluoro-5-(trifluoromethyl)benzyl) pyridin-4-yl)-1*H*-pyrazole-4-carboxylate (241 mg, 0.44 mmol) and hydrazine hydrate (0.06 mL, 1.31 mmol) according to the methods of Procedure 17, step 5. The crude product was used in the next step without further purification.

**LCMS (Method 9):** m/z 423.0 (ES+), at 2.24 min.

Step 6. Trimethylaluminium (0.18 mL, 0.36 mmol) was added to a solution of ethyl 5-(aminomethyl)-1-(2-(3-fluoro-5-(trifluoromethyl)benzyl)pyridin-4-yl)-1*H*-pyrazole-4-carboxylate 100 mg, 0.24 mmol) in toluene (4 mL) at 0 °C and the reaction mixture was heated at 80 °C for 2 h. The reaction mixture was quenched with water (2 mL), filtered through a pad of Celite and washed with DCM (2x20 mL). The organic layers were combined, dried ($Na_2SO_4$) and the solvent removed *in vacuo.* The residue was washed with $Et_2O$ (10 mL) and dried *in vacuo* to afford 1-(2-(3-fluoro-5-(trifluoromethyl)benzyl)pyridin-4-yl)-5,6-dihydropyrrolo[3,4-*c*]pyrazol-4(1*H*)-one as a white solid (38 mg, 43%). **Data in table 3.**

## Procedure 19:

### Example 49, 1-(2-((3-fluoro-5-(trifluoromethyl)phenyl)methyl-*d*)pyridin-4-yl)-1,5,6,7-tetrahydro-4*H*-pyrazolo[4,3-*c*]pyridin-4-one

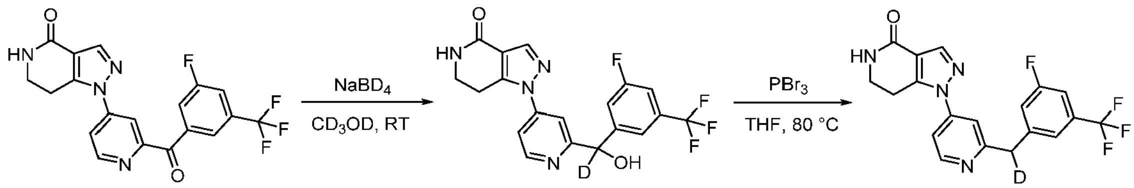


Step 1. $NaBD_4$ (16 mg, 0.37 mmol) was added to a solution of 1-(2-(3-fluoro-5-(trifluoromethyl)benzoyl)pyridin-4-yl)-1,5,6,7-tetrahydro-4*H*-pyrazolo[4,3-*c*]pyridin-4-one (Example 35, 100 mg, 0.24 mmol) in $CD_3OD$ (10 mL) and the reaction mixture was stirred at RT for 1 h. The reaction mixture was partitioned between sat. $NH_4Cl$ solution (20 mL) and EtOAc (20 mL). The organic layer was separated, dried ($Na_2SO_4$) and the solvent removed *in vacuo.* The residue was triturated with pet-ether (5 mL) and dried *in vacuo* to afford 1-(2-((3-fluoro-5-(trifluoromethyl)phenyl)(hydroxy)methyl-*d*) pyridin-4-yl)-1,5,6,7-tetrahydro-4*H*-pyrazolo[4,3-*c*]pyridin-4-one as a yellow solid (80 mg, 79%).

**LCMS (Method 7):** m/z 408.2 (ES+), at 1.80 min.

**$^1$H NMR:** (400 MHz, DMSO-$d_6$) δ: 8.64 (d, *J* = 5.6 Hz, 1H), 8.07 (s, 1H), 7.93 (d, *J* = 2.0 Hz, 1H), 7.71 (s, 1H), 7.63-7.51 (m, 4H), 6.63 (s, 1H), 3.46-3.43 (m, 2H), 3.33-3.27 (m, 2H).

Step 2. $PBr_3$ (322 mg, 1.19 mmol) was added dropwise to a solution of 1-(2-((3-fluoro-5-(trifluoromethyl)phenyl)(hydroxy)methyl-*d*)pyridin-4-yl)-1,5,6,7-tetrahydro-4*H*-pyrazolo[4,3-c]pyridin-4-one (80 mg, 0.19 mmol) in THF (10 mL) and the reaction mixture was heated at 80 °C for 4 h. The reaction mixture was partitioned between 10% $NaHCO_3$ solution (20 mL) and EtOAc (20 mL). The organic layer was separated, dried ($Na_2SO_4$) and the solvent removed *in vacuo.* The residue was purified by prep HPLC (Method 4). The solvent was removed *in vacuo* and the residue was partitioned between 10% $NaHCO_3$ solution (10 mL) and EtOAc (10 mL). Organic layer was separated, dried ($Na_2SO_4$) and the solvent removed *in vacuo* to afford 1-(2-((3-fluoro-5-(trifluoromethyl)phenyl)methyl-*d*)pyridin-4-yl)-1,5,6,7-tetrahydro-4*H*-pyrazolo[4,3-c]pyridin-4-one as a white solid (8 mg, 10%). **Data in table 3.**

## Procedure 20:

**Example 105, 1-(2-(3-fluoro-5-(trifluoromethyl)benzyl)pyridin-4-yl)-5-(2-hydroxyethyl)-1,5,6,7-tetrahydro-4*H*-pyrazolo[4,3-*c*]pyridin-4-one**

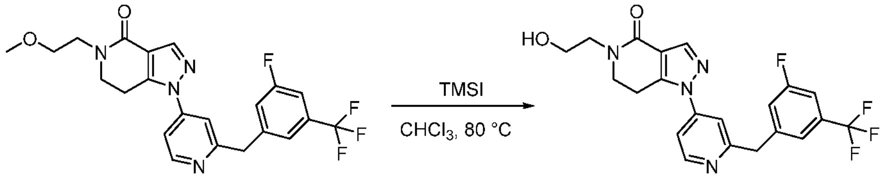


Iodotrimethylsilane (223 mg, 1.12 mmol) was added to a solution of 1-(2-(3-fluoro-5-(trifluoromethyl)benzyl) pyridin-4-yl)-5-(2-methoxyethyl)-1,5,6,7-tetrahydro-4*H*-pyrazolo[4,3-c]pyridin-4-one (Example 104, 100 mg, 0.223 mmol) in chloroform (10 mL) at RT and the reaction mixture was heated at 80 °C for 4 h. The reaction mixture was partitioned between water (20 mL) and DCM (20 mL). The aqueous layer was extracted with DCM (20 mL) and the combined organic layers were washed with water (10 mL) and brine solution (10 mL), dried over anhydrous ($Na_2SO_4$) and the solvent removed *in vacuo.* The residue was purified by gradient flash column chromatography eluting with 0-10% MeOH in DCM to afford 1-(2-(3-fluoro-5-(trifluoromethyl)benzyl)pyridin-4-yl)-5-(2-hydroxyethyl)-1,5,6,7-tetrahydro-4*H*-pyrazolo[4,3-*c*]pyridin-4-one as a white gum (24 mg, 24%). **Data in table 3.**

**<u>Procedure 21:</u>**

**Example 107, 1-(4-(3-fluoro-5-(trifluoromethyl)benzyl)pyridin-2-yl)-5-(2-hydroxyethyl)-1,5,6,7-tetrahydro-4*H*-pyrazolo[4,3-*c*]pyridin-4-one**

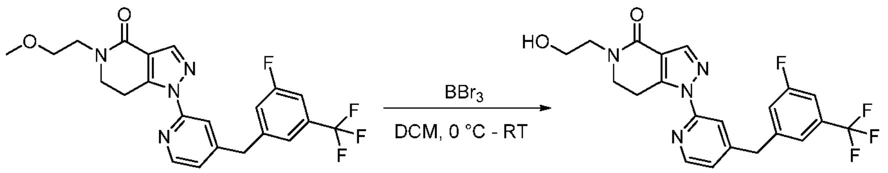


A solution of $BBr_3$ (1.0 M in DCM, 0.75 mL, 0.75 mmol) was added dropwise to a solution of 1-(4-(3-fluoro-5-(trifluoromethyl)benzyl)pyridin-2-yl)-5-(2-methoxyethyl)-1,5,6,7-tetrahydro-4*H*-pyrazolo[4,3-*c*]pyridin-4-one (Example 106, 65 mg, 0.15 mmol) in DCM (2 mL) at 0 °C. The reaction mixture was stirred at RT for 4h. The reaction mixture was diluted with water and the aqueous layer was extracted with DCM (2x20 mL). The combined organic layers were dried ($Na_2SO_4$) and the solvent removed *in vacuo.* The residue was purified by prep HPLC (Method 6 - gradient 40-75%) to afford 1-(4-(3-fluoro-5-(trifluoromethyl)benzyl)pyridin-2-yl)-5-(2-hydroxyethyl)-1,5,6,7-tetrahydro-4*H*-pyrazolo[4,3-*c*]pyridin-4-one as white solid (6 mg, 8%).

**<u>Procedure 22:</u>**

**Example 109, 1-(3-(3-fluoro-5-(trifluoromethyl)benzyl)phenyl)-1,5,6,7-tetrahydro-4*H*-pyrazolo[4,3-*c*]pyridin-4-one**

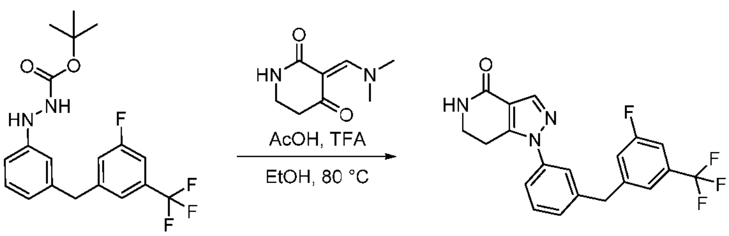

*tert*-Butyl 2-(3-(3-fluoro-5-(trifluoromethyl)benzyl)phenyl)hydrazine-1-carboxylate (181 mg, 0.47 mmol) and (*E*)-3-((dimethylamino)methylene)piperidine-2,4-dione (Intermediate 1, 95 mg, 0.57 mmol) were dissolved in ethanol (5 mL) and acetic acid (0.03 mL, 0.57 mmol) was added. The reaction mixture was heated at 80 °C for 3 hours. TFA (0.06 mL) was added and the reaction mixture heated at 80 °C for 2 hours. Further TFA (0.74 mL) was added and the reaction mixture heated at 80 °C overnight. The solvent was removed *in vacuo* and the residue partitioned between sat. $NaHCO_3$ soln (5 mL) and EtOAc (5 mL). The organic layer was removed and the aqueous layer extracted with EtOAc (2x5 mL), the combined organic layers dried (phase separator) and the solvent removed *in vacuo.* The residue was purified by gradient flash chromatography eluting with 2-7% MeOH in DCM. The residue was further purified via prep HPLC (Method 2 - 40-70% gradient) to give 1-(3-(3-fluoro-5-(trifluoromethyl)benzyl)phenyl)-1,5,6,7-tetrahydro-4*H*-pyrazolo[4,3-*c*]pyridin-4-one as a white solid (35 mg, 19%). **Data in table 3.**

**Procedure 23:**

**Example 111, 1-(2-((6-(difluoromethoxy)-5-fluoropyridin-3-yl)methyl)pyridin-4-yl)-3-methyl-1,5,6,7-tetrahydro-4*H*-pyrazolo[4,3-*c*]pyridin-4-one**

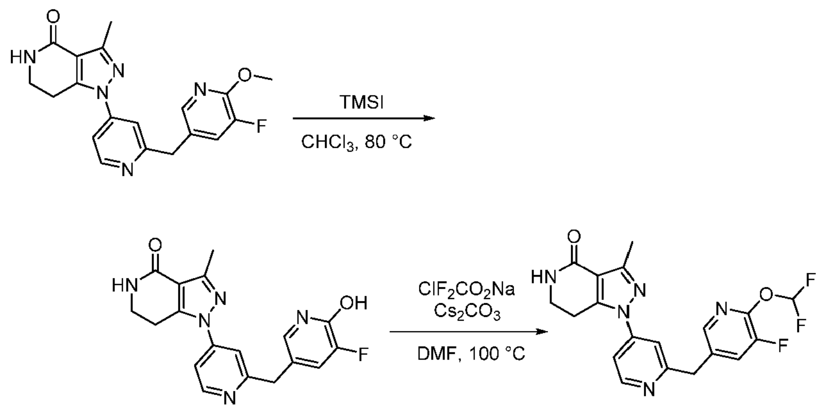


Step 1. 1-(2-((5-fluoro-6-hydroxypyridin-3-yl)methyl)pyridin-4-yl)-3-methyl-1,5,6,7-tetrahydro-4*H*-pyrazolo[4,3-c]pyridin-4-one (100 mg, 55%) was prepared from 1-(2-((5-fluoro-6-methoxypyridin-3-yl)methyl)pyridin-4-yl)-3-methyl-1,5,6,7-tetrahydro-4*H*-pyrazolo[4,3-*c*]pyridin-4-one (Example 93, 0.19 g, 0.52 mmol) using the methods of Procedure 20. **LCMS (Method 11):** m/z 354.1 (ES+), at 1.27 min.

**$^1$H NMR:** (400 MHz, DMSO-$d_6$) δ: 11.85 (s, 1H), 8.60 (d, *J* = 7.6 Hz, 1H), 7.58 (s, 1H), 7.46 - 7.44 (m, 3H), 7.22 (s, 1H), 3.92 (s, 2H), 3.47-3.40 (m, 2H), 3.15 (t, *J* = 8.8 Hz, 2H), 2.40 (s, 3H).

Step 2. $Cs_2CO_3$ (138 mg, 0.42 mmol) was added to a solution of 1-(2-((5-fluoro-6-hydroxypyridin-3-yl)methyl)pyridin-4-yl)-3-methyl-1,5,6,7-tetrahydro-4*H*-pyrazolo[4,3-*c*]pyridin-4-one (100 mg, 0.28 mmol) in DMF (10 mL), followed by the addition of sodium 2-chloro-2,2-difluoroacetate (44 mg, 0.282 mmol) and the reaction mixture was heated at 100 °C for 3 h. The reaction mixture was partitioned between water (50 mL) and 10% MeOH in DCM (50 mL). The organic layer was separated, washed with brine solution (50 mL), dried ($Na_2SO_4$) and the solvent removed *in vacuo.* The residue was purified by prep HPLC (Method 4). The residue was partitioned between 10% $NaHCO_3$ solution (25 mL) and EtOAc (25 mL). The organic layer was separated, dried ($Na_2SO_4$) and the solvent removed *in vacuo* to afford 1-(2-((6-(difluoromethoxy)-5-fluoropyridin-3-yl)methyl)pyridin-4-yl)-3-methyl-1,5,6,7-tetrahydro-4*H*-pyrazolo[4,3-*c*]pyridin-4-one as a white solid (10 mg, 9%). **Data in table 3.**

**Procedure 24:**

**Example 112, 2-(2-(3-fluoro-5-(trifluoromethyl)benzyl)pyridin-4-yl)-6,7-dihydro-2*H*-pyrazolo[4,3-*f*][1,4]oxazepin-4(5*H*)-one**

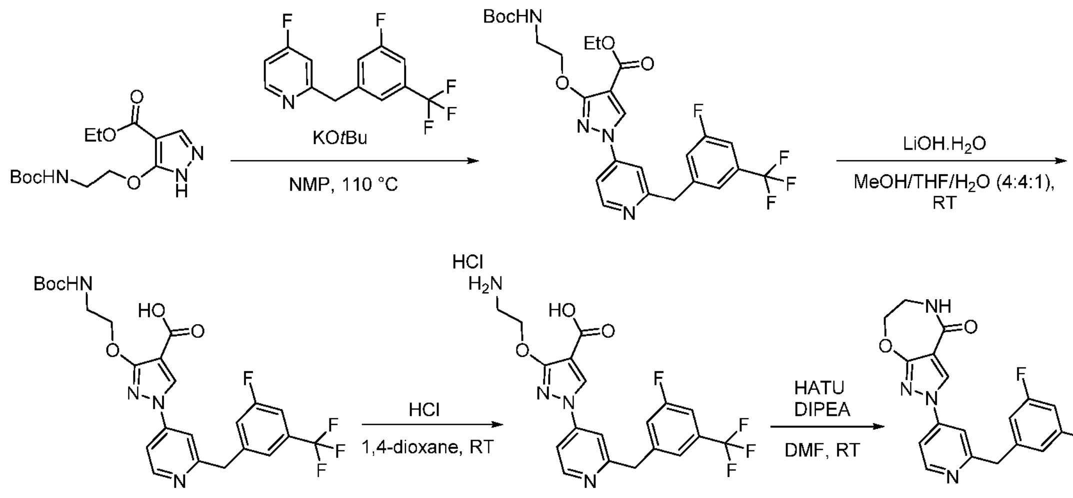


Step 1. Potassium *t*-butoxide (150 mg, 1.336 mmol) was added to a solution of ethyl 5-(2-((*tert*-butoxycarbonyl)amino)ethoxy)-1*H*-pyrazole-4-carboxylate (Intermediate 43, 200 mg, 0.67 mmol) and 4-fluoro-2-(3-fluoro-5-(trifluoromethyl)benzyl)pyridine (Intermediate 3, 183 mg, 0.67 mmol) in NMP (10 mL) at RT and the reaction mixture was heated at 110 °C for 16 h. The solvent was removed *in vacuo.* The residue was purified by gradient flash column chromatography eluting with 0-40% EtOAc in pet-ether to afford ethyl 3-(2-((*tert*-butoxycarbonyl)amino)ethoxy)-1-(2-(3-fluoro-5-(trifluoromethyl)benzyl)pyridin-4-yl)-1*H*-pyrazole-4-carboxylate as an off-white solid (200 mg, 54%).

**LCMS (Method 3):** m/z 553.1 (ES+), at 2.69 min.

**$^1$H NMR:** (400 MHz, DMSO-$d_6$) δ: 9.11 (s, 1H), 8.56 (d, *J* = 5.6 Hz, 1H), 7.96 (s, 1H), 7.76-7.74 (m, 1H), 7.58-7.52 (m, 3H), 6.98 (t, *J* = 5.6 Hz, 1H), 4.32-4.21 (m, 6H), 3.38-3.36 (m, 2H), 1.37 (s, 9H), 1.29 (t, *J* = 6.8 Hz, 3H).

Step 2. LiOH.$H_2O$ (27 mg, 0.63 mmol) was added to a solution of ethyl 3-(2-((*tert*-butoxycarbonyl)amino)ethoxy)-1-(2-(3-fluoro-5-(trifluoromethyl)benzyl)pyridin-4-yl)-1*H*-pyrazole-4-carboxylate (120 mg, 0.217 mmol) in MeOH:THF:$H_2O$ (2 mL:2 mL:0.5 mL) and the reaction mixture was stirred at RT for 6 h. The reaction mixture was diluted with water (20 mL), neutralised with 10% citric acid solution (10 mL) to pH~7 and extracted with EtOAc (2x30 mL). The combined organic layers were washed with brine solution (30 mL), dried ($Na_2SO_4$) and the solvent removed *in vacuo* to afford 3-(2-((*tert*-butoxycarbonyl)amino)ethoxy)-1-(2-(3-fluoro-5-(trifluoromethyl)benzyl)pyridin-4-yl)-1*H*-pyrazole-4-carboxylic acid as a yellow solid (110 mg, 95%).

**LCMS (Method 3):** m/z 525.1 (ES+), at 2.15 min.

**$^1$H NMR:** (400 MHz, DMSO-$d_6$) δ: 12.38 (s, 1H), 9.06 (s, 1H), 8.54 (d, *J* = 7.6 Hz, 1H), 7.93 (s, 1H), 7.93-7.71 (m, 1H), 7.58-7.51 (m, 3H), 7.00 (t, *J* = 5.2 Hz, 1H), 4.29-4.26 (m, 4H), 2.44-2.39 (m, 2H), 1.37 (s, 9H).

Step 3. HCl solution (4 M in 1,4-dioxane, 2.2 mL) was added to a suspension of 3-(2-((*tert*-butoxycarbonyl)amino)ethoxy)-1-(2-(3-fluoro-5-(trifluoromethyl)benzyl)pyridin-4-yl)-1*H*-pyrazole-4-carboxylic acid (110 mg, 0.21 mmol) in 1,4-dioxane (2 mL) and the reaction mixture was stirred at RT for 3h. The solvent was removed *in vacuo* to afford 3-(2-aminoethoxy)-1-(2-(3-fluoro-5-(trifluoromethyl)benzyl)pyridin-4-yl)-1*H*-pyrazole-4-carboxylic acid hydrochloride as a yellow solid (80 mg, 83%).

**LCMS (Method 3):** m/z 425.1 (ES+), at 1.16 min.

Step 4. DIPEA (48 mg, 0.38 mmol) and HATU (143 mg, 0.38 mmol) were added to a solution of 3-(2-aminoethoxy)-1-(2-(3-fluoro-5-(trifluoromethyl)benzyl)pyridin-4-yl)-1*H*-pyrazole-4-carboxylic acid hydrochloride (80 mg, 0.17 mmol) in DMF (1.6 mL) and the reaction mixture was stirred at RT for 8 h. The reaction mixture was diluted with water (50 mL) and extracted with EtOAc (2x30 mL). The combined organic layers were washed with brine solution (50 mL), dried ($Na_2SO_4$) and the solvent removed *in vacuo.* The residue was purified by prep HPLC (Method 4). The residue was partitioned between 10% $NaHCO_3$ solution (20 mL) and EtOAc (20 mL). The organic layer was separated, dried ($Na_2SO_4$) and the solvent removed *in vacuo* to afford 2-(2-(3-fluoro-5-(trifluoromethyl)benzyl)pyridin-4-yl)-6,7-dihydro-2*H*-pyrazolo[4,3-*f*][1,4]oxazepin-4(5*H*)-one as a white solid (30 mg, 42%). **Data in table 3.**

**Procedure 25:**

**Example 116, 1-(2-(3-fluoro-5-(trifluoromethyl)benzyl)pyridin-4-yl)-3-(trifluoromethyl)-1,5,6,7-tetrahydro-4*H*-pyrazolo[4,3-*c*]pyridin-4-one**

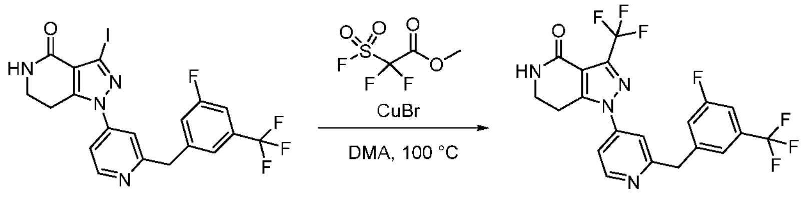


CuBr (8.3 mg, 0.058 mmol) was added to a solution of 1-(2-(3-fluoro-5-(trifluoromethyl)benzyl)pyridin-4-yl)-3-iodo-1,5,6,7-tetrahydro-4*H*-pyrazolo[4,3-*c*]pyridin-4-one (150 mg, 0.290 mmol) in DMA (8 mL) followed by the addition of methyl 2,2-difluoro-2-(fluorosulfonyl)acetate (334 mg, 1.74 mmol). The reaction mixture was heated in a microwave at 100 °C for 1 h. The reaction mixture was partitioned between water (10 mL) and EtOAc (20 mL). The organic layer was separated, dried ($Na_2SO_4$) and the solvent removed *in vacuo.* The residue was purified by prep HPLC (Method 4). The residue was partitioned between 10% $NaHCO_3$ solution (5 mL) and EtOAc (10 mL). The organic layer was separated, dried ($Na_2SO_4$) and the solvent removed *in vacuo* to afford 1-(2-(3-fluoro-5-(trifluoromethyl)benzyl)pyridin-4-yl)-3-(trifluoromethyl)-1,5,6,7-tetrahydro-4*H*-pyrazolo[4,3-*c*]pyridin-4-one as a white solid (14 mg, 10%). **Data in table 3.**

**Procedure 26:**

**Example 117, 1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one**

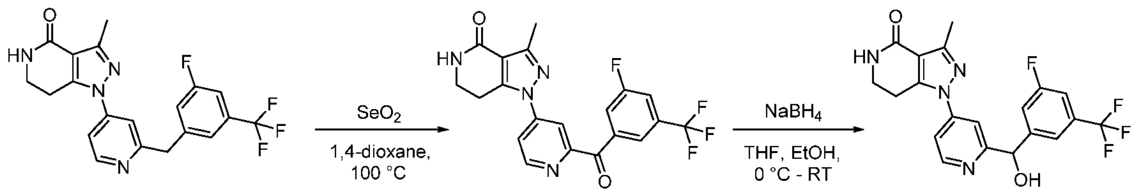


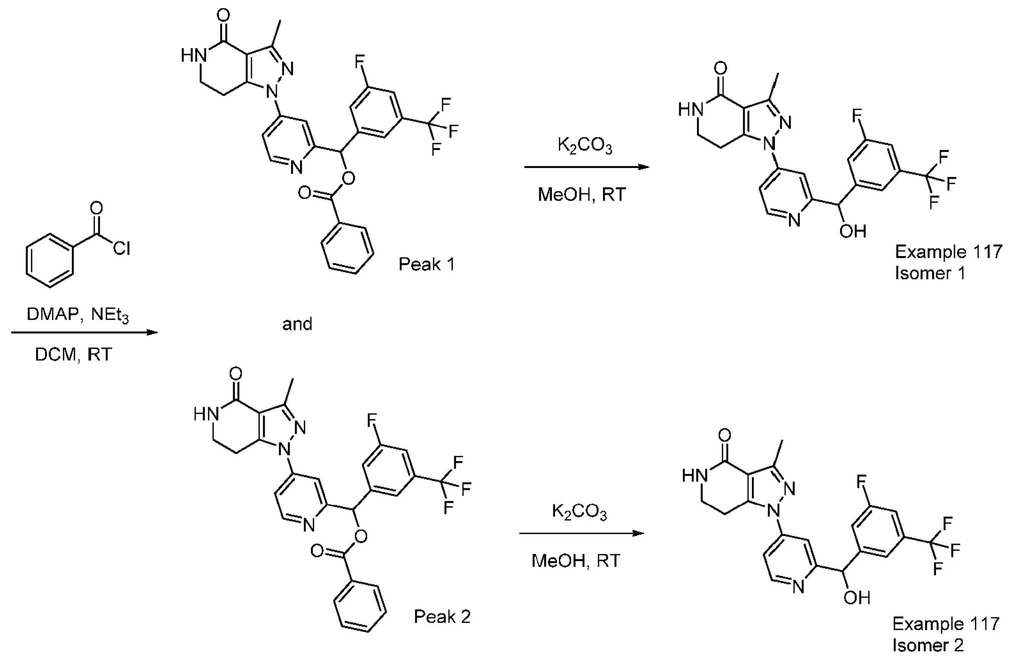

Peak 1
Example 117 Isomer 1
and
Peak 2
Example 117 Isomer 2

Steps 1 and 2. 1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one (300 mg, 58%) was prepared from 1-(2-(3-fluoro-5-(trifluoromethyl)benzyl)pyridin-4-yl)-3-methyl-1,5,6,7-tetrahydro-4*H*-pyrazolo[4,3-*c*]pyridin-4-one (Example 75, 500 mg, 1.23 mmol) using the methods of Procedure 11, steps 1 and 2.

**LCMS (Method 3):** m/z 421.1 (ES+), at 1.80 min.

**$^1$H NMR:** (400 MHz, DMSO-$d_6$) δ: 8.60 (d, *J* = 5.2 Hz, 1H), 7.90 (d, *J* = 2.4 Hz, 1H), 7.70 (s, 1H), 7.65-7.62 (m, 2H), 7.49-7.48 (m, 1H), 7.43 (s, 1H), 6.65 (d, *J* = 4.4 Hz, 1H), 5.94 (d, *J* = 4.4 Hz, 1H), 3.43-3.41 (m, 2H), 3.19-3.15 (m, 2H), 2.42 (s, 3H).

Step 3. $NEt_3$ (217 mg, 2.14 mmol) and DMAP (17.4 mg, 0.143 mmol) were added to a solution of 1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one (300 mg, 0.714 mmol) in DCM (10 mL) followed by the addition of benzoyl chloride (110 mg, 0.785 mmol) and the reaction mixture was stirred at RT for 15 h. The solvent was removed *in vacuo.* The residue was purified by gradient flash column chromatography eluting with 0-60% EtOAc in pet-ether. The residue was subjected to chiral SFC (Method 1 - isocratic run 20% co-solvent) to afford (3-fluoro-5-(trifluoromethyl)phenyl)(4-(3-methyl-4-oxo-4,5,6,7-tetrahydro-1*H*-pyrazolo[4,3-*c*]pyridin-1-yl)pyridin-2-yl)methyl benzoate as an off-white solid (Peak 1, 100 mg, 26%) and (3-fluoro-5-(trifluoromethyl)phenyl)(4-(3-methyl-4-oxo-4,5,6,7-tetrahydro-1*H*-pyrazolo[4,3-*c*]pyridin-1-yl)pyridin-2-yl)methyl benzoate as an off-white solid (Peak 2, 110 mg, 29%).

Peak 1:

**LCMS (Method 3):** m/z 525.1 (ES+), at 2.33 min.

**Chiral SFC purity analysis (Method 3 - isocratic run 20% co-solvent):** 4.25 min, 100%.

Peak 2:

**LCMS (Method 3):** m/z 525.1 (ES+), at 2.32 min.

**Chiral SFC purity analysis (Method 3 - isocratic run 20% co-solvent):** 4.76 min, 89%.

Step 4 (Peak 1). $K_2CO_3$ (79 mg, 0.572 mmol) was added to a solution of (3-fluoro-5-(trifluoromethyl)phenyl)(4-(3-methyl-4-oxo-4,5,6,7-tetrahydro-1*H*-pyrazolo[4,3-*c*]pyridin-1-yl)pyridin-2-yl)methyl benzoate (Peak 1, 100 mg, 0.191 mmol) in MeOH (1 mL) and the reaction mixture was stirred at RT for 2 h. The reaction mixture was partitioned between DCM (10 mL) and $H_2O$ (10 mL). The organic layer was separated, dried ($Na_2SO_4$) and the solvent removed *in vacuo.* The residue was purified by chiral SFC (Method 5 - isocratic run 20% co-solvent) to afford 1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one (Isomer 1) as an off-white solid (28 mg, 35%). **Data in table 3.**

Step 4 (Peak 2). $K_2CO_3$ (87 mg, 0.629 mmol) was added to a solution of (3-fluoro-5-(trifluoromethyl)phenyl)(4-(3-methyl-4-oxo-4,5,6,7-tetrahydro-1*H*-pyrazolo[4,3-*c*]pyridin-1-yl)pyridin-2-yl)methyl benzoate (Peak 2, 110 mg, 0.210 mmol) in MeOH (1 mL) and the reaction mixture was stirred at RT for 2 h. The reaction mixture was partitioned between DCM (10 mL) and $H_2O$ (10 mL). The organic layer was separated, dried ($Na_2SO_4$) and the solvent removed *in vacuo.* The residue was purified by chiral SFC (Method 5 - isocratic run 20% co-solvent) to afford 1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one (Isomer 2) as an off-white solid (41 mg, 46%). **Data in table 3.**

**Procedure 27:**

**Example 118, 1-(4-((3-fluoro-5-(trifluoromethyl)phenyl)(hydroxy)methyl)pyridin-2-yl)-3-methyl-1,5,6,7-tetrahydro-4*H*-pyrazolo[4,3-*c*]pyridin-4-one**

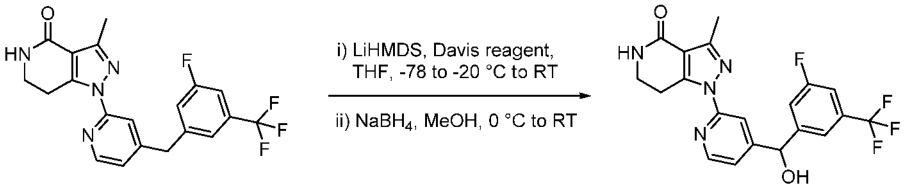


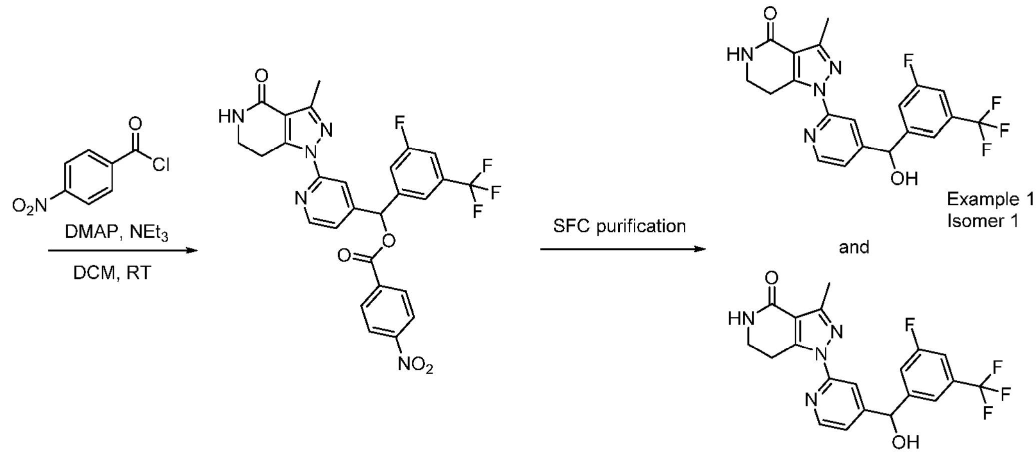


Example 118 Isomer 1

and

Example 118 Isomer 2

Step 1. LiHMDS solution (1 M in THF, 3.71 mL, 3.71 mmol) was added to a solution of 1-(4-(3-fluoro-5-(trifluoromethyl)benzyl)pyridin-2-yl)-3-methyl-1,5,6,7-tetrahydro-4*H*-pyrazolo[4,3-*c*]pyridin-4-one (Example 95, 500 mg, 1.23 mmol) in THF (5 mL) at -78 °C and stirred for 10 minutes, followed by the addition of Davis reagent (1 g, 3.71 mmol) at -20 °C. The reaction mixture was stirred at RT for 16 h. The reaction mixture was quenched with sat. $NH_4Cl$ solution (40 mL) and the aqueous layer extracted with EtOAc (2x50 mL). The combined organic layers were dried ($Na_2SO_4$) and the solvent removed *in vacuo.* The residue was purified by gradient flash column chromatography eluting with 0-100% EtOAc in pet-ether. After removal of the solvent *in vacuo* the residue was dissolved MeOH (5 mL) and sodium borohydride (52 mg, 0.69 mmol) was added at 0 °C. The reaction mixture was stirred at RT for 3 h. The reaction mixture was partitioned between EtOAc (20 mL) and water (20 mL). The organic layer was separated, dried ($Na_2SO_4$) and the solvent removed *in vacuo.* The residue was purified by gradient flash column chromatography eluting with 0-80% EtOAc in pet-ether to afford 1-(4-((3-fluoro-5-(trifluoromethyl)phenyl)(hydroxy)methyl)pyridin-2-yl)-3-methyl-1,5,6,7-tetrahydro-4*H*-pyrazolo[4,3-*c*]pyridin-4-one as an off-white solid (250 mg, 48%).

**LCMS (Method 6):** m/z 421.1 (ES+), at 2.12 min.

**$^1$H NMR:** (400 MHz, $CDCl_3$) δ: 8.42 (s, 1H), 8.05 (d, *J* = 2.4 Hz, 1H), 7.51 (s, 1H), 7.37-7.30 (m, 2H), 7.29-7.26 (m, 1H), 6.86-6.70 (m, 1H), 3.70-3.63 (m, 4H), 2.54 (s, 3H). 2 exchangeable protons not observed.

Step 2. 4-Nitrobenzoyl chloride (105 mg, 0.571 mmol) and $NEt_3$ (0.2 mL, 1.71 mmol) were added to a solution of 1-(4-((3-fluoro-5-(trifluoromethyl)phenyl)(hydroxy)methyl)pyridin-2-yl)-3-methyl-1,5,6,7-tetrahydro-4*H*-pyrazolo[4,3-*c*] pyridin-4-one (240 mg, 0.57 mmol) in DCM (5 mL) and the reaction mixture was stirred at RT for 3 h. The solvent was removed *in vacuo* and the residue was purified by prep HPLC (Method 1). The residue obtained was dissolved in EtOAC (50 mL) and washed with water (50 mL). The organic layer was separated, dried ($Na_2SO_4$) and the solvent removed *in vacuo.* The residue was subjected to chiral SFC (Method 7 - isocratic run 20% co-solvent). During evaporation of chiral SFC fractions, the 4-nitro benzoyl ester was hydrolysed therefore both elutions were further purified by gradient flash column chromatography eluting with 0-80% EtOAc in pet-ether to afford 1-(4-((3-fluoro-5-(trifluoromethyl)phenyl)(hydroxy)methyl)pyridin-2-yl)-3-methyl-1,5,6,7-tetrahydro-4*H*-pyrazolo[4,3-*c*]pyridin-4-one (Isomer 1) as an off-white solid (25 mg, 11%) and 1-(4-((3-fluoro-5-(trifluoromethyl)phenyl)(hydroxy)methyl)pyridin-2-yl)-3-methyl-1,5,6,7-tetrahydro-4*H*-pyrazolo[4,3-*c*]pyridin-4-one (Isomer 2) as an off-white solid (27 mg, 11%). **Data in table 3.**

Further examples prepared by the above procedures are detailed in Table 3.

**Table 2 - Intermediates**

| Intermediate | Name | Structure | Data |
|---|---|---|---|
| 1 | (*E*)-3-((dimethylamino)methylene)piperidine-2,4-dione | 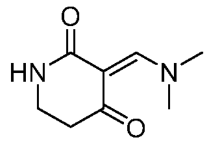 | **LCMS (Method 2):** m/z 169.2 (ES+), at 0.57 min & 0.73 min.<br>**$^1$H NMR:** (400 MHz, DMSO-$d_6$) δ: 7.83 (s, 1H), 7.15 (s, 1H), 3.29 (s, 3H), 3.17-3.15 (m, 2H), 3.10 (s, 3H), 2.30 (t, *J* = 6.4 Hz, 2H). |
| 2 | 2-((dimethylamino)methylene)cyclohexane-1,3-dione | 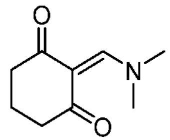 | **LCMS (Method 4):** m/z 168.2 (ES+), at 0.24 min.<br>**$^1$H NMR:** (400 MHz, DMSO-$d_6$) δ: 7.99 (s, 1H), 3.39 (s, 3H), 2.90 (s, 3H), 2.53-2.50 (m, 4H), 1.81 (t, *J* = 6.2 Hz, 2H). |
| 3 | 4-fluoro-2-(3-fluoro-5-(trifluoromethyl) benzyl)pyridine | 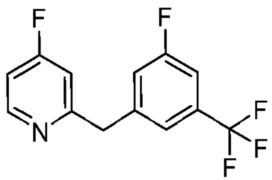 | **LCMS (Method 5):** m/z 274.1 (ES+), at 6.26 min.<br>**$^1$H NMR:** (400 MHz, DMSO-$d_6$) δ: 8.54 (dd, *J* = 9.1, 5.7 Hz, 1H), 7.60-7.45 (m, 3H), 7.36 (dd, *J* = 10.2, 2.5 Hz, 1H), 7.20 (ddd, *J* = 8.6, 5.7, 2.5 Hz, 1H), 4.24 (s, 2H). |
| 4 | 2-fluoro-4-(3-(trifluoromethyl)benzyl) pyridine | 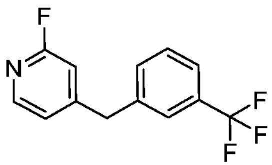 | **LCMS (Method 4):** m/z 256.2 (ES+), at 1.52 min.<br>**$^1$H NMR:** (400 MHz, Methanol-$d_4$) δ: 7.99 (dt, *J* = 5.2, 0.6 Hz, 1H), 7.52-7.35 (m, 4H), 7.06 (dddt, *J* = 5.2, 2.0, 1.3, 0.6 Hz, 1H), 6.83 (tq, *J* = 1.4, 0.7 Hz, 1H), 4.05 (s, 2H). |
| 5 | 2-fluoro-4-(3-fluoro-5-(trifluoromethyl) benzyl)pyridine | 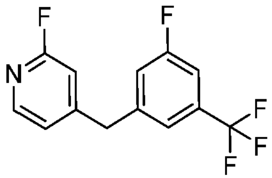 | **LCMS (Method 3):** m/z 274.0 (ES+), at 2.54 min.<br>**$^1$H NMR:** (400 MHz, DMSO-$d_6$) δ: 8.17 (d, *J* = 4.4 Hz, 1H), 7.65-7.51 (m, 3H), 7.29 (d, *J* = 1.2 Hz, 1H), 7.16 (s, 1H), 4.17 (d, *J* = 3.6 Hz, 2H). |
| 6 | 2-(3-fluoro-5-(trifluoromethyl)benzyl)-4-hydrazineylpyridine | 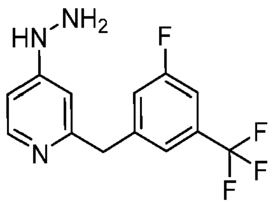 | **LCMS (Method 3):** m/z 286.1 (ES+), at 1.19 min.<br>**$^1$H NMR:** (400 MHz, DMSO-$d_6$) δ: 7.95 (d, *J* = 6.0 Hz, 1H), 7.56-7.21 (m, 4H), 6.59 (d, *J* = 1.6 Hz, 1H), 6.51-6.49 (m, 1H), 4.15 (s, 2H), 3.99 (s, 2H). |

(continued)

| Intermediate | Name | Structure | Data |
|---|---|---|---|
| 7 | 2-hydrazineyl-4-(3-(trifluoromethyl)benzyl)pyridine | 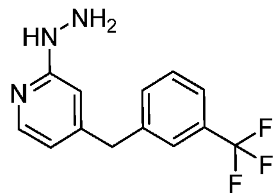 | **LCMS (Method 4):** m/z 268.2 (ES+), at 1.24 min.<br>**$^1$H NMR:** (400 MHz, DMSO-*d*$_6$) δ: 7.88 (dd, *J* = 5.2, 0.7 Hz, 1H), 7.69-7.43 (m, 4H), 7.36 (s, 1H), 6.57 (s, 1H), 6.43 (dd, *J* = 5.2, 1.6 Hz, 1H), 4.10 (br s, 2H), 3.93 (s, 2H). |
| 8 | 4-(3-fluoro-5-(trifluoromethyl)benzyl)-2-hydrazineylpyridine | 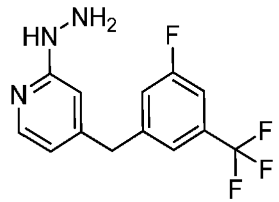 | **LCMS (Method 3):** m/z 286.2 (ES+), at 1.37 min.<br>**$^1$H NMR:** (400 MHz, DMSO-*d*$_6$) δ: 7.88 (d, *J* = 5.2 Hz, 1H), 7.53-7.44 (m, 3H), 7.36 (s, 1H), 6.57 (s, 1H), 6.45 - 6.44 (m, 1H), 4.07 (s, 2H), 3.95 (s, 2H). |
| 9 | 5-(3-fluoro-5-(trifluoromethyl)benzyl)-3-hydrazineylpyridazine | 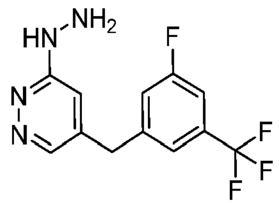 | **LCMS (Method 3):** m/z 287.1 (ES+), at 1.37 min.<br>**$^1$H NMR:** Not recorded. |
| 10 | 1-(6-chloropyridazin-4-yl)-1,5,6,7-tetrahydro-4*H*-pyrazolo[4,3-*c*]pyridin-4-one | 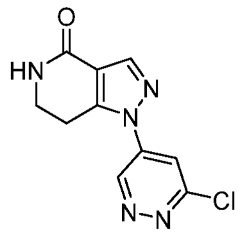 | **LCMS (Method 3):** m/z 250.0 (ES+), at 1.02 min.<br>**$^1$H NMR:** (400 MHz, DMSO-*d*$_6$) δ: 9.66 (d, *J* = 1.6 Hz, 1H), 8.20 (d, *J* = 1.4 Hz, 1H), 8.13 (s, 1H), 7.61 (s, 1H), 3.45-3.44 (m, 2H), 3.31-3.30 (m, 2H). |
| 11 | 1-(5-bromopyridin-3-yl)-1,5,6,7-tetrahydro-4*H*-pyrazolo[4,3-*c*]pyridin-4-one | 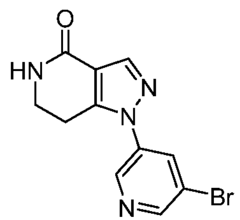 | **LCMS (Method 3):** m/z 293.1 (ES+), at 1.26 min.<br>**$^1$H NMR:** (400 MHz, DMSO-*d*$_6$) δ: 8.89-8.81 (m, 2H), 8.38-8.37 (m, 1H), 8.06 (s, 1H), 7.49 (s, 1H), 3.45-3.41 (m, 2H), 3.16-3.12 (m, 2H). |

(continued)

| Intermediate | Name | Structure | Data |
|---|---|---|---|
| 12 | 1-(4-bromopyridin-2-yl)-1,5,6,7-tetrahydro-4*H*-pyrazolo[4,3-*c*]pyridin-4-one | 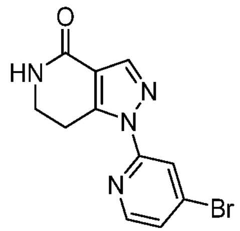 | **MS (Method 1):** m/z 293, 295 (ES+).<br>**$^1$H NMR:** (400 MHz, DMSO-$d_6$) δ: 8.41 (d, *J* = 5.3 Hz, 1H), 8.12 (s, 1H), 8.03 (s, 1H), 7.69 (d, *J* = 4.0 Hz, 1H), 7.49 (bs, 1H), 3.62-3.50 (m, 4H). |
| 13 | 1-(2-bromopyridin-4-yl)-1,5,6,7-tetrahydro-4*H*-pyrazolo[4,3-*c*]pyridin-4-one | 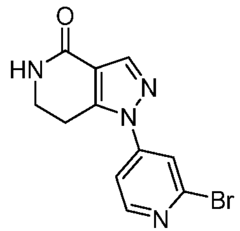 | **LCMS (Method 3):** m/z 293.1 (ES+), at 1.24 min.<br>**$^1$H NMR:** (400 MHz, DMSO-$d_6$) δ: 8.54 (d, *J* = 7.6 Hz, 1H), 8.09 (s, 1H), 7.90 (d, *J* = 2.0 Hz, 1H), 7.76-7.75 (m, 1H), 7.54 (s, 1H), 3.43 (d, *J* = 8.8 Hz, 2H), 3.23 (t, *J* = 8.8 Hz, 2H). |
| 14 | *tert*-butyl 1-(2-bromopyridin-4-yl)-4-oxo-1,4,6,7-tetrahydro-5*H*-pyrazolo[4,3-*c*]pyridine-5-carboxylate | 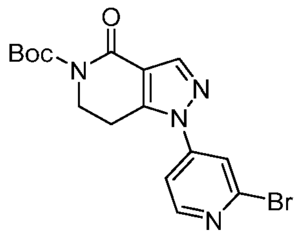 | **LCMS (Method 3):** m/z 393.1 (ES+), at 2.11 min.<br>**$^1$H NMR:** (400 MHz, DMSO-$d_6$) δ: 8.57 (d, *J* = 5.6 Hz, 1H), 8.25 (s, 1H), 7.91 (d, *J* = 1.6 Hz, 1H), 7.76-7.75 (m, 1H), 4.01 (t, *J* = 6.0 Hz, 2H), 3.36-3.34 (m, 2H), 1.49 (s, 9H). |
| 15 | 1,5,6,7-tetrahydro-4*H*-benzo[*d*][1,2,3]triazol-4-one | 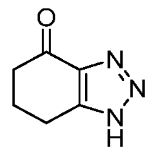 | **MS (Method 1):** m/z 138 (ES+).<br>**$^1$H NMR:** (400 MHz, DMSO-$d_6$) δ: 2.91 (t, *J* = 6.0 Hz, 2H), 2.56-2.43 (m, 2H), 213-202 (m, 2H). 1 exchangeable proton not observed. |
| 16 | 1,5,6,7-tetrahydro-4*H*-[1,2,3]triazolo[4,5-*c*]pyridin-4-one | 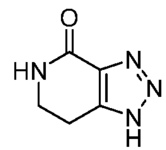 | **MS (Method 1):** m/z 139.0 (ES+).<br>**$^1$H NMR:** Not recorded. |

(continued)

| Intermediate | Name | Structure | Data |
|---|---|---|---|
| 17 | (*E*)-3-((dimethylamino)methylene)-6-methylpiperidine-2,4-dione | 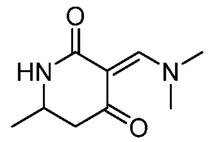 | **MS (Method 1):** m/z 183.0 (ES+).<br>**$^1$H NMR:** Not recorded. |
| 18 | 2-chloro-4-(3-fluoro-5-(trifluoromethyl) benzyl)pyridine | 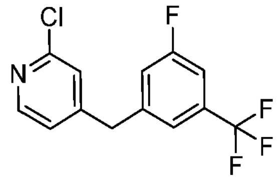 | **LCMS (Method 9):** m/z 290.2 (ES+), at 2.30 min.<br>**$^1$H NMR:** Not recorded. |
| 19 | ethyl (Z)-4-(*tert*-butoxy)-2-((dimethylamino)methylene)-3-oxobutanoate | 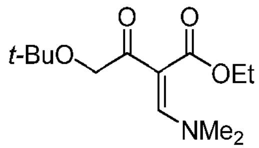 | **LCMS (Method 9):** m/z 258.2 (ES+), at 1.57 min.<br>**$^1$H NMR:** Not recorded. |
| 20 | (*E*)-3-((dimethylamino)methylene)-5-methylpiperidine-2,4-dione | 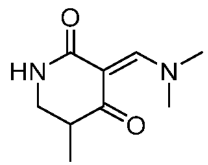 | **LCMS:** Not recorded.<br>**$^1$H NMR:** (400 MHz, DMSO-$d_6$) δ: 7.81 (s, 1H), 7.15 (s, 1H), 3.32 (s, 3H), 3.28-3.17 (m, 1H), 3.03 (s, 3 H), 2.89-2.88 (m, 1H), 2.32-2.28 (m, 1H), 0.97 (d, *J* = 6.9 Hz, 3H). |
| 21 | *tert*-butyl 1-(2-bromopyridin-4-yl)-4-oxo-1,4,6,7-tetrahydro-5*H*-pyrrolo [3,2-*c*]pyridine-5-carboxylate | 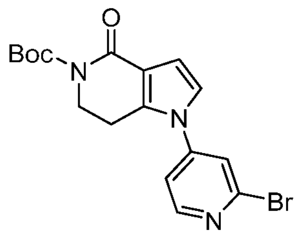 | **LCMS (Method 7):** m/z 336.0 (ES+), at 2.14 min.<br>**$^1$H NMR:** (400 MHz, DMSO-$d_6$) δ: 8.52 (d, *J* = 7.6 Hz, 1H), 7.87 (s, 1H), 7.62 (t, *J* = 2.8 Hz, 1H), 7.38 (d, *J* = 4.0 Hz, 1H), 6.64 (d, *J* = 4.4 Hz, 1H), 3.94 (t, *J* = 8.0 Hz, 2H), 3.09 (t, *J* = 8.0 Hz, 2H), 1.48 (s, 9H). |
| 22 | 1-(chloromethyl)-3-cyclopropyl-5-fluorobenzene | 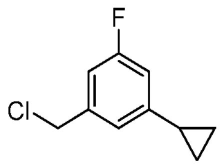 | **GCMS (Method 1):** m/z 184.0 (ES+), at 7.02 min.<br>**$^1$H NMR:** Not recorded. |

(continued)

| Intermediate | Name | Structure | Data |
|---|---|---|---|
| 23 | *tert*-butyl 1-(6-chloropyrimidin-4-yl)-4-oxo-1,4,6,7-tetrahydro-5*H*-pyrazolo [4,3-*c*]pyridine-5-carboxylate | 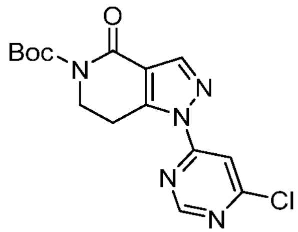 | **LCMS (Method 3):** m/z 348.0 (ES+), at 2.25 min.<br>**$^1$H NMR:** (400 MHz, DMSO-$d_6$) δ: 9.05 (d, *J* = 1.2 Hz, 1H), 8.32 (s, 1H), 8.08 (d, *J* = 0.8 Hz, 1H), 4.06-4.02 (m, 2H), 3.59 (t, *J* = 6.4 Hz, 2H), 1.49 (s, 9H). |
| 24 | 4-(chloromethyl)-1-(difluoromethoxy)-2-fluorobenzene | 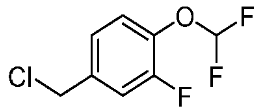 | **GCMS (Method 2):** m/z 210.0 (ES+), at 2.46 min.<br>**$^1$H NMR:** Not recorded. |
| 25 | 5-(bromomethyl)-1,2-difluoro-3-(trifluoromethyl)benzene | 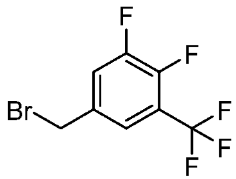 | **GCMS:** Not recorded.<br>**$^1$H NMR:** Not recorded.<br>**TLC:** $R_f$ = 0.7 (25% EtOAc in pet-ether) |
| 26 | 1-(chloromethyl)-3-(difluoromethyl)-5-fluorobenzene | 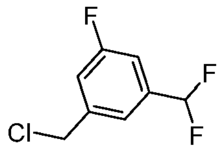 | **GCMS (Method 2):** m/z 193.9 (ES+), at 2.25 min.<br>**$^1$H NMR:** Not recorded. |
| 27 | 1-chloro-5-(chloromethyl)-2,3-difluorobenzene | 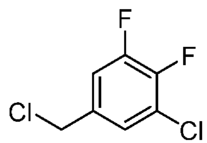 | **GCMS (Method 2):** m/z 197.0 (ES+), at 2.51 min.<br>**$^1$H NMR:** (400 MHz, DMSO-$d_6$) δ: 7.63-7.58 (m, 2H), 4.76 (s, 2H). |
| 28 | 5-(chloromethyl)-1,3-difluoro-2-(trifluoromethyl)benzene | 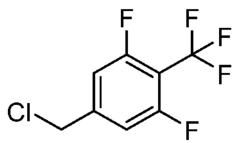 | **GCMS (Method 2):** m/z 230.0 (ES+), at 2.04 min.<br>**$^1$H NMR:** (400 MHz, $CDCl_3$) δ: 7.10 (s, 1H), 7.08 (s, 1H), 4.57 (s, 2H). |

(continued)

| Intermediate | Name | Structure | Data |
|---|---|---|---|
| 29 | 1-(chloromethyl)-3-fluoro-5-isopropyl-benzene | 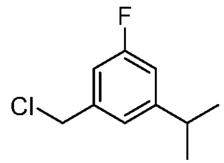 | **GCMS (Method 2):** m/z 186.0 (ES+), at 2.77 min. **$^1$H NMR:** (400 MHz, DMSO-$d_6$) δ: 7.18 (s, 1H), 7.11-7.07 (m, 2H), 4.75 (s, 2H), 2.96-2.89 (m, 1H), 1.20 (d, *J* = 6.9 Hz, 6H). |
| 30 | 1-(chloromethyl)-3-fluoro-5-(1-(trifluoro-methyl)cyclopropyl)benzene | 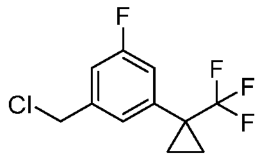 | **LCMS (Method 2):** m/z No ionisation observed (ES+), at 2.80 min. **$^1$H NMR:** (400 MHz, $CDCl_3$) δ: 7.28 (s, 1H), 7.16-7.11 (m, 2H), 4.58 (s, 2H), 1.40 (t, *J* = 6.8 Hz, 2H), 1.07 (t, *J* = 4.8 Hz, 2H). |
| 31 | 1-chloro-5-(chloromethyl)-2-fluoro-3-(trifluoromethyl)benzene | 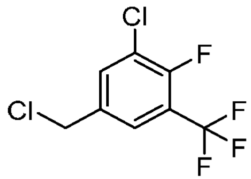 | **LCMS (Method 7):** m/z No ionisation observed (ES+), at 2.15 min. **$^1$H NMR:** Not recorded. |
| 32 | 5-(chloromethyl)-3-fluoro-2-methoxy-pyridine | 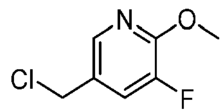 | **LCMS (Method 7):** m/z 176.1 (ES+), at 1.77 min. **$^1$H NMR:** (400 MHz, DMSO-$d_6$) δ: 8.08 (d, *J* = 2.0 Hz, 1H), 7.82-7.77 (m, 1H), 4.78 (s, 2H), 3.95 (s, 3H). |
| 33 | 5-(chloromethyl)-2-methoxypyridine | 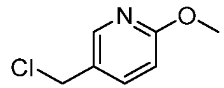 | **LCMS (Method 2):** m/z 157.7 (ES+), at 2.04 min. **$^1$H NMR:** (400 MHz, DMSO-$d_6$) δ: 8.24 (s, 1H), 7.80 (d, *J* = 3.6 Hz, 1H), 6.85 (d, *J* = 11.6 Hz, 1H), 4.77 (s, 2H), 3.86 (s, 3H). |
| 34 | 5-(chloromethyl)-2-(difluoromethoxy) pyridine | 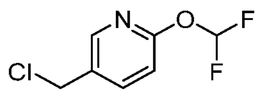 | **LCMS (Method 10):** m/z 194.1 (ES+), at 2.09 min. **$^1$H NMR:** (400 MHz, DMSO-$d_6$) δ: 8.35 (d, *J* = 2.4 Hz, 1H), 8.03-8.00 (m, 1H), 7.90-7.54 (m, 1H), 7.13 (d, *J* = 8.4 Hz, 1H), 4.83 (s, 2H). |
| 35 | 3-acetyl-1-(2,4-dimethoxybenzyl)-4-hydroxy-5,6-dihydropyridin-2(1*H*)-one | 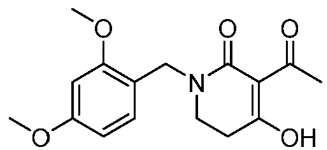 | **LCMS (Method 3):** m/z 306.0 (ES+), at 2.17 min. **$^1$H NMR:** (400 MHz, DMSO-$d_6$) δ: 7.12 (d, *J* = 8.0 Hz, 1H), 6.59 (d, *J* = 2.4 Hz, 1H), 6.52-6.50 (m, 1H), 4.53 (s, 2H), 3.80 (s, 3H), 3.76 (s, 3H), 3.42 (t, *J* = 6.8 Hz, 2H), 3.31 (s, 2H), 2.44 (s, 3H). 1 exchangeable proton not observed. |

(continued)

| Intermediate | Name | Structure | Data |
|---|---|---|---|
| 36 | 1-(2-bromopyridin-4-yl)-5-(2,4-dimethoxybenzyl)-3-methyl-1,5,6,7-tetrahydro-4*H*-pyrazolo[4,3-*c*]pyridin-4-one | 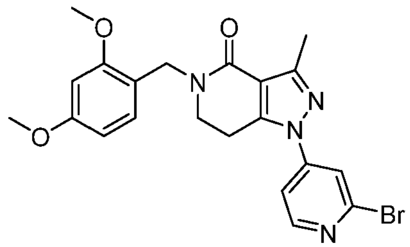 | **LCMS (Method 7):** m/z 457.1 (ES+), at 1.96 min. **$^1$H NMR:** (400 MHz, DMSO-$d_6$) δ: 8.49 (d, *J* = 7.2 Hz, 1H), 7.84 (d, *J* = 2.0 Hz, 1H), 7.70-7.68 (m, 1H), 7.11 (d, *J* = 11.6 Hz, 1H), 6.59 (d, *J* = 2.4 Hz, 1H), 6.51-6.48 (m, 1H), 4.51 (s, 2H), 3.81 (s, 3H), 3.75 (s, 3H), 3.54 (t, *J* = 9.2 Hz, 2H), 3.26 (t, *J* = 8.4 Hz, 2H), 2.43 (s, 3H). |
| 37 | 1-(5-bromopyridin-3-yl)-5-(2,4-dimethoxybenzyl)-3-methyl-1,5,6,7-tetrahydro-4*H*-pyrazolo[4,3-*c*]pyridin-4-one | 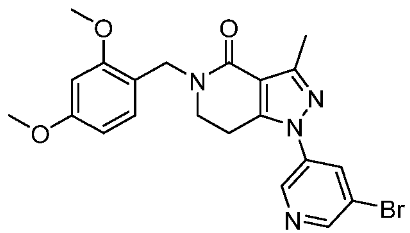 | **LCMS (Method 6):** m/z 457.1 (ES+), at 1.92 min. **$^1$H NMR:** (400 MHz, DMSO-$d_6$) δ: 8.84 (d, *J* = 2.0 Hz, 1H), 8.76 (d, *J* = 1.6 Hz, 1H), 8.31-8.30 (m, 1H), 7.12-7.10 (m, 1H), 6.59 (d, *J* = 2.4 Hz, 1H), 6.51-6.49 (m, 1H), 4.15 (s, 2H), 3.81-3.75 (m, 6H), 3.55-3.51 (m, 2H), 3.18-3.15 (m, 2H), 2.43 (s, 3H). |
| 38 | 1-(4-bromopyridin-2-yl)-5-(2,4-dimethoxybenzyl)-3-methyl-1,5,6,7-tetrahydro-4*H*-pyrazolo[4,3-*c*]pyridin-4-one | 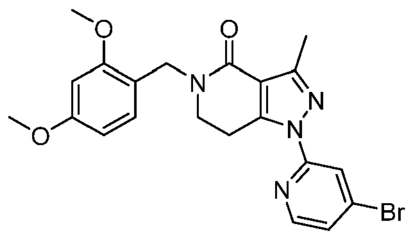 | **LCMS (Method 3):** m/z 459.0 (ES+), at 2.60 min. **$^1$H NMR:** (400 MHz, DMSO-$d_6$) δ: 8.37 (d, *J* = 5.6 Hz, 1H), 8.07 (d, *J* = 1.2 Hz, 1H), 7.65-7.63 (m, 1H), 7.10 (d, *J* = 8.4 Hz, 1H), 6.58 (d, *J* = 2.0 Hz, 1H), 6.51-6.48 (m, 1H), 4.51 (s, 2H), 3.81 (s, 3H), 3.75 (s, 3H), 3.56 (t, *J* = 13.6 Hz, 2H), 3.44 (t, *J* = 13.6 Hz, 2H), 2.44 (s, 3H). |
| 39 | 4-(3-(difluoromethyl)-5-fluorobenzyl)-2-hydrazineylpyridine | 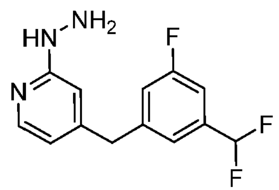 | **LCMS (Method 2):** m/z 267.9 (ES+), at 3.00 min. **$^1$H NMR:** Not recorded. |

(continued)

| Intermediate | Name | Structure | Data |
|---|---|---|---|
| 40 | 1-(5-fluoropyridin-3-yl)-1,5,6,7-tetrahydro-4*H*-pyrazolo[4,3-*c*]pyridin-4-one | 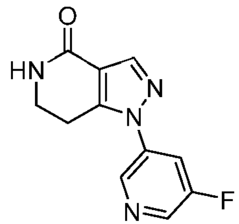 | **LCMS (Method 10):** m/z 233.2 (ES+), at 1.20 min.<br>**$^1$H NMR:** (400 MHz, DMSO-$d_6$) δ: 8.79 (s, 1H), 8.72-8.71 (m, 1H), 8.13-8.10 (m, 1H), 8.08 (s, 1H), 7.50 (s, 1H), 3.46-3.42 (m, 2H), 3.15 (t, *J* = 6.4 Hz, 2H). |
| 41 | *tert*-butyl 1-(2-bromo-5-methylpyridin-4-yl)-4-oxo-1,4,6,7-tetrahydro-5*H*-pyrazolo[4,3-c]pyridine-5-carboxylate | 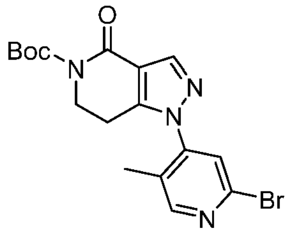 | **LCMS (Method 3):** m/z 407.0 (ES+), at 2.00 min.<br>**$^1$H NMR:** (400 MHz, DMSO-$d_6$) δ: 8.54 (s, 1H), 8.19 (s, 1H), 7.88 (s, 1H), 4.00 (t, *J* = 2.8 Hz, 2H), 3.00 (t, *J* = 6.0 Hz, 2H), 2.19 (s, 3H), 1.48 (s, 9H). |
| 42 | *tert*-butyl 2-(3-(3-fluoro-5-(trifluoromethyl)benzyl)phenyl)hydrazine-1-carboxylate | 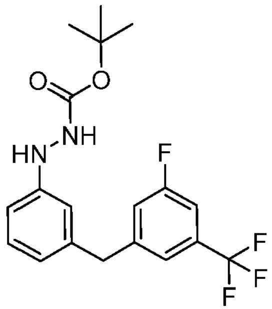 | **LCMS (Method 1):** m/z 407.0 (ES-), at 5.56 min.<br>**$^1$H NMR:** (400 MHz, DMSO-$d_6$) δ: 8.71 (s, 1H), 7.59-7.29 (m, 2H), 7.08 (t, *J* = 7.7 Hz, 1H), 6.62 (d, *J* = 7.4 Hz, 1H), 6.58-6.43 (m, 2H), 5.03 (s, 1H), 4.06 (s, 1H), 3.97 (s, 2H), 1.38 (d, *J* = 2.4 Hz, 9H). |
| 43 | ethyl 5-(2-((*tert*-butoxycarbonyl)amino)ethoxy)-1*H*-pyrazole-4-carboxylate | 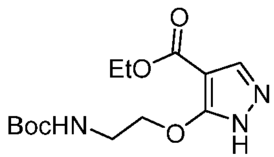 | **LCMS (Method 7):** m/z 298.0 (ES-), at 1.84 min.<br>**$^1$H NMR:** (400 MHz, $CDCl_3$) δ: 7.93 (s, 1H), 5.33 (s, 1H), 4.39-4.29 (m, 4H), 3.58 (t, *J* = 4.0 Hz, 2H), 1.57 (s, 9H), 1.37 (t, *J* = 7.2 Hz, 3H). 1 exchangeable proton not observed. |
| 44 | *tert*-butyl 3-(2-methoxyacetyl)-2,4-dioxopiperidine-1-carboxylate | 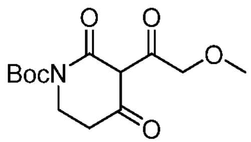 | **LCMS (Method 3):** m/z 284.1 (ES-), at 1.79 min.<br>**$^1$H NMR:** (400 MHz, DMSO-$d_6$) δ: 4.63 (s, 3H), 3.76 (s, 3H), 2.79-2.73 (m, 4H), 1.47 (s, 9H). |

(continued)

| Intermediate | Name | Structure | Data |
|---|---|---|---|
| 45 | 1-(2-(3-fluoro-5-(trifluoromethyl)benzyl) pyridin-4-yl)-3-iodo-1,5,6,7-tetrahydro-4*H*-pyrazolo[4,3-c]pyridin-4-one | 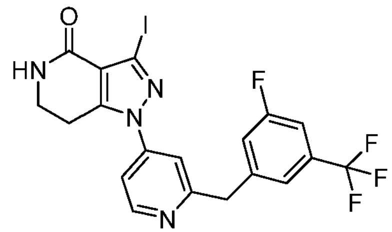 | **LCMS (Method 10):** m/z 517.0 (ES+), at 1.88 min.<br><br>**$^1$H NMR:** (400 MHz, DMSO-$d_6$) δ: 8.65 (d, *J* = 7.2 Hz, 1H), 7.71 (s, 1H), 7.62 (s, 1H), 7.56-7.48 (m, 4H), 4.32 (s, 2H), 3.40-3.26 (m, 2H), 3.21-3.16 (m, 2H). |

**Table 3 - Examples**

| Ex. No. | Name | Intermediate/procedure | $^1$H NMR | LCMS |
|---|---|---|---|---|
| 1 | 1-(2-(3-fluoro-5-(trifluoromethyl)benzyl)pyridin-4-yl)-1,5,6,7-tetrahydro-4*H*-pyrazolo[4,3-*c*]pyridin-4-one | Intermediates 1 and 6 Procedure 1 | (400 MHz, DMSO-$d_6$) δ: 8.65 (d, *J* = 5.2 Hz, 1H), 8.06 (s, 1H), 7.72 (d, *J* = 2.1 Hz, 1H), 7.63 (s, 1H), 7.57-7.52 (m, 4H), 4.33 (s, 2H), 3.43 (td, *J* = 6.7, 2.5 Hz, 2H), 3.20 (t, *J* = 6.7 Hz, 2H). | m/z 391.1 (M+H)+ (ES+), at 1.89 min, 95% (Method 3) |
| 2 | 1-(4-(3-(trifluoromethyl)benzyl)pyridin-2-yl)-1,5,6,7-tetrahydro-4*H*-pyrazolo[4,3-*c*]pyridin-4-one | Intermediates 1 and 7 Procedure 1 | (400 MHz, DMSO-$d_6$) δ: 8.42 (dd, *J* = 5.1, 0.7 Hz, 1H), 7.99 (s, 1H), 7.85 (dd, *J* = 1.6, 0.8 Hz, 1H), 7.75 (s, 1H), 7.71-7.52 (m, 3H), 7.46 (s, 1H), 7.35 (dd, *J* = 5.1, 1.5 Hz, 1H), 4.23 (s, 2H), 3.49-3.35 (m, 4H). | m/z 373.1 (M+H)+ (ES+), at 4.42 min, 100% (Method 1) |
| 3 | 5-methyl-1-(4-(3-(trifluoromethyl)benzyl)pyridin-2-yl)-1,5,6,7-tetrahydro-4*H*-pyrazolo[4,3-*c*]pyridin-4-one | Example 2 Procedure 2 | (400 MHz, DMSO-$d_6$) δ: 8.41 (d, *J* = 5.2 Hz, 1H), 7.98 (s, 1H), 7.85 (s, 1H), 7.74 (s, 1H), 7.70-7.50 (m, 3H), 7.33 (d, *J* = 5.1 Hz, 1H), 4.22 (s, 2H), 3.59 (t, *J* = 6.9 Hz, 2H), 3.49 (t, *J* = 6.9 Hz, 2H), 2.93 (s, 3H). | m/z 387.2 (M+H)+ (ES+), at 4.53 min, 97% (Method 1) |
| 4 | 1-(4-(3-fluoro-5-(trifluoromethyl)benzyl)pyridin-2-yl)-1,5,6,7-tetrahydro-4*H*-pyrazolo[4,3-*c*]pyridin-4-one | Intermediates 1 and 8 Procedure 1 | (400 MHz, DMSO-$d_6$) δ: 8.42 (d, *J* = 5.2 Hz, 1H), 7.99 (s, 1H), 7.88 (s, 1H), 7.65 (s, 1H), 7.61-7.55 (m, 2H), 7.45 (s, 1H), 7.37-7.35 (m, 1H), 4.23 (s, 2H), 3.43-3.39 (m, 4H). | m/z 391.1 (M+H)+ (ES+), at 2.16 min, 99% (Method 3) |
| 5 | 1-(4-(3-fluoro-5-(trifluoromethyl)benzyl)pyridin-2-yl)-5-methyl-1,5,6,7-tetrahydro-4*H*-pyrazolo[4,3-*c*]pyridin-4-one | Example 4 Procedure 2 | (400 MHz, DMSO-$d_6$) δ: 8.43 (d, *J* = 5.2 Hz, 1H), 7.99 (s, 1H), 7.90 (s, 1H), 7.60 (t, *J* = 11.8 Hz, 3H), 7.37 (d, *J* = 5.1 Hz, 1H), 4.24 (s, 2H), 3.62 (t, *J* = 7.3 Hz, 2H), 3.33 (s, 2H), 2.94 (s, 3H). | m/z 405.2 (M+H)+ (ES+), at 2.40 min, 99% (Method 3) |

(continued)

| Ex. No. | Name | Intermediate/procedure | $^1$H NMR | LCMS |
|---|---|---|---|---|
| 6 | 1-(4-((6-fluoro-5-methylpyridin-3-yl)methyl)pyridin-2-yl)-1,5,6,7-tetrahydro-4*H*-pyrazolo[4,3-*c*]pyridin-4-one | Intermediate 12 and CAS: 1260812-39-2 Procedure 3 | (400 MHz, DMSO-$d_6$) δ: 8.41 (d, *J* = 5.1 Hz, 1H), 8.05 (s, 1H), 7.98 (s, 1H), 7.83 (s, 1H), 7.80-7.71 (m, 1H), 7.45 (s, 1H), 7.36-7.26 (m, 1H), 4.10 (s, 2H), 3.51-3.35 (m, 4H), 2.20 (s, 3H). | m/z 338.2 (M+H)+ (ES+), at 4.83 min, 95% (Method 5) |
| 7 | 1-(4-((6-fluoro-5-methylpyridin-3-yl)methyl)pyridin-2-yl)-5-methyl-1,5,6,7-tetrahydro-4*H*-pyrazolo[4,3-*c*]pyridin-4-one | Example 6 Procedure 2 | (400 MHz, DMSO-$d_6$) δ: 8.41 (d, *J* = 5.1 Hz, 1H), 8.05 (d, *J* = 2.2 Hz, 1H), 7.98 (s, 1H), 7.83 (s, 1H), 7.81-7.73 (m, 1H), 7.31 (dd, *J* = 5.1, 1.6 Hz, 1H), 4.10 (s, 2H), 3.60 (t, *J* = 6.9 Hz, 2H), 3.49 (t, *J* = 6.9 Hz, 2H), 2.93 (s, 3H), 2.20 (s, 3H). | m/z 352.3 (M+H)+ (ES+), at 5.18 min, 100% (Method 5) |
| 8 | 2-(4-(3-(trifluoromethyl)benzyl)pyridin-2-yl)-2,5,6,7-tetrahydro-4*H*-pyrazolo[4,3-*c*]pyridin-4-one | Intermediate 7 and CAS: 50607-30-2 Procedure 4 | (400 MHz, DMSO-$d_6$) δ: 8.74 (d, *J* = 0.8 Hz, 1H), 8.48-8.36 (m, 1H), 7.86 (d, *J* = 1.2 Hz, 1H), 7.75 (s, 1H), 7.72-7.52 (m, 4H), 7.37-7.27 (m, 1H), 4.22 (s, 2H), 3.45 (td, *J* = 6.6, 2.6 Hz, 2H), 2.89 (t, *J* = 6.6 Hz, 2H). | m/z 373.2 (M+H)+ (ES+), at 4.15 min, 100% (Method 1) |
| 9 | 2-(2-(3-fluoro-5-(trifluoromethyl)benzyl)pyridin-4-yl)-2,5,6,7-tetrahydro-4*H*-pyrazolo[4,3-*c*]pyridin-4-one | Intermediate 6 and CAS: 50607-30-2 Procedure 4 | (400 MHz, DMSO-$d_6$) δ: 9.12 (s, 1H), 8.57 (d, *J* = 5.6 Hz, 1H), 8.00 (d, *J* = 2.0 Hz, 1H), 7.79 (t, *J* = 2.0 Hz, 1H), 7.69 (s, 1H), 7.60 (s, 1H), 7.54 (t, *J* = 2.4 Hz, 2H), 4.27 (s, 2H), 3.46 (t, *J* = 2.8 Hz, 2H), 2.90 (t, *J* = 6.4 Hz, 2H). | m/z 391.1 (M+H)+ (ES+), at 1.91 min, 99% (Method 2) |

(continued)

| Ex. No. | Name | Intermediate/procedure | $^1$H NMR | LCMS |
|---|---|---|---|---|
| 10 | 1-(4-(3-fluoro-5-(trifluoromethyl)benzyl)pyridin-2-yl)-5,6,7,8-tetrahydropyrazolo[4,3-*c*]azepin-4(1*H*)-one | Intermediates 2 and 8 Procedure 5 | (400 MHz, DMSO-$d_6$) δ: 8.44 (dd, *J* = 5.1, 0.7 Hz, 1H), 8.01 (s, 1H), 7.86-7.77 (m, 2H), 7.66 (d, *J* = 1.6 Hz, 1H), 7.58 (dd, *J* = 13.2, 9.5 Hz, 2H), 7.39 (dd, *J* = 5.1, 1.5 Hz, 1H), 4.23 (s, 2H), 3.28-3.20 (m, 2H), 1.96 (dd, *J* = 15.1, 6.2 Hz, 2H). 2 protons obscured by solvent peak. | m/z 405.2 (M+H)+ (ES+), at 4.24 min, 99% (Method 1) |
| 11 | 1-(4-(3-(trifluoromethyl)benzyl)pyridin-2-yl)-5,6,7,8-tetrahydropyrazolo [4,3-*c*]azepin-4(1*H*)-one | Intermediates 2 and 7 Procedure 5 | (400 MHz, DMSO-$d_6$) δ: 8.55-8.34 (m, 1H), 8.00 (s, 1H), 7.83 (d, *J* = 4.8 Hz, 1H), 7.80-7.74 (m, 2H), 7.61 (ddd, *J* = 21.1, 14.1, 7.5 Hz, 3H), 7.37 (dd, *J* = 5.1, 1.5 Hz, 1H), 4.22 (s, 2H), 3.26-3.13 (m, 2H), 2.04-1.84 (m, 2H). 2 protons obscured by solvent peak. | m/z 387.2 (M+H)+ (ES+), at 4.14 min, 100% (Method 1) |
| 12 | 1-(2-(3-fluoro-5-(trifluoromethyl)benzyl)pyridin-4-yl)-5,6,7,8-tetrahydropyrazolo[4,3-*c*]azepin-4(1*H*)-one | Intermediates 2 and 6 Procedure 5 | (400 MHz, DMSO-$d_6$) δ: 8.65 (d, *J* = 5.6 Hz, 1H), 8.05 (s, 1H), 7.83 (s, 1H), 7.71 (d, *J* = 1.6 Hz, 1H), 7.61 (s, 1H), 7.56-7.52 (m, 3H), 4.31 (s, 2H), 3.25-3.24 (m, 2H), 3.09 (t, *J* = 6.4 Hz, 2H), 1.98-1.93 (m, 2H). | m/z 405.2 (M+H)+ (ES+), at 1.94 min, 95% (Method 3) |
| 13 | 1-(4-(3-(trifluoromethyl)benzyl)pyridin-2-yl)-6,7-dihydro-1*H*-pyrazolo [4,3-*f*][1,4]oxazepin-4(5*H*)-one | Intermediate 7, CAS: 87-13-8 and 39684-80-5 Procedure 6 | (400 MHz, DMSO-$d_6$) δ: 9.27 (s, 1H), 8.34 (d, *J* = 5.2 Hz, 1H), 7.85 (s, 1H), 7.77 (s, 1H), 7.72 (s, 1H), 7.63-7.57 (m, 3H), 7.24 (d, *J* = 5.2 Hz, 1H), 4.41 (t, *J* = 3.2 Hz, 2H), 4.20 (s, 2H), 3.72 (t, 2H). | m/z 389.0 (M+H)+ (ES+), at 2.37 min, 99% (Method 3) |

(continued)

| Ex. No. | Name | Intermediate/procedure | $^1$H NMR | LCMS |
|---|---|---|---|---|
| 14 | 1-(4-(3-fluoro-5-(trifluoromethyl)benzyl)pyridin-2-yl)-6,7-dihydro-1*H*-pyrazolo[4,3-*f*][1,4]oxazepin-4(5*H*)-one | Intermediate 8, CAS: 87-13-8 and 39684-80-5 Procedure 6 | (400 MHz, DMSO-$d_6$) δ: 9.27 (s, 1H), 8.36 (d, *J* = 5.2 Hz, 1H), 7.87 (s, 1H), 7.82 (s, 1H), 7.63 (s, 1H), 7.58 (d, *J* = 11.2 Hz, 2H), 7.28 (t, *J* = 1.2 Hz, 1H), 4.42 (t, *J* = 3.6 Hz, 2H), 4.22 (s, 2H), 3.75-3.70 (m, 2H). | m/z 407.1 (M+H)+ (ES+), at 2.46 min, 99% (Method 3) |
| 15 | 1-(5-(3-fluoro-5-(trifluoromethyl)benzyl)pyridazin-3-yl)-1,5,6,7-tetrahydro-4*H*-pyrazolo[4,3-*c*]pyridin-4-one | Intermediates 1 and 9 Procedure 1 | (400 MHz, DMSO-$d_6$) δ: 9.28 (d, *J* = 2.0 Hz, 1H), 8.15 (d, *J* = 2.0 Hz, 1H), 8.10 (s, 1H), 7.72 (s, 1H), 7.66 (d, *J* = 10.0 Hz, 1H), 7.59 (d, *J* = 8.8 Hz, 1H), 7.53 (s, 1H), 4.28 (s, 2H), 3.45 (t, *J* = 6.8 Hz, 4H). | m/z 391.3 (M-H)- (ES-), at 1.94 min, 100% (Method 3) |
| 16 | 1-(5-(3-fluoro-5-(trifluoromethyl)benzyl)pyridin-3-yl)-1,5,6,7-tetrahydro-4*H*-pyrazolo[4,3-*c*]pyridin-4-one | Intermediate 11 and CAS: 239087-09-3 Procedure 7 | (400 MHz, DMSO-$d_6$) δ: 8.73 (d, *J* = 2.4 Hz, 1H), 8.68 (d, *J* = 2.0 Hz, 1H), 8.05-8.04 (m, 1H), 8.02 (s, 1H), 7.68 (s, 1H), 7.63-7.61 (m, 1H), 7.56-7.54 (m, 1H), 7.45 (s, 1H), 4.22 (s, 2H), 3.43-3.39 (m, 2H), 3.10-3.07 (m, 2H). | m/z 391.2 (M+H)+ (ES+), at 1.91 min, 98% (Method 3) |
| 17 | 1-(6-(3-fluoro-5-(trifluoromethyl)benzyl)pyridazin-4-yl)-1,5,6,7-tetrahydro-4*H*-pyrazolo[4,3-*c*]pyridin-4-one | Intermediate 10 and CAS: 239087-09-3 Procedure 7 | (400 MHz, DMSO-$d_6$) δ: 9.52 (d, *J* = 2.4 Hz, 1H), 8.15 (s, 1H), 7.99 (d, *J* = 2.4 Hz, 1H), 7.69 (s, 1H), 7.62-7.57 (m, 3H), 4.54 (s, 2H), 3.45-3.43 (m, 2H), 3.30-3.25 (m, 2H). | m/z 392.2 (M+H)+ (ES+), at 1.93 min, 99% (Method 3) |
| 18 | 1-(4-(3-fluoro-5-(trifluoromethyl)phenoxy)pyridin-2-yl)-1,5,6,7-tetrahydro-4*H*-pyrazolo[4,3-*c*]pyridin-4-one | Intermediate 12 and CAS: 172333-87-8 Procedure 8 | (400 MHz, DMSO-$d_6$) δ: 8.45 (d, *J* = 5.7 Hz, 1H), 7.98 (s, 1H), 7.72-7.66 (m, 2H), 7.61 (s, 1H), 7.48 (bs, 1H), 7.42 (d, *J* = 2.2 Hz, 1H), 7.11 (d, *J* = 5.6 2.2 Hz, 1H), 3.50-3.40 (m, 4H). | m/z 338.2 (M+H)+ (ES+), at 5.93 min, 100% (Method 5) |

(continued)

| Ex. No. | Name | Intermediate/procedure | $^1$H NMR | LCMS |
|---|---|---|---|---|
| 19 | 1-(2-(4-chlorobenzyl)pyridin-4-yl)-1,5,6,7-tetrahydro-4*H*-pyrazolo [4,3-*c*]pyridin-4-one | Intermediate 14 and CAS: 622-95-7<br>Procedure 9 | (400 MHz, DMSO-$d_6$) δ: 8.63 (d, *J* = 5.2 Hz, 1H), 8.04 (s, 1H), 7.62 (d, *J* = 2.0 Hz, 1H), 7.51-7.49 (m, 2H), 7.37 (s, 4H), 4.39 (s, 2H), 3.45-3.41 (m, 2H), 3.20-3.17 (m, 2H). | m/z 339.1 (M+H)+ (ES+), at 1.57 min, 98% (Method 3) |
| 20 | 1-(2-(3-chloro-5-fluorobenzyl)pyridin-4-yl)-1,5,6,7-tetrahydro-4*H*-pyrazolo[4,3-*c*]pyridin-4-one | Intermediate 14 and CAS: 493024-39-8<br>Procedure 9 | (400 MHz, DMSO-$d_6$) δ: 8.65 (d, *J* = 5.2 Hz, 1H), 8.06 (s, 1H), 7.69 (d, *J* = 2.0 Hz, 1H), 7.53-7.53 (m, 2H), 7.31-7.30 (m, 2H), 7.22 (d, *J* = 9.6 Hz, 1H), 4.22 (s, 2H), 3.44-3.43 (m, 2H), 3.20 (t, *J* = 6.8 Hz, 2H). | m/z 356.9 (M+H)+ (ES+), at 1.74 min, 100% (Method 3) |
| 21 | 1-(2-(3,5-difluorobenzyl)pyridin-4-yl)-1,5,6,7-tetrahydro-4*H*-pyrazolo [4,3-*c*]pyridin-4-one | Intermediate 14 and CAS: 141776-91-2<br>Procedure 9 | (400 MHz, DMSO-$d_6$) δ: 8.65 (d, *J* = 5.2 Hz, 1H), 8.05 (s, 1H), 7.67 (d, *J* = 2.0 Hz, 1H), 7.53-7.53 (m, 2H), 7.10-7.10 (m, 3H), 4.22 (s, 2H), 3.45 (d, *J* = 2.4 Hz, 2H), 3.20 (t, *J* = 6.8 Hz, 2H). | m/z 340.9 (M+H)+ (ES+), at 1.58 min, 100% (Method 3) |
| 22 | 1-(2-(3-(trifluoromethyl)benzyl)pyridin-4-yl)-1,5,6,7-tetrahydro-4*H*-pyrazolo[4,3-*c*]pyridin-4-one | Intermediate 14 and CAS: 402-23-3<br>Procedure 9 | (400 MHz, DMSO-$d_6$) δ: 8.64 (d, *J* = 7.2 Hz, 1H), 8.05 (s, 1H), 7.74 (s, 1H), 7.69-7.55 (m, 3H), 7.52-7.50 (m, 3H), 4.31 (s, 2H), 3.42-3.40 (m, 2H), 3.21-3.16 (m, 2H). | m/z 373.1 (M+H)+ (ES+), at 1.75 min, 99% (Method 3) |
| 23 | 1-(2-(4-fluoro-3-(trifluoromethyl)benzyl)pyridin-4-yl)-1,5,6,7-tetrahydro-4*H*-pyrazolo[4,3-*c*]pyridin-4-one | Intermediate 14 and CAS: 184970-26-1<br>Procedure 9 | (400 MHz, DMSO-$d_6$) δ: 8.64 (d, *J* = 5.2 Hz, 1H), 8.05 (s, 1H), 7.79 (d, *J* = 7.2 Hz, 1H), 7.74-7.72 (m, 2H), 7.52-7.52 (m, 3H), 4.28 (s, 2H), 3.45-3.44 (m, 2H), 3.19 (t, *J* = 6.8 Hz, 2H). | m/z 391.0 (M+H)+ (ES+), at 1.88 min, 99% (Method 2) |

(continued)

| Ex. No. | Name | Intermediate/procedure | $^1$H NMR | LCMS |
|---|---|---|---|---|
| 24 | 1-(2-(3,4,5-trifluorobenzyl)pyridin-4-yl)-1,5,6,7-tetrahydro-4*H*-pyrazolo [4,3-*c*]pyridin-4-one | Intermediate 14 and CAS: 220141-72-0<br><br>Procedure 9 | (400 MHz, DMSO-$d_6$) δ: 8.65 (d, *J* = 5.6 Hz, 1H), 8.06 (s, 1H), 7.67 (d, *J* = 2.0 Hz, 1H), 7.54-7.53 (m, 2H), 7.35-7.34 (m, 2H), 4.19 (s, 2H), 3.46-3.45 (m, 2H), 3.20 (t, *J* = 6.8 Hz, 2H). | m/z 359.1 (M+H)+ (ES+), at 1.69 min, 100% (Method 3) |
| 25 | 1-(2-(3-chloro-4-fluorobenzyl)pyridin-4-yl)-1,5,6,7-tetrahydro-4*H*-pyrazolo[4,3-*c*]pyridin-4-one | Intermediate 14 and CAS: 192702-01-5<br>Procedure 9 | (400 MHz, DMSO-$d_6$) δ: 8.64 (d, *J* = 5.6 Hz, 1H), 8.05 (s, 1H), 7.66 (d, *J* = 1.6 Hz, 1H), 7.58 (d, *J* = 7.6 Hz, 1H), 7.5 -7.51 (m, 2H), 7.36-7.36 (m, 2H), 4.19 (s, 2H), 3.45-3.44 (m, 2H), 3.19 (t, *J* = 6.4 Hz, 2H). | m/z 357.1 (M+H)+ (ES+), at 1.68 min, 100% (Method 3) |
| 26 | 1-(2-(3-fluoro-5-methylbenzyl)pyridin-4-yl)-1,5,6,7-tetrahydro-4*H*-pyrazolo[4,3-*c*]pyridin-4-one | Intermediate 14 and CAS: 212268-39-8<br>Procedure 9 | (400 MHz, $CDCl_3$) δ: 8.71 (d, *J* = 5.2 Hz, 1H), 8.14 (s, 1H), 7.37-7.35 (m, 2H), 6.92 (s, 1H), 6.80 (d, *J* = 18.4 Hz, 2H), 5.58 (s, 1H), 4.21 (s, 2H), 3.67-3.63 (m, 2H), 3.15-3.12 (m, 2H), 2.35 (s, 3H). | m/z 337.1 (M+H)+ (ES+), at 1.69 min, 100% (Method 2) |
| 27 | 1-(2-(3-chloro-5-(trifluoromethyl)benzyl)pyridin-4-yl)-1,5,6,7-tetrahydro-4*H*-pyrazolo[4,3-*c*]pyridin-4-one | Intermediate 14 and CAS: 886496-91-9<br>Procedure 9 | (400 MHz, DMSO-$d_6$) δ: 8.65 (d, *J* = 5.2 Hz, 1H), 8.06 (s, 1H), 7.78 (s, 1H), 7.73 (t, *J* = 6.0 Hz, 3H), 7.54-7.50 (m, 2H), 4.32 (s, 2H), 3.46-3.27 (m, 2H), 3.20 (t, *J* = 6.8 Hz, 2H). | m/z 407.0 (M+H)+ (ES+), at 2.03 min, 100% (Method 2) |
| 28 | 1-(2-(3-methyl-5-(trifluoromethyl)benzyl)pyridin-4-yl)-1,5,6,7-tetrahydro-4*H*-pyrazolo[4,3-*c*]pyridin-4-one | Intermediate 14 and CAS: 116070-36-1<br>Procedure 9 | (400 MHz, DMSO-$d_6$) δ: 8.67 (d, *J* = 5.6 Hz, 1H), 8.07 (s, 1H), 7.72 (d, *J* = 2.0 Hz, 1H), 7.57-7.51 (m, 3H), 7.48 (s, 1H), 7.42 (s, 1H), 4.28 (s, 2H), 3.45-3.41 (m, 2H), 3.19 (t, *J* = 6.4 Hz, 2H), 2.37 (s, 3H). | m/z 387.1 (M+H)+ (ES+), at 1.94 min, 98% (Method 2) |

(continued)

| Ex. No. | Name | Intermediate/procedure | $^1$H NMR | LCMS |
|---|---|---|---|---|
| 29 | 1-(2-(2-fluoro-3-(trifluoromethyl)benzyl)pyridin-4-yl)-1,5,6,7-tetrahydro-4*H*-pyrazolo[4,3-*c*]pyridin-4-one | Intermediate 14 and CAS: 184970-25-0 Procedure 9 | (400 MHz, DMSO-$d_6$) δ: 8.63 (d, *J* = 5.6 Hz, 1H), 8.06 (s, 1H), 7.76-7.69 (m, 1H), 7.66 (d, *J* = 2.0 Hz, 2H), 7.54-7.50 (m, 2H), 7.39 (t, *J* = 7.6 Hz, 1H), 4.34 (s, 2H), 3.46-3.42 (m, 2H), 3.19 (t, *J* = 6.4 Hz, 2H). | m/z 391.1 (M+H)+ (ES+), at 1.87 min, 99% (Method 2) |
| 30 | 1-(2-(2-fluoro-5-(trifluoromethyl)benzyl)pyridin-4-yl)-1,5,6,7-tetrahydro-4*H*-pyrazolo[4,3-*c*]pyridin-4-one | Intermediate 14 and CAS: 220239-69-0 Procedure 9 | (400 MHz, DMSO-$d_6$) δ: 8.62 (d, *J* = 5.6 Hz, 1H), 8.08 (d, *J* = 17.6 Hz, 1H), 7.87 (d, *J* = 5.6 Hz, 1H), 7.72 (d, *J* = 3.6 Hz, 1H), 7.66 (s, 1H), 7.52 (d, *J* = 6.0 Hz, 2H), 7.47-7.42 (m, 1H), 4.34 (s, 2H), 3.45-3.42 (m, 2H), 3.20-3.17 (m, 2H). | m/z 391.0 (M+H)+ (ES+), at 1.90 min, 94% (Method 2) |
| 31 | 1-(2-(3,5-dichlorobenzyl)pyridin-4-yl)-1,5,6,7-tetrahydro-4*H*-pyrazolo[4,3-*c*]pyridin-4-one | Intermediate 14 and CAS: 7778-01-0 Procedure 9 | (400 MHz, DMSO-$d_6$) δ: 8.65 (d, *J* = 5.6 Hz, 1H), 8.06 (s, 1H), 7.70 (d, *J* = 1.6 Hz, 1H), 7.53-7.51 (m, 2H), 7.48-7.44 (m, 3H), 4.21 (s, 2H), 3.45-3.38 (m, 2H), 3.22-3.17 (m, 2H). | m/z 372.9 (M+H)+ (ES+), at 1.94 min, 100% (Method 2) |
| 32 | 1-(2-(4-(trifluoromethyl)benzyl)pyridin-4-yl)-1,5,6,7-tetrahydro-4*H*-pyrazolo[4,3-*c*]pyridin-4-one | Intermediate 14 and CAS: 402-49-3 Procedure 9 | (400 MHz, DMSO-$d_6$) δ: 8.64 (d, *J* = 5.2 Hz, 1H), 8.05 (s, 1H), 7.68 (t, *J* = 2.4 Hz, 3H), 7.57 (d, *J* = 8.0 Hz, 2H), 7.52-7.52 (m, 2H), 4.30 (s, 2H), 3.45-3.44 (m, 2H), 3.19 (t, *J* = 6.8 Hz, 2H). | m/z 373.2 (M+H)+ (ES+), at 1.81 min, 99% (Method 3) |

(continued)

| Ex. No. | Name | Intermediate/procedure | $^1$H NMR | LCMS |
|---|---|---|---|---|
| 33 | 3-((4-(4-oxo-4,5,6,7-tetrahydro-1*H*-pyrazolo[4,3-*c*]pyridin-1-yl)pyridin-2-yl)methyl)-5-(trifluoromethyl)benzonitrile | Intermediate 14 and CAS: 954123-46-7 Procedure 10 | (400 MHz, DMSO-$d_6$) $\delta$: 8.65 (d, *J* = 5.6 Hz, 1H), 8.22 (s, 1H), 8.17 (s, 1H), 8.12 (s, 1H), 8.07 (s, 1H), 7.73 (d, *J* = 1.6 Hz, 1H), 7.55-7.51 (m, 2H), 4.38 (s, 2H), 3.46-3.42 (m, 2H), 3.20 (t, *J* = 6.8 Hz, 2H). | m/z 397.9 (M+H)+ (ES+), at 2.64 min, 99% (Method 6) |
| 34 | 1-(2-(3,5-bis(trifluoromethyl)benzyl) pyridin-4-yl)-1,5,6,7-tetrahydro-4*H*-pyrazolo[4,3-*c*]pyridin-4-one | Intermediate 14 and CAS: 32247-96-4 Procedure 9 | (400 MHz, DMSO-$d_6$) $\delta$: 8.65 (d, *J* = 5.6 Hz, 1H), 8.11 (s, 2H), 8.06 (s, 1H), 7.98 (s, 1H), 7.77-7.76 (m, 1H), 7.54-7.54 (m, 2H), 4.43 (s, 2H), 3.45-3.44 (m, 2H), 3.20 (t, *J* = 6.8 Hz, 2H). | m/z 441.0 (M+H)+ (ES+), at 1.20 min, 98% (Method 2) |
| 35 | 1-(2-(3-fluoro-5-(trifluoromethyl)benzoyl)pyridin-4-yl)-1,5,6,7-tetrahydro-4*H*-pyrazolo[4,3-*c*]pyridin-4-one | Example 1 Procedure 11 | (400 MHz, DMSO-$d_6$) $\delta$: 8.91 (d, *J* = 5.6 Hz, 1H), 8.32 (d, *J* = 2.0 Hz, 1H), 8.21-8.18 (m, 2H), 8.15 (s, 1H), 8.09 (d, *J* = 8.4 Hz, 1H), 8.01-7.99 (m, 1H), 7.57 (s, 1H), 3.48-3.46 (m, 2H), 3.30 (t, *J* = 6.4 Hz, 2H). | m/z 405.0 (M+H)+ (ES+), at 2.05 min, 98% (Method 2) |
| 36 | 1-(2-((3-fluoro-5-(trifluoromethyl)phenyl)(hydroxy)methyl)pyridin-4-yl)-1,5,6,7-tetrahydro-4*H*-pyrazolo [4,3-*c*]pyridin-4-one | Example 1 Procedure 11 | (400 MHz, DMSO-$d_6$) $\delta$: 8.64 (d, *J* = 5.6 Hz, 1H), 8.07 (s, 1H), 7.93 (d, *J* = 2.0 Hz, 1H), 7.70 (s, 1H), 7.63-7.52 (m, 4H), 6.66 (d, *J* = 4.0 Hz, 1H), 5.96 (d, *J* = 2.8 Hz, 1H), 3.45-3.43 (m, 2H), 3.22-3.21 (m, 2H). | m/z 407.0 (M+H)+ (ES+), at 1.76 min, 98% (Method 2) |

(continued)

| Ex. No. | Name | Intermediate/procedure | $^1$H NMR | LCMS |
|---|---|---|---|---|
| 36 Isomer 1 | 1-(2-((3-fluoro-5-(trifluoromethyl)phenyl)(hydroxy)methyl)pyridin-4-yl)-1,5,6,7-tetrahydro-4*H*-pyrazolo[4,3-*c*]pyridin-4-one<br><br>(Single enantiomer of unknown absolute stereochemistry) | Example 36<br><br>Chiral SFC method 1 (Isocratic run 20% co-solvent)<br><br>Chiral SFC purity analysis: 4.75 min, 100% (Method 3 - isocratic run 20% co-solvent) | (400 MHz, DMSO-$d_6$) δ: 8.64 (d, *J* = 5.2 Hz, 1H), 8.07 (s, 1H), 7.93 (d, *J* = 2.0 Hz, 1H), 7.70 (s, 1H), 7.55-7.53 (m, 4H), 6.66 (d, *J* = 4.8 Hz, 1H), 5.96 (d, *J* = 4.8 Hz, 1H), 3.45-3.36 (m, 2H), 3.22-3.21 (m, 2H). | m/z 406.9 (M+H)+ (ES+), at 2.09 min, 99% (Method 2) |
| 36 Isomer 2 | 1-(2-((3-fluoro-5-(trifluoromethyl)phenyl)(hydroxy)methyl)pyridin-4-yl)-1,5,6,7-tetrahydro-4*H*-pyrazolo[4,3-*c*]pyridin-4-one (Single enantiomer of unknown absolute stereochemistry) | Example 36<br>Chiral SFC method 1 (Isocratic run 20% co-solvent)<br><br>Chiral SFC purity analysis: 5.60 min, 99% (Method 3 - isocratic run 20% co-solvent) | (400 MHz, DMSO-$d_6$) δ: 8.64 (d, *J* = 5.2 Hz, 1H), 8.07 (s, 1H), 7.93 (d, *J* = 2.0 Hz, 1H), 7.70 (s, 1H), 7.63-7.52 (m, 4H), 6.66 (d, *J* = 4.4 Hz, 1H), 5.96 (d, *J* = 4.4 Hz, 1H), 3.46-3.43 (m, 2H), 3.24-3.21 (m, 2H). | m/z 406.9 (M+H)+ (ES+), at 2.09 min, 99% (Method 2) |
| 37 | 1-(2-(fluoro(3-fluoro-5-(trifluoromethyl)phenylmethyl)pyridin-4-yl)-1,5,6,7-tetrahydro-4*H*-pyrazolo[4,3-*c*]pyridin-4-one | Example 1<br>Procedure 11 | (400 MHz, DMSO-$d_6$) δ: 8.73 (d, *J* = 5.2 Hz, 1H), 8.10 (s, 1H), 7.97 (d, *J* = 2.0 Hz, 1H), 7.77-7.67 (m, 4H), 7.54 (s, 1H), 6.96 (d, *J* = 45.2 Hz, 1H), 3.47-3.43 (m, 2H), 3.33-3.30 (m, 2H). | m/z 409.2 (M+H)+ (ES+), at 2.21 min, 97% (Method 7) |
| 38 | 1-(2-(difluoro(3-fluoro-5-(trifluoromethyl)phenylmethyl)pyridin-4-yl)-1,5,6,7-tetrahydro-4*H*-pyrazolo[4,3-*c*]pyridin-4-one | Example 35<br>Procedure 12 | (400 MHz, DMSO-$d_6$) δ: 8.81 (d, *J* = 5.2 Hz, 1H), 8.17 (d, *J* = 2.0 Hz, 1H), 8.14 (s, 1H), 7.97-7.91 (m, 2H), 7.86-7.83 (m, 2H), 7.56 (s, 1H), 3.48-3.44 (m, 2H), 3.30-3.27 (m, 2H). | m/z 427.1 (M+H)+ (ES+), at 2.22 min, 93% (Method 3) |
| 39 | 1-(2-((3-fluoro-5-(trifluoromethyl)phenyl)methyl-$d_2$)pyridin-4-yl)-1,5,6,7-tetrahydro-4*H*-pyrazolo[4,3-*c*]pyridin-4-one | Example 1<br>Procedure 13 | (400 MHz, DMSO-$d_6$) δ: 8.67 (d, *J* = 7.6 Hz, 1H), 8.06 (s, 1H), 7.84 (s, 1H), 7.63 (s, 1H), 7.55-7.50 (m, 4H), 3.46-3.42 (m, 2H), 3.22-3.17 (m, 2H). | m/z 393.0 (M+H)+ (ES+), at 1.94 min, 100% (Method 7) |

(continued)

| Ex. No. | Name | Intermediate/procedure | $^1$H NMR | LCMS |
|---|---|---|---|---|
| 40 | 1-(2-(3-fluoro-5-(trifluoromethyl)benzyl)pyridin-4-yl)-6-methyl-1,5,6,7-tetrahydro-4*H*-pyrazolo[4,3-*c*]pyridin-4-one | Intermediates 6 and 17<br>Procedure 1 | (400 MHz, DMSO-$d_6$) δ: 8.64 (d, *J* = 5.4 Hz, 1H), 8.04 (s, 1H), 7.69 (d, *J* = 1.8 Hz, 1H), 7.62 (s, 1H), 7.57-7.48 (m, 4H), 4.32 (s, 2H), 3.77 (dt, *J* = 11.4, 5.9 Hz, 1H), 3.21 (dd, *J* = 16.4, 5 Hz, 1H), 2.97 (dd, *J* = 16.5, 10.6 Hz, 1H), 1.23 (d, *J* = 6.4 Hz, 3H). | m/z 405.2 (M+H)+ (ES+), at 5.51 min, 98% (Method 5) |
| 40 Isomer 1 | 1-(2-(3-fluoro-5-(trifluoromethyl)benzyl)pyridin-4-yl)-6-methyl-1,5,6,7-tetrahydro-4*H*-pyrazolo[4,3-*c*]pyridin-4-one (Single enantiomer of unknown absolute stereochemistry) | Example 40<br>Chiral SFC method 2 (Isocratic run 30% co-solvent)<br>Chiral SFC purity analysis: 1.94 min, 99% (Method 4 - gradient run 3-50% co-solvent) | (400 MHz, DMSO-$d_6$) δ: 8.65 (dd, *J* = 5.5, 0.6 Hz, 1H), 8.05 (s, 1H), 7.70 (dd, *J* = 2.2, 0.7 Hz, 1H), 7.63 (td, *J* = 1.5, 0.8 Hz, 1H), 7.60-7.37 (m, 4H), 4.33 (s, 2H), 3.86-3.71 (m, 1H), 3.20 (dd, *J* = 16.4, 5.1 Hz, 1H), 2.98 (dd, *J* = 16.5, 10.6 Hz, 1H), 1.24 (d, *J* = 6.4 Hz, 3H). | m/z 405.2 (M+H)+ (ES+), at 3.97 min, 100% (Method 1) |
| 40 Isomer 2 | 1-(2-(3-fluoro-5-(trifluoromethyl)benzyl)pyridin-4-yl)-6-methyl-1,5,6,7-tetrahydro-4*H*-pyrazolo[4,3-*c*]pyridin-4-one (Single enantiomer of unknown absolute stereochemistry) | Example 40<br>Chiral SFC method 2 (Isocratic run 30% co-solvent)<br>Chiral SFC purity analysis: 2.02 min, 99% (Method 4 - gradient run 3-50% co-solvent) | (400 MHz, DMSO-$d_6$) δ: 8.65 (dd, *J* = 5.5, 0.6 Hz, 1H), 8.05 (s, 1H), 7.70 (dd, *J* = 2.1, 0.7 Hz, 1H), 7.63 (tt, *J* = 1.6, 0.7 Hz, 1H), 7.60-7.38 (m, 4H), 4.33 (s, 2H), 3.84-3.71 (m, 1H), 3.20 (dd, *J* = 16.4, 5.1 Hz, 1H), 2.98 (dd, *J* = 16.5, 10.6 Hz, 1H), 1.24 (d, *J* = 6.5 Hz, 3H). | m/z 405.1 (M+H)+ (ES+), at 3.97 min, 100% (Method 1) |

(continued)

| Ex. No. | Name | Intermediate/procedure | $^1$H NMR | LCMS |
|---|---|---|---|---|
| 41 | 1-(4-(3-(trifluoromethyl)benzyl)pyridin-2-yl)-5,6,7,8-tetrahydro-[1,2,3]triazolo[4,5-*c*]azepin-4(1*H*)-one | Intermediates 4 and 15 Procedure 14 | (400 MHz, DMSO-$d_6$) δ: 8.54 (d, *J* = 5.0 Hz, 1H), 8.09 (t, *J* = 4.9 Hz, 1H), 7.93 (s, 1H), 7.78 (s, 1H), 7.67 (d, *J* = 7.3 Hz, 1H), 7.64- 7.55 (m, 2H), 7.52 (d, *J* = 4.7 Hz, 1H), 4.26 (s, 2H), 3.26-3.19 (m, 4H), 2.00-1.94 (m, 2H). | m/z 388.2 (M+H)+ (ES+), at 5.47 min, 99% (Method 8) |
| 42 | 1-(4-(3-(trifluoromethyl)benzyl)pyridin-2-yl)-1,5,6,7-tetrahydro-4*H*-[1,2,3]triazolo[4,5-*c*]pyridin-4-one | Intermediates 4 and 16 Procedure 15 | (400 MHz, DMSO-$d_6$) δ: 8.53 (d, *J* = 4.9 Hz, 1H), 8.07 (bs, 1H),7.82-7.72 (m, 2H), 7.72-7.65 (m, 1H), 7.63-7.53 (m, 2H), 7.50 (d, *J* = 4.8 Hz, 1H), 4.27 (s, 2H), 3.51-3.45 (m, 2H), 3.43-3.37 (m, 2H). | m/z 374.0 (M+H)+ (ES+), at 5.65 min, 100% (Method 5) |
| 43 | 2-(4-(3-(trifluoromethyl)benzyl)pyridin-2-yl)-2,5,6,7-tetrahydro-4*H*-[1,2,3]triazolo[4,5-*c*]pyridin-4-one | Intermediates 4 and 16 Procedure 15 | (400 MHz, DMSO-$d_6$) δ: 8.50 (d, *J* = 4.9 Hz, 1H), 8.12 (bs, 1H), 7.98 (s, 1H), 7.76 (s, 1H), 7.70-7.63 (m, 1H), 7.62-7.55 (m, 2H), 7.45 (d, *J* = 4.5 Hz, 1H), 4.25 (s, 2H), 3.60-3.50 (m, 2H), 3.10-3.00 (m, 2H). | m/z 374.1 (M+H)+ (ES+), at 5.44 min, 100% (Method 5) |
| 44 | 5-methyl-2-(4-(3-(trifluoromethyl)benzyl)pyridin-2-yl)-2,5,6,7-tetrahydro-4*H*-[1,2,3]triazolo[4,5-*c*]pyridin-4-one | Example 43 Procedure 2 | (400 MHz, DMSO-$d_6$) δ: 8.49 (d, *J* = 5.0 Hz, 1H), 7.99 (s, 1H), 7.77 (s, 1H), 7.66 (d, *J* = 7.3 Hz, 1H), 7.59 (dt, *J* = 15.1, 7.6 Hz, 2H), 7.49-7.40 (m, 1H), 4.26 (s, 2H), 3.73 (t, *J* = 6.8 Hz, 2H), 3.13 (t, *J* = 6.8 Hz, 2H), 3.03 (s, 3H). | m/z 388.1 (M+H)+ (ES+), at 5.62 min, 99% (Method 5) |

(continued)

| Ex. No. | Name | Intermediate/procedure | $^1$H NMR | LCMS |
|---|---|---|---|---|
| 45 | 2-(4-(3-(trifluoromethyl)benzyl)pyridin-2-yl)-5,6,7,8-tetrahydro-[1,2,3]triazolo[4,5-*c*]azepin-4(2*H*)-one | Intermediates 4 and 15 Procedure 14 | (400 MHz, DMSO-$d_6$) δ: 8.49 (d, *J* = 4.9 Hz, 1H), 8.29 (t, *J* = 4.8 Hz, 1H), 7.97 (s, 1H), 7.75 (s, 1H), 7.65 (d, *J* = 7.2 Hz, 1H), 7.62- 7.55 (m, 2H), 7.45 (d, *J* = 4.8 Hz, 1H), 4.26 (s, 2H), 3.26 (t, *J* = 4.4 Hz, 2H), 3.06 (t, *J* = 6.7 Hz, 2H), 2.05-1.98 (m, 2H). | m/z 388.2 (M+H)+ (ES+), at 5.74 min, 99% (Method 8) |
| 46 | 1-(4-(3-fluoro-5-(trifluoromethyl)benzyl)pyridin-2-yl)-5,6,7,8-tetrahydro-[1,2,3]triazolo[4,5-*c*]azepin-4(1*H*)-one | Intermediate 18 and CAS: 5220-49-5 Procedure 16 | (400 MHz, DMSO-$d_6$) δ: 8.55 (d, *J* = 5.0 Hz, 1H), 8.09 (t, *J* = 4.8 Hz, 1H), 7.97 (s, 1H), 7.69 (s, 1H), 7.66-7.61 (m, 1H), 7.59-7.54 (m, 2H), 4.27 (s, 2H), 3.28-3.20 (m, 4H), 2.02-1.91 (m, 2H). | m/z 406.1 (M+H)+ (ES+), at 5.59 min, 96% (Method 5) |
| 47 | 1-(4-(3-fluoro-5-(trifluoromethyl)benzyl)pyridin-2-yl)-1,7-dihydropyrazolo[4,3-*d*][1,2]oxazin-4(5*H*)-one | Intermediates 8 and 19 Procedure 17 | (400 MHz, DMSO-$d_6$) δ: 10.68 (s, 1H), 8.41 (d, *J* = 5.08 Hz, 1H), 8.19 (s, 1H), 7.93 (s, 1H), 7.66 (s, 1H), 7.63-7.54 (m, 2H), 7.41-7.36 (m, 1H), 5.52 (s, 2H), 4.25 (s, 2H). | m/z 393.0 (M+H)+ (ES+), at 6.51 min, 99% (Method 8) |
| 48 | 1-(2-(3-fluoro-5-(trifluoromethyl)benzyl)pyridin-4-yl)-5,6-dihydropyrrolo[3,4-*c*]pyrazol-4(1*H*)-one | Intermediates 6 and 19 Procedure 18 | (400 MHz, DMSO-$d_6$) δ: 8.61 (d, *J* = 5.6 Hz, 1H), 8.30 (s, 1H), 8.03 (s, 1H), 7.71 (d, *J* = 2.0 Hz, 1H), 7.61 (s, 1H), 7.56-7.49 (m, 3H), 4.79 (s, 2H), 4.31 (s, 2H). | m/z 377.0 (M+H)+ (ES+), at 5.35 min, 96% (Method 5) |
| 49 | 1-(2-((3-fluoro-5-(trifluoromethyl)phenyl)methyl-*d*)pyridin-4-yl)-1,5,6,7-tetrahydro-4*H*-pyrazolo[4,3-*c*]pyridin-4-one | Example 35 Procedure 19 | (400 MHz, DMSO-$d_6$) δ: 8.66 (d, *J* = 5.6 Hz, 1H), 8.07 (d, *J* = 4.8 Hz, 1H), 7.73 (d, *J* = 1.6 Hz, 1H), 7.63 (s, 1H), 7.57-7.53 (m, 3H), 7.50 (s, 1H), 4.32 (d, *J* = 8.4 Hz, 1H), 3.52-3.43 (m, 2H), 3.22-3.18 (m, 2H). | m/z 392.0 (M+H)+ (ES+), at 1.91 min, 96% (Method 7) |

(continued)

| Ex. No. | Name | Intermediate/procedure | $^1$H NMR | LCMS |
|---|---|---|---|---|
| 50 | 1-(2-(3-fluoro-5-(trifluoromethyl)benzyl)pyridin-4-yl)-7-methyl-1,5,6,7-tetrahydro-4*H*-pyrazolo[4,3-*c*]pyridin-4-one | Intermediates 6 and 20<br>Procedure 1 | (400 MHz, DMSO-$d_6$) δ: 8.66 (d, *J* = 5.5 Hz, 1H), 8.03 (s, 1H), 7.74 (d, *J* = 2.1 Hz, 1H), 7.65-7.43 (m, 5H), 4.33 (s, 2H), 3.66 (dd, *J* = 12.6, 4.7 Hz, 1H), 3.63-3.53 (m, 1H), 3.17 (ddd, *J* = 12.5, 4.5, 2.1 Hz, 1H), 1.11 (d, *J* = 6.8 Hz, 3H). | m/z 405.2 (M+H)+ (ES+), at 5.22 min, 97% (Method 8) |
| 51 | 1-(2-(3-fluoro-5-(trifluoromethyl)benzyl)pyridin-4-yl)-1,5,6,7-tetrahydro-4*H*-pyrrolo[3,2-*c*]pyridin-4-one | Intermediate 21 and CAS: 239087-09-3<br>Procedures 7 and 10, step 4 | (400 MHz, DMSO-$d_6$) δ: 8.61 (d, *J* = 5.2 Hz, 1H), 7.63 (s, 1H), 7.58 (d, *J* = 1.6 Hz, 1H), 7.55-7.53 (m, 2H), 7.40 (d, *J* = 2.0 Hz, 1H), 7.23 (d, *J* = 3.2 Hz, 1H), 7.20 (s, 1H), 6.52 (d, *J* = 3.2 Hz, 1H), 4.28 (s, 2H), 3.38 (t, *J* = 2.4 Hz, 2H), 2.94 (t, *J* = 6.8 Hz, 2H). | m/z 389.9 (M+H)+ (ES+), at 2.11 min, 97% (Method 2) |
| 52 | 1-(2-(3-fluoro-5-(trifluoromethyl)phenoxy)pyridin-4-yl)-1,5,6,7-tetrahydro-4*H*-pyrazolo[4,3-*c*]pyridin-4-one | Intermediate 13 and CAS: 172333-87-8<br>Procedure 8 | (400 MHz, DMSO-$d_6$) δ: 8.33 (d, *J* = 5.6 Hz, 1H), 8.09 (s, 1H), 7.63-7.56 (m, 2H), 7.55-7.50 (m, 3H), 7.38 (d, *J* = 1.6 Hz, 1H), 3.44 (t, *J* = 6.8 Hz, 2H), 2.24 (t, *J* = 6.7 Hz, 2H). | m/z 393.2 (M+H)+ (ES+), at 5.64 min, 96% (Method 5) |
| 53 | 1-(2-(3-cyclopropyl-5-fluorobenzyl)pyridin-4-yl)-1,5,6,7-tetrahydro-4*H*-pyrazolo[4,3-*c*]pyridin-4-one | Intermediates 14 and 22<br>Procedure 9 | (400 MHz, DMSO-$d_6$) δ: 8.64 (d, *J* = 5.6 Hz, 1H), 8.05 (s, 1H), 7.62 (d, *J* = 1.6 Hz, 1H), 7.51-7.50 (m, 2H), 6.9 - 6.93 (m, 2H), 6.73-6.72 (m, 1H), 4.14 (s, 2H), 3.45-3.44 (m, 2H), 3.18 (t, *J* = 6.8 Hz, 2H), 1.95-1.94 (m, 1H), 0.98-0.97 (m, 2H), 0.69 (d, *J* = 4.4 Hz, 2H). | m/z 363.1 (M+H)+ (ES+), at 1.88 min, 100% (Method 2) |

(continued)

| Ex. No. | Name | Intermediate/procedure | $^{1}$H NMR | LCMS |
|---|---|---|---|---|
| 54 | 1-(2-(3-fluoro-4-(trifluoromethyl)benzyl)pyridin-4-yl)-1,5,6,7-tetrahydro-4*H*-pyrazolo[4,3-*c*]pyridin-4-one | Intermediate 14 and CAS: 213203-65-7 Procedure 9 | (400 MHz, $CDCl_3$) δ: 8.73 (d, *J* = 5.6 Hz, 1H), 8.16 (s, 1H), 7.60-7.54 (m, 1H), 7.52 (d, *J* = 1.6 Hz, 1H), 7.37 (d, *J* = 3.6 Hz, 1H), 7.24-7.17 (m, 2H), 5.56 (s, 1H), 4.32 (s, 2H), 3.70-3.66 (m, 2H), 3.21-3.18 (m, 2H). | m/z 391.2 (M+H)+ (ES+), at 1.97 min, 97% (Method 3) |
| 55 | 1-(2-(4-methoxy-3-(trifluoromethyl) benzyl)pyridin-4-yl)-1,5,6,7-tetrahydro-4*H*-pyrazolo[4,3-*c*]pyridin-4-one | Intermediate 14 and CAS: 261951-89-7 Procedure 9 | (400 MHz, $CDCl_3$) δ: 8.71 (d, *J* = 5.6 Hz, 1H), 8.14 (s, 1H), 7.52 (d, *J* = 1.6 Hz, 1H), 7.48 (d, *J* = 8.8 Hz, 1H), 7.37 (d, *J* = 5.6 Hz, 1H), 7.34 (s, 1H), 7.00 (d, *J* = 8.4 Hz, 1H), 5.52 (s, 1H), 4.25 (s, 2H), 3.92 (s, 3H), 3.67-3.66 (m, 2H), 3.13 (t, *J* = 6.8 Hz, 2H). | m/z 403.2 (M+H)+ (ES+), at 1.71 min, 99% (Method 3) |
| 56 | 1-(6-(3-fluoro-5-(trifluoromethyl)benzyl)pyrimidin-4-yl)-1,5,6,7-tetrahydro-4*H*-pyrazolo[4,3-*c*]pyridin-4-one | Intermediate 23 and CAS: 239087-09-3 Procedure 9 | (400 MHz, DMSO-$d_6$) δ: 9.04 (d, *J* = 1.2 Hz, 1H), 8.12 (s, 1H), 8.01 (d, *J* = 0.8 Hz, 1H), 7.66 (s, 1H), 7.61-7.56 (m, 3H), 4.36 (s, 2H), 3.52-3.42 (m, 4H). | m/z 392.1 (M+H)+ (ES+), at 2.01 min, 95% (Method 3) |
| 57 | 1-(2-(3-methoxy-5-(trifluoromethyl) benzyl)pyridin-4-yl)-1,5,6,7-tetrahydro-4*H*-pyrazolo[4,3-*c*]pyridin-4-one | Intermediate 14 and CAS: 916421-00-6 Procedure 9 | (400 MHz, DMSO-$d_6$) δ: 8.65 (d, *J* = 5.6 Hz, 1H), 8.05 (s, 1H), 7.68 (d, *J* = 2.0 Hz, 1H), 7.52-7.52 (m, 2H), 7.30 (s, 1H), 7.24 (s, 1H), 7.10 (s, 1H), 4.26 (s, 2H), 3.82 (s, 3H), 3.45-3.44 (m, 2H), 3.19 (t, *J* = 6.8 Hz, 2H). | m/z 402.9 (M+H)+ (ES+), at 2.07 min, 99% (Method 2) |
| 58 | 1-(2-(4-chloro-3,5-difluorobenzyl)pyridin-4-yl)-1,5,6,7-tetrahydro-4*H*-pyrazolo[4,3-*c*]pyridin-4-one | Intermediate 14 and CAS: 1400991-56-1 Procedure 9 | (400 MHz, DMSO-$d_6$) δ: 8.65 (d, *J* = 5.6 Hz, 1H), 8.06 (s, 1H), 7.69 (d, *J* = 1.6 Hz, 1H), 7.54-7.52 (m, 2H), 7.34-7.32 (m, 2H), 4.23 (s, 2H), 3.44-3.42 (m, 2H), 3.22-3.19 (m, 2H). | m/z 375.0 (M+H)+ (ES+), at 1.89 min, 98% (Method 7) |

(continued)

| Ex. No. | Name | Intermediate/procedure | $^1H$ NMR | LCMS |
|---|---|---|---|---|
| 59 | 1-(2-(benzo[*b*]thiophen-5-ylmethyl) pyridin-4-yl)-1,5,6,7-tetrahydro-4*H*-pyrazolo[4,3-*c*]pyridin-4-one | Intermediate 14 and CAS: 10133-22-9 Procedure 9 | (400 MHz, DMSO-$d_6$) δ: 8.63 (t, *J* = 5.2 Hz, 1H), 8.03 (s, 1H), 7.93 (d, *J* = 8.4 Hz, 1H), 7.84 (d, *J* = 1.2 Hz, 1H), 7.74 (d, *J* = 5.2 Hz, 1H), 7.63 (d, *J* = 2.0 Hz, 1H), 7.50-7.48 (m, 2H), 7.43-7.42 (m, 1H), 7.37-7.35 (m, 1H), 4.31 (s, 2H), 3.43-3.40 (m, 2H), 3.17 (t, *J* = 6.8 Hz, 2H). | m/z 361.1 (M+H)+ (ES+), at 1.91 min, 99% (Method 6) |
| 60 | 1-(2-(3-methoxy-4-(trifluoromethyl) benzyl)pyridin-4-yl)-1,5,6,7-tetrahydro-4*H*-pyrazolo[4,3-*c*]pyridin-4-one | Intermediate 14 and CAS: 853367-87-0 Procedure 9 | (400 MHz, DMSO-$d_6$) δ: 8.65 (d, *J* = 5.6 Hz, 1H), 8.05 (s, 1H), 7.67 (d, *J* = 1.6 Hz, 1H), 7.55-7.51 (m, 3H), 7.29 (s, 1H), 7.03 (d, *J* = 8.4 Hz, 1H), 4.26 (s, 2H), 3.88 (s, 3H), 3.44-3.41 (m, 2H), 3.19 (t, *J* = 6.8 Hz, 2H). | m/z 403.1 (M+H)+ (ES+), at 2.01 min, 100% (Method 6) |
| 61 | 1-(2-(3-fluoro-5-(pentafluoro-λ$^6$-sulfaneyl)benzyl)pyridin-4-yl)-1,5,6,7-tetrahydro-4*H*-pyrazolo[4,3-*c*]pyridin-4-one | Intermediate 14 and CAS: 1240257-20-8 Procedure 9 | (400 MHz, DMSO-$d_6$) δ: 8.66 (d, *J* = 5.6 Hz, 1H), 8.06 (s, 1H), 7.84 (s, 1H), 7.81-7.77 (m, 1H), 7.73 (d, *J* = 2.0 Hz, 1H), 7.58 (d, *J* = 9.2 Hz, 1H), 7.55-7.52 (m, 2H), 4.35 (s, 2H), 3.45-3.41 (m, 2H), 3.20 (t, *J* = 6.8 Hz, 2H). | m/z 449.1 (M+H)+ (ES+), at 2.13 min, 100% (Method 6) |
| 62 | 1-(2-(3-(difluoromethoxy)-5-fluorobenzyl)pyridin-4-yl)-1,5,6,7-tetrahydro-4*H*-pyrazolo[4,3-*c*]pyridin-4-one | Intermediate 14 and CAS: 1017779-39-3 Procedure 9 | (400 MHz, DMSO-$d_6$) δ: 8.65 (d, *J* = 5.2 Hz, 1H), 8.06 (s, 1H), 7.67 (d, *J* = 2.0 Hz, 1H), 7.53-7.52 (m, 2H), 7.47-7.28 (m, 1H), 7.12-7.08 (m, 1H), 7.05 (s, 1H), 7.01-6.98 (m, 1H), 4.22 (s, 2H), 3.45-3.41 (m, 2H), 3.19 (t, *J* = 6.4 Hz, 2H). | m/z 389.1 (M+H)+ (ES+), at 1.93 min, 100% (Method 6) |

(continued)

| Ex. No. | Name | Intermediate/procedure | $^1$H NMR | LCMS |
|---|---|---|---|---|
| 63 | 1-(2-(4-(difluoromethoxy)-3-fluorobenzyl)pyridin-4-yl)-1,5,6,7-tetrahydro-4*H*-pyrazolo[4,3-*c*]pyridin-4-one | Intermediates 14 and 24 Procedures 9, step 1 and 2, and 10, step 4 | (400 MHz, DMSO-$d_6$) δ: 8.64 (d, *J* = 5.6 Hz, 1H), 8.05 (s, 1H), 7.66 (d, *J* = 2.0 Hz, 1H), 7.52-7.50 (m, 2H), 7.41-7.38 (m, 1H), 7.33-7.29 (m, 1H), 7.22-7.02 (m, 2H), 4.20 (s, 2H), 3.45-3.41 (m, 2H), 3.21-3.17 (m, 2H). | m/z 389.1 (M+H)+ (ES+), at 1.67 min, 97% (Method 3) |
| 64 | 1-(2-(3,4-difluoro-5-(trifluoromethyl) benzyl)pyridin-4-yl)-1,5,6,7-tetrahydro-4*H*-pyrazolo[4,3-*c*]pyridin-4-one | Intermediates 14 and 25 Procedures 9, step 1 and 2, and 10, step 4 | (400 MHz, DMSO-$d_6$) δ: 8.65 (d, *J* = 5.6 Hz, 1H), 8.06 (s, 1H), 7.86-7.81 (m, 1H), 7.71-7.70 (m, 1H), 7.66-7.65 (m, 1H), 7.54-7.51 (m, 2H), 4.29 (s, 2H), 3.46-3.42 (m, 2H), 3.24-3.18 (m, 2H). | m/z 409.2 (M+H)+ (ES+), at 2.03 min, 99% (Method 2) |
| 65 | 1-(2-(3-(difluoromethyl)-5-fluorobenzyl)pyridin-4-yl)-1,5,6,7-tetrahydro-4*H*-pyrazolo[4,3-*c*]pyridin-4-one | Intermediates 14 and 26 Procedures 9, step 1 and 2, and 10, step 4 | (400 MHz, DMSO-$d_6$) δ: 8.65 (d, *J* = 5.6 Hz, 1H), 8.06 (s, 1H), 7.69 (d, *J* = 2.0 Hz, 1H), 7.53-7.51 (m, 2H), 7.41 (d, *J* = 12.4 Hz, 2H), 7.29 (d, *J* = 8.8 Hz, 1H), 7.03 (t, *J* = 55.6 Hz, 1H), 4.28 (s, 2H), 3.45-3.41 (m, 2H), 3.19 (t, *J* = 6.8 Hz, 2H). | m/z 373.1 (M+H)+ (ES+), at 1.47 min, 99% (Method 7) |
| 66 | 1-(2-(3-chloro-4,5-difluorobenzyl)pyridin-4-yl)-1,5,6,7-tetrahydro-4*H*-pyrazolo[4,3-*c*]pyridin-4-one | Intermediates 14 and 27 Procedures 9, step 1 and 2, and 10, step 4 | (400 MHz, DMSO-$d_6$) δ: 8.65 (d, *J* = 5.6 Hz, 1H), 8.06 (s, 1H), 7.68 (d, *J* = 2.0 Hz, 1H), 7.54-7.54 (m, 4H), 4.20 (s, 2H), 3.46-3.42 (m, 2H), 3.20 (t, *J* = 6.8 Hz, 2H). | m/z 375.1 (M+H)+ (ES+), at 1.59 min, 98% (Method 7) |

(continued)

| Ex. No. | Name | Intermediate/procedure | $^1$H NMR | LCMS |
|---|---|---|---|---|
| 67 | 1-(2-(quinolin-3-ylmethyl)pyridin-4-yl)-1,5,6,7-tetrahydro-4*H*-pyrazolo[4,3-*c*]pyridin-4-one | Intermediate 14 and CAS: 120277-70-5<br>Procedures 9, step 1 and 2, and 10, step 4 | (400 MHz, DMSO-$d_6$) δ: 8.93 (d, *J* = 2.4 Hz, 1H), 8.65 (d, *J* = 5.6 Hz, 1H), 8.27 (d, *J* = 1.6 Hz, 1H), 8.04 (s, 1H), 7.99 (s, 1H), 7.94 (d, *J* = 0.8 Hz, 1H), 7.75-7.70 (m, 2H), 7.61-7.57 (m, 1H), 7.53-7.51 (m, 2H), 4.43 (s, 2H), 3.43-3.41 (m, 2H), 3.20 (t, *J* = 6.4 Hz, 2H). | m/z 356.2 (M+H)+ (ES+), at 0.97 min, 99% (Method 3) |
| 68 | 1-(2-(4-chloro-3-fluoro-5-(trifluoromethyl)benzyl)pyridin-4-yl)-1,5,6,7-tetrahydro-4*H*-pyrazolo[4,3-*c*]pyridin-4-one | Intermediate 14 and CAS: 1431329-80-4<br>Procedures 9, step 1 and 2, and 10, step 4 | (400 MHz, DMSO-$d_6$) δ: 8.65 (d, *J* = 5.6 Hz, 1H), 8.06 (s, 1H), 7.80-7.72 (m, 3H), 7.55-7.51 (m, 2H), 4.33 (s, 2H), 3.46-3.42 (m, 2H), 3.20 (t, *J* = 6.4 Hz, 2H). | m/z 424.9 (M+H)+ (ES+), at 2.26 min, 94% (Method 2) |
| 69 | 1-(2-(benzo[*b*]thiophen-2-ylmethyl)pyridin-4-yl)-1,5,6,7-tetrahydro-4*H*-pyrazolo[4,3-*c*]pyridin-4-one | Intermediate 14 and CAS: 2076-88-2<br>Procedures 9, step 1 and 2, and 10, step 4 | (400 MHz, DMSO-$d_6$) δ: 8.68 (d, *J* = 5.2 Hz, 1H), 8.05 (s, 1H), 7.88-7.86 (m, 1H), 7.78-7.74 (m, 2H), 7.56-7.50 (m, 2H), 7.35-7.28 (m, 3H), 4.50 (s, 2H), 3.45-3.41 (m, 2H), 3.20 (t, *J* = 6.8 Hz, 2H). | m/z 361.1 (M+H)+ (ES+), at 1.71 min, 97% (Method 7) |
| 70 | 1-(2-(3,5-difluoro-4-(trifluoromethyl)benzyl)pyridin-4-yl)-1,5,6,7-tetrahydro-4*H*-pyrazolo[4,3-*c*]pyridin-4-one | Intermediates 14 and 28<br>Procedure 9 | (400 MHz, DMSO-$d_6$) δ: 8.66 (d, *J* = 5.6 Hz, 1H), 8.07 (s, 1H), 7.73 (d, *J* = 1.6 Hz, 1H), 7.56-7.52 (m, 2H), 7.40-7.38 (m, 2H), 4.31 (s, 2H), 3.46-3.42 (m, 2H), 3.21 (t, *J* = 6.4 Hz, 2H). | m/z 409.2 (M+H)+ (ES+), at 1.71 min, 98% (Method 7) |

(continued)

| Ex. No. | Name | Intermediate/procedure | $^1$H NMR | LCMS |
|---|---|---|---|---|
| 71 | 1-(2-(3-fluoro-5-isopropylbenzyl)pyridin-4-yl)-1,5,6,7-tetrahydro-4*H*-pyrazolo[4,3-*c*]pyridin-4-one | Intermediates 14 and 29 Procedure 9 | (400 MHz, DMSO-$d_6$) δ: 8.64 (d, *J* = 5.2 Hz, 1H), 8.05 (s, 1H), 7.63-7.51 (m, 1H), 7.50-7.50 (m, 2H), 7.09 (s, 1H), 6.97-6.92 (m, 2H), 4.17 (s, 2H), 3.44-3.40 (m, 2H), 3.18 (t, *J* = 6.8 Hz, 2H), 2.90-2.86 (m, 1H), 1.18 (d, *J* = 6.9 Hz, 6H). | m/z 365.0 (M+H)+ (ES+), at 2.11 min, 100% (Method 2) |
| 72 | 1-(2-((5-fluoro-6-methoxypyridin-3-yl) methyl)pyridin-4-yl)-1,5,6,7-tetrahydro-4*H*-pyrazolo[4,3-*c*]pyridin-4-one | Intermediates 14 and 32 Procedures 9, step 1 and 2, and 10, step 4 | (400 MHz, DMSO-$d_6$) δ: 8.72 (d, *J* = 5.6 Hz, 1H), 8.16 (s, 1H), 7.92 (d, *J* = 2.0 Hz, 1H), 7.49 (s, 1H), 7.38- 733 (m, 2H), 5.59 (s, 1H), 4.22 (s, 2H), 4.03 (s, 3H), 3.70-3.66 (m, 2H), 3.19 (t, *J* = 6.8 Hz, 2H). | m/z 354.0 (M+H)+ (ES+), at 1.48 min, 99% (Method 7) |
| 73 | 1-(2-(3-chloro-4-fluoro-5-(trifluoromethyl)benzyl)pyridin-4-yl)-1,5,6,7-tetrahydro-4*H*-pyrazolo[4,3-*c*]pyridin-4-one | Intermediates 14 and 31 Procedures 9, step 1 and 2, and 10, step 4 | (400 MHz, DMSO-$d_6$) δ: 8.65 (d, *J* = 5.6 Hz, 1H), 8.07 (s, 1H), 7.98-7.96 (m, 1H), 7.82-7.80 (m, 1H), 7.72 (d, *J* = 1.6 Hz, 1H), 7.54-7.51 (m, 2H), 4.30 (s, 2H), 3.44-3.42 (m, 2H), 3.20 (t, *J* = 6.8 Hz, 2H). | m/z 425.1 (M+H)+ (ES+), at 1.77 min, 99% (Method 7) |
| 74 | 1-(2-(3-fluoro-5-(1-(trifluoromethyl)cyclopropyl)benzyl)pyridin-4-yl)-1,5,6,7-tetrahydro-4*H*-pyrazolo[4,3-*c*]pyridin-4-one | Intermediates 14 and 30 Procedures 9, step 1 and 2, and 10, step 4 | (400 MHz, DMSO-$d_6$) δ: : 8.65 (t, *J* = 5.2 Hz, 1H), 8.05 (s, 1H), 7.74-7.66 (m, 1H), 7.53-7.51 (m, 2H), 7.35 (s, 1H), 7.21-7.14 (m, 2H), 4.23 (s, 2H), 3.45-3.41 (m, 2H), 3.17 (t, *J* = 6.4 Hz, 2H), 1.38-1.33 (m, 2H), 0.90-0.85 (m, 2H). | m/z 431.2 (M+H)+ (ES+), at 1.90 min, 96% (Method 2) |

(continued)

| Ex. No. | Name | Intermediate/procedure | $^1$H NMR | LCMS |
|---|---|---|---|---|
| 75 | 1-(2-(3-fluoro-5-(trifluoromethyl)benzyl)pyridin-4-yl)-3-methyl-1,5,6,7-tetrahydro-4*H*-pyrazolo[4,3-*c*]pyridin-4-one | Intermediate 36 and CAS: 239087-09-3 Procedures 9, step 1 and 2, and 10, step 4 | (400 MHz, DMSO-$d_6$) δ: 8.61 (d, *J* = 5.6 Hz, 1H), 7.70 (d, *J* = 2.0 Hz, 1H), 7.62 (s, 1H), 7.56-7.53 (m, 2H), 7.48-7.46 (m, 1H), 7.43 (s, 1H), 4.29 (s, 2H), 3.42-3.38 (m, 2H), 3.18-3.14 (m, 2H), 2.41 (s, 3H). | m/z 404.9 (M+H)+ (ES+), at 2.13 min, 98% (Method 2) |
| 76 | 1-(2-((6-fluoro-5-methylpyridin-3-yl)methyl)pyridin-4-yl)-3-methyl-1,5,6,7-tetrahydro-4*H*-pyrazolo[4,3-*c*]pyridin-4-one | Intermediate 36 and CAS: 1260812-39-2 Procedures 9, step 1 and 2, and 10, step 4 | (400 MHz, DMSO-$d_6$) δ: 8.60 (d, *J* = 5.2 Hz, 1H), 8.03 (s, 1H), 7.78 (d, *J* = 9.6 Hz, 1H), 7.64 (d, *J* = 2.0 Hz, 1H), 7.47-7.43 (m, 2H), 4.17 (s, 2H), 3.43-3.39 (m, 2H), 3.16 (t, *J* = 6.8 Hz, 2H), 2.41 (s, 3H), 2.21 (s, 3H). | m/z 352.2 (M+H)+ (ES+), at 1.11 min, 99% (Method 7) |
| 77 | 3-methyl-1-(2-(3,4,5-trifluorobenzyl)pyridin-4-yl)-1,5,6,7-tetrahydro-4*H*-pyrazolo[4,3-*c*]pyridin-4-one | Intermediate 36 and CAS: 220141-72-0 Procedures 9, step 1 and 2, and 10, step 4 | (400 MHz, DMSO-$d_6$) δ: 8.61 (d, *J* = 5.2 Hz, 1H), 7.64 (d, *J* = 2.0 Hz, 1H), 7.48-7.46 (m, 2H), 7.33-7.29 (m, 2H), 4.18 (s, 2H), 3.41-3.39 (m, 2H), 3.18-3.15 (m, 2H), 2.41 (s, 3H). | m/z 373.2 (M+H)+ (ES+), at 1.80 min, 100% (Method 7) |
| 78 | 3-methyl-1-(2-(3-(trifluoromethyl)benzyl)pyridin-4-yl)-1,5,6,7-tetrahydro-4*H*-pyrazolo[4,3-*c*]pyridin-4-one | Intermediate 36 and CAS: 402-23-3 Procedures 9, step 1 and 2, and 10, step 4 | (400 MHz, DMSO-$d_6$) δ: 8.61 (d, *J* = 5.6 Hz, 1H), 7.74 (s, 1H), 7.66 (t, *J* = 1.6 Hz, 2H), 7.60-7.53 (m, 2H), 7.47-7.42 (m, 2H), 4.29 (s, 2H), 3.40-3.38 (m, 2H), 3.15 (t, *J* = 6.4 Hz, 2H), 2.40 (s, 3H). | m/z 387.2 (M+H)+ (ES+), at 1.48 min, 100% (Method 2) |
| 79 | 1-(2-(3-chloro-5-fluorobenzyl)pyridin-4-yl)-3-methyl-1,5,6,7-tetrahydro-4*H*-pyrazolo[4,3-*c*]pyridin-4-one | Intermediate 36 and CAS: 493024-39-8 Procedures 9, step 1 and 2, and 10, step 4 | (400 MHz, DMSO-$d_6$) δ: 8.61 (d, *J* = 5.6 Hz, 1H), 7.66 (d, *J* = 1.6 Hz, 1H), 7.48-7.43 (m, 2H), 7.30-7.20 (m, 3H), 4.20 (s, 2H), 3.41-3.39 (m, 2H), 3.17 (t, *J* = 6.8 Hz, 2H), 2.41 (s, 3H). | m/z 371.1 (M+H)+ (ES+), at 1.99 min, 100% (Method 7) |

(continued)

| Ex. No. | Name | Intermediate/procedure | $^{1}$H NMR | LCMS |
|---|---|---|---|---|
| 80 | 1-(2-(3-chloro-4-fluorobenzyl)pyridin-4-yl)-3-methyl-1,5,6,7-tetrahydro-4*H*-pyrazolo[4,3-*c*]pyridin-4-one | Intermediate 36 and CAS: 192702-01-5 Procedures 9, step 1 and 2, and 10, step 4 | (400 MHz, DMSO-$d_6$) δ: 8.60 (d, *J* = 5.6 Hz, 1H), 7.64 (d, *J* = 1.6 Hz, 1H), 7.58 (d, *J* = 7.6 Hz, 1H), 7.46-7.44 (m, 2H), 7.35 (d, *J* = 8.4 Hz, 2H), 4.17 (s, 2H), 3.43-3.39 (m, 2H), 3.15 (t, *J* = 6.8 Hz, 2H), 2.40 (s, 3H). | m/z 370.9 (M+H)+ (ES+), at 1.58 min, 97% (Method 2) |
| 81 | 1-(2-(4-(difluoromethoxy)-3-fluorobenzyl)pyridin-4-yl)-3-methyl-1,5,6,7-tetrahydro-4*H*-pyrazolo[4,3-*c*]pyridin-4-one | Intermediate 36 and 24 Procedures 9, step 1 and 2, and 10, step 4 | (400 MHz, DMSO-$d_6$) δ: 8.60 (d, *J* = 7.2 Hz, 1H), 7.63 (d, *J* = 2.4 Hz, 1H), 7.47-6.95 (m, 6H), 4.18 (s, 2H), 3.41-3.38 (m, 2H), 3.17-3.12 (m, 2H), 2.27 (s, 3H). | m/z 402.9 (M+H)+ (ES+), at 2.05 min, 98% (Method 2) |
| 82 | 1-(5-(3-fluoro-5-(trifluoromethyl)benzyl)pyridin-3-yl)-3-methyl-1,5,6,7-tetrahydro-4*H*-pyrazolo[4,3-*c*]pyridin-4-one | Intermediate 37 and CAS: 239087-09-3 Procedures 3 and 10, step 4 | (400 MHz, DMSO-$d_6$) δ: 8.68-8.64 (m, 2H), 8.02 (s, 1H), 7.68-7.53 (m, 3H), 7.35 (s, 1H), 4.21 (s, 2H), 3.39-3.37 (m, 2H), 3.06-3.02 (m, 2H), 2.28 (s, 3H). | m/z 405.2 (M+H)+ (ES+), at 1.98 min, 99% (Method 6) |
| 83 | 1-(4-((6-fluoro-5-methylpyridin-3-yl)methyl)pyridin-2-yl)-3-methyl-1,5,6,7-tetrahydro-4*H*-pyrazolo[4,3-*c*]pyridin-4-one | Intermediate 38 and CAS: 1260812-39-2 Procedures 3 and 10, step 4 | (400 MHz, DMSO-$d_6$) δ: 8.38 (d, *J* = 5.2 Hz, 1H), 8.06 (s, 1H), 7.78 (t, *J* = 16.0 Hz, 2H), 7.38 (s, 1H), 7.27 (d, *J* = 4.0 Hz, 1H), 4.09 (s, 2H), 3.74-3.41 (m, 4H), 2.41 (s, 3H), 2.21 (s, 3H). | m/z 352.2 (M+H)+ (ES+), at 1.64 min, 98% (Method 2) |
| 84 | 1-(2-(3-chloro-4,5-difluorobenzyl)pyridin-4-yl)-3-methyl-1,5,6,7-tetrahydro-4H-pyrazolo[4,3-c]pyridin-4-one | Intermediates 36 and 27 Procedures 9, step 1 and 2, and 10, step 4 | (400 MHz, DMSO-$d_6$) δ: 8.61 (d, *J* = 5.6 Hz, 1H), 7.66 (d, *J* = 2.0 Hz, 1H), 7.48-7.42 (m, 4H), 4.18 (s, 2H), 3.43-3.41 (m, 2H), 3.18-3.15 (m, 2H), 2.50 (s, 3H). | m/z 389.2 (M+H)+ (ES+), at 1.52 min, 100% (Method 2) |

(continued)

| Ex. No. | Name | Intermediate/procedure | $^1$H NMR | LCMS |
|---|---|---|---|---|
| 85 | 3-fluoro-5-((4-(3-methyl-4-oxo-4,5,6,7-tetrahydro-1*H*-pyrazolo[4,3-*c*]pyridin-1-yl)pyridin-2-yl)methyl)benzonitrile | Intermediate 36 and CAS: 853368-35-1 Procedures 9, step 1 and 2, and 10, step 4 | (400 MHz, DMSO-$d_6$) δ: 8.61 (d, *J* = 5.2 Hz, 1H), 7.73-7.67 (m, 3H), 7.61 (d, *J* = 10.0 Hz, 1H), 7.49-7.43 (m, 2H), 4.27 (s, 2H), 3.41 (t, *J* = 1.6 Hz, 2H), 3.17 (t, *J* = 6.8 Hz, 2H), 2.41 (s, 3H). | m/z 362.2 (M+H)+ (ES+), at 1.34 min, 99% (Method 7) |
| 86 | 1-(2-(3,4-difluoro-5-(trifluoromethyl) benzyl)pyridin-4-yl)-3-methyl-1,5,6,7-tetrahydro-4*H*-pyrazolo[4,3-*c*]pyridin-4-one | Intermediates 36 and 25 Procedures 9, step 1 and 2, and 10, step 4 | (400 MHz, DMSO-$d_6$) δ: 8.61 (d, *J* = 5.6 Hz, 1H), 7.85-7.80 (m, 1H), 7.69-7.64 (m, 2H), 7.48-7.43 (m, 2H), 4.27 (s, 2H), 3.42-3.34 (m, 2H), 3.14-3.14 (m, 2H), 2.41 (s, 3H). | m/z 423.0 (M+H)+ (ES+), at 1.90 min, 100% (Method 3) |
| 87 | 1-(2-(4-chloro-3-fluoro-5-(trifluoromethyl)benzyl)pyridin-4-yl)-3-methyl-1,5,6,7-tetrahydro-4*H*-pyrazolo [4,3-*c*]pyridin-4-one | Intermediate 36 and CAS: 1431329-80-4 Procedures 9, step 1 and 2, and 10, step 4 | (400 MHz, DMSO-$d_6$) δ: 8.62-8.61 (m, 1H), 7.79-7.77 (m, 2H), 7.70 (d, *J* = 1.6 Hz, 1H), 7.48 (dd, *J* = 2.4, 5.4 Hz, 1H), 7.42 (s, 1H), 4.31 (s, 2H), 3.41 (td, *J* = 6.8, 2.4 Hz, 2H), 3.16 (t, *J* = 6.8 Hz, 2H), 2.41 (s, 3H). | m/z 439.0 (M+H)+ (ES+), at 2.10 min, 100% (Method 7) |
| 88 | 1-(2-(3-chloro-4-fluoro-5-(trifluoromethyl)benzyl)pyridin-4-yl)-3-methyl-1,5,6,7-tetrahydro-4*H*-pyrazolo [4,3-*c*]pyridin-4-one | Intermediates 36 and 31 Procedures 9, step 1 and 2, and 10, step 4 | (400 MHz, DMSO-$d_6$) δ: 8.68 (d, *J* = 5.6 Hz, 1H), 7.54-7.30 (m, 4H), 5.39 (s, 1H), 4.25 (s, 2H), 3.66-3.63 (m, 2H), 3.16 (t, *J* = 6.4 Hz, 2H), 2.59 (s, 3H). | m/z 439.1 (M+H)+ (ES+), at 1.96 min, 95% (Method 2) |
| 89 | 1-(2-(3-(difluoromethoxy)-5-fluorobenzyl)pyridin-4-yl)-3-methyl-1,5,6,7-tetrahydro-4*H*-pyrazolo[4,3-*c*]pyridin-4-one | Intermediate 36 and CAS: 1017779-39-3 Procedures 9, step 1 and 2, and 10, step 4 | (400 MHz, DMSO-$d_6$) δ: 8.61 (d, *J* = 5.6 Hz, 1H), 7.65 (s, 1H), 7.64-7.46 (m, 1H), 7.42 (s, 1H), 7.27 (t, *J* = 72.4 Hz, 1H), 7.10-7.08 (m, 1H), 7.10-7.01 (m, 1H), 6.99 (m, 1 H), 4.20 (s, 2H), 3.42-3.38 (m, 2H), 3.15 (t, *J* = 6.4 Hz, 2H), 2.41 (s, 3H). | m/z 403.2 (M+H)+ (ES+), at 1.55 min, 98% (Method 7) |

(continued)

| Ex. No. | Name | Intermediate/procedure | $^1$H NMR | LCMS |
|---|---|---|---|---|
| 90 | 1-(2-(4-chloro-3,5-difluorobenzyl)pyridin-4-yl)-3-methyl-1,5,6,7-tetrahydro-4*H*-pyrazolo[4,3-*c*]pyridin-4-one | Intermediate 36 and CAS: 1400991-56-1 Procedures 9, step 1 and 2, and 10, step 4 | (400 MHz, DMSO-$d_6$) δ: 8.61 (d, *J* = 5.6 Hz, 1H), 7.66 (d, *J* = 2.0 Hz, 1H), 7.49-7.43 (m, 2H), 7.34-7.31 (m, 2H), 4.22 (s, 2H), 3.43-3.39 (m, 2H), 3.17 (t, *J* = 6.0 Hz, 2H), 2.41 (s, 3H). | m/z 389.0 (M+H)+ (ES+), at 1.92 min, 98% (Method 7) |
| 91 | 1-(2-(3-(difluoromethyl)-5-fluorobenzyl)pyridin-4-yl)-3-methyl-1,5,6,7-tetrahydro-4*H*-pyrazolo[4,3-*c*]pyridin-4-one | Intermediates 36 and 26 Procedures 9, step 1 and 2, and 10, step 4 | (400 MHz, DMSO-$d_6$) δ: 8.61 (d, *J* = 5.6 Hz, 1H), 7.67 (s, 1H), 7.48-7.46 (m, 1H), 7.42-7.38 (m, 3H), 7.30-7.28 (m, 1H), 7.03 (t, *J* = 55.6 Hz, 1H), 4.27 (s, 2H), 3.43-3.39 (m, 2H), 3.15 (t, *J* = 6.8 Hz, 2H), 2.41 (s, 3H). | m/z 387.1 (M+H)+ (ES+), at 1.92 min, 96% (Method 3) |
| 92 | 1-(2-((6-methoxypyridin-3-yl)methyl)pyridin-4-yl)-3-methyl-1,5,6,7-tetrahydro-4*H*-pyrazolo[4,3-*c*]pyridin-4-one | Intermediates 36 and 33 Procedures 9, step 1 and 2, and 10, step 4 | (400 MHz, DMSO-$d_6$) δ: 8.59 (d, *J* = 5.6 Hz, 1H), 8.15 (d, *J* = 2.0 Hz, 1H), 7.67-7.59 (m, 2H), 7.45-7.42 (m, 2H), 6.77-6.75 (m, 1H), 4.11 (s, 2H), 3.81 (s, 3H), 3.42-3.35 (m, 2H), 3.15 (t, *J* = 6.8 Hz, 2H), 2.40 (s, 3H). | m/z 350.2 (M+H)+ (ES+), at 0.97 min, 100% (Method 7) |
| 93 | 1-(2-((5-fluoro-6-methoxypyridin-3-yl)methyl)pyridin-4-yl)-3-methyl-1,5,6,7-tetrahydro-4*H*-pyrazolo[4,3-*c*]pyridin-4-one | Intermediates 36 and 32 Procedures 9, step 1 and 2, and 10, step 4 | (400 MHz, DMSO-$d_6$) δ: 8.59 (d, *J* = 5.2 Hz, 1H), 7.98 (d, *J* = 2.0 Hz, 1H), 7.67-7.62 (m, 2H), 7.46-7.42 (m, 2H), 4.15 (s, 2H), 3.91 (s, 3H), 3.43-3.39 (m, 2H), 3.16 (t, *J* = 6.8 Hz, 2H), 2.33 (s, 3H). | m/z 368.1 (M+H)+ (ES+), at 1.30 min, 98% (Method 7) |
| 94 | 1-(2-((6-(difluoromethoxy)pyridin-3-yl)methyl)pyridin-4-yl)-3-methyl-1,5,6,7-tetrahydro-4*H*-pyrazolo[4,3-*c*]pyridin-4-one | Intermediates 36 and 34 Procedures 9, step 1 and 2, and 10, step 4 | (400 MHz, DMSO-$d_6$) δ: 8.59 (d, *J* = 5.6 Hz, 1H), 8.26 (d, *J* = 2.0 Hz, 1H), 7.89-7.86 (m, 1H), 7.66 (t, *J* = 8.4 Hz, 2H), 7.49-7.42 (m, 2H), 7.04 (d, *J* = 8.4 Hz, 1H), 4.20 (s, 2H), 3.43-3.39 (m, 2H), 3.16 (t, *J* = 6.8 Hz, 2H), 2.41 (s, 3H). | m/z 385.9 (M+H)+ (ES+), at 1.90 min, 97% (Method 2) |

(continued)

| Ex. No. | Name | Intermediate/procedure | $^1$H NMR | LCMS |
|---|---|---|---|---|
| 95 | 1-(4-(3-fluoro-5-(trifluoromethyl)benzyl)pyridin-2-yl)-3-methyl-1,5,6,7-tetrahydro-4*H*-pyrazolo[4,3-*c*]pyridin-4-one | Intermediates 8 and 35 Procedures 1 and 10, step 4 | (400 MHz, DMSO-$d_6$) δ: 8.39 (d, *J* = 5.2 Hz, 1H), 7.88 (s, 1H), 7.65 (s, 1H), 7.58-7.56 (m, 2H), 7.38 (s, 1H), 7.33-7.31 (m, 1H), 4.23 (s, 2H), 3.42-3.33 (m, 4H), 2.34 (s, 3H). | m/z 405.1 (M+H)+ (ES+), at 2.32 min, 96% (Method 7) |
| 96 | 3-fluoro-5-((4-(4-oxo-4,5,6,7-tetrahydro-1*H*-pyrazolo[4,3-*c*]pyridin-1-yl)pyridin-2-yl)methyl)benzonitrile | Intermediate 14 and CAS: 216755-57-6 Procedure 10 | (400 MHz, DMSO-$d_6$) δ: 8.65 (d, *J* = 5.6 Hz, 1H), 8.06 (s, 1H), 7.74-7.70 (m, 3H), 7.63-7.61 (m, 1H), 7.60-7.51 (m, 2H), 4.28 (s, 2H), 3.46-3.42 (m, 2H), 3.22-3.12 (m, 2H). | m/z 348.2 (M+H)+ (ES+), at 1.60 min, 100% (Method 6) |
| 97 | 1-(2-(3-fluoro-5-(trifluoromethyl)benzyl)pyridin-4-yl)-5,6,7,8-tetrahydro-[1,2,3]triazolo[4,5-*c*]azepin-4(1*H*)-one | Intermediates 3 and 15 Procedure 14 | (400 MHz, DMSO-$d_6$) δ: 8.77 (d, *J* = 5.4 Hz, 1H), 8.11 (t, *J* = 5.3 Hz, 1H), 7.82 (d, *J* = 2.0 Hz, 1H), 7.72-7.59 (m, 2H), 7.59-7.42 (m, 2H), 4.36 (s, 2H), 3.35-3.19 (m, 2H), 3.04 (t, *J* = 6.5 Hz, 2H), 1.98 (dd, *J* = 9.4, 5.6 Hz, 2H). | m/z 406.1 (M+H)+ (ES+), at 5.18 min, 98% (Method 5) |
| 98 | 2-(4-(3-fluoro-5-(trifluoromethyl)benzyl)pyridin-2-yl)-2,5,6,7-tetrahydro-4*H*-[1,2,3]triazolo[4,5-*c*]pyridin-4-one | Intermediates 5 and 16 Procedure 15 | (400 MHz, DMSO-$d_6$) δ: 8.51 (d, *J* = 5.0 Hz, 1H), 8.13 (s, 1H), 8.03 (d, *J* = 1.3 Hz, 1H), 7.67 (s, 1H), 7.62 (dd, *J* = 9.8, 2.4 Hz, 1H), 7.56 (dd, *J* = 8.9, 2.0 Hz, 1H), 7.48 (dd, *J* = 5.0, 1.5 Hz, 1H), 4.27 (s, 2H), 3.53 (td, *J* = 6.7, 2.8 Hz, 2H), 3.05 (t, *J* = 6.7 Hz, 2H). | m/z 392.1 (M+H)+ (ES+), at 5.52 min, 99% (Method 5) |

(continued)

| Ex. No. | Name | Intermediate/procedure | 1H NMR | LCMS |
|---|---|---|---|---|
| 99 | 1-(4-(3-fluoro-5-(trifluoromethyl)benzyl)pyridin-2-yl)-1,5,6,7-tetrahydro-4*H*-[1,2,3]triazolo[4,5-*c*]pyridin-4-one | Intermediates 5 and 16 Procedure 15 | (400 MHz, DMSO-$d_6$) δ: 8.53 (d, *J* = 5.1 Hz, 1H), 8.13 (s, 1H), 7.78 (s, 1H), 7.70 (s, 1H), 7.69-7.62 (m, 1H), 7.62-7.50 (m, 2H), 4.28 (s, 2H), 3.48 (td, *J* = 7.0, 5.9, 2.1 Hz, 2H), 3.39 (dd, *J* = 7.2, 5.5 Hz, 2H). | m/z 392.1 (M+H)+ (ES+), at 5.71 min, 99% (Method 5) |
| 100 | 2-(2-(3-fluoro-5-(trifluoromethyl)benzyl)pyridin-4-yl)-2,5,6,7-tetrahydro-4*H*-[1,2,3]triazolo[4,5-*c*]pyridin-4-one | Intermediates 3 and 16 Procedure 15 | (400 MHz, DMSO-$d_6$) δ: 8.76 (d, *J* = 5.4 Hz, 1H), 7.86 (d, *J* = 2.0 Hz, 1H), 7.82 (s, 1H), 7.64 (dd, J = 5.5, 2.0 Hz, 2H), 7.55 (t, *J* = 8.4 Hz, 2H), 4.36 (s, 2H), 3.49 (td, J = 6.8, 2.6 Hz, 2H), 3.24 (t, *J* = 6.8 Hz, 2H). | m/z 392.1 (M+H)+ (ES+), at 5.24 min, 98% (Method 5) |
| 101 | 1-(2-(3-fluoro-5-(trifluoromethyl)benzyl)pyridin-4-yl)-1,5,6,7-tetrahydro-4*H*-[1,2,3]triazolo[4,5-*c*]pyridin-4-one | Intermediates 3 and 16 Procedure 15 | (400 MHz, DMSO-$d_6$) δ: 8.68 (d, *J* = 5.5 Hz, 1H), 8.21 (d, *J* = 3.0 Hz, 1H), 8.07 (d, *J* = 2.1 Hz, 1H), 7.86 (dd, *J* = 5.5, 2.1 Hz, 1H), 7.63 (s, 1H), 7.61-7.49 (m, 2H), 4.35 (s, 2H), 3.54 (td, *J* = 6.7, 2.8 Hz, 2H), 3.07 (t, *J* = 6.7 Hz, 2H). | m/z 392.1 (M+H)+ (ES+), at 5.64 min, 96% (Method 5) |
| 102 | 1-(4-(3-(difluoromethyl)-5-fluorobenzyl)pyridin-2-yl)-1,5,6,7-tetrahydro-4*H*-pyrazolo[4,3-c]pyridin-4-one | Intermediates 1 and 39 Procedure 1 | (400 MHz, DMSO-$d_6$) δ: 8.43 (d, *J* = 5.2 Hz, 1H), 7.99 (s, 1H), 7.86 (s, 1H), 7.46-7.31 (m, 5H), 7.03 (t, *J* = 55.6 Hz, 1H), 4.20 (s, 2H), 3.44 - 3.44 (m, 4H). | m/z 373.1 (M+H)+ (ES+), at 1.91 min, 100% (Method 3) |
| 103 | 1-(5-(3-fluoro-5-(trifluoromethyl)phenoxy)pyridin-3-yl)-1,5,6,7-tetrahydro-4*H*-pyrazolo[4,3-*c*]pyridin-4-one | Intermediate 40 and CAS: 172333-87-8 Procedure 8 (2 equivalents KOtBu substituted for 3 equivalents of $Cs_2CO_3$) | (400 MHz, DMSO-$d_6$) δ: 8.76 (s, 1H), 8.58 (d, *J* = 2.4 Hz, 1H), 8.04 (s, 1H), 7.89 (d, *J* = 2.4 Hz, 1H), 7.58- 7.56 (m, 1H), 7.51-7.47 (m, 3H), 3.44-3.40 (m, 2H), 3.12 (t, *J* = 6.4 Hz, 2H). | m/z 393.1 (M+H)+ (ES+), at 1.73 min, 98% (Method 7) |

(continued)

| Ex. No. | Name | Intermediate/procedure | $^1$H NMR | LCMS |
|---|---|---|---|---|
| 104 | 1-(2-(3-fluoro-5-(trifluoromethyl)benzyl)pyridin-4-yl)-5-(2-methoxyethyl)-1,5,6,7-tetrahydro-4*H*-pyrazolo [4,3-*c*]pyridin-4-one | Intermediate 13 and CAS: 6482-24-2 and 239087-09-3 Procedures 2 (DMF substituted for THF) and 9, step 1 and 2 | (400 MHz, DMSO-$d_6$) δ: 8.65 (d, *J* = 5.2 Hz, 1H), 8.06 (s, 1H), 7.72 (d, *J* = 1.6 Hz, 1H), 7.63 (s, 1H), 7.55-7.52 (m, 3H), 4.32 (s, 2H), 3.68-3.65 (m, 2H), 3.61-3.58 (m, 2H), 3.51-3.49 (m, 2H), 3.29-3.25 (m, 5H). | m/z 449.0 (M+H)+ (ES+), at 2.23 min, 100% (Method 2) |
| 105 | 1-(2-(3-fluoro-5-(trifluoromethyl)benzyl)pyridin-4-yl)-5-(2-hydroxyethyl)-1,5,6,7-tetrahydro-4*H*-pyrazolo[4,3-*c*]pyridin-4-one | Example 104 Procedure 20 | (400 MHz, DMSO-$d_6$) δ: 8.65 (d, *J* = 5.6 Hz, 1H), 8.06 (s, 1H), 7.72 (d, *J* = 1.6 Hz, 1H), 7.63-7.75 (m, 4H), 4.77-4.74 (m, 1H), 4.33 (s, 2H), 3.71-3.68 (m, 2H), 3.56-3.48 (m, 4H), 3.29-3.26 (m, 2H). | m/z 435.1 (M+H)+ (ES+), at 1.76 min, 99% (Method 3) |
| 106 | 1-(4-(3-fluoro-5-(trifluoromethyl)benzyl)pyridin-2-yl)-5-(2-methoxyethyl)-1,5,6,7-tetrahydro-4*H*-pyrazolo [4,3-*c*]pyridin-4-one | Intermediate 12 and CAS: 6482-24-2 and 239087-09-3 Procedures 2 (DMF substituted for THF) and 9, step 1 (additionally with 3 equivalents of LiCl) and 2 (additionally with 0.1 equivalents of CuI) | (400 MHz, DMSO-$d_6$) δ: 8.42 (d, *J* = 5.1 Hz, 1H), 7.99 (s, 1H), 7.89 (s, 1H), 7.65 (s, 1H), 7.58 (dd, J = 14.0, 9.3 Hz, 2H), 7.36 (d, *J* = 5.2 Hz, 1H), 4.23 (s, 2H), 3.65 (t, *J* = 6.9 Hz, 2H), 3.57 (t, *J* = 5.6 Hz, 2H), 3.47 (dt, J = 16.8, 6.2 Hz, 4H), 3.26 (s, 3H). | m/z 449.3 (M+H)+ (ES+), at 6.31 min, 100% (Method 5) |
| 107 | 1-(4-(3-fluoro-5-(trifluoromethyl)benzyl)pyridin-2-yl)-5-(2-hydroxyethyl)-1,5,6,7-tetrahydro-4*H*-pyrazolo[4,3-*c*]pyridin-4-one | Example 106 Procedure 21 | (400 MHz, DMSO-$d_6$) δ: 8.42 (d, *J* = 5.1 Hz, 1H), 7.99 (s, 1H), 7.89 (s, 1H), 7.65 (s, 1H), 7.58 (dd, J = 14.1, 9.4 Hz, 2H), 7.46-7.25 (m, 1H), 4.72 (t, *J* = 5.4 Hz, 1H), 4.23 (s, 2H), 3.68 (t, *J* = 6.9 Hz, 2H), 3.55 (q, *J* = 5.8 Hz, 2H), 3.51-3.39 (m, 4H). | m/z 435.2 (M+H)+ (ES+), at 5.94 min, 100% (Method 5) |

(continued)

| Ex. No. | Name | Intermediate/procedure | $^1$H NMR | LCMS |
|---|---|---|---|---|
| 108 | 1-(2-(3-fluoro-5-(trifluoromethyl)benzyl)-5-methylpyridin-4-yl)-1,5,6,7-tetrahydro-4*H*-pyrazolo[4,3-*c*]pyridin-4-one | Intermediate 41 and CAS: 239087-09-3<br>Procedures 9, step 1 and 2, and 10, step 4 | (400 MHz, DMSO-$d_6$) δ: 8.60 (s, 1H), 8.01 (s, 1H), 7.59 (s, 1H), 7.54-7.52 (m, 3H), 7.45 (s, 1H), 4.26 (s, 2H), 3.43-3.39 (m, 2H), 2.84 (t, *J* = 6.8 Hz, 2H), 2.18 (s, 3H). | m/z 405.1 (M+H)+ (ES+), at 1.93 min, 100% (Method 10) |
| 109 | 1-(3-(3-fluoro-5-(trifluoromethyl)benzyl)phenyl)-1,5,6,7-tetrahydro-4*H*-pyrazolo[4,3-*c*]pyridin-4-one | Intermediates 1 and 42<br>Procedure 22 | (400 MHz, DMSO-$d_6$) δ: 7.95 (s, 1H), 7.69-7.34 (m, 8H), 4.17 (s, 2H), 3.40 (td, *J* = 6.8, 2.5 Hz, 2H), 3.04 (t, *J* = 6.8 Hz, 2H). | m/z 390.0 (M+H)+ (ES+), at 4.33 min, 95% (Method 1) |
| 110 | 1-(2-((6-(difluoromethoxy)-5-fluoropyridin-3-yl)methyl)pyridin-4-yl)-1,5,6,7-tetrahydro-4*H*-pyrazolo[4,3-*c*]pyridin-4-one | Intermediates 14 and 32<br>Procedures 9, step 1 and 2, and 23 | (400 MHz, DMSO-$d_6$) δ: 8.64 (d, *J* = 5.2 Hz, 1H), 8.10 (d, *J* = 2.0 Hz, 1H), 8.06 (s, 1H), 7.93 (t, *J* = 10.8 Hz, 1H), 7.70 (t, *J* = 3.6 Hz, 2H), 7.54-7.53 (m, 2H), 4.25 (s, 2H), 3.45 (t, *J* = 2.4 Hz, 2H), 3.20 (t, *J* = 6.8 Hz, 2H). | m/z 390.0 (M+H)+ (ES+), at 1.52 min, 96% (Method 3) |
| 111 | 1-(2-((6-(difluoromethoxy)-5-fluoropyridin-3-yl)methyl)pyridin-4-yl)-3-methyl-1,5,6,7-tetrahydro-4*H*-pyrazolo[4,3-*c*]pyridin-4-one | Example 93<br>Procedure 23 | (400 MHz, DMSO-$d_6$) δ: 8.60 (d, *J* = 5.6 Hz, 1H), 8.10 (s, 1H), 7.94 (d, *J* = 1.6 Hz, 1H), 7.70-7.67 (m, 2H), 7.52 (s, 1H), 7.47 (t, *J* = 3.2 Hz, 1H), 4.23 (s, 2H), 3.42-3.41 (m, 2H), 3.17 (t, *J* = 6.8 Hz, 2H), 2.41 (s, 3H). | m/z 404.1 (M+H)+ (ES+), at 1.58 min, 98% (Method 3) |
| 112 | 2-(2-(3-fluoro-5-(trifluoromethyl)benzyl)pyridin-4-yl)-6,7-dihydro-2*H*-pyrazolo[4,3-*f*][1,4]oxazepin-4(5*H*)-one | Intermediates 3 and 43<br>Procedure 24 | (400 MHz, DMSO-$d_6$) δ: 9.04 (s, 1H), 8.54 (d, *J* = 5.6 Hz, 1H), 8.06 (t, *J* = 4.0 Hz, 1H), 7.95 (d, *J* = 2.0 Hz, 1H), 7.75-7.73 (m, 1H), 7.60 (s, 1H), 7.56-7.52 (m, 2H), 4.42-4.40 (m, 2H), 4.25 (s, 2H), 3.49-3.46 (m, 2H). | m/z 407.1 (M+H)+ (ES+), at 1.75 min, 99% (Method 3) |

(continued)

| Ex. No. | Name | Intermediate/procedure | $^1$H NMR | LCMS |
|---|---|---|---|---|
| 113 | 1-(4-(3-fluoro-5-(trifluoromethyl)benzyl)pyridin-2-yl)-3-(hydroxymethyl)-1,5,6,7-tetrahydro-4*H*-pyrazolo[4,3-*c*]pyridin-4-one | Intermediates 8 and 44 Procedures 1 and 20 | (400 MHz, DMSO-$d_6$) δ: 8.42 (d, *J* = 5.2 Hz, 1H), 7.89 (s, 1H), 7.83-7.81 (m, 1H), 7.65 (s, 1H), 7.57-7.55 (m, 2H), 7.35 (d, *J* = 5.2 Hz, 1H), 5.76 (t, *J* = 6.0 Hz, 1H), 4.65 (d, *J* = 6.4 Hz, 2H), 4.24 (s, 2H), 3.46-3.45 (m, 4H). | m/z 420.9 (M+H)+ (ES+), at 2.57 min, 99% (Method 2) |
| 114 | 1-(2-(3-fluoro-5-(trifluoromethyl)benzyl)pyridin-4-yl)-3-(hydroxymethyl)-1,5,6,7-tetrahydro-4*H*-pyrazolo[4,3-*c*]pyridin-4-one | Intermediates 6 and 44 Procedures 1 and 20 | (400 MHz, DMSO-$d_6$) δ: 8.65 (d, *J* = 5.6 Hz, 1H), 7.82 (s, 1H), 7.71 (d, *J* = 2.0 Hz, 1H), 7.62 (s, 1H), 7.56-7.50 (m, 3H), 5.69 (t, *J* = 6.4 Hz, 1H), 4.65 (d, *J* = 6.4 Hz, 2H), 4.32 (s, 2H), 3.47 (t, *J* = 6.8 Hz, 2H), 3.20 (t, *J* = 6.8 Hz, 2H). | m/z 420.9 (M+H)+ (ES+), at 2.06 min, 97% (Method 2) |
| 115 | 1-(2-(3-fluoro-5-(trifluoromethyl)benzyl)pyridin-4-yl)-4-oxo-4,5,6,7-tetrahydro-1*H*-pyrazolo[4,3-c]pyridine-3-carbonitrile | Intermediate 45 Procedure 10, step 3 | (400 MHz, DMSO-$d_6$) δ: 8.73 (d, *J* = 5.2 Hz, 1H), 7.94 (s, 1H), 7.81 (s, 1H), 7.63-7.55 (m, 4H), 4.35 (s, 2H), 3.47 (d, *J* = 6.4 Hz, 2H), 3.25-3.22 (m, 2H). | m/z 416.0 (M+H)+ (ES+), at 2.23 min, 98% (Method 3) |
| 116 | 1-(2-(3-fluoro-5-(trifluoromethyl)benzyl)pyridin-4-yl)-3-(trifluoromethyl)-1,5,6,7-tetrahydro-4*H*-pyrazolo[4,3-*c*]pyridin-4-one | Intermediate 45 Procedure 25 | (400 MHz, DMSO-$d_6$) δ: 8.71 (d, *J* = 5.6 Hz, 1H), 7.78 (d, *J* = 8.4 Hz, 2H), 7.63-7.53 (m, 4H), 4.35 (s, 2H), 3.45-3.33 (m, 2H), 3.20 (t, *J* = 6.8 Hz, 2H). | m/z 459.0 (M+H)+ (ES+), at 2.38 min, 98% (Method 12) |
| 117 Isomer 1 | 1-(2-((3-fluoro-5-(trifluoromethyl)phenyl)(hydroxy)methyl)pyridin-4-yl)-3-methyl-1,5,6,7-tetrahydro-4*H*-pyrazolo [4,3-*c*]pyridin-4-one (Single enantiomer of unknown absolute stereochemistry) | Example 75 Procedure 26<br>Chiral SFC purity analysis: 4.91 min, 99% (Method 6 - isocratic run 20% cosolvent) | (400 MHz, DMSO-$d_6$) δ: 8.60 (d, *J* = 5.2 Hz, 1H), 7.90 (d, *J* = 2.4 Hz, 1H), 7.70 (s, 1H), 7.62-7.56 (m, 2H), 7.49-7.47 (m, 1H), 7.44 (s, 1H), 6.66 (s, 1H), 5.94 (s, 1H), 3.43-3.40 (m, 2H), 3.20-3.15 (m, 2H), 2.42 (s, 3H). | m/z 421.0 (M+H)+ (ES+), at 1.68 min, 99% (Method 3) |

(continued)

| Ex. No. | Name | Intermediate/procedure | $^1$H NMR | LCMS |
|---|---|---|---|---|
| 117 Isomer 2 | 1-(2-((3-fluoro-5-(trifluoromethyl)phenyl)(hydroxy)methyl)pyridin-4-yl)-3-methyl-1,5,6,7-tetrahydro-4*H*-pyrazolo [4,3-*c*]pyridin-4-one (Single enantiomer of unknown absolute stereochemistry) | Example 75 Procedure 26<br>Chiral SFC purity analysis: 5.54 min, 99% (Method 6 - isocratic run 20% cosolvent) | (400 MHz, DMSO-$d_6$) δ: 8.60 (d, *J* = 5.2 Hz, 1H), 7.90 (d, *J* = 2.4 Hz, 1H), 7.70 (s, 1H), 7.62-7.56 (m, 2H), 7.49-7.47 (m, 1H), 7.44 (s, 1H), 6.66 (s, 1H), 5.94 (s, 1H), 3.43-3.40 (m, 2H), 3.20-3.15 (m, 2H), 2.42 (s, 3H). | m/z 421.0 (M+H)+ (ES+), at 1.68 min, 100% (Method 3) |
| 118 Isomer 1 | 1-(4-((3-fluoro-5-(trifluoromethyl)phenyl)(hydroxy)methyl)pyridin-2-yl)-3-methyl-1,5,6,7-tetrahydro-4*H*-pyrazolo [4,3-*c*]pyridin-4-one (Single enantiomer of unknown absolute stereochemistry) | Example 95 Procedure 27<br>Chiral SFC purity analysis: Could not be determined - isomers not separable under available methods | (400 MHz, DMSO-$d_6$) δ: 8.41 (t, *J* = 5.2 Hz, 1H), 8.02 (s, 1H), 7.72 (s, 1H), 7.66-7.59 (m, 2H), 7.42-7.37 (m, 2H), 6.63 (d, *J* = 4.4 Hz, 1H), 6.02 (d, *J* = 4.0 Hz, 1H), 3.41-3.33 (m, 4H), 2.42 (s, 3H). | m/z 420.9 (M+H)+ (ES+), at 2.46 min, 98% (Method 2) |
| 118 Isomer 2 | 1-(4-((3-fluoro-5-(trifluoromethyl)phenyl)(hydroxy)methyl)pyridin-2-yl)-3-methyl-1,5,6,7-tetrahydro-4*H*-pyrazolo [4,3-*c*]pyridin-4-one (Single enantiomer of unknown absolute stereochemistry) | Example 95 Procedure 27<br>Chiral SFC purity analysis: Could not be determined - isomers not separable under available methods | (400 MHz, DMSO-$d_6$) δ: 8.41 (t, *J* = 5.2 Hz, 1H), 8.02 (s, 1H), 7.72 (s, 1H), 7.66-7.59 (m, 2H), 7.42-7.37 (m, 2H), 6.63 (d, *J* = 4.4 Hz, 1H), 6.02 (d, *J* = 4.0 Hz, 1H), 3.41-3.33 (m, 4H), 2.42 (s, 3H). | m/z 420.9 (M+H)+ (ES+), at 2.46 min, 99% (Method 2) |

**BIOLOGICAL ACTIVITY**

**GPR52 Agonist Functional cAMP Assay**

HEKf suspension cells were infected for 24 h with 0.1% v/v human GPR52 expressing BacMam virus, a modified baculovirus designed for mammalian gene expression. Following BacMam infection, cells were pelleted by centrifugation (335g, 5 min), resuspended in cell freezing medium (Sigma) and frozen at -150 °C until required. On experiment day, 25 nL GPR52 compound dilutions, prepared in DMSO, were stamped onto proxiplates (PerkinElmer) by a LabCyte ECHO acoustic dispenser. Frozen cells were thawed and resuspended in assay stimulation buffer (Cisbio) containing 0.5mM 3-iso-butyl-1-methylxanthine (IBMX, Sigma) to achieve a density of 2000 cells per well. 10 μl cells were added to assay plates using a Multidrop Combi Reagent Dispenser (ThermoFisher) before centrifugation (335g, 1 min). Cells were incubated with compounds at 37 °C for 30 min prior to addition of cAMP detection reagents (HiRange cAMP kit, Cisbio) which were prepared according to the manufacturer's instructions. Plates were shaken for 1 h at room temperature before reading on a PHERAstar FS plate reader (BMG Labtech) using standard HTRF settings. HTRF ratios were obtained by dividing the acceptor emissions (665 nm) by the donor emissions (620 nm) and multiplying by 10,000. Data were normalised to DMSO (0%) and maximal 3-(2-(3-chloro-5-fluorobenzyl)benzo[b]thiophen-7-yl)-N-(2-methoxyethyl)benzamide (compound **7m** in *J. Med. Chem.,* **2014,** *57*, 5226) responses (100%) and fit to a 4-parameter logistical fit to generate agonist $pEC_{50}$s and maximal responses which are presented in **Table 4** below.

Table 4 - GPR52 $pEC_{50}$ data

| Ex. No. | $pEC_{50}$ average | $E_{max}$ (%) |
|---|---|---|
| 1 | 7.5 | 97 |
| 2 | 7.2 | 86 |
| 3 | 7.3 | 104 |
| 4 | 8.0 | 95 |
| 5 | 7.6 | 96 |
| 6 | 6.3 | 82 |
| 7 | 6.7 | 83 |
| 8 | 6.5 | 75 |
| 9 | 6.3 | 89 |
| 10 | 8.1 | 90 |
| 11 | 7.2 | 86 |
| 12 | 7.3 | 92 |
| 13 | 7.6 | 89 |
| 14 | 8.0 | 103 |
| 15 | 6.9 | 95 |
| 16 | 7.0 | 95 |
| 17 | 5.9 | 96 |
| 18 | 7.1 | 82 |
| 19 | 6.2 | 73 |
| 20 | 6.8 | 101 |
| 21 | 5.9 | 100 |
| 22 | 6.7 | 91 |
| 23 | 6.8 | 90 |
| 24 | 6.5 | 95 |
| 25 | 6.8 | 89 |
| 26 | 7.1 | 95 |
| 27 | 7.1 | 90 |
| 28 | 7.0 | 88 |
| 29 | 5.6 | 57 |
| 30 | 6.1 | 77 |
| 31 | 6.9 | 95 |
| 32 | 6.4 | 61 |
| 33 | 5.5 | 58 |
| 34 | 6.0 | 54 |
| 35 | 5.5 | 40 |
| 36 | 6.3 | 92 |
| 36 Isomer 1 | 5.8 | 72 |
| 36 Isomer 2 | 6.6 | 94 |
| 37 | 6.9 | 93 |

(continued)

| Ex. No. | $pEC_{50}$ average | $E_{max}$ (%) |
|---|---|---|
| 38 | 6.2 | 55 |
| 39 | 7.4 | 90 |
| 40 | 7.0 | 93 |
| 40 Isomer 1 | 7.5 | 95 |
| 40 Isomer 2 | 6.9 | 83 |
| 41 | 6.1 | 90 |
| 42 | 6.1 | 90 |
| 43 | 6.6 | 86 |
| 44 | 5.7 | 74 |
| 45 | 6.2 | 75 |
| 46 | 6.7 | 90 |
| 47 | 7.1 | 81 |
| 48 | 6.6 | 83 |
| 49 | 7.4 | 98 |
| 50 | 7.4 | 95 |
| 51 | 6.9 | 95 |
| 52 | 5.8 | 69 |
| 53 | 8.2 | 100 |
| 54 | 6.4 | 63 |
| 55 | 6.4 | 69 |
| 56 | 6.9 | 98 |
| 57 | 6.1 | 44 |
| 58 | 7.0 | 79 |
| 59 | 7.1 | 79 |
| 60 | 6.4 | 83 |
| 61 | 7.3 | 99 |
| 62 | 7.1 | 94 |
| 63 | 6.9 | 70 |
| 64 | 7.7 | 94 |
| 65 | 7.7 | 94 |
| 66 | 7.6 | 100 |
| 67 | 5.8 | 53 |
| 68 | 7.4 | 91 |
| 69 | 6.9 | 60 |
| 70 | 6.5 | 74 |
| 71 | 7.7 | 91 |
| 72 | 5.9 | 83 |
| 73 | 7.3 | 88 |
| 74 | 6.3 | 53 |

(continued)

| Ex. No. | $pEC_{50}$ average | $E_{max}$ (%) |
|---|---|---|
| 75 | 8.4 | 98 |
| 76 | 7.1 | 94 |
| 77 | 7.8 | 95 |
| 78 | 7.7 | 94 |
| 79 | 7.9 | 99 |
| 80 | 8.0 | 97 |
| 81 | 8.0 | 70 |
| 82 | 8.0 | 101 |
| 83 | 7.6 | 93 |
| 84 | 8.6 | 95 |
| 85 | 7.2 | 96 |
| 86 | 8.5 | 102 |
| 87 | 8.2 | 95 |
| 88 | 8.3 | 100 |
| 89 | 8.2 | 92 |
| 90 | 7.7 | 87 |
| 91 | 8.5 | 97 |
| 92 | 6.2 | 74 |
| 93 | 7.0 | 83 |
| 94 | 6.4 | 78 |
| 95 | 8.2 | 98 |
| 96 | 6.1 | 94 |
| 97 | 5.6 | 90 |
| 98 | 6.8 | 92 |
| 99 | 6.9 | 95 |
| 100 | 6.2 | 92 |
| 101 | 6.0 | 61 |
| 102 | 8.0 | 98 |
| 103 | 6.0 | 68 |
| 104 | 7.0 | 94 |
| 105 | 7.2 | 97 |
| 106 | 7.5 | 86 |
| 107 | 7.9 | 99 |
| 108 | 7.1 | 80 |
| 109 | 7.9 | 81 |
| 110 | 6.3 | 74 |
| 111 | 7.2 | 87 |
| 112 | 6.4 | 77 |
| 113 | 7.8 | 95 |

(continued)

| Ex. No. | $pEC_{50}$ average | $E_{max}$ (%) |
|---|---|---|
| 114 | 7.4 | 93 |
| 115 | 7.6 | 91 |
| 116 | 8.3 | 95 |
| 117 Isomer 1 | 6.6 | 83 |
| 117 Isomer 2 | 7.6 | 103 |
| 118 Isomer 1 | 6.9 | 94 |
| 118 Isomer 2 | 8.1 | 101 |

**Pharmacokinetic profiling**

The pharmacokinetic profiles of Example 1 were assessed in male Sprague-Dawley rats via intravenous (IV) and oral *(per os,* PO) routes of delivery. Pharmacokinetic data (mean values ± standard deviation) for Example 1 of the invention are detailed in Table 5.

*Methods:* For pharmacokinetic analysis, groups of three male Sprague-Dawley rats, ranging in weight between 200 and 230 g, were administered a single dose of Example 1 via IV or PO route, using the doses, dose volumes and vehicles specified in Table 5. Following dosing, blood samples were taken at several time points (pre-dose, 2 min, 5 min, 15 min, 30 min, 1 h, 3 h, 6 h, 12 h and 24 h for IV administration and pre-dose, 5 min, 15 min, 30 min, 1 hr, 2 h, 4 h, 8 h, 12 h and 24 h for PO administration) via serial tail vein bleeds, and centrifuged to separate plasma for analysis by LC-MS/MS. WinNonlin v8.2 statistics software (Pharsight Corporation, California, USA) was used to generate pharmacokinetic parameters using non-compartmental analysis.

**Brain penetration**

Plasma and brain exposure were evaluated to assess the brain penetration of Example 1, following IV administration. Unbound brain-to-plasma ratio ($K_{p,uu}$) was calculated, as detailed in Table 5, following experimental determination of binding in rat plasma and brain homogenate.

*Methods:* For brain penetration assessment, male Sprague-Dawley rats (n=3) were administered a single 1 mg/kg dose (formulated in 10% DMAC + 10% Solutol HS15 + 80% saline) via the IV route. After 10 min post-dose, animals were sacrificed and brains extracted, homogenised with 2 volumes (w/v) of 50 mM sodium phosphate buffer (pH 7.4), and analysed by LC-MS/MS. Blood samples were removed at the same time point via tail vein bleed, centrifuged and the plasma analysed by LC-MS/MS.

To permit calculation of unbound brain-to-plasma ratio ($K_{p,uu}$), test compound binding in rat plasma and brain homogenate was performed, using Rapid Equilibrium Dialysis (RED). Test compound prepared in DMSO (1 μM final, 0.2% DMSO) was added to (i) undiluted male Sprague Dawley rat plasma and (ii) rat brain tissue homogenised with 2 volumes (w/v) of sodium phosphate buffer (pH 7.4), and dialysed against phosphate buffer for 5 h at 37 °C. After incubation, the contents of each plasma/brain and buffer compartment were removed and mixed with equal volumes of control dialysed buffer or plasma/brain to maintain matrix similarity for analysis. Proteins were then precipitated by the addition of acetonitrile containing an analytical internal standard (allowing ratio of test compound versus internal standard to be derived), centrifuged and the supernatant removed for analysis by LC-MS/MS. Fraction unbound ($F_u$) in plasma and brain was calculated using the following formula, then used to correct total plasma and brain concentrations to derive the $K_{p,uu}$:

Fraction bound = (Total plasma or brain ratio) - (Total buffer ratio) / Total plasma or brain ratio

$$\text{Fraction unbound } (F_u\text{,brain or plasma}) = 1 - \text{Fraction bound}$$

For correction of dilution in brain binding assay:

$$\text{Undiluted } F_u\text{,brain} = (1 / \text{dilution factor}) / ((1 / F_u \text{ diluted})) - 1) + (1 / \text{dilution factor})$$

Where dilution factor = 4

**Table 5 - Pharmacokinetic profiles and brain penetration of Example 1**

| | Rat IV pharmacokinetics (n=3) | | | |
|---|---|---|---|---|
| | Dose (mg/kg) | Dose volume (mL/kg) | Dosing vehicle | Clearance (mL/min/kg) |
| Example 1 | 1 | 5 | 10 % DMAC + 10 % Solutol HS15 + 80 % saline | 16.4 ± 1.0 |
| | Rat PO pharmacokinetics (n=3) | | | |
| | Dose (mg/kg) | Dose volume (mL/kg) | Dosing vehicle | Bioavailability (%) |
| Example 1 | 3 | 5 | 10 % DMAC + 10 % Solutol HS15 + 80 % water | 40.4 ± 6.0 |
| | Rat IV brain penetration, 10 min (n=3) | | | |
| | Dose (mg/kg) | Dose volume (mL/kg) | Dosing vehicle | Kp, uu |
| Example 1 | 1 | 5 | 10 % DMAC + 10 % Solutol HS15 + saline | 0.35 ± 0.03 |

**Amphetamine-induced locomotor activity in rat**

The rat amphetamine-induced locomotor hyperactivity test is used to model the enhanced mesolimbic dopaminergic activity thought to contribute to psychosis in humans. The effects of Example 1 and FTBMT *(J. Pharmacol. Exp. Ther.,* **2017,** *363*, 253) were evaluated in amphetamine-induced hyperlocomotion in the rat, using risperidone as a positive control. Male Sprague-Dawley rats (200-250 g) were housed in groups with a 12 h light/dark cycle (lights on at 07.00), at an ambient temperature of 21±2 °C and with standard pelleted diet and water *ad libitum*. Testing was carried out in the light phase. On the day of the experiment, animals were habituated to the locomotor cages for a 60-minute period.

Subsequently, they were dosed with vehicle, Example 1 (1, 3 and 10 mg/kg) or FTBMT (10 mg/kg) by the oral route or with risperidone by the subcutaneous route and returned to the appropriate locomotor cage. Example 1 was formulated in a vehicle of 10% DMAC, 10% solutol (Kolliphor HS15) and 80% water (v/v/v), FTBMT was formulated in 0.5% HPMC/ 0.1% Tween 80 in de-ionised water and risperidone was dissolved in acidified saline. Sixty minutes later, animals were dosed with vehicle (saline) or D-amphetamine by the subcutaneous route. Locomotor activity was assessed for a two hour period after D-amphetamine treatment. Data are back-transformed means, adjusted for differences between treatment groups in activity during the 30 minutes prior to treatment with test compound or vehicle (n=10-12). Analysis was by general linear model with treatment, cohort and rack as factors. SEMs were calculated from the residuals of the statistical model. Example 1 was compared to D-amphetamine by Williams' test. As shown in figure 1, treatment with Example 1 caused a reduction of the D-amphetamine-induced hyperlocomotor response which reached statistical significance at 10 mg/kg, but not 1 or 3 mg/kg. FTBMT showed a trend towards a reduced D-amphetamine-induced hyperlocomotor response but statistical significance was not reached.

**Subchronic PCP-induced reversal learning deficit in rat**

Subchronic treatment with PCP in the rat results in long-lasting neurobiological alterations and dysfunction in a range of behaviours relevant to neuropsychiatric disorders. Deficits in cognitive flexibility following subchronic PCP treatment have been demonstrated using the reversal learning paradigm (*Neuropharmacology,* **2017,** *142,* 41). The ability of Example 1 to attenuate the subchronic PCP induced cognitive deficit was assessed in female Lister Hooded rats in a manner comparable to the $D_1$ receptor agonist SKF-38393.

Adult female Lister Hooded rats were housed in groups of 5 under standard laboratory conditions under a 12 h light: dark cycle, lights on at 07.00 and food restricted to 90% of free-feeding bodyweight. Testing was carried out in the light phase. Rats underwent operant training firstly to press the left/right lever for a food reward in the presence or absence of a visual cue, and then to respond to the opposite contingency. The reversal learning task was introduced as follows. On the day before each reversal task session, a full 30-minute operant training session was conducted in order to ensure stable responding. In the initial task, animals were first exposed to a five-minute period during which the contingency (cue position relative to active lever) was the same as for the operant training session. This was followed by a 2-minute time-out period, which was signalled by the house-light being turned off. In the subsequent 5-minute period (reversal task), the contingency

was reversed. Responses made on the correct and incorrect levers were recorded. Animals undertook several of these reversal learning task sessions before the beginning of the drug studies in order to ensure that they attained a stable level of performance in both phases of the task. In general, 4-6 sessions were required before this was achieved and in total, animals required about 6 weeks of training to reach this point. At this stage, training ceased and rats were treated with PCP (2 mg/kg, IP or vehicle 0.9% saline, IP) for 7 days followed by at least 7-days washout period.

On the day of testing, rats were randomly assigned to six treatment groups (n=8-10 per group) to receive an acute treatment with Example 1 (3, 10 and 30 mg/kg, PO), SKF-38393 (6 mg/kg, IP) or vehicle. Example 1 was dissolved in 10% DMAC, 10% Solutol (kolliphor H515) and 80% water and administered in a volume of 5 ml/kg *via* the oral route, 2 hours prior to testing. SKF-38393 was dissolved in 0.9% NaCl and administered IP in a volume of 1 ml/kg, 60 min prior to testing. In the initial phase, the reward contingency was the same as the previous day and in the reversal phase the reward contingency was reversed. Animals underwent 1 reversal session following the first phase (initial phase): i.e. initial phase 5 mins, time out of 2 mins, reversal phase 1 for 5 mins, end of experiment. Rats were randomized into a crossover design using Microsoft Excel 2010 random number generator whereby each rat within a cage receives a different treatment both within and between experiments.

Data are presented as mean total number of lever presses (+ SEM) and percent correct response (+ SEM). The percent correct response data were used to determine whether there was a significant effect of drugs on response accuracy; these data were Arc-Sin transformed prior to analysis. Statistical significance was assumed when $P<0.05$ and was determined as follows: one-way ANOVA (IBM SPSS Statistics 22) was performed in order to detect main effect of drug treatment in the initial and reversal tasks. Where a significant effect was detected, a post-hoc LSD t-test was performed in order to compare treatment groups versus the appropriate control in the reversal phase of the test.

Subchronic PCP treatment produced a significant ($P<0.001$) deficit in reversal learning, measured by a significant and selective reduction in percent correct responding in the reversal stage of the test with initial phase performance unaffected. Treatment with Example 1 at 10 and 30 mg/kg (but not 3 mg/kg), and the positive control compound, SKF-38393, significantly ($P<0.05$- $P<0.001$) reversed the deficit compared to the vehicle-treated subchronic PCP group (figure 2). There were no significant effects of Example 1 or SKF-38393 on the total number of lever presses (figure 3), indicating that any pro-cognitive effects were produced without a reduction in motivation or motor control.

## Brief Description of the Figures

**Figure 1:** The effect of acute treatment with Example 1 (1, 3 and 10 mg/kg, PO) and risperidone (0.6 mg/kg, SC) on D-amphetamine-induced hyperlocomotor activity. Significant differences vs D-amphetamine are represented as *p<0.05, **p<0.01, ***p<0.001.

**Figure 2:** The effect of acute treatment with Example 1 (3, 10 and 30 mg/kg, PO, 2 h prior to testing) and SKF38393 (6 mg/kg, IP, 60 min prior to testing) in subchronic PCP (scPCP) treated rats (2 mg/kg, IP twice daily for seven days, followed by at least a 7-day washout period) on performance in the reversal learning task. Data are shown as mean ± s.e.m. % correct responding (n=8-10) and were analysed by ANOVA and post-hoc Student's t-test. Significant reduction in percent correct responding in the reversal phase compared with the vehicle group **P<0.01, ***P<0.001. Improvement in responding compared to scPCP alone in the reversal phase; #P<0.05- # # # P<0.001.

**Figure 3:** The effect of acute treatment with Example 1 (3, 10 and 30 mg/kg, PO, 2 h prior to testing) and SKF38393 (6 mg/kg, IP, 60 min prior to testing) in subchronic PCP (scPCP) treated rats (2 mg/kg, IP) twice daily for seven days, followed by at least a 7-day washout period) on performance in the reversal learning task. Data are shown as the mean total number of lever presses, correct responses are shown as clear bars and incorrect responses are shown as shaded bars (n=8-10).

## Claims

1. A compound of Formula (1):

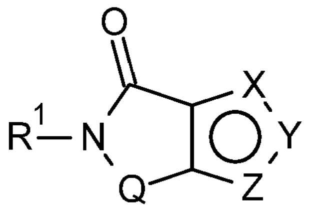

(1)

or a salt thereof, wherein;
X is N or $CR^2$;
Y is N, $NR^3$ or $CR^2$;
Z is N or $NR^3$;
wherein one but not both of Y and Z is $NR^3$;
Q is selected from $-CR^4R^5-$, $-CR^4R^5CR^6R^7-$, $-CR^4R^5CR^6R^7CR^8R^9-$, $-CR^4R^5CR^6R^7O-$, $-OCR^4R^5-$ and $-OCR^4R^5CR^6R^7-$;
R' is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;
$R^2$ is H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;
$R^4$, $R^5$, $R^6$, $R^7$, $R^8$ and $R^9$ are independently selected from H and $C_{1-3}$ alkyl;
wherein $R^3$ is a group of the formula:

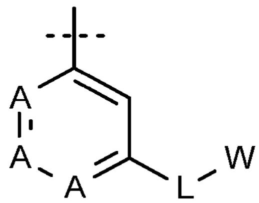

wherein, each A is independently N or $CR^{10}$;
L is selected from $CH_2$, CHD, $CD_2$, CHF, $CF_2$, C=O, CHOH, O and NH;
$R^{10}$ is selected from H, halo and $C_{1-3}$ alkyl optionally substituted with 1 to 6 fluorine atoms;
and W is either:

(i) a 6-membered optionally substituted aryl or a 6-membered optionally substituted heteroaryl ring;
or
(ii) a 9-10-membered optionally substituted heterobicyclic ring system, where one or more of the rings is aromatic.

**2.** The compound according to claim 1, wherein W is a 6-membered optionally substituted aryl or 6-membered optionally substituted heteroaryl ring.

**3.** The compound according to claim 1, which is

(i) a compound of formula (2a), (2b), (2c), (2d), (2e), (2f) or (2g):

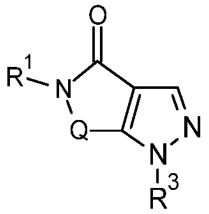 (2a); 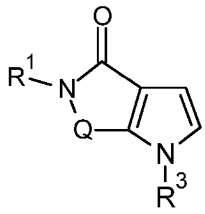 (2b); 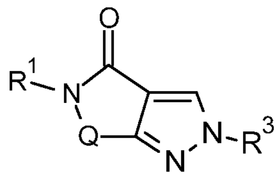 (2c);

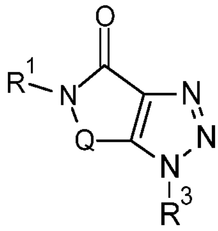 (2d); 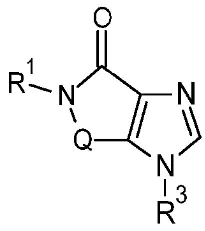 (2e); 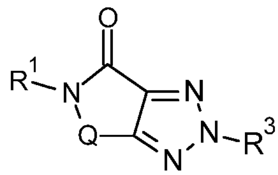 (2f);

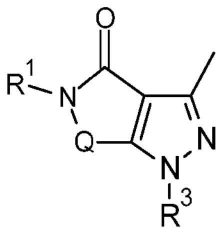 (2g);

or a salt thereof; or

(ii) a compound of formula (11a):

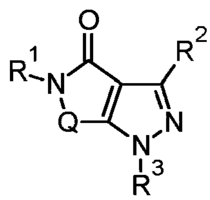 (11a)

or a salt thereof.

**4.** The compound according to any one of claims 1 to 3 wherein

(i) $R^3$ is a group of the formula:

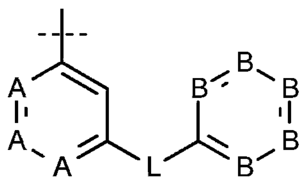

wherein, each A is independently N or $CR^{10}$;
L is selected from $CH_2$, CHD, $CD_2$, CHF, $CF_2$, C=O, CHOH, O and NH;
each B is independently N, $CR^{11}$, $CR^{12}$, $CR^{13}$, $CR^{15}$ or $CR^{16}$;
$R^{10}$ is selected from H, halo and $C_{1-3}$ alkyl optionally substituted with 1 to 6 fluorine atoms;
$R^{11}$, $R^{12}$, $R^{13}$, $R^{15}$ and $R^{16}$ are independently selected from H, CN, $SF_5$, halo, a $C_{1-6}$ saturated hydrocarbon group optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms

and $C_{3-6}$ cycloalkyl optionally substituted with 1 to 6 fluorine atoms or optionally substituted with $CF_3$; preferably wherein

(ii) $R^3$ is selected from the group consisting of:

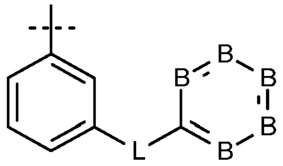
;
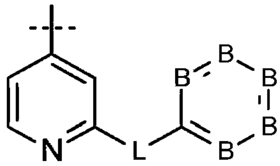
;
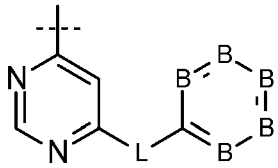
;
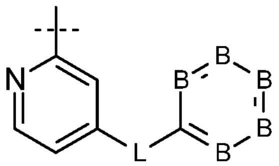
;
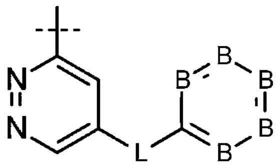
;
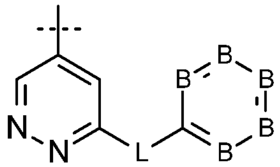
;
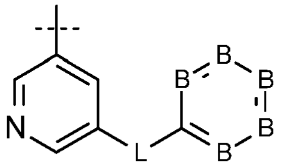
;
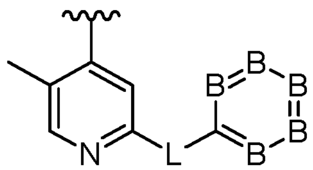
.

5. The compound according to any one of claims 1 to 4, wherein L is $CH_2$.

6. The compound according to claims 4 or 5, wherein the group:

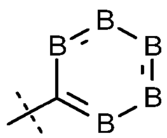

is selected from the group consisting of:

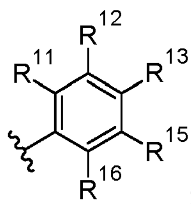
;
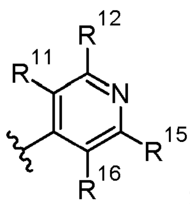
;
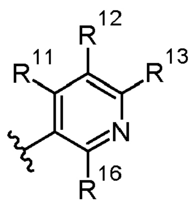
.

7. **The** compound according to any one of claims 1 to 6, wherein Q is selected from the group consisting of $-CH_2CH_2-$; $-CH_2CH_2CH_2-$; $-CH_2-$; $-OCH_2-$; $-CH_2CH_2O-$, $-CH(CH_3)CH_2-$ and $-CH_2CH(CH_3)-$; preferably wherein Q is $-CH_2CH_2-$.

8. The compound according to claim 1 which is a compound of formula (13a), (13b), (13c), (13d), (13e), (13f), (13g), (13h), (13i) or (13j):

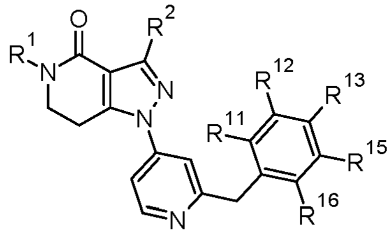 (13a); 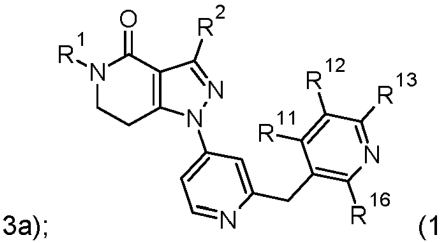 (13b);

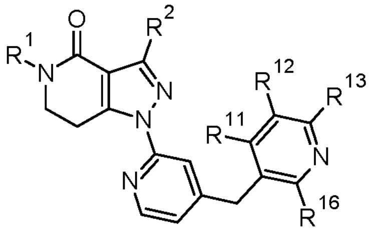 (13c); 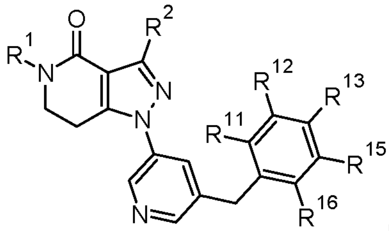 (13d);

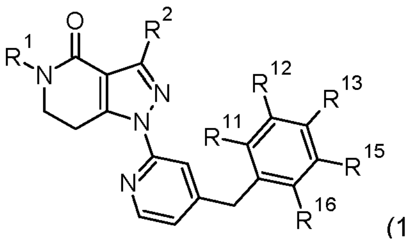 (13e); 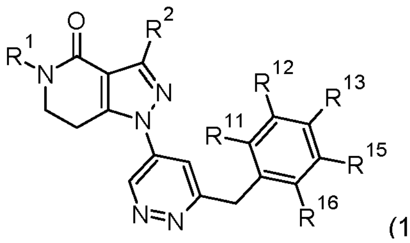 (13f);

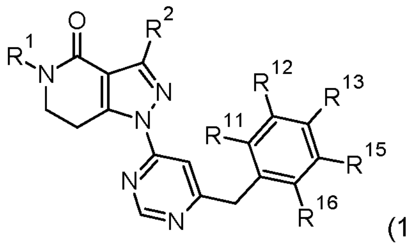 (13g); 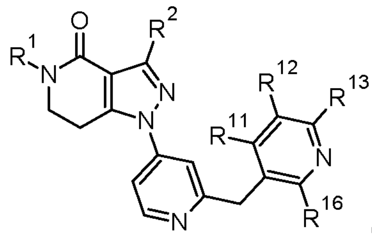 (13h);

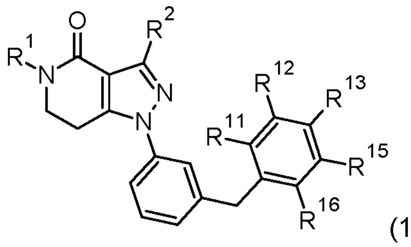 (13i); 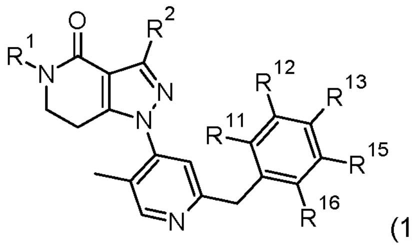 (13j);

or a salt thereof, wherein;

$R^1$ is H, $C_{1-6}$ alkyl optionally substituted with 1 to 6 fluorine atoms, $C_{3-6}$ cycloalkyl optionally substituted with 1 to 6 fluorine atoms, a group $-C(R^{14})_2C(R^{18})_2OR^{17}$ or a group $-C(R^{14})_2C(R^{19})_2OH$, wherein each $R^{14}$ is independently H, F or $C_{1-3}$ alkyl optionally substituted with 1 to 6 fluorine atoms, $R^{17}$ is $C_{1-3}$ alkyl optionally substituted with 1 to 6 fluorine atoms, each $R^{18}$ is independently H, F or $C_{1-3}$ alkyl optionally substituted with 1 to 6 fluorine atoms and each $R^{19}$ is independently H or $C_{1-3}$ alkyl optionally substituted with 1 to 6 fluorine atoms, and wherein $R^{17}$ and one $R^{14}$ may be joined to form an oxolane or oxetane ring;

$R^2$ is H, halo, CN, $C_{1-6}$ alkyl optionally substituted with OH or 1 to 6 fluorine atoms, $C_{1-6}$ alkoxy optionally substituted with OH or 1 to 6 fluorine atoms or $C_{3-6}$ cycloalkyl optionally substituted with OH or 1 to 6 fluorine atoms, wherein one atom of the $C_{1-6}$ alkyl, $C_{1-6}$ alkoxy or $C_{3-6}$ cycloalkyl group may be optionally replaced by a heteroatom selected from O, N, S and oxidised forms thereof;

$R^{11}$, $R^{12}$, $R^{13}$, $R^{15}$ and $R^{16}$ are independently selected from H, CN, $SF_5$, halo, a $C_{1-6}$ saturated hydrocarbon group

optionally substituted with 1 to 6 fluorine atoms wherein one atom of the $C_{1-6}$ saturated hydrocarbon group is optionally replaced by a heteroatom selected from O, N, S and oxidised forms thereof, $OC_{1-6}$ alkyl optionally substituted with 1 to 6 fluorine atoms, $SO_2C_{1-6}$ alkyl optionally substituted with 1 to 6 fluorine atoms and $C_{3-6}$ cycloalkyl optionally substituted with 1 to 6 fluorine atoms or optionally substituted with $CF_3$.

**9.** The compound according to claim 4 which is a compound of formula (6a):

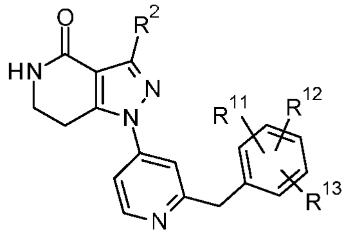

(6a)

or a salt thereof.

**10.** The compound according to any one of claims 1 to 8, wherein $R^1$ is H, methyl, - $CH_2CH_2OCH_3$ or $-CH_2CH_2OH$.

**11.** The compound according to any one of claims 1 to 10 wherein $R^2$ is selected from H, methyl, F, Cl, CN, $CF_3$ and $CH_2OH$; preferably wherein $R^2$ is H or methyl.

**12.** The compound according to any one of claims 4 to 11, wherein $R^{11}$, $R^{12}$, $R^{13}$, $R^{15}$ and $R^{16}$ are independently selected from H, CN, F, Cl, methyl, isopropyl, cyclopropyl, $CF_3$, $CF_2H$, $OCF_2H$, OMe and

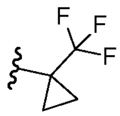

;

preferably wherein $R^{11}$, $R^{12}$, $R^{13}$, $R^{15}$ and $R^{16}$ are independently selected from H, F and $CF_3$.

**13.** The compound according to claim 1 which is selected from the group consisting of:

1-(2-(3-fluoro-5-(trifluoromethyl)benzyl)pyridin-4-yl)-1,5,6,7-tetrahydro-4*H*-pyrazolo[4,3-*c*]pyridin-4-one;
1-(4-(3-(trifluoromethyl)benzyl)pyridin-2-yl)-1,5,6,7-tetrahydro-4*H*-pyrazolo[4,3-*c*]pyridin-4-one;
5-methyl-1-(4-(3-(trifluoromethyl)benzyl)pyridin-2-yl)-1,5,6,7-tetrahydro-4*H*-pyrazolo[4,3-*c*]pyridin-4-one;
1-(4-(3-fluoro-5-(trifluoromethyl)benzyl)pyridin-2-yl)-1,5,6,7-tetrahydro-4*H*-pyrazolo[4,3-*c*]pyridin-4-one;
1-(4-(3-fluoro-5-(trifluoromethyl)benzyl)pyridin-2-yl)-5-methyl-1,5,6,7-tetrahydro-4*H*-pyrazolo[4,3-*c*]pyridin-4-one;
1-(4-((6-fluoro-5-methylpyridin-3-yl)methyl)pyridin-2-yl)-1,5,6,7-tetrahydro-4*H*-pyrazolo[4,3-*c*]pyridin-4-one;
1-(4-((6-fluoro-5-methylpyridin-3-yl)methyl)pyridin-2-yl)-5-methyl-1,5,6,7-tetrahydro-4*H*-pyrazolo[4,3-*c*]pyridin-4-one;
2-(4-(3-(trifluoromethyl)benzyl)pyridin-2-yl)-2,5,6,7-tetrahydro-4*H*-pyrazolo[4,3-*c*]pyridin-4-one;
2-(2-(3-fluoro-5-(trifluoromethyl)benzyl)pyridin-4-yl)-2,5,6,7-tetrahydro-4*H*-pyrazolo[4,3-*c*]pyridin-4-one;
1-(4-(3-fluoro-5-(trifluoromethyl)benzyl)pyridin-2-yl)-5,6,7,8-tetrahydropyrazolo[4,3-c]azepin-4(1*H*)-one;
1-(4-(3-(trifluoromethyl)benzyl)pyridin-2-yl)-5,6,7,8-tetrahydropyrazolo[4,3-*c*]azepin-4(1*H*)-one;
1-(2-(3-fluoro-5-(trifluoromethyl)benzyl)pyridin-4-yl)-5,6,7,8-tetrahydropyrazolo[4,3-c]azepin-4(1*H*)-one;
1-(4-(3-(trifluoromethyl)benzyl)pyridin-2-yl)-6,7-dihydro-1*H*-pyrazolo[4,3-*f*][1,4]oxazepin-4(5*H*)-one;
1-(4-(3-fluoro-5-(trifluoromethyl)benzyl)pyridin-2-yl)-6,7-dihydro-1*H*-pyrazolo[4,3-*f*][1,4]oxazepin-4(5*H*)-one;
1-(5-(3-fluoro-5-(trifluoromethyl)benzyl)pyridazin-3-yl)-1,5,6,7-tetrahydro-4*H*-pyrazolo[4,3-*c*]pyridin-4-one;
1-(5-(3-fluoro-5-(trifluoromethyl)benzyl)pyridin-3-yl)-1,5,6,7-tetrahydro-4*H*-pyrazolo[4,3-*c*]pyridin-4-one;
1-(6-(3-fluoro-5-(trifluoromethyl)benzyl)pyridazin-4-yl)-1,5,6,7-tetrahydro-4*H*-pyrazolo[4,3-*c*]pyridin-4-one;
1-{4-[3-fluoro-5-(trifluoromethyl)phenoxy]pyridin-2-yl}-1,5,6,7-tetrahydro-4*H*-pyrazolo[4,3-*c*]pyridin-4-one;
1-(2-(4-chlorobenzyl)pyridin-4-yl)-1,5,6,7-tetrahydro-4*H*-pyrazolo[4,3-*c*]pyridin-4-one;
1-(2-(3-chloro-5-fluorobenzyl)pyridin-4-yl)-1,5,6,7-tetrahydro-4*H*-pyrazolo[4,3-*c*]pyridin-4-one;

1-(2-(3,5-difluorobenzyl)pyridin-4-yl)-1,5,6,7-tetrahydro-4*H*-pyrazolo[4,3-*c*]pyridin-4-one;
1-(2-(3-(trifluoromethyl)benzyl)pyridin-4-yl)-1,5,6,7-tetrahydro-4*H*-pyrazolo[4,3-*c*]pyridin-4-one;
1-(2-(4-fluoro-3-(trifluoromethyl)benzyl)pyridin-4-yl)-1,5,6,7-tetrahydro-4*H*-pyrazolo[4,3-*c*]pyridin-4-one;
1-(2-(3,4,5-trifluorobenzyl)pyridin-4-yl)-1,5,6,7-tetrahydro-4*H*-pyrazolo[4,3-*c*]pyridin-4-one;
1-(2-(3-chloro-4-fluorobenzyl)pyridin-4-yl)-1,5,6,7-tetrahydro-4*H*-pyrazolo[4,3-*c*]pyridin-4-one;
1-(2-(3-fluoro-5-methylbenzyl)pyridin-4-yl)-1,5,6,7-tetrahydro-4*H*-pyrazolo[4,3-*c*]pyridin-4-one;
1-(2-(3-chloro-5-(trifluoromethyl)benzyl)pyridin-4-yl)-1,5,6,7-tetrahydro-4*H*-pyrazolo[4,3-*c*]pyridin-4-one;
1-(2-(3-methyl-5-(trifluoromethyl)benzyl)pyridin-4-yl)-1,5,6,7-tetrahydro-4*H*-pyrazolo[4,3-*c*]pyridin-4-one;
1-(2-(2-fluoro-3-(trifluoromethyl)benzyl)pyridin-4-yl)-1,5,6,7-tetrahydro-4*H*-pyrazolo[4,3-*c*]pyridin-4-one;
1-(2-(2-fluoro-5-(trifluoromethyl)benzyl)pyridin-4-yl)-1,5,6,7-tetrahydro-4*H*-pyrazolo[4,3-*c*]pyridin-4-one;
1-(2-(3,5-dichlorobenzyl)pyridin-4-yl)-1,5,6,7-tetrahydro-4*H*-pyrazolo[4,3-*c*]pyridin-4-one;
1-(2-(4-(trifluoromethyl)benzyl)pyridin-4-yl)-1,5,6,7-tetrahydro-4*H*-pyrazolo[4,3-*c*]pyridin-4-one;
3-((4-(4-oxo-4,5,6,7-tetrahydro-1*H*-pyrazolo[4,3-*c*]pyridin-1-yl)pyridin-2-yl)methyl)-5-(trifluoromethyl)benzonitrile;
1-(2-(3,5-bis(trifluoromethyl)benzyl)pyridin-4-yl)-1,5,6,7-tetrahydro-4*H*-pyrazolo[4,3-*c*]pyridin-4-one;
1-(2-(3-fluoro-5-(trifluoromethyl)benzoyl)pyridin-4-yl)-1,5,6,7-tetrahydro-4*H*-pyrazolo[4,3-*c*]pyridin-4-one;
1-(2-((3-fluoro-5-(trifluoromethyl)phenyl)(hydroxy)methyl)pyridin-4-yl)-1,5,6,7-tetrahydro-4*H*-pyrazolo[4,3-*c*] pyridin-4-one;
1-(2-(fluoro(3-fluoro-5-(trifluoromethyl)phenyl)methyl)pyridin-4-yl)-1,5,6,7-tetrahydro-4*H*-pyrazolo[4,3-*c*]pyridin-4-one;
1-(2-(difluoro(3-fluoro-5-(trifluoromethyl)phenyl)methyl)pyridin-4-yl)-1,5,6,7-tetrahydro-4*H*-pyrazolo[4,3-*c*]pyridin-4-one;
1-(2-((3-fluoro-5-(trifluoromethyl)phenyl)methyl-$d_2$)pyridin-4-yl)-1,5,6,7-tetrahydro-4*H*-pyrazolo[4,3-]pyridin-4-one;
1-(2-(3-fluoro-5-(trifluoromethyl)benzyl)pyridin-4-yl)-6-methyl-1,5,6,7-tetrahydro-4*H*-pyrazolo[4,3-*c*]pyridin-4-one;
1-(4-(3-(trifluoromethyl)benzyl)pyridin-2-yl)-5,6,7,8-tetrahydro-[1,2,3]triazolo[4,5-c]azepin-4(1*H*)-one;
1-(4-(3-(trifluoromethyl)benzyl)pyridin-2-yl)-1,5,6,7-tetrahydro-4*H*-[1,2,3]triazolo[4,5-*c*]pyridin-4-one;
2-(4-(3-(trifluoromethyl)benzyl)pyridin-2-yl)-2,5,6,7-tetrahydro-4*H*-[1,2,3]triazolo[4,5-*c*]pyridin-4-one;
5-methyl-2-(4-(3-(trifluoromethyl)benzyl)pyridin-2-yl)-2,5,6,7-tetrahydro-4*H*-[1,2,3]triazolo[4,5-*c*]pyridin-4-one;
2-(4-(3-(trifluoromethyl)benzyl)pyridin-2-yl)-5,6,7,8-tetrahydro-[1,2,3]triazolo[4,5-c]azepin-4(2*H*)-one;
1-(4-(3-fluoro-5-(trifluoromethyl)benzyl)pyridin-2-yl)-5,6,7,8-tetrahydro-[1,2,3]triazolo[4,5-*c*]azepin-4(1*H*)-one;
1-(4-(3-fluoro-5-(trifluoromethyl)benzyl)pyridin-2-yl)-1,7-dihydropyrazolo[4,3-*d*][1,2]oxazin-4(5*H*)-one;
1-(2-(3-fluoro-5-(trifluoromethyl)benzyl)pyridin-4-yl)-5,6-dihydropyrrolo[3,4-*c*]pyrazol-4(1*H*)-one;
1-(2-((3-fluoro-5-(trifluoromethyl)phenyl)methyl-*d*)pyridin-4-yl)-1,5,6,7-tetrahydro-4*H*-pyrazolo[4,3-*c*]pyridin-4-one;
1-(2-(3-fluoro-5-(trifluoromethyl)benzyl)pyridin-4-yl)-7-methyl-1,5,6,7-tetrahydro-4*H*-pyrazolo[4,3-*c*]pyridin-4-one;
1-(2-(3-fluoro-5-(trifluoromethyl)benzyl)pyridin-4-yl)-1,5,6,7-tetrahydro-4*H*-pyrrolo[3,2-*c*]pyridin-4-one;
1-(2-(3-fluoro-5-(trifluoromethyl)phenoxy)pyridin-4-yl)-1,5,6,7-tetrahydro-4*H*-pyrazolo[4,3-*c*]pyridin-4-one;
1-(2-(3-cyclopropyl-5-fluorobenzyl)pyridin-4-yl)-1,5,6,7-tetrahydro-4*H*-pyrazolo[4,3-*c*]pyridin-4-one;
1-(2-(3-fluoro-4-(trifluoromethyl)benzyl)pyridin-4-yl)-1,5,6,7-tetrahydro-4*H*-pyrazolo[4,3-*c*]pyridin-4-one;
1-(2-(4-methoxy-3-(trifluoromethyl)benzyl)pyridin-4-yl)-1,5,6,7-tetrahydro-4*H*-pyrazolo[4,3-*c*]pyridin-4-one;
1-(6-(3-fluoro-5-(trifluoromethyl)benzyl)pyrimidin-4-yl)-1,5,6,7-tetrahydro-4*H*-pyrazolo[4,3-*c*]pyridin-4-one;
1-(2-(3-methoxy-5-(trifluoromethyl)benzyl)pyridin-4-yl)-1,5,6,7-tetrahydro-4*H*-pyrazolo[4,3-*c*]pyridin-4-one;
1-(2-(4-chloro-3,5-difluorobenzyl)pyridin-4-yl)-1,5,6,7-tetrahydro-4*H*-pyrazolo[4,3-*c*]pyridin-4-one;
1-(2-(benzo[b]thiophen-5-ylmethyl)pyridin-4-yl)-1,5,6,7-tetrahydro-4*H*-pyrazolo[4,3-*c*]pyridin-4-one;
1-(2-(3-methoxy-4-(trifluoromethyl)benzyl)pyridin-4-yl)-1,5,6,7-tetrahydro-4*H*-pyrazolo[4,3-*c*]pyridin-4-one;
1-(2-(3-fluoro-5-(pentafluoro-$\lambda_6$-sulfaneyl)benzyl)pyridin-4-yl)-1,5,6,7-tetrahydro-4*H*-pyrazolo[4,3-*c*]pyridin-4-one;
1-(2-(3-(difluoromethoxy)-5-fluorobenzyl)pyridin-4-yl)-1,5,6,7-tetrahydro-4*H*-pyrazolo[4,3-*c*]pyridin-4-one;
1-(2-(4-(difluoromethoxy)-3-fluorobenzyl)pyridin-4-yl)-1,5,6,7-tetrahydro-4*H*-pyrazolo[4,3-*c*]pyridin-4-one;
1-(2-(3,4-difluoro-5-(trifluoromethyl)benzyl)pyridin-4-yl)-1,5,6,7-tetrahydro-4*H*-pyrazolo[4,3-*c*]pyridin-4-one;
1-(2-(3-(difluoromethyl)-5-fluorobenzyl)pyridin-4-yl)-1,5,6,7-tetrahydro-4*H*-pyrazolo[4,3-*c*]pyridin-4-one;
1-(2-(3-chloro-4,5-difluorobenzyl)pyridin-4-yl)-1,5,6,7-tetrahydro-4*H*-pyrazolo[4,3-*c*]pyridin-4-one;
1-(2-(quinolin-3-ylmethyl)pyridin-4-yl)-1,5,6,7-tetrahydro-4*H*-pyrazolo[4,3-*c*]pyridin-4-one;
1-(2-(4-chloro-3-fluoro-5-(trifluoromethyl)benzyl)pyridin-4-yl)-1,5,6,7-tetrahydro-4*H*-pyrazolo[4,3-*c*]pyridin-4-one;

1-(2-(benzo[*b*]thiophen-2-ylmethyl)pyridin-4-yl)-1,5,6,7-tetrahydro-4*H*-pyrazolo[4,3-*c*]pyridin-4-one;
1-(2-(3,5-difluoro-4-(trifluoromethyl)benzyl)pyridin-4-yl)-1,5,6,7-tetrahydro-4*H*-pyrazolo[4,3-*c*]pyridin-4-one;
1-(2-(3-fluoro-5-isopropylbenzyl)pyridin-4-yl)-1,5,6,7-tetrahydro-4*H*-pyrazolo[4,3-*c*]pyridin-4-one;
1-(2-((5-fluoro-6-methoxypyridin-3-yl)methyl)pyridin-4-yl)-1,5,6,7-tetrahydro-4*H*-pyrazolo[4,3-*c*]pyridin-4-one;
1-(2-(3-chloro-4-fluoro-5-(trifluoromethyl)benzyl)pyridin-4-yl)-1,5,6,7-tetrahydro-4*H*-pyrazolo[4,3-*c*]pyridin-4-one;
1-(2-(3-fluoro-5-(1-(trifluoromethyl)cyclopropyl)benzyl)pyridin-4-yl)-1,5,6,7-tetrahydro-4*H*-pyrazolo[4,3-*c*]pyridin-4-one;
1-(2-(3-fluoro-5-(trifluoromethyl)benzyl)pyridin-4-yl)-3-methyl-1,5,6,7-tetrahydro-4*H*-pyrazolo[4,3-*c*]pyridin-4-one;
1-(2-((6-fluoro-5-methylpyridin-3-yl)methyl)pyridin-4-yl)-3-methyl-1,5,6,7-tetrahydro-4*H*-pyrazolo[4,3-*c*]pyridin-4-one;
3-methyl-1-(2-(3,4,5-trifluorobenzyl)pyridin-4-yl)-1,5,6,7-tetrahydro-4*H*-pyrazolo[4,3-*c*]pyridin-4-one;
3-methyl-1-(2-(3-(trifluoromethyl)benzyl)pyridin-4-yl)-1,5,6,7-tetrahydro-4*H*-pyrazolo[4,3-*c*]pyridin-4-one;
1-(2-(3-chloro-5-fluorobenzyl)pyridin-4-yl)-3-methyl-1,5,6,7-tetrahydro-4*H*-pyrazolo[4,3-*c*]pyridin-4-one;
1-(2-(3-chloro-4-fluorobenzyl)pyridin-4-yl)-3-methyl-1,5,6,7-tetrahydro-4*H*-pyrazolo[4,3-*c*]pyridin-4-one;
1-(2-(4-(difluoromethoxy)-3-fluorobenzyl)pyridin-4-yl)-3-methyl-1,5,6,7-tetrahydro-4*H*-pyrazolo[4,3-*c*]pyridin-4-one;
1-(5-(3-fluoro-5-(trifluoromethyl)benzyl)pyridin-3-yl)-3-methyl-1,5,6,7-tetrahydro-4*H*-pyrazolo[4,3-*c*]pyridin-4-one;
1-(4-((6-fluoro-5-methylpyridin-3-yl)methyl)pyridin-2-yl)-3-methyl-1,5,6,7-tetrahydro-4*H*-pyrazolo[4,3-*c*]pyridin-4-one;
1-(2-(3-chloro-4,5-difluorobenzyl)pyridin-4-yl)-3-methyl-1,5,6,7-tetrahydro-4*H*-pyrazolo[4,3-*c*]pyridin-4-one;
3-fluoro-5-((4-(3-methyl-4-oxo-4,5,6,7-tetrahydro-1*H*-pyrazolo[4,3-*c*]pyridin-1 - yl)pyridin-2-yl)methyl)benzonitrile;
1-(2-(3,4-difluoro-5-(trifluoromethyl)benzyl)pyridin-4-yl)-3-methyl-1,5,6,7-tetrahydro-4*H*-pyrazolo[4,3-*c*]pyridin-4-one;
1-(2-(4-chloro-3-fluoro-5-(trifluoromethyl)benzyl)pyridin-4-yl)-3-methyl-1,5,6,7-tetrahydro-4*H*-pyrazolo[4,3-*c*]pyridin-4-one;
1-(2-(3-chloro-4-fluoro-5-(trifluoromethyl)benzyl)pyridin-4-yl)-3-methyl-1,5,6,7-tetrahydro-4*H*-pyrazolo[4,3-*c*]pyridin-4-one;
1-(2-(3-(difluoromethoxy)-5-fluorobenzyl)pyridin-4-yl)-3-methyl-1,5,6,7-tetrahydro-4*H*-pyrazolo[4,3-*c*]pyridin-4-one;
1-(2-(4-chloro-3,5-difluorobenzyl)pyridin-4-yl)-3-methyl-1,5,6,7-tetrahydro-4*H*-pyrazolo[4,3-*c*]pyridin-4-one;
1-(2-(3-(difluoromethyl)-5-fluorobenzyl)pyridin-4-yl)-3-methyl-1,5,6,7-tetrahydro-4*H*-pyrazolo[4,3-*c*]pyridin-4-one;
1-(2-((6-methoxypyridin-3-yl)methyl)pyridin-4-yl)-3-methyl-1,5,6,7-tetrahydro-4*H*-pyrazolo[4,3-*c*]pyridin-4-one;
1-(2-((5-fluoro-6-methoxypyridin-3-yl)methyl)pyridin-4-yl)-3-methyl-1,5,6,7-tetrahydro-4*H*-pyrazolo[4,3-*c*]pyridin-4-one;
1-(2-((6-(difluoromethoxy)pyridin-3-yl)methyl)pyridin-4-yl)-3-methyl-1,5,6,7-tetrahydro-4*H*-pyrazolo[4,3-*c*]pyridin-4-one;
1-(4-(3-fluoro-5-(trifluoromethyl)benzyl)pyridin-2-yl)-3-methyl-1,5,6,7-tetrahydro-4*H*-pyrazolo[4,3-*c*]pyridin-4-one;
3-fluoro-5-((4-(4-oxo-4,5,6,7-tetrahydro-1*H*-pyrazolo[4,3-*c*]pyridin-1-yl)pyridin-2-yl)methyl)benzonitrile;
1-(2-(3-fluoro-5-(trifluoromethyl)benzyl)pyridin-4-yl)-5,6,7,8-tetrahydro-[1,2,3]triazolo[4,5-*c*]azepin-4(1*H*)-one;
2-(4-(3-fluoro-5-(trifluoromethyl)benzyl)pyridin-2-yl)-2,5,6,7-tetrahydro-4*H*-[1,2,3]triazolo[4,5-*c*]pyridin-4-one;
1-(4-(3-fluoro-5-(trifluoromethyl)benzyl)pyridin-2-yl)-1,5,6,7-tetrahydro-4*H*-[1,2,3]triazolo[4,5-*c*]pyridin-4-one;
1-(2-(3-fluoro-5-(trifluoromethyl)benzyl)pyridin-4-yl)-1,5,6,7-tetrahydro-4*H*-[1,2,3]triazolo[4,5-*c*]pyridin-4-one;
2-(2-(3-fluoro-5-(trifluoromethyl)benzyl)pyridin-4-yl)-2,5,6,7-tetrahydro-4*H*-[1,2,3]triazolo[4,5-*c*]pyridin-4-one;
1-(4-(3-(difluoromethyl)-5-fluorobenzyl)pyridin-2-yl)-1,5,6,7-tetrahydro-4*H*-pyrazolo[4,3-*c*]pyridin-4-one;
1-(5-(3-fluoro-5-(trifluoromethyl)phenoxy)pyridin-3-yl)-1,5,6,7-tetrahydro-4*H*-pyrazolo[4,3-*c*]pyridin-4-one;
1-(2-(3-fluoro-5-(trifluoromethyl)benzyl)pyridin-4-yl)-5-(2-methoxyethyl)-1,5,6,7-tetrahydro-4*H*-pyrazolo[4,3-*c*]pyridin-4-one;
1-(2-(3-fluoro-5-(trifluoromethyl)benzyl)pyridin-4-yl)-5-(2-hydroxyethyl)-1,5,6,7-tetrahydro-4*H*-pyrazolo[4,3-*c*]pyridin-4-one;
1-(4-(3-fluoro-5-(trifluoromethyl)benzyl)pyridin-2-yl)-5-(2-methoxyethyl)-1,5,6,7-tetrahydro-4*H*-pyrazolo[4,3-*c*]pyridin-4-one;
1-(4-(3-fluoro-5-(trifluoromethyl)benzyl)pyridin-2-yl)-5-(2-hydroxyethyl)-1,5,6,7-tetrahydro-4*H*-pyrazolo[4,3-*c*]

pyridin-4-one;
1-(2-(3-fluoro-5-(trifluoromethyl)benzyl)-5-methylpyridin-4-yl)-1,5,6,7-tetrahydro-4*H*-pyrazolo[4,3-*c*]pyridin-4-one;
1-(3-(3-fluoro-5-(trifluoromethyl)benzyl)phenyl)-1,5,6,7-tetrahydro-4*H*-pyrazolo[4,3-c]pyridin-4-one;
1-(2-((6-(difluoromethoxy)-5-fluoropyridin-3-yl)methyl)pyridin-4-yl)-1,5,6,7-tetrahydro-4*H*-pyrazolo[4,3-*c*]pyridin-4-one;
1-(2-((6-(difluoromethoxy)-5-fluoropyridin-3-yl)methyl)pyridin-4-yl)-3-methyl-1,5,6,7-tetrahydro-4*H*-pyrazolo[4,3-*c*]pyridin-4-one;
2-(2-(3-fluoro-5-(trifluoromethyl)benzyl)pyridin-4-yl)-6,7-dihydro-2*H*-pyrazolo[4,3-*f*][1,4]oxazepin-4(5*H*)-one;
1-(4-(3-fluoro-5-(trifluoromethyl)benzyl)pyridin-2-yl)-3-(hydroxymethyl)-1,5,6,7-tetrahydro-4*H*-pyrazolo[4,3-*c*]pyridin-4-one;
1-(2-(3-fluoro-5-(trifluoromethyl)benzyl)pyridin-4-yl)-3-(hydroxymethyl)-1,5,6,7-tetrahydro-4*H*-pyrazolo[4,3-*c*]pyridin-4-one;
1-(2-(3-fluoro-5-(trifluoromethyl)benzyl)pyridin-4-yl)-4-oxo-4,5,6,7-tetrahydro-1*H*-pyrazolo[4,3-*c*]pyridine-3-carbonitrile;
1-(2-(3-fluoro-5-(trifluoromethyl)benzyl)pyridin-4-yl)-3-(trifluoromethyl)-1,5,6,7-tetrahydro-4*H*-pyrazolo[4,3-*c*]pyridin-4-one;
1-(2-((3-fluoro-5-(trifluoromethyl)phenyl)(hydroxy)methyl)pyridin-4-yl)-3-methyl-1,5,6,7-tetrahydro-4*H*-pyrazolo[4,3-*c*]pyridin-4-one;
1-(4-((3-fluoro-5-(trifluoromethyl)phenyl)(hydroxy)methyl)pyridin-2-yl)-3-methyl-1,5,6,7-tetrahydro-4*H*-pyrazolo[4,3-*c*]pyridin-4-one;
or a salt thereof.

**14.** The compound according to claim 1 wherein said compound is:

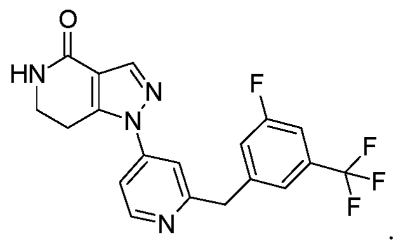

.

**15.** The compound according to claim 1 wherein said compound is:

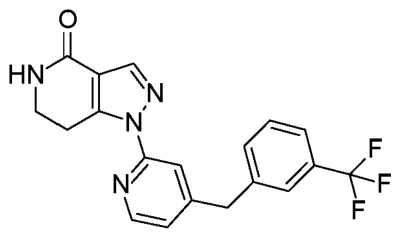

.

**16.** The compound according to claim 1 wherein said compound is:

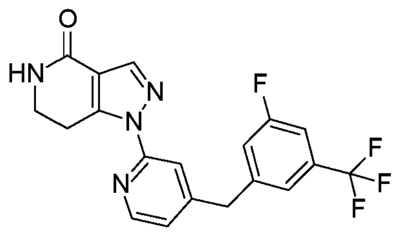

.

**17.** The compound according to claim 1 wherein said compound is:

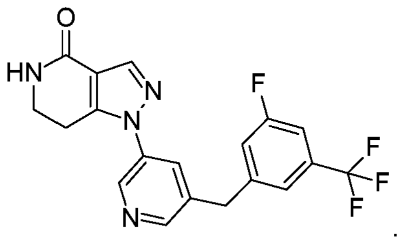

18. The compound according to claim 1 wherein said compound is:

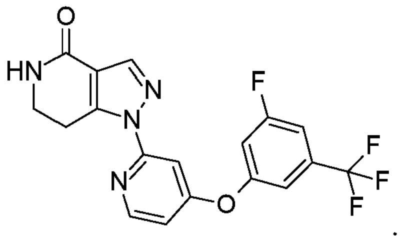

19. The compound according to claim 1 wherein said compound is:

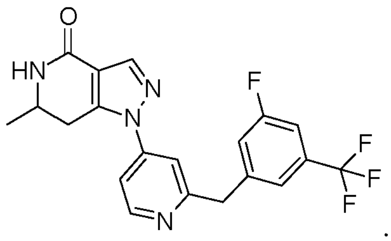

20. The compound according to claim 1 wherein said compound is:

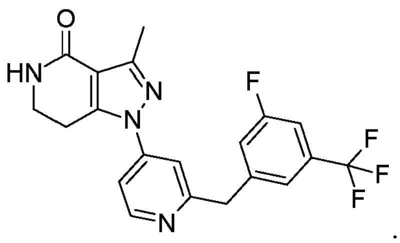

21. The compound according to claim 1 wherein said compound is:

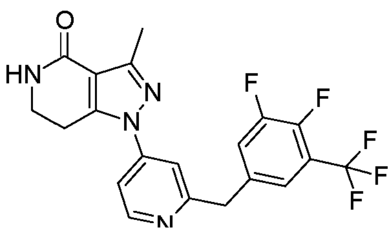

22. The compound according to claim 1 wherein said compound is:

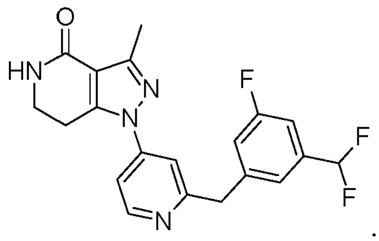

23. The compound according to claim 1 wherein said compound is:

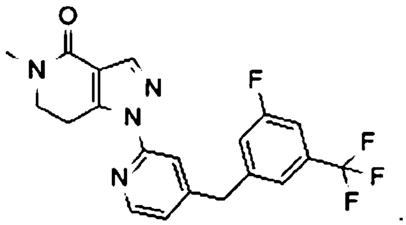

24. The compound according to claim 1 wherein said compound is:

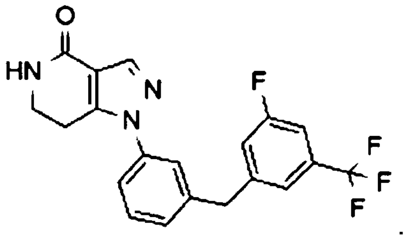

25. The compound according to claim 1 wherein said compound is:

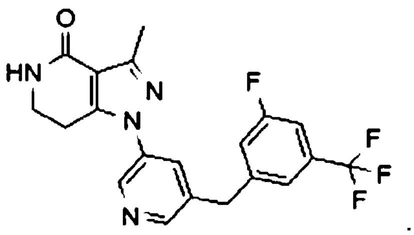

26. The compound according to claim 1 wherein said compound is:

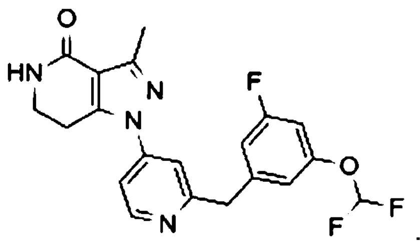

27. A salt of a compound as defined in any one of claims 14 to 26.

28. A pharmaceutical composition comprising a compound or salt as defined in any one of claims 1 to 27 and a pharmaceutically acceptable excipient.

29. The compound or salt according to any one of claims 1 to 27 or composition according to claim 28 for use in medicine.

30. The compound according to any one of claims 1 to 27 or composition according to claim 28 for use in the treatment of psychiatric disorders; neuropsychiatric disorders; neurodegenerative disorders; psychotic disorders; cognitive disorders; neurocognitive disorders; extrapyramidal disorders; movement disorders; motor disorders; hyperkinetic movement disorders; catatonia; mood disorders; depressive disorders; anxiety disorders; obsessive-compulsive disorder (OCD); autism spectrum disorders; depressive disorders; hypothalamic disorders; pituitary disorders; prolactin-related disorders; trauma- or stressor-related disorders; disruptive, impulse-control or conduct disorders; sleep-wake disorders; substance-related disorders; addictive disorders; behavioral disorders; hypofrontality; abnormalities in the tuberoinfundibular, mesolimbic, mesocortical, or nigrostriatal pathway; decreased activity in the striatum; cortical dysfunction; neurocognitive dysfunction or conditions or symptoms related thereto.

31. The compound or composition for use according to claim 30, wherein the disorder or symptom is selected from schizophrenia, positive symptoms of schizophrenia, negative symptoms of schizophrenia, cognitive symptoms of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, anhedonia, agitation, Alzheimer's disease, Parkinson's disease, Huntington's disease, vascular dementia, Lewy body disease, frontotemporal dementia, Tourette's syndrome, hyperprolactinemia, pituitary adenoma, prolactinoma, craniopharyngioma, Cushing's disease, diabetes insipidus, non-functioning tumours, obesity, posttraumatic stress disorder (PTSD), akathisia and associated movements, athetosis, ataxia, ballismus, hemiballismus, chorea, choreoathetosis, dyskinesia, tardive dyskinesia, neuroleptic-induced dyskinesia, myoclonus, mirror movement disorder, paroxysmal kinesigenic dyskinesia, restless legs syndrome, spasms, stereotypic movement disorder, sterotypy, Tic disorder, tremor, Wilson's disease, schizotypal personality disorder, delusional disorder, brief psychotic disorder, schizophreniform disorder, schizoaffective disorder, substance- or medication-induced psychotic disorder, delusions, hallucinations, disorganized thinking, grossly disorganized or abnormal motor behavior, catatonia, major depressive disorder, bipolar I disorder, bipolar II disorder, cyclothymic disorder, substance- or medication-induced bipolar and related disorders, bipolar and related disorders due to another medical condition, separation anxiety disorder, selective mutism, specific phobia, social anxiety disorder, panic disorder, agoraphobia, generalized anxiety disorder, substance- or medication-induced anxiety disorder, anxiety disorders due to another medical condition, delirium, major neurocognitive disorder, minor neurocognitive disorder, amnesia, dementia, developmental coordination disorder, stereotypic movement disorder, a post-stroke effect, dentatorubral-pallidoluysian atrophy, diminished emotional expression, avolition, alogia and asociality;
preferably wherein the disorder or symptom is selected from schizophrenia, positive symptoms of schizophrenia, negative symptoms of schizophrenia, cognitive symptoms of schizophrenia, depression, attention-deficit hyperactivity disorder (ADHD), generalised anxiety disorder, obsessive-compulsive disorder (OCD), panic disorder, bipolar disorder, addiction/impulse-control disorders, autism spectrum disorders, psychosis, neurocognitive disorder, delirium, anhedonia, agitation, Alzheimer's disease, Parkinson's disease, Huntington's disease, vascular dementia, Lewy body disease, frontotemporal dementia, Tourette's syndrome, hyperprolactinemia, obesity, and posttraumatic stress disorder (PTSD).

## Patentansprüche

1. Verbindung der Formel (1):

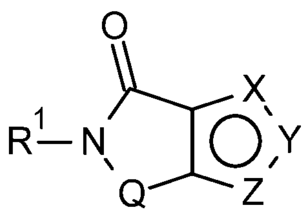

(1)

oder ein Salz davon, wobei;
X N oder $CR^2$ ist;
Y N, $NR^3$ oder $CR^2$ ist;
Z N oder $NR^3$ ist;
wobei eines, aber nicht beide von Y und Z $NR^3$ ist;
Q ausgewählt ist aus: $-CR^4R^5-$, $-CR^4R^5CR^6R^7-$, $-CR^4R^5CR^6R^7CR^8R^9-$, $-CR^4R^5CR^6R^7O-$, $-OCR^4R^5-$ und

$-OCR^4R^5CR^6R^7-$;
$R^1$ ist: H, $C_{1-6}$ -Alkyl, optional substituiert mit 1 bis 6 Fluoratomen, $C_{3-6}$-Cycloalkyl, optional substituiert mit 1 bis 6 Fluoratomen, eine Gruppe $-C(R^{14})_2C(R^{18})_2OR^{17}$ oder eine Gruppe - $C(R^{14})_2C(R^{19})_2OH$ ist, wobei jedes $R^{14}$ unabhängig voneinander H, F oder $C_{1-3}$-Alkyl ist, das optional mit 1 bis 6 Fluoratomen substituiert ist, $R^{17}$ $C_{1-3}$-Alkyl ist, das optional mit 1 bis 6 Fluoratomen substituiert ist, jedes $R^{18}$ unabhängig voneinander H, F oder $C_{1-3}$-Alkyl ist, das optional mit 1 bis 6 Fluoratomen substituiert ist, und jedes $R^{19}$ ist unabhängig voneinander H oder $C_{1-3}$-Alkyl, das optional mit 1 bis 6 Fluoratomen substituiert ist, und wobei $R^{17}$ und ein $R^{14}$ miteinander verbunden sein können, um einen Oxolan- oder Oxetanring zu bilden; $R^2$ ist: H, Halogen, CN, $C_{1-6}$-Alkyl, optional substituiert mit OH oder 1 bis 6 Fluoratomen, $C_{1-6}$-Alkoxy, optional substituiert mit OH oder 1 bis 6 Fluoratomen, oder $C_{3-6}$-Cycloalkyl, optional substituiert mit OH oder 1 bis 6 Fluoratomen, wobei ein Atom der $C_{1-6}$-Alkyl-, $C_{1-6}$-Alkoxy- oder $C_{3-6}$-Cycloalkylgruppe optional durch ein Heteroatom ersetzt sein kann, das aus O, N, S und oxidierten Formen davon ausgewählt ist;
$R^4$, $R^5$, $R^6$, $R^7$, $R^8$ und $R^9$ unabhängig voneinander aus H und $C_{1-3}$-Alkyl ausgewählt sind; wobei $R^3$ eine Gruppe der folgenden Formel ist:

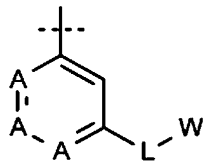

wobei jedes A unabhängig voneinander N oder $CR^{10}$ ist;
L aus $CH_2$, CHD, $CD_2$, CHF, $CF_2$, C=O, CHOH, O und NH ausgewählt ist;
$R^{10}$ aus H, Halogen und $C_{1-3}$-Alkyl, das optional mit 1 bis 6 Fluoratomen substituiert ist, ausgewählt ist;
und W ist entweder:

(i) ein 6-gliedriger, optional substituierter Arylring oder ein 6-gliedriger, optional substituierter Heteroarylring; oder
(ii) ein 9- bis 10-gliedriges, optional substituiertes heterobizyklisches Ringsystem, wobei einer oder mehrere der Ringe aromatisch sind.

2. Verbindung nach Anspruch 1, wobei W ein 6-gliedriger, optional substituierter Aryl- oder 6-gliedriger, optional substituierter Heteroarylring ist.

3. Verbindung nach Anspruch 1, die Folgendes ist

(i) eine Verbindung der Formel (2a), (2b), (2c), (2d), (2e), (2f) oder (2g):

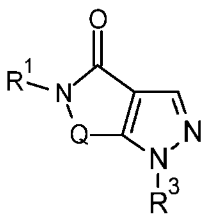 (2a); 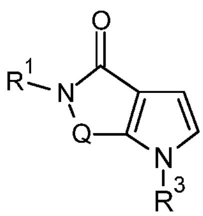 (2b); 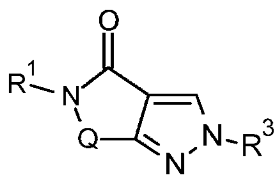 (2c);

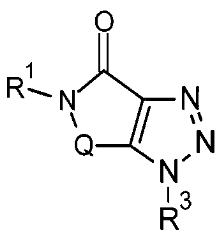 (2d); 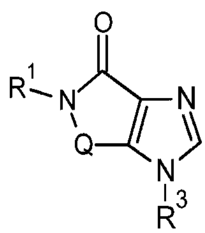 (2e); 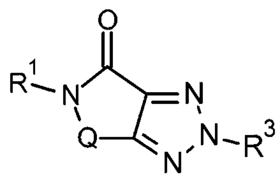 (2f);

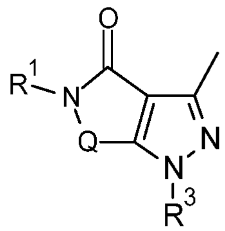

(2g);

oder ein Salz davon; oder

(ii) eine Verbindung der Formel (11a):

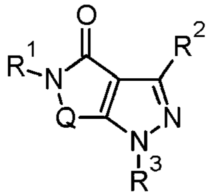

(11a)

oder ein Salz davon.

**4.** Verbindung nach einem der Ansprüche 1 bis 3, wobei

(i) $R^3$ eine Gruppe der folgenden Formel ist:

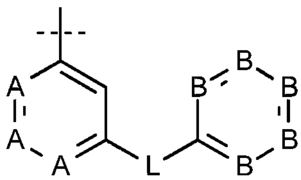

wobei jedes A unabhängig voneinander N oder $CR^{10}$ ist;
L aus $CH_2$, CHD, $CD_2$, CHF, $CF_2$, C=O, CHOH, O und NH ausgewählt ist; jedes B unabhängig voneinander N, $CR^{11}$, $CR^{12}$, $CR^{13}$, $CR^{15}$ oder $CR^{16}$ ist;
$R^{10}$ aus H, Halogen und $C_{1-3}$-Alkyl, das optional mit 1 bis 6 Fluoratomen substituiert ist, ausgewählt ist;
$R^{11}$, $R^{12}$, $R^{13}$, $R^{15}$ und $R^{16}$ unabhängig voneinander ausgewählt sind aus: H, CN, $SF_5$, Halogen, einer gesättigten $C_{1-6}$ -Kohlenwasserstoffgruppe, die optional mit 1 bis 6 Fluoratomen substituiert ist, wobei ein Atom der gesättigten $C_{1-6}$-Kohlenwasserstoffgruppe optional durch ein Heteroatom ersetzt ist, das ausgewählt ist aus: O, N, S und oxidierten Formen davon, $OC_{1-6}$-Alkyl, optional substituiert mit 1 bis 6 Fluoratomen, $SO_2C_{1-6}$-Alkyl, optional substituiert mit 1 bis 6 Fluoratomen, und $C_{3-6}$-Cycloalkyl, optional substituiert mit 1 bis 6 Fluoratomen oder optional substituiert mit $CF_3$; vorzugsweise wobei

(ii) $R^3$ ausgewählt ist aus der Gruppe bestehend aus:

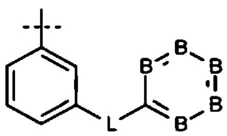 ; 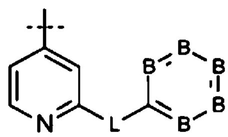 ; 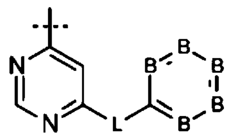 ;

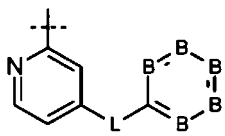 ; 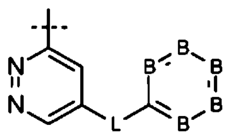 ; 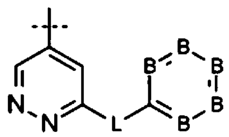 ;

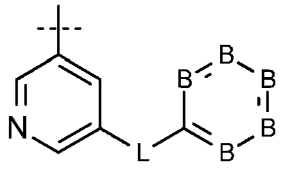 ; 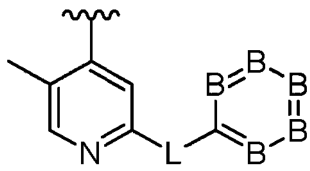 .

5. Verbindung nach einem der Ansprüche 1 bis 4, wobei L $CH_2$ ist.

6. Verbindung nach Anspruch 4 oder 5, wobei die Gruppe:

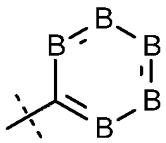

ausgewählt ist aus der Gruppe bestehend aus:

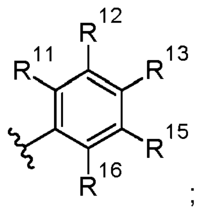 ; 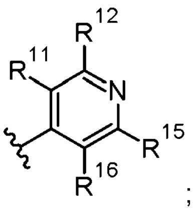 ; 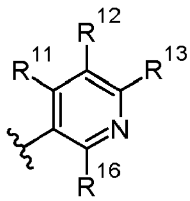 .

7. Verbindung nach einem der Ansprüche 1 bis 6, wobei Q aus der Gruppe ausgewählt ist, die aus $-CH_2CH_2-$; $-CH_2CH_2CH_2-$; $-CH_2-$; $-OCH_2-$; $-CH_2CH_2O-$, $-CH(CH_3)CH_2-$ und - $CH_2CH(CH_3)$-besteht; wobei Q vorzugsweise $-CH_2CH_2-$ ist.

8. Verbindung nach Anspruch 1, die eine Verbindung der Formel (13a), (13b), (13c), (13d), (13e), (13f), (13g), (13H), (13i) oder (13j) ist:

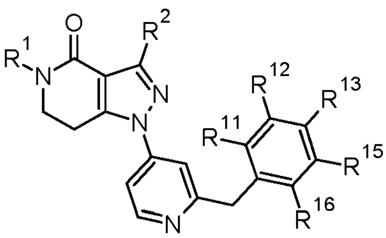 (13a);

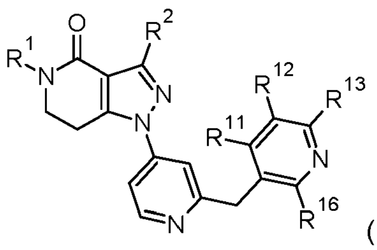 (13b);

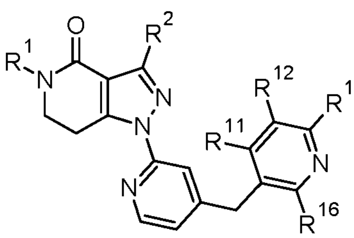 (13c);

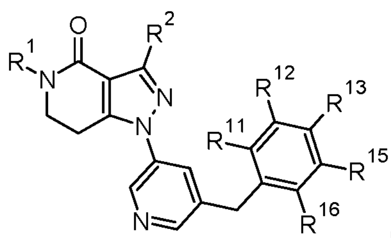 (13d);

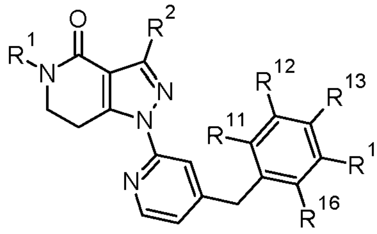
(13e);

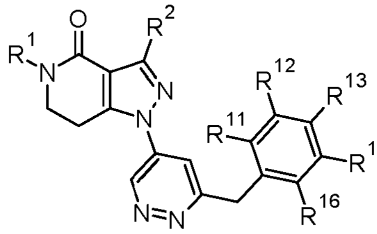
(13f);

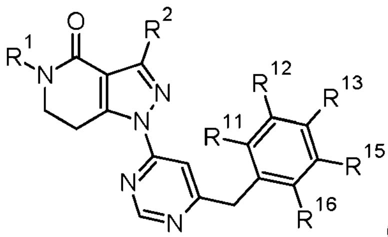
(13g);

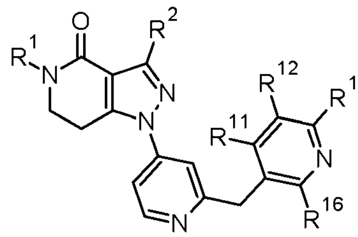
(13h);

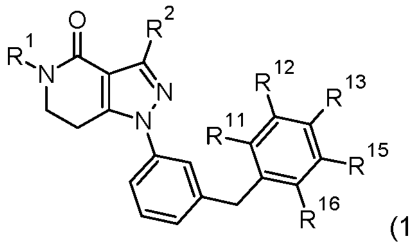
(13i);

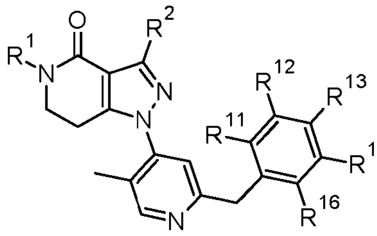
(13j);

oder ein Salz davon, wobei;

$R^1$ ist: H, $C_{1-6}$-Alkyl, optional substituiert mit 1 bis 6 Fluoratomen, $C_{3-6}$-Cycloalkyl, optional substituiert mit 1 bis 6 Fluoratomen, eine Gruppe -$C(R^{14})_2C(R^{18})_2OR^{17}$ oder eine Gruppe - $C(R^{14})_2C(R^{19})_2OH$, wobei jedes $R^{14}$ unabhängig voneinander H, F oder $C_{1-3}$-Alkyl ist, das optional mit 1 bis 6 Fluoratomen substituiert ist, $R^{17}$ $C_{1-3}$-Alkyl ist, das optional mit 1 bis 6 Fluoratomen substituiert ist, jedes $R^{18}$ unabhängig voneinander H, F oder $C_{1-3}$-Alkyl ist, das optional mit 1 bis 6 Fluoratomen substituiert ist, und jedes $R^{19}$ unabhängig voneinander H oder $C_{1-3}$-Alkyl ist, das optional mit 1 bis 6 Fluoratomen substituiert ist, und wobei $R^{17}$ und ein $R^{14}$ miteinander verbunden sein können, um einen Oxolan- oder Oxetanring zu bilden; $R^2$ ist: H, Halogen, CN, $C_{1-6}$-Alkyl, optional substituiert mit OH oder 1 bis 6 Fluoratomen, $C_{1-6}$-Alkoxy, optional substituiert mit OH oder 1 bis 6 Fluoratomen, oder $C_{3-6}$-Cycloalkyl, optional substituiert mit OH oder 1 bis 6 Fluoratomen, wobei ein Atom der $C_{1-6}$-Alkyl-, $C_{1-6}$-Alkoxy- oder $C_{3-6}$-Cycloalkylgruppe optional durch ein Heteroatom ersetzt sein kann, das aus O, N, S und oxidierten Formen davon ausgewählt ist;

$R^{11}$, $R^{12}$, $R^{13}$, $R^{15}$ und $R^{18}$ unabhängig voneinander ausgewählt sind aus: H, CN, $SF_5$, Halogen, einer gesättigten $C_{1-6}$-Kohlenwasserstoffgruppe, die optional mit 1 bis 6 Fluoratomen substituiert ist, wobei ein Atom der gesättigten $C_{1-6}$-Kohlenwasserstoffgruppe optional durch ein Heteroatom ersetzt ist, das ausgewählt ist aus: O, N, S und oxidierten Formen davon, $OC_{1-6}$-Alkyl, optional substituiert mit 1 bis 6 Fluoratomen, $SO_2C_{1-6}$-Alkyl, optional substituiert mit 1 bis 6 Fluoratomen, und $C_{3-6}$-Cycloalkyl, optional substituiert mit 1 bis 6 Fluoratomen oder optional substituiert mit $CF_3$.

**9.** Verbindung nach Anspruch 4, die eine Verbindung der Formel (6a) ist:

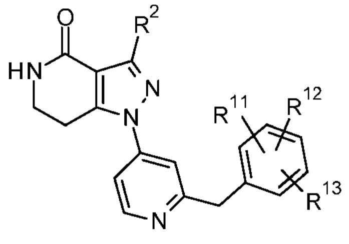
(6a)

oder ein Salz davon.

**10.** Verbindung nach einem der Ansprüche 1 bis 8, wobei $R^1$ H, Methyl, $-CH_2CH_2OCH_3$ oder $-CH_2CH_2OH$ ist.

**11.** Verbindung nach einem der Ansprüche 1 bis 10, wobei $R^2$ aus H, Methyl, F, Cl, CN, $CF_3$ und $CH_2OH$ ausgewählt ist; vorzugsweise wobei $R^2$ H oder Methyl ist.

**12.** Verbindung nach einem der Ansprüche 4 bis 11, wobei $R^{11}$, $R^{12}$, $R^{13}$, $R^{15}$ und $R^{16}$ unabhängig voneinander ausgewählt sind aus: H, CN, F, Cl, Methyl, Isopropyl, Cyclopropyl, $CF_3$, $CF_2H$, $OCF_2H$, OMe und

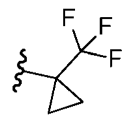

;

vorzugsweise wobei $R^{11}$, $R^{12}$, $R^{13}$, $R^{15}$ und $R^{16}$ unabhängig voneinander aus H, F und $CF_3$ ausgewählt sind.

**13.** Verbindung nach Anspruch 1, die ausgewählt ist aus der Gruppe bestehend aus:

1-(2-(3-Fluor-5-(trifluormethyl)benzyl)pyridin-4-yl)-1,5,6,7-tetrahydro-4*H*-pyrazolo[4,3-c]pyridin-4-on;
1-(4-(3-(Trifluormethyl)benzyl)pyridin-2-yl)-1,5,6,7-tetrahydro-4*H*-pyrazolo[4,3-c]pyridin-4-on;
5-Methyl-1-(4-(3-(trifluormethyl)benzyl)pyridin-2-yl)-1,5,6,7-tetrahydro-*4H*-pyrazolo[4,3-c]pyridin-4-on;
1-(4-(3-Fluor-5-(trifluormethyl)benzyl)pyridin-2-yl)-1,5,6,7-tetrahydro-*4H*-pyrazolo[4,3-c]pyridin-4-on;
1-(4-(3-Fluor-5-(trifluormethyl)benzyl)pyridin-2-yl)-5-methyl-1,5,6,7-tetrahydro-*4H*-pyrazolo[4,3-c]pyridin-4-on;
1-(4-((6-Fluor-5-methylpyridin-3-yl)methyl)pyridin-2-yl)-1,5,6,7-tetrahydro-*4H*-pyrazolo[4,3-c]pyridin-4-on;
1-(4-((6-Fluor-5-methylpyridin-3-yl)methyl)pyridin-2-yl)-5-methyl-1,5,6,7-tetrahydro-*4H*-pyrazolo[4,3-c]pyridin-4-on;
2-(4-(3-(Trifluormethyl)benzyl)pyridin-2-yl)-2,5,6,7-tetrahydro-4*H*-pyrazolo[4,3-*c*]pyridin-4-on;
2-(2-(3-Fluor-5-(trifluormethyl)benzyl)pyridin-4-yl)-2,5,6,7-tetrahydro-4*H*-pyrazolo[4,3-c]pyridin-4-on;
1-(4-(3-Fluor-5-(trifluormethyl)benzyl)pyridin-2-yl)-5,6,7,8-tetrahydropyrazolo[4,3-*c*]azepin-4(*1H*)-on;
1-(4-(3-(Trifluormethyl)benzyl)pyridin-2-yl)-5,6,7,8-tetrahydropyrazolo[4,3-c]azepin-4(*1H*)-on;
1-(2-(3-Fluor-5-(trifluormethyl)benzyl)pyridin-4-yl)-5,6,7,8-tetrahydropyrazolo[4,3-*c*]azepin-4(*1H*)-on;
1-(4-(3-(Trifluormethyl)benzyl)pyridin-2-yl)-6,7-dihydro-1*H*-pyrazolo[4,3-*f*][1,4]oxazepin-4(5*H*)-on;
1-(4-(3-Fluor-5-(trifluormethyl)benzyl)pyridin-2-yl)-6,7-dihydro-1*H*-pyrazolo[4,3-*f*][1,4]oxazepin-4(5*H*)-on;
1-(5-(3-Fluor-5-(trifluormethyl)benzyl)pyridazin-3-yl)-1,5,6,7-tetrahydro-*4H*-pyrazolo[4,3-c]pyridin-4-on;
1-(5-(3-Fluor-5-(trifluormethyl)benzyl)pyridin-3-yl)-1,5,6,7-tetrahydro-*4H*-pyrazolo[4,3-c]pyridin-4-on;
1-(6-(3-Fluor-5-(trifluormethyl)benzyl)pyridazin-4-yl)-1,5,6,7-tetrahydro-*4H*-pyrazolo[4,3-c]pyridin-4-on;
1-{4-[3-fluoro-5-(trifluoromethyl)phenoxy]pyridin-2-yl}-1,5,6,7-tetrahydro-4H-pyrazolo[4,3-c]pyridin-4-on;
1-(2-(4-Chlorbenzyl)pyridin-4-yl)-1,5,6,7-tetrahydro-4*H*-pyrazolo[4,3-c]pyridin-4-on;
1-(2-(3-Chlor-5-fluorbenzyl)pyridin-4-yl)-1,5,6,7-tetrahydro-4*H*-pyrazolo[4,3-c]pyridin-4-on;
1-(2-(3,5-Difluorbenzyl)pyridin-4-yl)-1,5,6,7-tetrahydro-*4H*-pyrazolo[4,3-c]pyridin-4- on;
1-(2-(3-(Trifluormethyl)benzyl)pyridin-4-yl)-1,5,6,7-tetrahydro-4*H*-pyrazolo[4,3-*c*]pyridin-4-on;
1-(2-(4-Fluor-3-(trifluormethyl)benzyl)pyridin-4-yl)-1,5,6,7-tetrahydro-*4H*-pyrazolo[4,3-c]pyridin-4-on;
1-(2-(3,4,5-Trifluorbenzyl)pyridin-4-yl)-1,5,6,7-tetrahydro-*4H*-pyrazolo[4,3-c]pyridin-4- on;
1-(2-(3-Chlor-4-fluorbenzyl)pyridin-4-yl)-1,5,6,7-tetrahydro-4*H*-pyrazolo[4,3-c]pyridin-4-on;
1 -(2-(3-Fluor-5-methylbenzyl)pyridin-4-yl)-1,5,6,7-tetrahydro-*4H*-pyrazolo[4,3-*c*]pyridin-4-on;
1-(2-(3-Chlor-5-(trifluormethyl)benzyl)pyridin-4-yl)-1,5,6,7-tetrahydro-*4H*-pyrazolo[4,3-c]pyridin-4-on;
1-(2-(3-Methyl-5-(trifluormethyl)benzyl)pyridin-4-yl)-1,5,6,7-tetrahydro-*4H*-pyrazolo[4,3-c]pyridin-4-on;
1-(2-(2-Fluor-3-(trifluormethyl)benzyl)pyridin-4-yl)-1,5,6,7-tetrahydro-*4H*-pyrazolo[4,3-c]pyridin-4-on;
1-(2-(2-Fluor-5-(trifluormethyl)benzyl)pyridin-4-yl)-1,5,6,7-tetrahydro-*4H*-pyrazolo[4,3-c]pyridin-4-on;
1-(2-(3,5-Dichlorbenzyl)pyridin-4-yl)-1,5,6,7-tetrahydro-*4H*-pyrazolo[4,3-c]pyridin-4- on;
1-(2-(4-(Trifluormethyl)benzyl)pyridin-4-yl)-1,5,6,7-tetrahydro-*4H*-pyrazolo[*4,3-c*]pyridin-4-on;
3-((4-(4-Oxo-4,5,6,7-tetrahydro-1*H*-pyrazolo[4,3-c]pyridin-1-yl)pyridin-2-yl)methyl)-5-(trifluoromethyl)benzonitril;
1-(2-(3,5-Bis(trifluormethyl)benzyl)pyridin-4-yl)-1,5,6,7-tetrahydro-*4H*-pyrazolo[4,3-*c*]pyridin-4-on;
1-(2-(3-Fluor-5-(trifluormethyl)benzoyl)pyridin-4-yl)-1,5,6,7-tetrahydro-*4H*-pyrazolo[4,3-c]pyridin-4-on;
1-(2-((3-Fluor-5-(trifluormethyl)phenyl)(hydroxy)methyl)pyridin-4-yl)-1,5,6,7-tetrahydro-*4H*-pyrazolo[4,3-c]pyridin-4-on;

1-(2-(Fluor(3-fluor-5-(trifluormethyl)phenyl)methyl)pyridin-4-yl)-1,5,6,7-tetrahydro-*4H*-pyrazolo[4,3-c]pyridin-4-on;
1-(2-(Difluor(3-fluor-5-(trifluormethyl)phenyl)methyl)pyridin-4-yl)-1,5,6,7-tetrahydro-*4H*-pyrazolo[4,3-c]pyridin-4-on;
1-(2-((3-Fluor-5-(trifluoromethyl)phenyl)methyl-$d_2$)pyridin-4-yl)-1,5,6,7-tetrahydro-*4H*-pyrazolo[4,3-]pyridin-4-on;
1-(2-(3-Fluor-5-(trifluormethyl)benzyl)pyridin-4-yl)-6-methyl-1,5,6,7-tetrahydro-*4H*-pyrazolo[4,3-c]pyridin-4-on;
1-(4-(3-(Trifluormethyl)benzyl)pyridin-2-yl)-5,6,7,8-tetrahydro-[1,2,3]triazolo[4,5-c]azepin-4*(1H)*-on;
1-(4-(3-(Trifluormethyl)benzyl)pyridin-2-yl)-1,5,6,7-tetrahydro-*4H*-[1,2,3]triazolo[4,5-c]pyridin-4-on;
2-(4-(3-(Fluormethyl)benzyl)pyridin-2-yl)-2,5,6,7-tetrahydro-*4H*-[1,2,3]triazolo[4,5-c]pyridin-4-on;
5-Methyl-2-(4-(3-(trifluormethyl)benzyl)pyridin-2-yl)-2,5,6,7-tetrahydro-*4H*-[1.2.3] triazolo[4,5-c]pyridin-4-on;
2-(4-(3-(Trifluormethyl)benzyl)pyridin-2-yl)-5,6,7,8-tetrahydro-[1,2,3]triazolo[4,5-c] azepin-4(*2H)*-on;
1-(4-(3-Fluor-5-(trifluormethyl)benzyl)pyridin-2-yl)-5,6,7,8-tetrahydro-[1.2.3] triazolo[4,5-c]azepin-4*(1H)*-on;
1-(4-(3-Fluor-5-(trifluormethyl)benzyl)pyridin-2-yl)-1,7-dihydropyrazolo[4,3-d] [1,2]Oxazin-4(5*H)*-on;
1-(2-(3-Fluor-5-(trifluormethyl)benzyl)pyridin-4-yl)-5,6-dihydropyrrolo[3,4-c]pyrazol-4*(1H)*-on;
1-(2-((3-Fluor-5-(trifluormethyl)phenyl)methyl-d)pyridin-4-yl)-1,5,6,7-tetrahydro-*4H*-pyrazolo[4,3-c]pyridin-4-on;
1-(2-(3-Fluor-5-(trifluormethyl)benzyl)pyridin-4-yl)-7-methyl-1,5,6,7-tetrahydro-*4H*-pyrazolo[4,3-c]pyridin-4-on;
1-(2-(3-Fluor-5-(trifluormethyl)benzyl)pyridin-4-yl)-1,5,6,7-tetrahydro-*4H*-pyrrolo[3,2-c]pyridin-4-on;
1-(2-(3-Fluor-5-(trifluormethyl)phenoxy)pyridin-4-yl)-1,5,6,7-tetrahydro-*4H*-pyrazolo[4,3-c]pyridin-4-on;
1-(2-(3-Cyclopropyl-5-fluorbenzyl)pyridin-4-yl)-1,5,6,7-tetrahydro-*4H*-pyrazolo[4,3-c]pyridin-4-on;
1-(2-(3-Fluor-4-(trifluormethyl)benzyl)pyridin-4-yl)-1,5,6,7-tetrahydro-*4H*-pyrazolo[4,3-c]pyridin-4-on;
1-(2-(4-Methoxy-3-(trifluormethyl)benzyl)pyridin-4-yl)-1,5,6,7-tetrahydro-*4H*-pyrazolo[4,3-c]pyridin-4-on;
1-(6-(3-Fluor-5-(trifluormethyl)benzyl)pyrimidin-4-yl)-1,5,6,7-tetrahydro-*4H*-pyrazolo[4,3-c]pyridin-4-on;
1-(2-(3-Methoxy-5-(trifluormethyl)benzyl)pyridin-4-yl)-1,5,6,7-tetrahydro-*4H*-pyrazolo[4,3-c]pyridin-4-on;
1-(2-(4-Chlor-3,5-difluorbenzyl)pyridin-4-yl)-1,5,6,7-tetrahydro-*4H*-pyrazolo[4,3-*c*]pyridin-4-on;
1-(2-(Benzo[b]thiophen-5-ylmethyl)pyridin-4-yl)-1,5,6,7-tetrahydro-*4H*-pyrazolo[4,3-*c*]pyridin-4-on;
1-(2-(3-Methoxy-4-(trifluormethyl)benzyl)pyridin-4-yl)-1,5,6,7-tetrahydro-*4H*-pyrazolo[4,3-c]pyridin-4-on;
1-(2-(3-Fluor-5-(pentafluoro-$\lambda^6$-sulfanyl)benzyl)pyridin-4-yl)-1,5,6,7-tetrahydro-*4H*-pyrazolo[4,3-c]pyridin-4-on;
1-(2-(3-(Difluormethoxy)-5-fluorbenzyl)pyridin-4-yl)-1,5,6,7-tetrahydro-*4H*-pyrazolo[4,3-c]pyridin-4-on;
1-(2-(4-(Difluormethoxy)-3-fluorbenzyl)pyridin-4-yl)-1,5,6,7-tetrahydro-*4H*-pyrazolo[4,3-c]pyridin-4-on;
1-(2-(3,4-Difluor-5-(trifluormethyl)benzyl)pyridin-4-yl)-1,5,6,7-tetrahydro-*4H*-pyrazolo[4,3-c]pyridin-4-on;
1-(2-(3-(Difluormethyl)-5-fluorbenzyl)pyridin-4-yl)-1,5,6,7-tetrahydro-*4H*-pyrazolo[4,3-c]pyridin-4-on;
1-(2-(3-Chlor-4,5-difluorbenzyl)pyridin-4-yl)-1,5,6,7-tetrahydro-*4H*-pyrazolo[4,3-*c*]pyridin-4-on;
1-(2-(Chinolin-3-ylmethyl)pyridin-4-yl)-1,5,6,7-tetrahydro-*4H*-pyrazolo[4,3-c]pyridin-4- on;
1-(2-(4-Chlor-3-fluor-5-(trifluormethyl)benzyl)pyridin-4-yl)-1,5,6,7-tetrahydro-*4H*-pyrazolo[4,3-c]pyridin-4-on;
1-(2-(Benzo[*b*]thiophen-2-ylmethyl)pyridin-4-yl)-1,5,6,7-tetrahydro-*4H*-pyrazolo[4,3-c]pyridin-4-on;
1-(2-(3,5-Difluor-4-(trifluormethyl)benzyl)pyridin-4-yl)-1,5,6,7-tetrahydro-*4H*-pyrazolo[4,3-c]pyridin-4-on;
1-(2-(3-Fluor-5-isopropylbenzyl)pyridin-4-yl)-1,5,6,7-tetrahydro-*4H*-pyrazolo[4,3-*c*]pyridin-4-on;
1-(2-((5-Fluor-6-methoxypyridin-3-yl)methyl)pyridin-4-yl)-1,5,6,7-tetrahydro-*4H*-pyrazolo[4,3-c]pyridin-4-on;
1-(2-(3-Chlor-4-fluor-5-(trifluormethyl)benzyl)pyridin-4-yl)-1,5,6,7-tetrahydro-*4H*-pyrazolo[4,3-c]pyridin-4-on;
1-(2-(3-Fluor-5-(1-(trifluormethyl)cyclopropyl)benzyl)pyridin-4-yl)-1,5,6,7-tetrahydro-*4H*-pyrazolo[4,3-c]pyridin-4-on;
1-(2-(3-Fluor-5-(trifluormethyl)benzyl)pyridin-4-yl)-3-methyl-1,5,6,7-tetrahydro-*4H*-pyrazolo[4,3-c]pyridin-4-on;
1-(2-((6-Fluor-5-methylpyridin-3-yl)methyl)pyridin-4-yl)-3-methyl-1,5,6,7-tetrahydro-*4H*-pyrazolo[4,3-c]pyridin-4-on;
3-Methyl-1-(2-(3,4,5-trifluorbenzyl)pyridin-4-yl)-1,5,6,7-tetrahydro-*4H*-pyrazolo[4,3-*c*]pyridin-4-on;
3-Methyl-1-(2-(3-(trifluormethyl)benzyl)pyridin-4-yl)-1,5,6,7-tetrahydro-*4H*-pyrazolo[4,3-c]pyridin-4-on;
1-(2-(3-Chlor-5-fluorbenzyl)pyridin-4-yl)-3-methyl-1,5,6,7-tetrahydro-*4H*-pyrazolo[4,3-c]pyridin-4-on;
1-(2-(3-Chlor-4-fluorbenzyl)pyridin-4-yl)-3-methyl-1,5,6,7-tetrahydro-*4H*-pyrazolo[4,3-c]pyridin-4-on;
1-(2-(4-(Difluormethoxy)-3-fluorbenzyl)pyridin-4-yl)-3-methyl-1,5,6,7-tetrahydro-*4H*-pyrazolo[4,3-c]pyridin-4-on;
1-(5-(3-Fluor-5-(trifluormethyl)benzyl)pyridin-3-yl)-3-methyl-1,5,6,7-tetrahydro-*4H*-pyrazolo[4,3-c]pyridin-4-on;
1-(4-((6-Fluor-5-methylpyndin-3-yl)methyl)pyridin-2-yl)-3-methyl-1,5,6,7-tetrahydro-*4H*-pyrazolo[4,3-c]pyridin-4-on;
1-(2-(3-Chlor-4,5-difluorbenzyl)pyridin-4-yl)-3-methyl-1,5,6,7-tetrahydro-*4H*-pyrazolo[4,3-c]pyridin-4-on;
3-Fluor-5-((4-(3-methyl-4-oxo-4,5,6,7-tetrahydro-1*H*-pyrazolo[4,3-c]pyridin-1-yl)pyridin-2-yl)methyl)benzonitril;
1-(2-(3,4-Difluor-5-(trifluormethyl)benzyl)pyridin-4-yl)-3-methyl-1,5,6,7-tetrahydro-*4H*-pyrazolo[4,3-c]pyridin-4-on;
1-(2-(4-Chlor-3-fluor-5-(trifluormethyl)benzyl)pyridin-4-yl)-3-methyl-1,5,6,7-tetrahydro-*4H*-pyrazolo[4,3-c]pyridin-4-on;
1-(2-(3-Chlor-4-fluor-5-(trifluormethyl)benzyl)pyridin-4-yl)-3-methyl-1,5,6,7-tetrahydro-*4H*-pyrazolo[4,3-c]pyridin-4-on;
1-(2-(3-(Difluormethoxy)-5-fluorbenzyl)pyridin-4-yl)-3-methyl-1,5,6,7-tetrahydro-*4H*-pyrazolo[4,3-c]pyridin-4-on;
1-(2-(4-Chlor-3,5-difluorbenzyl)pyridin-4-yl)-3-methyl-1,5,6,7-tetrahydro-*4H*-pyrazolo[4,3-c]pyridin-4-on;
1-(2-(3-(Difluormethyl)-5-fluorbenzyl)pyridin-4-yl)-3-methyl-1,5,6,7-tetrahydro-*4H*-pyrazolo[4,3-c]pyridin-4-on;
1-(2-((6-Methoxypyridin-3-yl)methyl)pyndin-4-yl)-3-methyl-1,5,6,7-tetrahydro-*4H*-pyrazolo[4,3-c]pyridin-4-on;
1-(2-((5-Fluor-6-methoxypyndin-3-yl)methyl)pyridin-4-yl)-3-methyl-1,5,6,7-tetrahydro-*4H*-pyrazolo[4,3-c]pyridin-4-on;
1-(2-((6-(Difluormethoxy)pyridin-3-yl)methyl)pyndin-4-yl)-3-methyl-1,5,6,7-tetrahydro-*4H*-pyrazolo[4,3-c]pyridin-4-on;
1-(4-(3-Fluor-5-(trifluormethyl)benzyl)pyridin-2-yl)-3-methyl-1,5,6,7-tetrahydro-*4H*-pyrazolo[4,3-c]pyridin-4-on;
3-Fluor-5-((4-(4-oxo-4,5,6,7-tetrahydro-1*H*-pyrazolo[4,3-c]pyridin-1-yl)pyridin-2-yl)methyl)benzonitril;
1-(2-(3-Fluor-5-(trifluormethyl)benzyl)pyridin-4-yl)-5,6,7,8-tetrahydro-[1,2,3]triazolo[4,5-c]azepin-*4(1H)*-on;
2-(4-(3-Fluor-5-(trifluormethyl)benzyl)pyridin-2-yl)-2,5,6,7-tetrahydro-*4H*-[1,2,3]triazolo[4,5-c]pyridin-4-on;
1-(4-(3-Fluor-5-(trifluormethyl)benzyl)pyridin-2-yl)-1,5,6,7-tetrahydro-*4H*-[1,2,3]triazolo[4,5-c]pyridin-4-on;
1-(2-(3-Fluor-5-(trifluormethyl)benzyl)pyridin-4-yl)-1,5,6,7-tetrahydro-*4H*-[1,2,3]triazolo[4,5-c]pyridin-4-on;
2-(2-(3-Fluor-5-(trifluormethyl)benzyl)pyridin-4-yl)-2,5,6,7-tetrahydro-*4H*-[1,2,3]triazolo[4,5-c]pyridin-4-on;
1-(4-(3-(Difluormethyl)-5-fluorbenzyl)pyridin-2-yl)-1,5,6,7-tetrahydro-*4H*-pyrazolo[4,3-c]pyridin-4-on;
1-(5-(3-Fluor-5-(trifluormethyl)phenoxy)pyridin-3-yl)-1,5,6,7-tetrahydro-*4H*-pyrazolo[4,3-c]pyridin-4-on;
1-(2-(3-Fluor-5-(trifluormethyl)benzyl)pyridin-4-yl)-5-(2-methoxyethyl)-1,5,6,7-tetrahydro-*4H*-pyrazolo[4,3-c] pyridin-4-on;
1-(2-(3-Fluor-5-(trifluormethyl)benzyl)pyridin-4-yl)-5-(2-hydroxyethyl)-1,5,6,7-tetrahydro-*4H*-pyrazolo[4,3-c]pyridin-4-on;
1-(4-(3-Fluor-5-(trifluormethyl)benzyl)pyridin-2-yl)-5-(2-methoxyethyl)-1,5,6,7-tetrahydro-*4H*-pyrazolo[4,3-c] pyridin-4-on;
1-(4-(3-Fluor-5-(trifluormethyl)benzyl)pyridin-2-yl)-5-(2-hydroxyethyl)-1,5,6,7-tetrahydro-*4H*-pyrazolo[4,3-c]pyridin-4-on;
1-(2-(3-Fluor-5-(trifluormethyl)benzyl)-5-methylpyridin-4-yl)-1,5,6,7-tetrahydro-*4H*-pyrazolo[4,3-c]pyridin-4-on;
1-(3-(3-Fluor-5-(trifluormethyl)benzyl)phenyl)-1,5,6,7-tetrahydro-*4H*-pyrazolo[4,3-*c*]pyridin-4-on;
1-(2-((6-(Difluormethoxy)-5-fluorpyridin-3-yl)methyl)pyridin-4-yl)-1,5,6,7-tetrahydro-*4H*-pyrazolo[4,3-c]pyridin-4-on;
1-(2-((6-(Difluormethoxy)-5-fluorpyridin-3-yl)methyl)pyridin-4-yl)-3-methyl-1,5,6,7-tetrahydro-*4H*-pyrazolo[4,3-c]pyridin-4-on;
2-(2-(3-Fluor-5-(trifluormethyl)benzyl)pyridin-4-yl)-6,7-dihydro-*2H*-pyrazolo[4,3-*f*][1,4]oxazepin-4(*5H)*-on;
1-(4-(3-Fluor-5-(trifluormethyl)benzyl)pyridin-2-yl)-3-(hydroxymethyl)-1,5,6,7-tetrahydro-*4H*-pyrazolo[4,3-c]pyridin-4-on;
1-(2-(3-Fluor-5-(trifluormethyl)benzyl)pyridin-4-yl)-3-(hydroxymethyl)-1,5,6,7-tetrahydro-*4H*-pyrazolo[4,3-c]pyridin-4-on;
1-(2-(3-Fluor-5-(trifluormethyl)benzyl)pyridin-4-yl)-4-oxo-4,5,6,7-tetrahydro-1*H*-pyrazolo[4,3-c]pyridin-3-carbonitril;
1-(2-(3-Fluor-5-(trifluormethyl)benzyl)pyridin-4-yl)-3-(trifluormethyl)-1,5,6,7-tetrahydro-*4H*-pyrazolo[4,3-c]pyridin-4-on;
1-(2-((3-Fluor-5-(trifluormethyl)phenyl)(hydroxy)methyl)pyridin-4-yl)-3-methyl-1,5,6,7-tetrahydro-*4H*-pyrazolo [4,3-c]pyridin-4-on;
1-(4-((3-Fluor-5-(trifluormethyl)phenyl)(hydroxy)methyl)pyridin-2-yl)-3-methyl-1,5,6,7-tetrahydro-*4H*-pyrazolo [4,3-c]pyridin-4-on;
oder ein Salz davon.

**14.** Verbindung nach Anspruch 1, wobei die Verbindung die folgende ist:

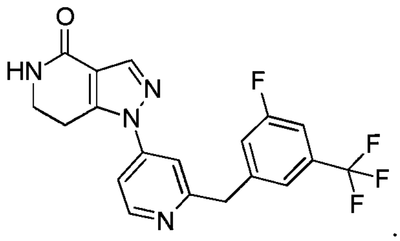

15. Verbindung nach Anspruch 1, wobei die Verbindung die folgende ist:

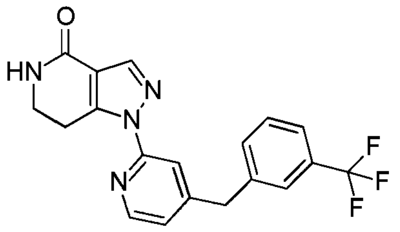

16. Verbindung nach Anspruch 1, wobei die Verbindung die folgende ist:

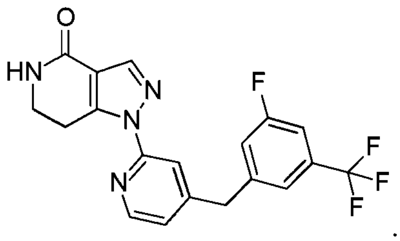

17. Verbindung nach Anspruch 1, wobei die Verbindung die folgende ist:

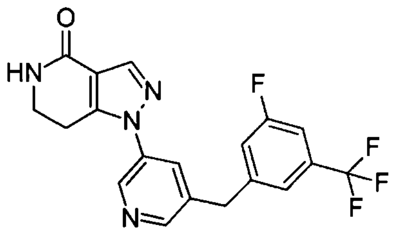

18. Verbindung nach Anspruch 1, wobei die Verbindung die folgende ist:

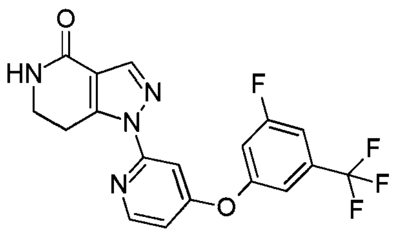

19. Verbindung nach Anspruch 1, wobei die Verbindung die folgende ist:

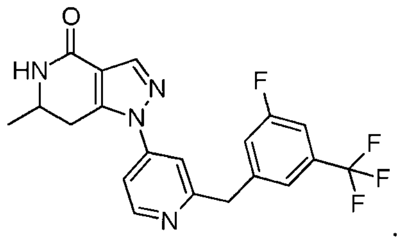

20. Verbindung nach Anspruch 1, wobei die Verbindung die folgende ist:

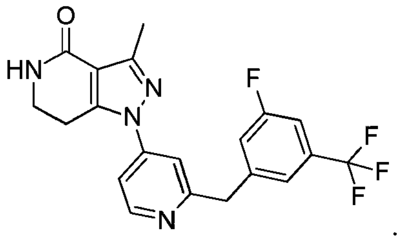

21. Verbindung nach Anspruch 1, wobei die Verbindung die folgende ist:

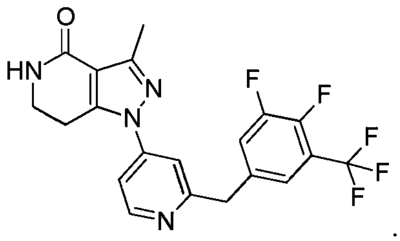

22. Verbindung nach Anspruch 1, wobei die Verbindung die folgende ist:

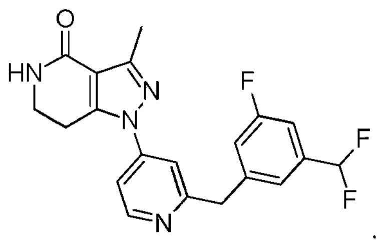

23. Verbindung nach Anspruch 1, wobei die Verbindung die folgende ist:

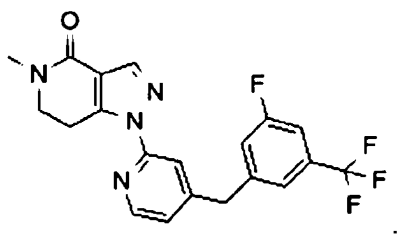

24. Verbindung nach Anspruch 1, wobei die Verbindung die folgende ist:

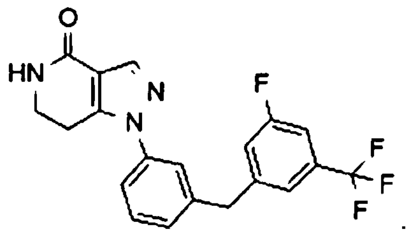

25. Verbindung nach Anspruch 1, wobei die Verbindung die folgende ist:

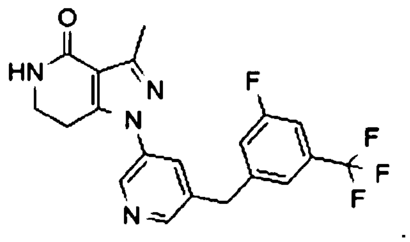

26. Verbindung nach Anspruch 1, wobei die Verbindung die folgende ist:

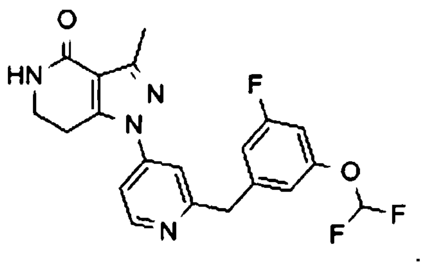

27. Salz einer Verbindung nach einem der Ansprüche 14 bis 26.

28. Pharmazeutische Zusammensetzung, umfassend eine Verbindung oder ein Salz, wie in einem der Ansprüche 1 bis 27 definiert, und einen pharmazeutisch annehmbaren Hilfsstoff.

29. Verbindung oder Salz nach einem der Ansprüche 1 bis 27 oder Zusammensetzung nach Anspruch 28 zur Verwendung in der Medizin.

30. Verbindung nach einem der Ansprüche 1 bis 27 oder Zusammensetzung nach Anspruch 28 zur Verwendung bei der Behandlung von psychiatrischen Störungen; neuropsychiatrischen Störungen; neurodegenerativen Störungen; psychotischen Störungen; kognitiven Störungen; neurokognitiven Störungen; extrapyramidalen Störungen; Bewegungsstörungen; motorischen Störungen; hyperkinetischen Bewegungsstörungen; Katatonie; Gemütsstörungen; depressiven Störungen; Angststörungen; Zwangsstörungen (OCD); Autismus-Spektrum-Störungen; depressiven Störungen; hypothalamischen Störungen; Hypophysenstörungen; prolaktinbedingten Störungen; trauma- oder stressbedingten Störungen; Störungen des Sozialverhaltens, Impulskontrollstörungen oder Verhaltensstörungen; Schlaf-Wach-Störungen; substanzbedingten Störungen; Suchterkrankungen; Verhaltensstörungen; Hypofrontalität; Anomalien in dem tuberoinfundibulären, mesolimbischen, mesokortikalen oder nigrostriatalen Pfad; verminderter Aktivität im Striatum; kortikaler Dysfunktion; neurokognitiver Dysfunktion oder damit verbundenen Zuständen oder Symptomen.

31. Verbindung oder Zusammensetzung zur Verwendung nach Anspruch 30, wobei die Störung oder das Symptom aus Folgendem ausgewählt ist: Schizophrenie, positive Symptomen der Schizophrenie, negative Symptomen der Schizophrenie, kognitive Symptomen der Schizophrenie, Depression, Aufmerksamkeitsdefizit-/Hyperaktivitätsstörung (ADHS), generalisierte Angststörung, Zwangsstörungen (OCD), Panikstörungen, bipolare Störungen, Suchterkrankungen/Impulskontrollstörungen, Autismus-Spektrum-Störungen, Psychosen, Anhedonie, Unruhezuständen, Alzheimer-Krankheit, Parkinson-Krankheit, Huntington-Krankheit, vaskuläre Demenz, Lewy-Körper-Demenz, frontotemporaler Demenz, Tourette-Syndrom, Hyperprolaktinämie, Hypophysenadenom, Prolaktinom, Kraniopharyngeom, Morbus Cushing, Diabetes insipidus, nicht-funktionierende Tumoren, Adipositas, posttraumatische Belastungsstörung (PTBS), Akathisie und damit verbundene Bewegungen, Athetose, Ataxie, Ballismus, Hemiballismus, Chorea, Choreoathetose, Dyskinesie, tardive Dyskinesie, neuroleptisch induzierte Dyskinesie, Myoklonus, Spiegelbewegungsstörung, paroxysmaler kinesigener Dyskinesie, Restless-Legs-Syndrom, Spasmen, stereotyper Bewegungsstörung, Stereotypie, Tic-Störung, Tremor, Morbus Wilson, schizotype Persönlichkeitsstörung, wahnhafte Störung, kurzfristige psychotische Störung, schizophreniforme Störung, schizoaffektive Störung, substanz- oder medikamenteninduzierte psychotischer Störung, Wahnvorstellungen, Halluzinationen, desorganisiertes Denken, grob unorganisiertes oder abnormales motorisches Verhalten, Katatonie, schwere depressiver Störung, bipolare I-Störung, bipolare II-Störung, zyklothymische Störung, substanz- oder medikamenteninduzierte bipolare und verwandte Störungen, bipolare und verwandte Störungen aufgrund einer anderen medizinischen Erkrankung, Trennungsangststörung, selektiver Mutismus, spezifische Phobie, soziale Angststörung, Panikstörung, Agoraphobie, generalisierte Angststörung, substanz- oder medikamenteninduzierte Angststörung, Angststörungen aufgrund einer anderen Erkrankung, Delirium, schwere neurokognitive Störung, leichte neurokognitive Störung, Amnesie, Demenz, Entwicklungskoordinationsstörung, stereotype Bewegungsstörung, ein Post-Schlaganfall-Effekt, dentatorubral-pallidoluysianische Atrophie, verminderter emotionaler Ausdruck, Antriebslosigkeit, Alogie und Asozialität;
wobei die Störung oder das Symptom vorzugsweise aus Folgendem ausgewählt ist: Schizophrenie, positive Symptome der Schizophrenie, negative Symptome der Schizophrenie, kognitive Symptome der Schizophrenie, Depression, Aufmerksamkeitsdefizit-/Hyperaktivitätsstörung (ADHS), generalisierte Angststörung, Zwangsstörung (OCD), Panikstörung, bipolare Störung, Suchterkrankungen/Impulskontrollstörungen, Autismus-Spektrum-Störungen, Psychose, neurokognitive Störung, Delirium, Anhedonie, Unruhezustände, Alzheimer-Krankheit, Parkinson-Krankheit, Huntington-Krankheit, vaskuläre Demenz, Lewy-Körper-Demenz, frontotemporale Demenz, Tourette-Syndrom, Hyperprolaktinämie, Adipositas und posttraumatische Belastungsstörung (PTBS).

## Revendications

1. Composé de formule (1) :

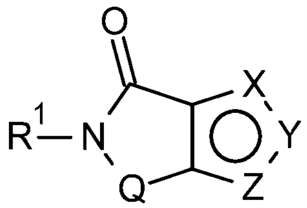

(1)

ou un sel de celui-ci, dans lequel ;
X est N ou $CR^2$ ;
Y est N, $NR^3$ ou $CR^2$ ;
Z est N ou $NR^3$ ;
dans lequel un mais pas à la fois Y et Z est $NR^3$ ;
Q est sélectionné parmi $-CR^4R^5-$, $-CR^4R^5CR^6R^7-$, $-CR^4R^5CR^6R^7CR^8R^9-$, $-CR^4R^5CR^6R^7O-$, $-OCR^4R^5-$ et $-OCR^4R^5CR^6R^7-$ ;
$R^1$ est H, $C_{1-6}$ alkyle facultativement substitué par 1 à 6 atomes de fluor, $C_{3-6}$ cycloalkyle facultativement substitué par 1 à 6 atomes de fluor, un groupe $-C(R^{14})_2C(R^{18})_2OR^{17}$ ou un groupe $-C(R^{14})_2C(R^{19})_2OH$, dans lequel chaque $R^{14}$ est indépendamment H, F ou $C_{1-3}$ alkyle facultativement substitué par 1 à 6 atomes de fluor, $R^{17}$ est $C_{1-3}$ alkyle facultativement substitué par 1 à 6 atomes de fluor, chaque $R^{18}$ est indépendamment H, F ou $C_{1-3}$ alkyle facultativement substitué par 1 à 6 atomes de fluor et chaque $R^{19}$ est indépendamment H ou $C_{1-3}$ alkyle facultativement substitué par 1 à 6 atomes de fluor, et dans lequel $R^{17}$ et un $R^{14}$ peuvent être joints pour former un cycle d'oxolane ou d'oxétane ;
$R^2$ est H, halo, CN, $C_{1-6}$ alkyle facultativement substitué par OH ou 1 à 6 atomes de fluor, $C_{1-6}$ alcoxy facultativement substitué par OH ou 1 à 6 atomes de fluor ou $C_{3-6}$ cycloalkyle facultativement substitué par OH ou 1 à 6 atomes de fluor, dans lequel un atome du groupe $C_{1-6}$ alkyle, $C_{1-6}$ alcoxy ou $C_{3-6}$ cycloalkyle peut être facultativement remplacé par un hétéroatome sélectionné parmi O, N, S et des formes oxydées de ceux-ci ;
$R^4$, $R^5$, $R^6$, $R^7$, $R^8$ et $R^9$ sont sélectionnés indépendamment parmi H et $C_{1-3}$ alkyle ; dans lequel $R^3$ est un groupe de la formule :

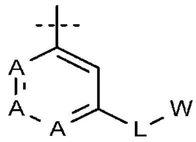

dans lequel chaque A est indépendamment N ou $CR^{10}$ ;
L est sélectionné parmi $CH_2$, CHD, $CD_2$, CHF, $CF_2$, C=O, CHOH, O et NH ;
$R^{10}$ est sélectionné parmi H, halo et $C_{1-3}$ alkyle facultativement substitué par 1 à 6 atomes de fluor ;
et W est soit :

(i) un cycle aryle facultativement substitué à 6 chaînons ou un cycle hétéroaryle facultativement substitué à 6 chaînons ;
ou
(ii) un système de cycles hétérocycliques facultativement substitués à 9-10 chaînons, où un ou plusieurs des cycles est aromatique.

**2.** Composé selon la revendication 1, dans lequel W est un cycle aryle facultativement substitué à 6 chaînons ou un cycle hétéroaryle facultativement substitué à 6 chaînons.

**3.** Composé selon la revendication 1, qui est

(i) un composé de formule (2a), (2b), (2c), (2d), (2e), (2f) ou (2g) :

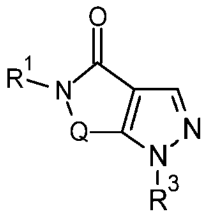 (2a); 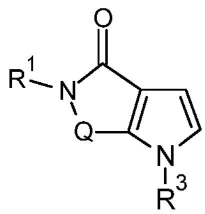 (2b); 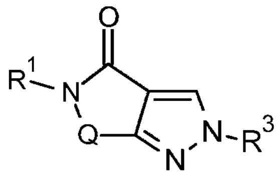 (2c);

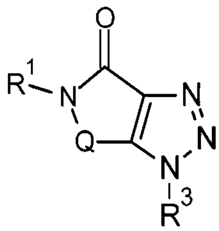 (2d); 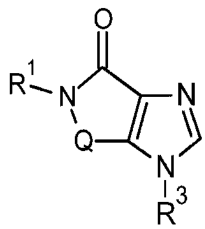 (2e); 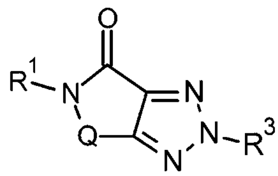 (2f);

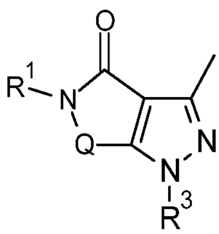 (2g);

ou un sel de celui-ci ; ou
(ii) un composé de formule (11a) :

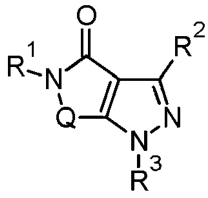

(11a)

ou un sel de celui-ci.

**4.** Composé selon l'une quelconque des revendications 1 à 3 dans lequel

(i) $R^3$ est un groupe de la formule :

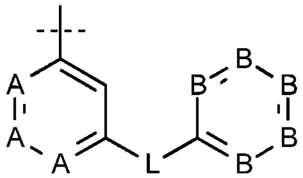

dans lequel chaque A est indépendamment N ou $CR^{10}$ ;
L est sélectionné parmi $CH_2$, CHD, $CD_2$, CHF, $CF_2$, C=O, CHOH, O et NH ; chaque B est indépendamment N, $CR^{11}$, $CR^{12}$, $CR^{13}$, $CR^{15}$ ou $CR^{16}$ ;
$R^{10}$ est sélectionné parmi H, halo et $C_{1-3}$ alkyle facultativement substitué par 1 à 6 atomes de fluor ;
$R^{11}$, $R^{12}$, $R^{13}$, $R^{15}$ et $R^{16}$ sont indépendamment sélectionnés parmi H, CN, $SF_5$, halo, un groupe d'hydrocarbures saturés $C_{1-6}$ facultativement substitué par 1 à 6 atomes de fluor dans lequel un atome du groupe d'hydrocarbures saturés $C_{1-6}$ est facultativement remplacé par un hétéroatome sélectionné parmi O, N, S et des formes oxydées de ceux-ci, $OC_{1-6}$ alkyle facultativement substitué par 1 à 6 atomes de fluor, $SO_2C_{1-6}$ alkyle facultativement substitué par 1 à 6 atomes de fluor et $C_{3-6}$ cycloalkyle facultativement substitué par 1 à 6 atomes de fluor ou facultativement substitué par $CF_3$ ; de préférence dans lequel

(ii) $R^3$ est sélectionné dans le groupe consistant en :

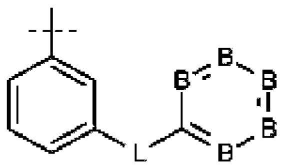 ; 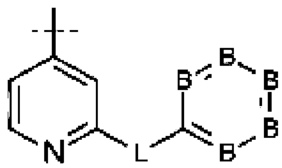 ; 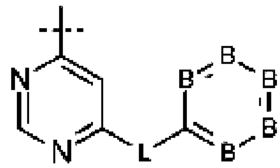 ;

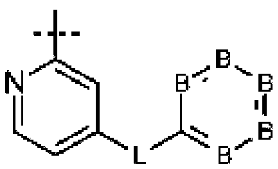 ; 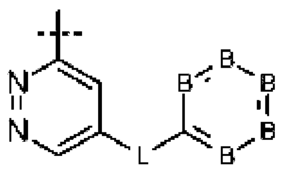 ; 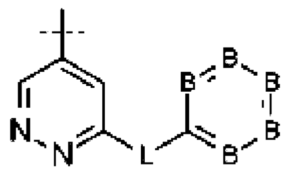 ;

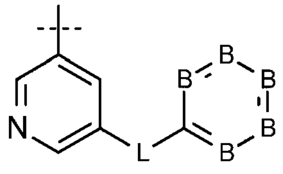 ; 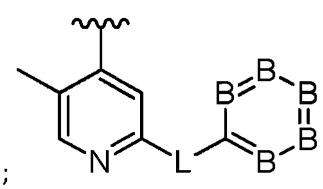 .

**5.** Composé selon l'une quelconque des revendications 1 à 4, dans lequel L est $CH_2$.

**6.** Composé selon la revendication 4 ou la revendication 5, dans lequel le groupe :

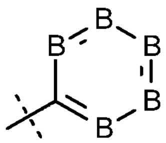

est sélectionné dans le groupe consistant en :

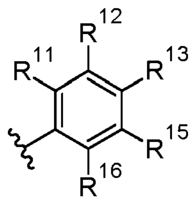 ; 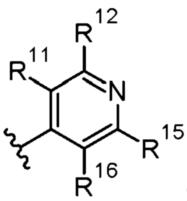 ; 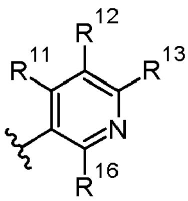 .

**7.** Composé selon l'une quelconque des revendications 1 à 6, dans lequel Q est sélectionné dans le groupe consistant en $-CH_2CH_2-$ ; $-CH_2CH_2CH_2-$ ; $-CH_2-$ ; $-OCH_2-$ ; $-CH_2CH_2O-$, $-CH(CH_3)CH_2-$ et $-CH_2CH(CH_3)-$ ; de préférence dans lequel Q est $-CH_2CH_2-$.

**8.** Composé selon la revendication 1 qui est un composé de formule (13a), (13b), (13c), (13d), (13e), (13f), (13g), (13h), (13i) ou (13j) :

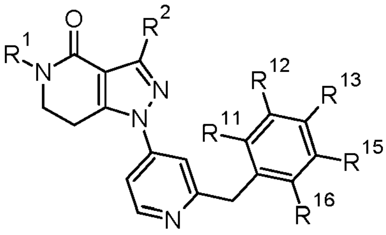 (13a);

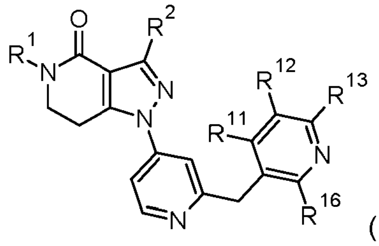 (13b);

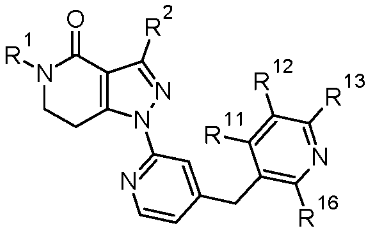 (13c);

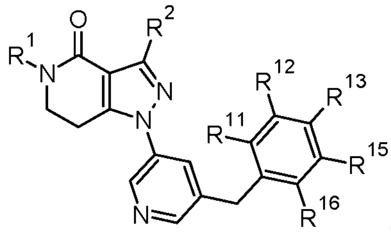 (13d);

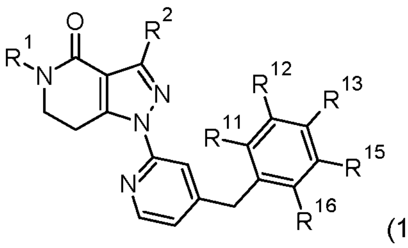 (13e);

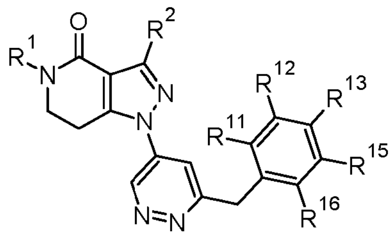 (13f);

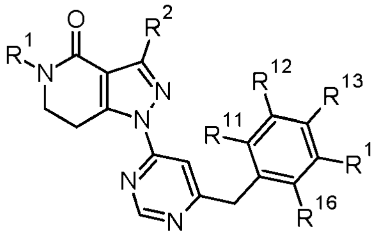
(13g);

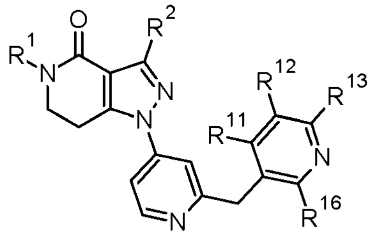
(13h);

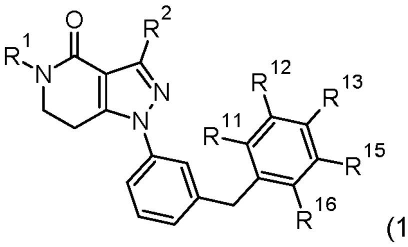
(13i);

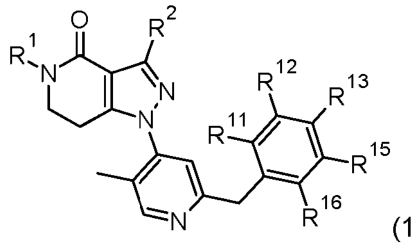
(13j);

ou un sel de celui-ci, dans lequel ;

$R^1$ est H, $C_{1-6}$ alkyle facultativement substitué par 1 à 6 atomes de fluor, $C_{3-6}$ cycloalkyle facultativement substitué par 1 à 6 atomes de fluor, un groupe $-C(R^{14})_2C(R^{18})_2OR^{17}$ ou un groupe $-C(R^{14})_2C(R^{19})_2OH$, dans lequel chaque $R^{14}$ est indépendamment H, F ou $C_{1-3}$ alkyle facultativement substitué par 1 à 6 atomes de fluor, $R^{17}$ est $C_{1-3}$ alkyle facultativement substitué par 1 à 6 atomes de fluor, chaque $R^{18}$ est indépendamment H, F ou $C_{1-3}$ alkyle facultativement substitué par 1 à 6 atomes de fluor et chaque $R^{19}$ est indépendamment H ou $C_{1-3}$ alkyle facultativement substitué par 1 à 6 atomes de fluor, et dans lequel $R^{17}$ et un $R^{14}$ peuvent être joints pour former un cycle d'oxolane ou d'oxétane ;

$R^2$ est H, halo, CN, $C_{1-6}$ alkyle facultativement substitué par OH ou 1 à 6 atomes de fluor, $C_{1-6}$ alcoxy facultativement substitué par OH ou 1 à 6 atomes de fluor ou $C_{3-6}$ cycloalkyle facultativement substitué par OH ou 1 à 6 atomes de fluor, dans lequel un atome du groupe $C_{1-6}$ alkyle, $C_{1-6}$ alcoxy ou $C_{3-6}$ cycloalkyle peut être facultativement remplacé par un hétéroatome sélectionné parmi O, N, S et des formes oxydées de ceux-ci ;

$R^{11}$, $R^{12}$, $R^{13}$, $R^{15}$ et $R^{16}$ sont indépendamment sélectionnés parmi H, CN, $SF_5$, halo, un groupe d'hydrocarbures saturés $C_{1-6}$ facultativement substitué par 1 à 6 atomes de fluor dans lequel un atome du groupe d'hydrocarbures saturés $C_{1-6}$ est facultativement remplacé par un hétéroatome sélectionné parmi O, N, S et des formes oxydées de ceux-ci, $OC_{1-6}$ alkyle facultativement substitué par 1 à 6 atomes de fluor, $SO_2C_{1-6}$ alkyle facultativement substitué par 1 à 6 atomes de fluor et $C_{3-6}$ cycloalkyle facultativement substitué par 1 à 6 atomes de fluor ou facultativement substitué par $CF_3$.

9. Composé selon la revendication 4 qui est un composé de formule (6a) :

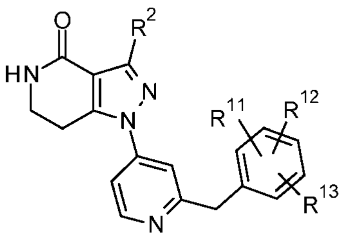
(6a)

ou un sel de celui-ci.

10. Composé selon l'une quelconque des revendications 1 à 8, dans lequel $R^1$ est H, méthyl, - $CH_2CH_2OCH_3$ ou $-CH_2CH_2OH$.

11. Composé selon l'une quelconque des revendications 1 à 10 dans lequel $R^2$ est sélectionné parmi H, méthyl, F, Cl, CN, $CF_3$ et $CH_2OH$ ; de préférence dans lequel $R^2$ est H ou méthyl.

12. Composé selon l'une quelconque des revendications 4 à 11, dans lequel $R^{11}$, $R^{12}$, $R^{13}$, $R^{15}$ et $R^{16}$ sont indépen-

damment sélectionnés parmi H, CN, F, Cl, méthyl, isopropyle, cyclopropyle, $CF_3$, $CF_2H$, $OCF_2H$, OMe et

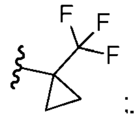

;

de préférence dans lequel $R^{11}$, $R^{12}$, $R^{13}$, $R^{15}$ et $R^{16}$ sont sélectionnés indépendamment parmi H, F et $CF_3$.

**13.** Composé selon la revendication 1 qui est sélectionné dans le groupe consistant en :

1-(2-(3-fluoro-5-(trifluorométhyl)benzyl)pyridine-4-yl)-1,5,6,7-tétrahydro-4*H*-pyrazolo[4,3-c]pyridine-4-one ;
1-(4-(3-(trifluorométhyl)benzyl)pyridine-2-yl)-1,5,6,7-tétrahydro-4*H*-pyrazolo[4,3-c]pyridine-4-one ;
5-méthyl-1-(4-(3-(trifluorométhyl)benzyl)pyridine-2-yl)-1,5,6,7-tétrahydro-4*H*-pyrazolo[4,3-c]pyridine-4-one ;
1-(4-(3-fluoro-5-(trifluorométhyl)benzyl)pyridine-2-yl)-1,5,6,7-tétrahydro-4*H*-pyrazolo[4,3-c]pyridine-4-one ;
1-(4-(3-fluoro-5-(trifluorométhyl)benzyl)pyridine-2-yl)-5-méthyl-1,5,6,7-tétrahydro-4*H*-pyrazolo[4,3-c]pyridine-4-one ;
1-(4-((6-fluoro-5-méthylpyridine-3-yl)méthyl)pyridine-2-yl)-1,5,6,7-tétrahydro-4*H*-pyrazolo[4,3-c]pyridine-4-one ;
1-(4-((6-fluoro-5-méthylpyridine-3-yl)méthyl)pyridine-2-yl)-5-méthyl-1,5,6,7-tétrahydro-4*H*-pyrazolo[4,3-c]pyridine-4-one ;
2-(4-(3-(trifluorométhyl)benzyl)pyridine-2-yl)-2,5,6,7-tétrahydro-4*H*-pyrazolo[4,3-c]pyridine-4-one ;
2-(2-(3-fluoro-5-(trifluorométhyl)benzyl)pyridine-4-yl)-2,5,6,7-tétrahydro-4*H*-pyrazolo[4,3-c]pyridine-4-one ;
1-(4-(3-fluoro-5-(trifluorométhyl)benzyl)pyridine-2-yl)-5,6,7,8-tétrahydropyrazolo[4,3-c]azépine-4(1*H*)-one ;
1-(4-(3-(trifluorométhyl)benzyl)pyridine-2-yl)-5,6,7,8-tétrahydropyrazolo[4,3-c]azépine-4(1*H*)-one ;
1-(2-(3-fluoro-5-(trifluorométhyl)benzyl)pyridine-4-yl)-5,6,7,8-tétrahydropyrazolo[*4,3*-c]azépine-4(1*H*)-one ;
1-(4-(3-(trifluorométhyl)benzyl)pyridine-2-yl)-6,7-dihydro-1*H*-pyrazolo[4,3-f] [1,4]oxazépine-4(5*H*)-one ;
1-(4-(3-fluoro-5-(trifluorométhyl)benzyl)pyridine-2-yl)-6,7-dihydro-1*H*-pyrazolo[4,3-*f*][1,4]oxazépine-4(5H)-one;
1-(5-(3-fluoro-5-(trifluorométhyl)benzyl)pyridazine-3-yl)-1,5,6,7-tétrahydro-*4H*-pyrazolo[4,3-c]pyridine-4-one ;
1-(5-(3-fluoro-5-(trifluorométhyl)benzyl)pyridine-3-yl)-1,5,6,7-tétrahydro-4*H*-pyrazolo[4,3-c]pyridine-4-one ;
1-(6-(3-fluoro-5-(trifluorométhyl)benzyl)pyridazine-4-yl)-1,5,6,7-tétrahydro-4*H*-pyrazolo[4,3-c]pyridine-4-one ;
1-{4-[3-fluoro-5-(trifluorométhyl)phénoxy]pyridine-2-yl}-1,5,6,7-tétrahydro-4*H*-pyrazolo[4,3-c]pyridine-4-one ;
1-(2-(4-chlorobenzyl)pyridine-4-yl)-1,5,6,7-tétrahydro-4*H*-pyrazolo[4,3-c]pyridine-4-one ;
1-(2-(3-chloro-5-fluorobenzyl)pyridine-4-yl)-1,5,6,7-tétrahydro-4*H*-pyrazolo[4,3-c]pyridine-4-one ;
1-(2-(3,5-difluorobenzyl)pyridine-4-yl)-1,5,6,7-tétrahydro-4*H*-pyrazolo[4,3-c]pyridine-4-one ;
1-(2-(3-(trifluorométhyl)benzyl)pyridine-4-yl)-1,5,6,7-tétrahydro-4*H*-pyrazolo[4,3-c]pyridine-4-one ;
1-(2-(4-fluoro-3-(trifluorométhyl)benzyl)pyridine-4-yl)-1,5,6,7-tétrahydro-4*H*-pyrazolo[4,3-c]pyridine-4-one ;
1-(2-(3,4,5-trifluorobenzyl)pyridine-4-yl)-1,5,6,7-tétrahydro-4*H*-pyrazolo[4,3-c]pyridine-4-one ;
1-(2-(3-chloro-4-fluorobenzyl)pyridine-4-yl)-1,5,6,7-tétrahydro-4*H*-pyrazolo[4,3-c]pyridine-4-one ;
1 -(2-(3-fluoro-5-méthylbenzyl)pyridine-4-yl)-1,5,6,7-tétrahydro-4*H*-pyrazolo[4,3-c]pyridine-4-one ;
1-(2-(3-chloro-5-(trifluorométhyl)benzyl)pyridine-4-yl)-1,5,6,7-tétrahydro-4*H*-pyrazolo[4,3-c]pyridine-4-one ;
1-(2-(3-méthyl-5-(trifluorométhyl)benzyl)pyridine-4-yl)-1,5,6,7-tétrahydro-4*H*-pyrazolo[4,3-c]pyridine-4-one ;
1-(2-(2-fluoro-3-(trifluorométhyl)benzyl)pyridine-4-yl)-1,5,6,7-tétrahydro-4*H*-pyrazolo[4,3-c]pyridine-4-one ;
1-(2-(2-fluoro-5-(trifluorométhyl)benzyl)pyridine-4-yl)-1,5,6,7-tétrahydro-4*H*-pyrazolo[4,3-c]pyridine-4-one ;
1-(2-(3,5-dichlorobenzyl)pyridine-4-yl)-1,5,6,7-tétrahydro-4*H*-pyrazolo[4,3-c]pyridine-4-one ;
1-(2-(4-(trifluorométhyl)benzyl)pyridine-4-yl)-1,5,6,7-tétrahydro-4*H*-pyrazolo[4,3-c]pyridine-4-one ;
3-((4-(4-oxo-4,5,6,7-tétrahydro-1*H*-pyrazolo[4,3-c]pyridine-1-yl)pyridine-2-yl)méthyl)-5-(trifluorométhyl)benzonitrile ;
1-(2-(3,5-bis(trifluorométhyl)benzyl)pyridine-4-yl)-1,5,6,7-tétrahydro-4*H*-pyrazolo[4,3-c]pyridine-4-one ;
1-(2-(3-fluoro-5-(trifluorométhyl)benzoyle)pyridine-4-yl)-1,5,6,7-tétrahydro-4*H*-pyrazolo[4,3-c]pyridine-4-one ;
1-(2-((3-fluoro-5-(trifluorométhyl)phényl)(hydroxy)métyl)pyridine-4-yl)-1,5,6,7-tétrahydro-4*H*-pyrazolo[4,3-c]pyridine-4-one ;
1-(2-(fluoro(3-fluoro-5-(trifluorométhyl)phényl)méthyl)pyridine-4-yl)-1,5,6,7-tétrahydro-4H-pyrazolo[4,3-c]pyridine-4-one ;
1-(2-(difluoro(3-fluoro-5-(trifluorométhyl)phényl)méthyl)pyridine-4-yl)-1,5,6,7-tétrahydro-4*H*-pyrazolo[4,3-c]pyridine-4-one ;
1-(2-((3-fluoro-5-(trifluorométhyl)phényl)méthyl-$d_2$)pyridine-4-yl)-1,5,6,7-tétrahydro-*4H*-pyrazolo[4,3-]pyridine-4-one ;

1-(2-(3-fluoro-5-(trifluorométhyl)benzyl)pyridine-4-yl)-6-méthyl-1,5,6,7-tétrahydro-*4H*-pyrazolo[4,3-c]pyridine-4-one ;
1-(4-(3-(trifluorométhyl)benzyl)pyridine-2-yl)-5,6,7,8-tétrahydro-[1,2,3]triazolo[4,5-c]azépine-4(1*H*)-one ;
1-(4-(3-(trifluorométhyle)benzyl)pyridine-2-yl)-1,5,6,7-tétrahydro-*4H*-[1,2,3]triazolo[4,5-c]pyridine-4-one ;
2-(4-(3-(trifluorométhyl)benzyl)pyridine-2-yl)-2,5,6,7-tétrahydro-*4H*-[1,2,3]triazolo[4,5-c]pyridine-4-one ;
5-méthyl-2-(4-(3-(trifluorométhyl)benzyl)pyridine-2-yl)-2,5,6,7-tétrahydro-*4H*-[1.2.3]triazolo[4,5-c]pyridine-4-one ;
2-(4-(3-(trifluorométhyl)benzyl)pyridine-2-yl)-5,6,7,8-tétrahydro-[1,2,3]triazolo[4,5-c]azépine-4(2*H*)-one ;
1-(4-(3-fluoro-5-(trifluorométhyl)benzyl)pyridine-2-yl)-5,6,7,8-tétrahydro-[1.2.3]triazolo[4,5-c]azépine-4(1*H*)-one ;
1-(4-(3-fluoro-5-(trifluorométhyl)benzyl)pyridine-2-yl)-1,7-dihydropyrazolo[4,3-d][1,2]oxazine-4(5*H*)-one ;
1-(2-(3-fluoro-5-(trifluorométhyl)benzyl)pyridine-4-yl)-5,6-dihydropyrrolo[3,4-c]pyrazol-4(1*H*)-one ;
1-(2-((3-fluoro-5-(trifluorométhyl)phényl)méthyl-d)pyridine-4-yl)-1,5,6,7-tétrahydro-*4H*-pyrazolo[4,3-c]pyridine-4-one ;
1-(2-(3-fluoro-5-(trifluorométhyl)benzyl)pyridine-4-yl)-7-méthyl-1,5,6,7-tétrahydro-*4H*-pyrazolo[4,3-c]pyridine-4-one ;
1-(2-(3-fluoro-5-(trifluorométhyl)benzyl)pyridine-4-yl)-1,5,6,7-tétrahydro-*4H*-pyrrolo[3,2-c]pyridine-4-one ;
1-(2-(3-fluoro-5-(trifluorométhyl)phénoxy)pyridine-4-yl)-1,5,6,7-tétrahydro-*4H*-pyrazolo[4,3-c]pyridine-4-one ;
1-(2-(3-cyclopropyl-5-fluorobenzyl)pyridine-4-yl)-1,5,6,7-tétrahydro-*4H*-pyrazolo[4,3-c]pyridine-4-one ;
1-(2-(3-fluoro-4-(trifluorométhyl)benzyl)pyridine-4-yl)-1,5,6,7-tétrahydro-*4H*-pyrazolo[4,3-c]pyridine-4-one ;
1-(2-(4-méthoxy-3-(trifluorométhyl)benzyl)pyridine-4-yl)-1,5,6,7-tétrahydro-*4H*-pyrazolo[4,3-c]pyridine-4-one ;
1-(6-(3-fluoro-5-(trifluorométhyl)benzyl)pyrimidin-4-yl)-1,5,6,7-tétrahydro-*4H*-pyrazolo[4,3-c]pyridine-4-one ;
1-(2-(3-méthoxy-5-(trifluorométhyl)benzyl)pyridine-4-yl)-1,5,6,7-tétrahydro-*4H*-pyrazolo[4,3-c]pyridine-4-one ;
1-(2-(4-chloro-3,5-difluorobenzyl)pyridine-4-yl)-1,5,6,7-tétrahydro-*4H*-pyrazolo[4,3-c]pyridine-4-one ;
1-(2-(benzo[b]thiophène-5-ylméthyl)pyridine-4-yl)-1,5,6,7-tétrahydro-*4H*-pyrazolo[4,3-c]pyridine-4-one ;
1-(2-(3-méthoxy-4-(trifluorométhyl)benzyl)pyridine-4-yl)-1,5,6,7-tétrahydro-*4H*-pyrazolo[4,3-c]pyridine-4-one ;
1-(2-(3-fluoro-5-(pentafluoro-$\lambda^6$-sulfaneyl)benzyl)pyridine-4-yl)-1,5,6,7-tétrahydro-*4H*-pyrazolo[4,3-c]pyridine-4-one ;
1-(2-(3-(difluorométhoxy)-5-fluorobenzyl)pyridine-4-yl)-1,5,6,7-tétrahydro-*4H*-pyrazolo[4,3-c]pyridine-4-one ;
1-(2-(4-(difluorométhoxy)-3-fluorobenzyl)pyridine-4-yl)-1,5,6,7-tétrahydro-*4H*-pyrazolo[4,3-c]pyridine-4-one ;
1-(2-(3,4-difluoro-5-(trifluorométhyl)benzyl)pyridine-4-yl)-1,5,6,7-tétrahydro-*4H*-pyrazolo[4,3-c]pyridine-4-one ;
1-(2-(3-(difluorométhyle)-5-fluorobenzyl)pyridine-4-yl)-1,5,6,7-tétrahydro-*4H*-pyrazolo[4,3-c]pyridine-4-one ;
1-(2-(3-chloro-4,5-difluorobenzyl)pyridine-4-yl)-1,5,6,7-tétrahydro-*4H*-pyrazolo[4,3-c]pyridine-4-one ;
1-(2-(quinoline-3-ylméthyl)pyridine-4-yl)-1,5,6,7-tétrahydro-*4H*-pyrazolo[4,3-c]pyridine-4-one ;
1-(2-(4-chloro-3-fluoro-5-(trifluorométhyl)benzyl)pyridine-4-yl)-1,5,6,7-tétrahydro-*4H*-pyrazolo[4,3-c]pyridine-4-one ;
1-(2-(benzo[*b*]thiophène-2-ylméthyl)pyridine-4-yl)-1,5,6,7-tétrahydro-*4H*-pyrazolo[4,3-c]pyridine-4-one ;
1-(2-(3,5-difluoro-4-(trifluorométhyl)benzyl)pyridine-4-yl)-1,5,6,7-tétrahydro-*4H*-pyrazolo[4,3-c]pyridine-4-one ;
1-(2-(3-fluoro-5-isopropylbenzyl)pyridine-4-yl)-1,5,6,7-tétrahydro-*4H*-pyrazolo[4,3-c]pyridine-4-one ;
1-(2-((5-fluoro-6-méthoxypridinine-3-yl)méthyl)pridinine-4-yl)-1,5,6,7-tétrahydro-*4H*-pyrazolo[4,3-c]pyridine-4-one ;
1-(2-(3-chloro-4-fluoro-5-(trifluorométhyl)benzyl)pyridine-4-yl)-1,5,6,7-tétrahydro-*4H*-pyrazolo[4,3-c]pyridine-4-one ;
1-(2-(3-fluoro-5-(1-(trifluorométhyl)cyclopropyle)benzyl)pyridine-4-yl)-1,5,6,7-tétrahydro-4*H*-pyrazolo[4,3-c]pyridine-4-one ;
1-(2-(3-fluoro-5-(trifluorométhyl)benzyl)pyridine-4-yl)-3-méthyl-1,5,6,7-tétrahydro-*4H*-pyrazolo[4,3-c]pyridine-4-one ;
1-(2-((6-fluoro-5-méthylpyridine-3-yl)méthyl)pyridine-4-yl)-3-méthyl-1,5,6,7-tétrahydro-*4H*-pyrazolo[4,3-c]pyridine-4-one ;
3-méthyl-1-(2-(3,4,5-trifluorobenzyl)pyridine-4-yl)-1,5,6,7-tétrahydro-*4H*-pyrazolo[*4,3*-c]pyridine-4-one ;
3-méthyl-1-(2-(3-(trifluorométhyl)benzyl)pyridine-4-yl)-1,5,6,7-tétrahydro-*4H*-pyrazolo[4,3-c]pyridine-4-one ;
1-(2-(3-chloro-5-fluorobenzyl)pyridine-4-yl)-3-méthyl-1,5,6,7-tétrahydro-*4H*-pyrazolo[4,3-c]pyridine-4-one ;
1-(2-(3-chloro-4-fluorobenzyl)pyridine-4-yl)-3-méthyl-1,5,6,7-tétrahydro-*4H*-pyrazolo[4,3-c]pyridine-4-one ;
1-(2-(4-(difluorométhoxy)-3-fluorobenzyl)pyridine-4-yl)-3-méthyl-1,5,6,7-tétrahydro-*4H*-pyrazolo[4,3-c]pyridine-4-one ;
1-(5-(3-fluoro-5-(trifluorométhyl)benzyl)pyridine-3-yl)-3-méthyl-1,5,6,7-tétrahydro-*4H*-pyrazolo[4,3-c]pyridine-4-one ;
1-(4-((6-fluoro-5-méthylpyridine-3-yl)méthyl)pyridine-2-yl)-3-méthyl-1,5,6,7-tétrahydro-*4H*-pyrazolo[4,3-c]pyridine-4-one ;
1-(2-(3-chloro-4,5-difluorobenzyl)pyridine-4-yl)-3-méthyl-1,5,6,7-tétrahydro-*4H*-pyrazolo[4,3-c]pyridine-4-one ;
3-fluoro-5-((4-(3-méthyl-4-oxo-4,5,6,7-tétrahydro-1*H*-pyrazolo[4,3-c]pyridine-1-yl)pyridine-2-yl)méthyl)benzonitrile ;
1-(2-(3,4-difluoro-5-(trifluorométhyl)benzyl)pyridine-4-yl)-3-méthyl-1,5,6,7-tétrahydro-*4H*-pyrazolo[4,3-c]pyridine-4-one ;
1-(2-(4-chloro-3-fluoro-5-(trifluorométhyl)benzyl)pyridine-4-yl)-3-méthyl-1,5,6,7-tétrahydro-4*H*-pyrazolo[4,3-c]pyridine-4-one ;
1-(2-(3-chloro-4-fluoro-5-(trifluorométhyl)benzyl)pyridine-4-yl)-3-méthyl-1,5,6,7-tétrahydro-4*H*-pyrazolo[4,3-c]pyridine-4-one ;
1-(2-(3-(difluorométhoxy)-5-fluorobenzyl)pyridine-4-yl)-3-méthyl-1,5,6,7-tétrahydro-*4H*-pyrazolo[4,3-c]pyridine-4-one ;
1-(2-(4-chloro-3,5-difluorobenzyl)pyridine-4-yl)-3-méthyl-1,5,6,7-tétrahydro-*4H*-pyrazolo[4,3-c]pyridine-4-one ;
1-(2-(3-(difluorométhyle)-5-fluorobenzyl)pyridine-4-yl)-3-méthyl-1,5,6,7-tétrahydro-*4H*-pyrazolo[4,3-c]pyridine-4-one ;
1-(2-((6-méthoxypyridine-3-yl)méthyl)pyridine-4-yl)-3-méthyl-1,5,6,7-tétrahydro-*4H*-pyrazolo[4,3-c]pyridine-4-one ;
1-(2-((5-fluoro-6-méthoxypyridine-3-yl)méthyl)pyridine-4-yl)-3-méthyl-1,5,6,7-tétrahydro-4*H*-pyrazolo[4,3-c]pyridine-4-one ;
1-(2-((6-(difluorométhoxy)pyridine-3-yl)méthyl)pyridine-4-yl)-3-méthyl-1,5,6,7-tétrahydro-4*H*-pyrazolo[4,3-c]pyridine-4-one ;
1-(4-(3-fluoro-5-(trifluorométhyl)benzyl)pyridine-2-yl)-3-méthyl-1,5,6,7-tétrahydro-*4H*-pyrazolo[4,3-c]pyridine-4-one ;
3-fluoro-5-((4-(4-oxo-4,5,6,7-tétrahydro-1*H*-pyrazolo[4,3-c]pyridine-1-yl)pyridine-2-yl)méthyl)benzonitrile ;
1-(2-(3-fluoro-5-(trifluorométhyl)benzyl)pyridine-4-yl)-5,6,7,8-tétrahydro-[1,2,3]triazolo[4,5-c]azépine-4(1*H*)-one ;
2-(4-(3-fluoro-5-(trifluorométhyl)benzyl)pyridine-2-yl)-2,5,6,7-tétrahydro-*4H*-[1,2,3]triazolo[4,5-c]pyridine-4-one ;
1-(4-(3-fluoro-5-(trifluorométhyl)benzyl)pyridine-2-yl)-1,5,6,7-tétrahydro-*4H*-[1,2,3]triazolo[4,5-c]pyridine-4-one ;
1-(2-(3-fluoro-5-(trifluorométhyl)benzyl)pyridine-4-yl)-1,5,6,7-tétrahydro-*4H*-[1,2,3]triazolo[4,5-c]pyridine-4-one ;
2-(2-(3-fluoro-5-(trifluorométhyl)benzyl)pyridine-4-yl)-2,5,6,7-tétrahydro-*4H*-[1,2,3]triazolo[4,5-c]pyridine-4-one ;
1-(4-(3-(difluorométhyle)-5-fluorobenzyl)pyridine-2-yl)-1,5,6,7-tétrahydro-4H-pyrazolo[4,3-c]pyridine-4-one ;
1-(5-(3-fluoro-5-(trifluorométhyl)phénoxy)pyridine-3-yl)-1,5,6,7-tétrahydro-*4H*-pyrazolo[4,3-c]pyridine-4-one ;
1-(2-(3-fluoro-5-(trifluorométhyl)benzyl)pyridine-4-yl)-5-(2-méthoxyéthyle)-1,5,6,7-tétrahydro-*4H*-pyrazolo[4,3-c]pyridine-4-one ;
1-(2-(3-fluoro-5-(trifluorométhyl)benzyl)pyridine-4-yl)-5-(2-hydroxyéthyle)-1,5,6,7-tétrahydro-*4H*-pyrazolo[4,3-c]pyridine-4-one ;
1-(4-(3-fluoro-5-(trifluorométhyl)benzyl)pyridine-2-yl)-5-(2-méthoxyéthyle)-1,5,6,7-tétrahydro-*4H*-pyrazolo[4,3-c]pyridine-4-one ;
1-(4-(3-fluoro-5-(trifluorométhyl)benzyl)pyridine-2-yl)-5-(2-hydroxyéthyle)-1,5,6,7-tétrahydro-*4H*-pyrazolo[4,3-c]pyridine-4-one ;
1-(2-(3-fluoro-5-(trifluorométhyl)benzyl)-5-méthylpyridine-4-yl)-1,5,6,7-tétrahydro-*4H*-pyrazolo[4,3-c]pyridine-4-one ;
1-(3-(3-fluoro-5-(trifluorométhyl)benzyl)phényl)-1,5,6,7-tétrahydro-4H-pyrazolo[4,3-*c*]pyridine-4-one ;
1-(2-((6-(difluorométhoxy)-5-fluoropyridine-3-yl)méthyl)pyridine-4-yl)-1,5,6,7-tétrahydro-4*H*-pyrazolo[4,3-c]pyridine-4-one ;
1-(2-((6-(difluorométhoxy)-5-fluoropyridine-3-yl)méthyl)pyridine-4-yl)-3-méthyl-1,5,6,7-tétrahydro-*4H*-pyrazolo[4,3-c]pyridine-4-one ;
2-(2-(3-fluoro-5-(trifluorométhyl)benzyl)pyridine-4-yl)-6,7-dihydro-*2H*-pyrazolo[4,3-*f*][1,4]oxazépine-4(5H)-one;
1-(4-(3-fluoro-5-(trifluorométhyl)benzyl)pyridine-2-yl)-3-(hydroxyméthyl)-1,5,6,7-tétrahydro-4*H*-pyrazolo[4,3-c]pyridine-4-one ;
1-(2-(3-fluoro-5-(trifluorométhyl)benzyl)pyridine-4-yl)-3-(hydroxyméthyl)-1,5,6,7-tétrahydro-4*H*-pyrazolo[4,3-c]pyridine-4-one ;
1-(2-(3-fluoro-5-(trifluorométhyl)benzyl)pyridine-4-yl)-4-oxo-4,5,6,7-tétrahydro-1*H*-pyrazolo[4,3-c]pyridine-3-carbonitrile ;

1-(2-(3-fluoro-5-(trifluorométhyl)benzyl)pyridine-4-yl)-3-(trifluorométhyl)-1,5,6,7-tétrahydro-4H-pyrazolo[4,3-c]pyridine-4-one ;
1-(2-((3-fluoro-5-(trifluorométhyl)phényl)(hydroxy)méthyl)pyridine-4-yl)-3-méthyl-1,5,6,7-tétrahydro-*4H*-pyrazolo[4,3-c]pyridine-4-one ;
1-(4-((3-fluoro-5-(trifluorométhyl)phényl)(hydroxy)méthyl)pyridine-2-yl)-3-méthyl-1,5,6,7-tétrahydro-*4H*-pyrazolo[4,3-c]pyridine-4-one ;
ou un sel de celui-ci.

**14.** Composé selon la revendication 1 dans lequel ledit composé est :

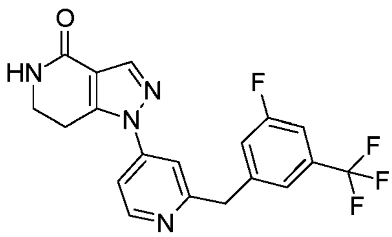

.

**15.** Composé selon la revendication 1 dans lequel ledit composé est :

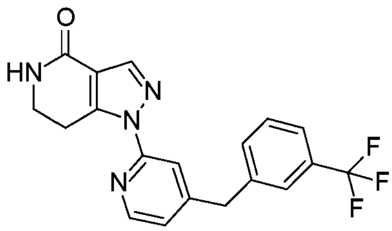

.

**16.** Composé selon la revendication 1 dans lequel ledit composé est :

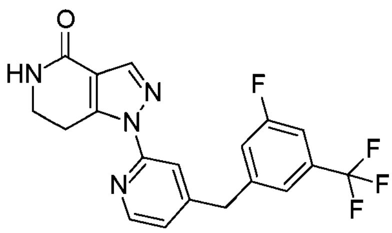

.

**17.** Composé selon la revendication 1 dans lequel ledit composé est :

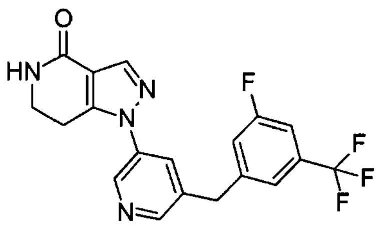

.

**18.** Composé selon la revendication 1 dans lequel ledit composé est :

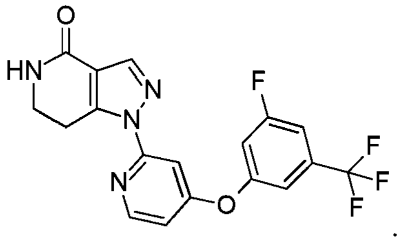

**19.** Composé selon la revendication 1 dans lequel ledit composé est :

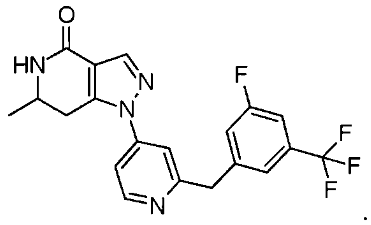

**20.** Composé selon la revendication 1 dans lequel ledit composé est :

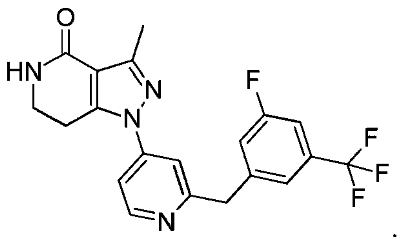

**21.** Composé selon la revendication 1 dans lequel ledit composé est :

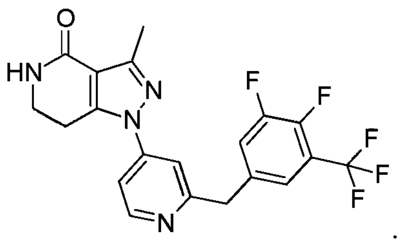

**22.** Composé selon la revendication 1 dans lequel ledit composé est :

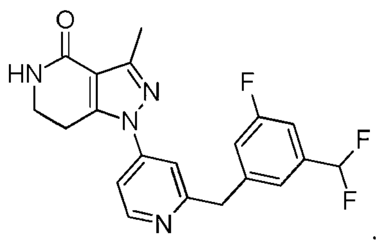

**23.** Composé selon la revendication 1 dans lequel ledit composé est :

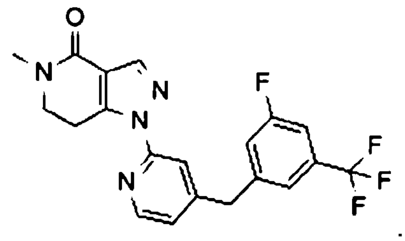

**24.** Composé selon la revendication 1 dans lequel ledit composé est :

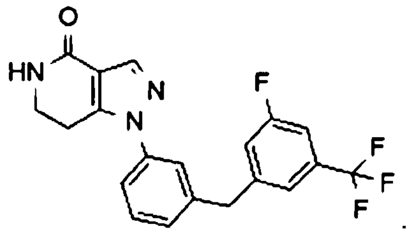

**25.** Composé selon la revendication 1 dans lequel ledit composé est :

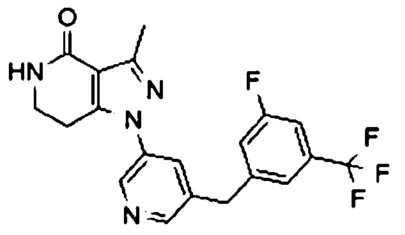

**26.** Composé selon la revendication 1 dans lequel ledit composé est :

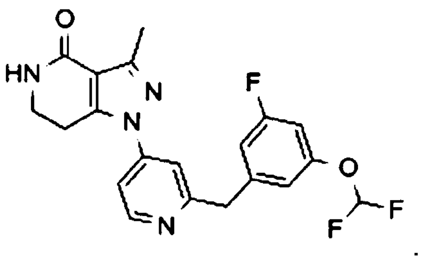

**27.** Sel d'un composé tel que défini dans l'une quelconque des revendications 14 à 26.

**28.** Composition pharmaceutique comprenant un composé ou sel tel que défini dans l'une quelconque des revendications 1 à 27 et un excipient pharmaceutiquement acceptable.

**29.** Composé ou sel selon l'une quelconque des revendications 1 à 27 ou composition selon la revendication 28 pour son utilisation en médecine.

**30.** Composé selon l'une quelconque des revendications 1 à 27 ou composition selon la revendication 28 pour son utilisation dans le traitement de troubles psychiatriques ; troubles neuropsychiatriques ; troubles neurodégénératifs ; troubles psychotiques ; troubles cognitifs ; troubles neurocognitifs ; troubles extrapyramidaux ; troubles du mouvement ; troubles moteurs ;

troubles du mouvement hypercinétique ; catatonie ; troubles de l'humeur ; troubles dépressifs ; troubles anxieux ; trouble obsessionnel-compulsif (TOC) ; troubles du spectre autistique ; troubles dépressifs ; troubles hypothalamiques ; troubles hypophysaires ; troubles liés à la prolactine ; troubles liés au traumatisme ou au stress ;

troubles perturbateurs, du contrôle des impulsions ou de la conduite ; troubles du sommeil-éveil ; troubles liés aux substances ; troubles addictifs ;

troubles comportementaux ; hypofrontalité ; anomalies de la voie tubéro-infundibulaire, mésolimbique, mésocorticale ou nigrostriatale ; diminution de l'activité dans le striatum ; dysfonctionnement cortical ; dysfonctionnement neurocognitif ou affections ou symptômes associés.

**31.** Composé ou composition pour son utilisation selon la revendication 30, dans lequel le trouble ou symptôme est sélectionné parmi les schizophrénie, symptômes positifs de schizophrénie, symptômes négatifs de schizophrénie, symptômes cognitifs de schizophrénie, dépression, trouble du déficit de l'attention et de l'hyperactivité (TDAH), trouble anxieux généralisé,

trouble obsessionnel-compulsif (TOC), trouble panique, trouble bipolaire, troubles de la dépendance/du contrôle des impulsions, troubles du spectre autistique, psychose, anhédonie, agitation, maladie d'Alzheimer, maladie de Parkinson, maladie de Huntington, démence vasculaire, maladie à corps de Lewy, démence frontotemporale, syndrome de Tourette, hyperprolactinémie,

adénome hypophysaire, prolactinome, craniopharyngiome, maladie de Cushing, diabète insipide, tumeurs non fonctionnelles, obésité, trouble de stress post-traumatique (TSPT), acathisie et mouvements associés, athétose, ataxie, ballisme, hémiballisme,

chorée, choréoathétose, dyskinésie, dyskinésie tardive, dyskinésie induite par le neuroleptique, myoclonie, trouble du mouvement du miroir, dyskinésie paroxystique kinésigène, syndrome des jambes sans repos, spasmes, trouble du mouvement stéréotypique, stéréotypie, trouble des tics, tremblement, maladie de Wilson, trouble de la personnalité schizotypique, trouble délirant,

trouble psychotique bref, trouble schizophréniforme, trouble schizoaffectif, trouble psychotique induit par une substance ou un médicament, délires, hallucinations, pensée désorganisée,

comportement moteur fortement désorganisé ou anormal, catatonie, trouble dépressif majeur, trouble bipolaire I, trouble bipolaire II, trouble cyclothymique, troubles bipolaires et apparentés induits par une substance ou un médicament, troubles bipolaires et apparentés dus à une autre pathologie, trouble d'anxiété séparative, mutisme sélectif, phobie spécifique,

trouble anxieux social, trouble panique, agoraphobie, trouble anxieux généralisé, trouble anxieux induit par une substance ou un médicament, troubles anxieux dus à une autre affection médicale, délire, trouble neurocognitif majeur, trouble neurocognitif mineur, amnésie, démence, trouble de la coordination du développement, trouble stéréotypique des mouvements,

un effet post-accident vasculaire cérébral, une atrophie dentaturo-rubro-pallido-luysienne, une diminution de l'expression émotionnelle, une avolition, une alogie et une associalité ; dans lequel le trouble ou symptôme est de préférence sélectionné parmi les schizophrénie, symptômes positifs de schizophrénie, symptômes négatifs de schizophrénie, symptômes cognitifs de schizophrénie, dépression, trouble d'hyperactivité avec déficit de l'attention (TDAH),

trouble anxieux généralisé, trouble obsessionnel compulsif (TOC), trouble panique, trouble bipolaire, troubles de la dépendance/du contrôle des impulsions, troubles du spectre autistique, psychose, trouble neurocognitif, délire, anhédonie, agitation, maladie d'Alzheimer, maladie de Parkinson, maladie de Huntington, démence vasculaire, maladie à corps de Lewy,

démence frontotemporelle, syndrome de Tourette, hyperprolactinémie, obésité et trouble de stress post-traumatique (TSPT).

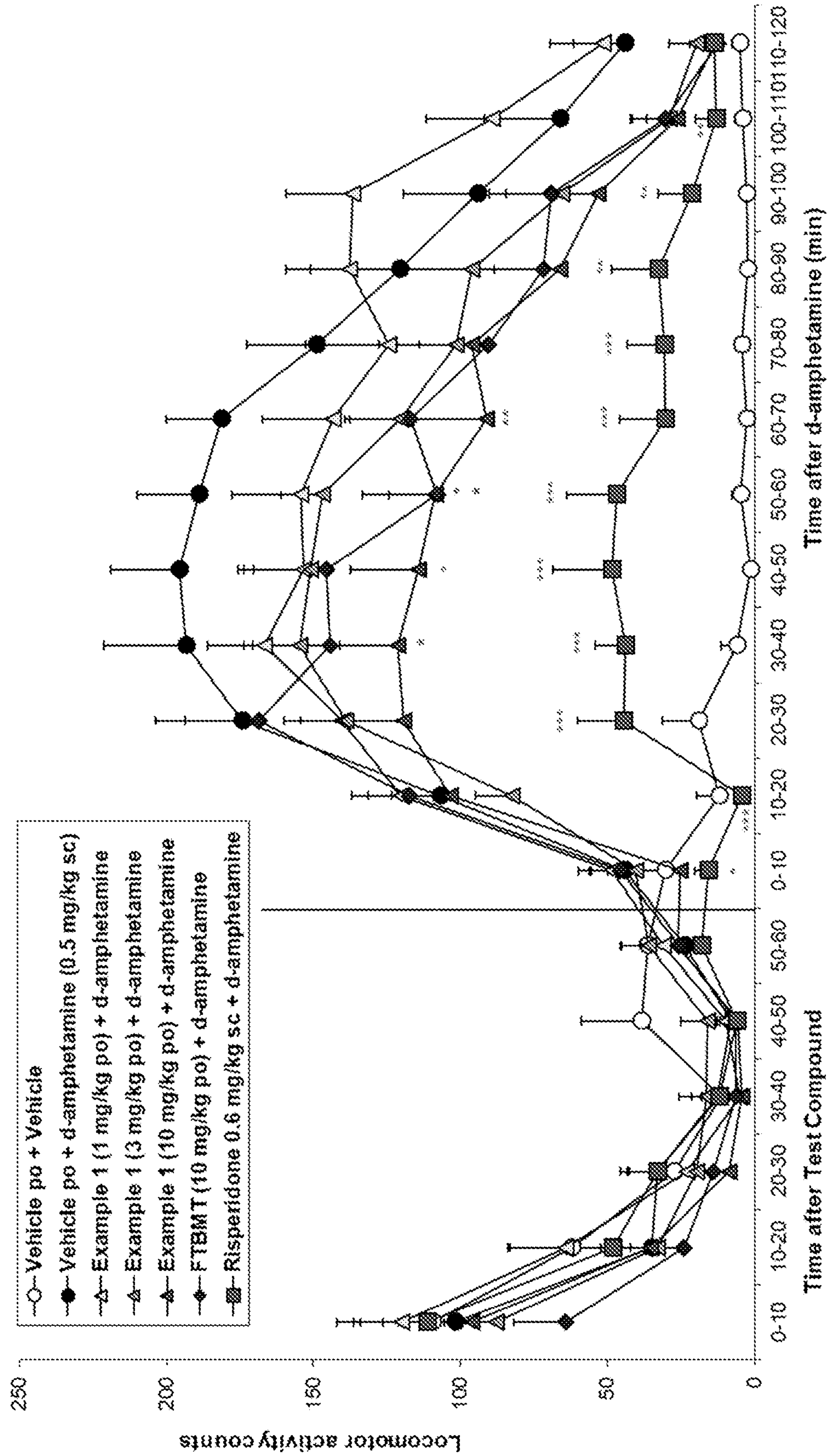
Vehicle po + Vehicle
Vehicle po + d-amphetamine (0.5 mg/kg sc)
Example 1 (1 mg/kg po) + d-amphetamine
Example 1 (3 mg/kg po) + d-amphetamine
Example 1 (10 mg/kg po) + d-amphetamine
FTBMT (10 mg/kg po) + d-amphetamine
Risperidone 0.6 mg/kg sc + d-amphetamine
Locomotor activity counts
Time after Test Compound
Time after d-amphetamine (min)

Figure 1

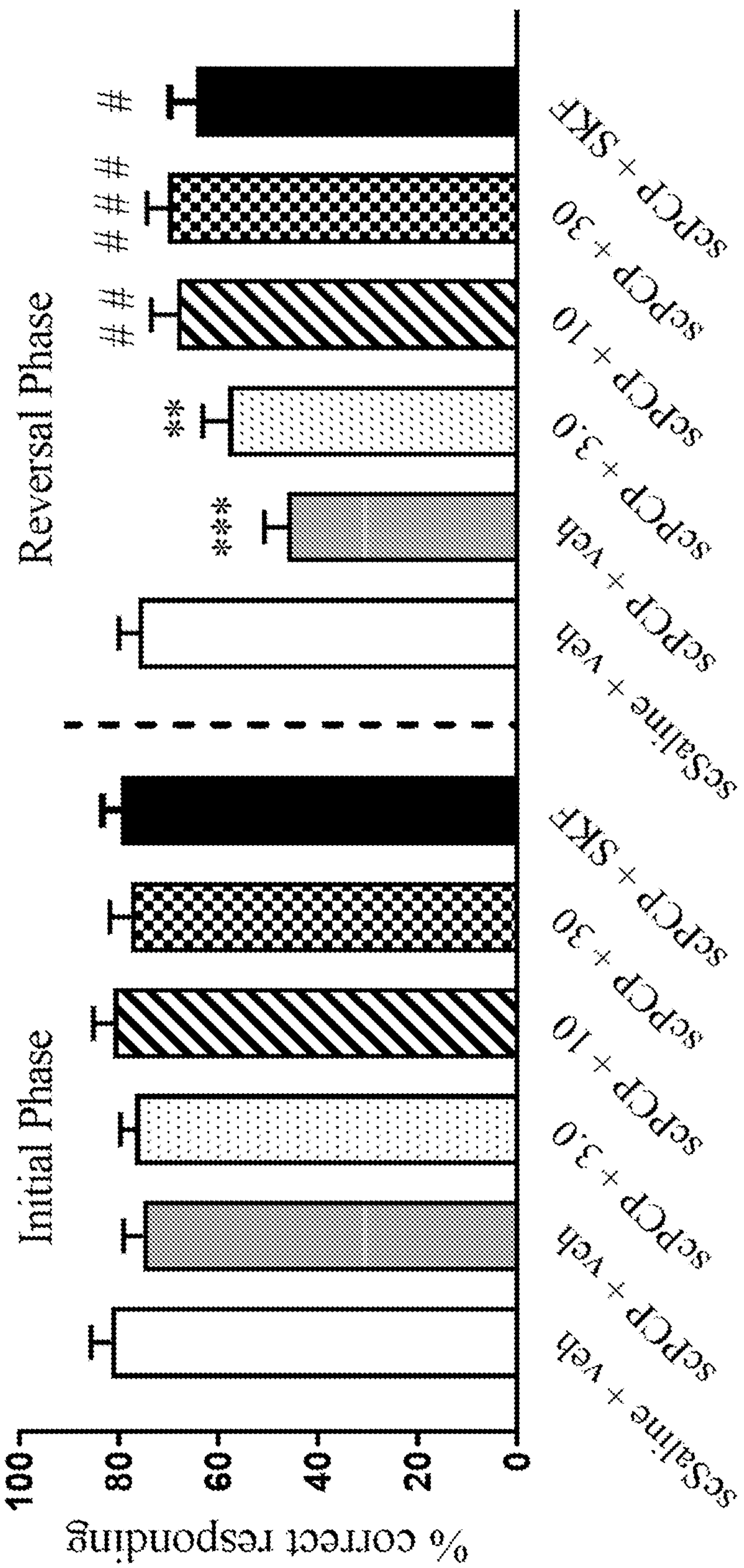
Initial Phase
Reversal Phase
% correct responding
0
20
40
60
80
100
scSaline + veh
scPCP + veh
scPCP + 3.0
scPCP + 10
scPCP + 30
scPCP + SKF
***
**
##
###
#

Figure 2

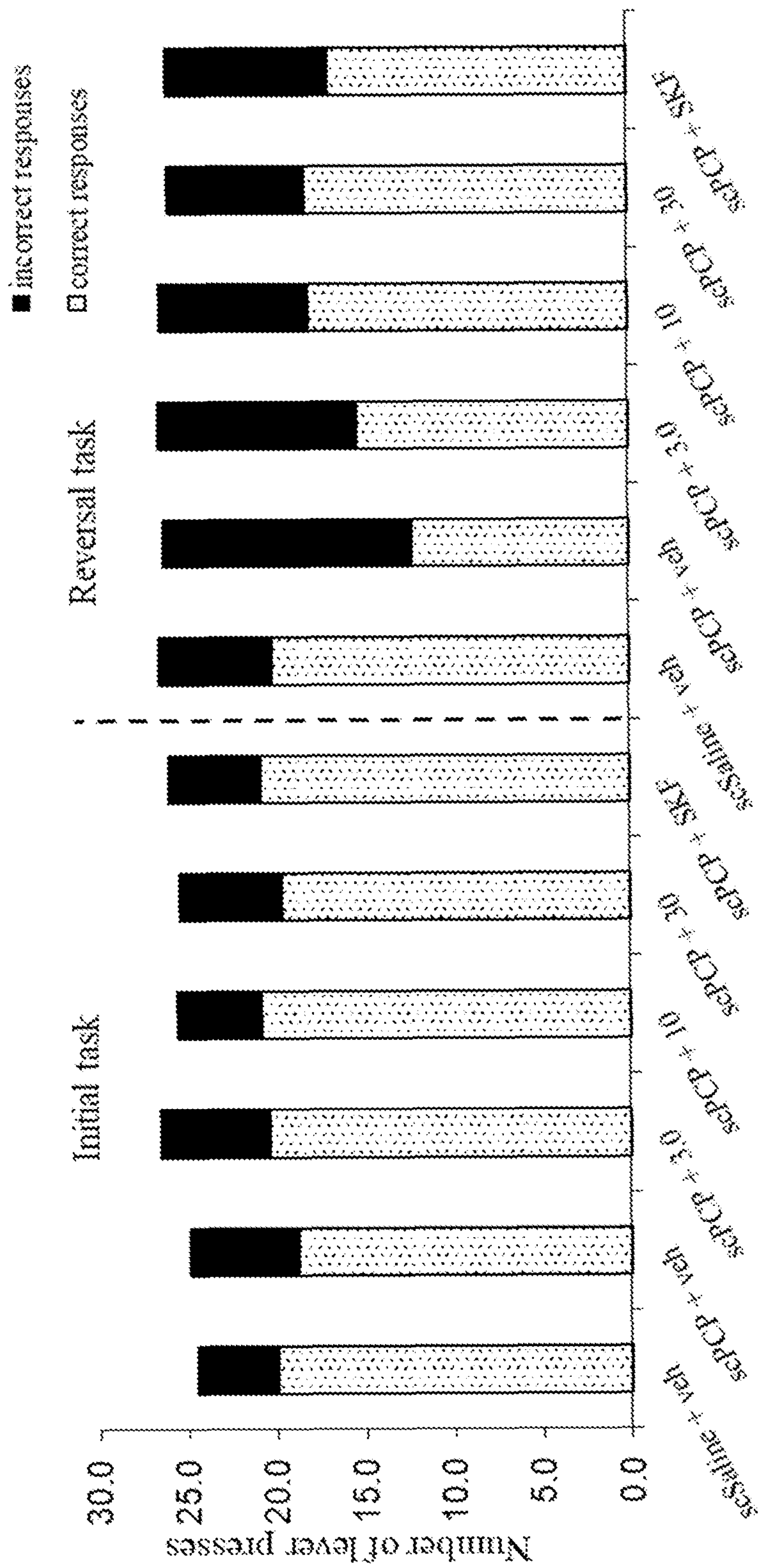
incorrect responses
correct responses
Initial task
Reversal task
Number of lever presses
30.0
25.0
20.0
15.0
10.0
5.0
0.0
scSaline + veh
scPCP + veh
scPCP + 3.0
scPCP + 10
scPCP + 30
scPCP + SKF
scSaline + veh
scPCP + veh
scPCP + 3.0
scPCP + 10
scPCP + 30
scPCP + SKF

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016176571 A, Xiong


### Non-patent literature cited in the description


- **KOMATSU et al.** *PLoS One*, 2014, vol. 9, e90134
- **BRYN et al.** Solid-State Chemistry of Drugs. SSCI, Inc of West Lafayette, 1999
- Remington's Pharmaceutical Sciences. Mack Publishing Company